# EUROPÄISCHE PATENTSCHRIFT

(11) **EP 0 842 944 B1**
(45) Veröffentlichungstag und Bekanntmachung des Hinweises auf die Patenterteilung: **09.07.2003**
(21) Anmeldenummer: 97119639.9
(22) Anmeldetag: 10.11.1997
(51) Int. Cl.: C07K 5/023, C07K 5/117, A61K 38/06, A61K 38/07

(54) **Heterocyclen als Inhibitoren der Leukozytenadhäsion und VLA-4-Antagonisten**
Heterocycles as inhibitors of leukocyte adhesion and as antagonists of VLA-4
Hétérocycles à titre d'inhibiteurs d'adhésion des leucocytes et d'antogonistes de VLA-4

(30) Priorität: 15.11.1996 DE 19647382
(43) Veröffentlichungstag der Anmeldung: 20.05.1998
(73) Patentinhaber: HOECHST AKTIENGESELLSCHAFT, 65929 Frankfurt am Main (DE)
(72) Erfinder: Stilz, Hans Ulrich, Dr., 65929 Frankfurt (DE); Wehner, Volkmar, Dr., 97657 Sandberg (DE); Hüls, Christoph, Dr., 55263 Wackernheim (DE); Seiffge, Dirk, Dr., 55246 Mainz-Kostheim (DE)

(56) Entgegenhaltungen:
- EP-A- 0 580 008
- EP-A- 0 903 353
- WO-A-95/14008
- WO-A-95/15973
- WO-A-96/22966

## Beschreibung

Gegenstand der vorliegenden Erfindung sind Verbindungen der Formel I 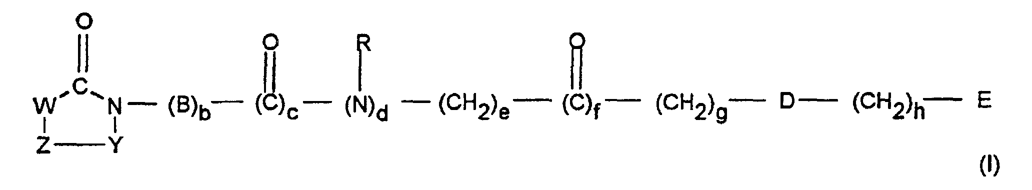 als Inhibitoren der Adhäsion und Migration von Leukozyten und/oder Antagonisten des zur Gruppe der Integrine gehörenden Adhäsionsrezeptors VLA-4. Die Erfindung betrifft die Verwendung von Verbindungen der Formel I und von pharmazeutischen Zubereitungen, die solche Verbindungen enthalten, zur Behandlung oder Prophylaxe von Krankheiten, die durch ein unerwünschtes Ausmaß an Leukozytenadhäsion und/oder Leukozytenmigration verursacht werden oder die damit verbunden sind oder bei denen Zell-Zell- oder Zell-Matrix-Interaktionen eine Rolle spielen, die auf Wechselwirkungen von VLA-4-Rezeptoren mit ihren Liganden beruhen, beispielsweise von Entzündungsprozessen, der rheumatoiden Arthritis oder von allergischen Erkrankungen, ebenso wie die Verwendung von Verbindungen der Formel I zur Herstellung von Arzneimitteln zur Anwendung bei solchen Krankheiten. Ferner betrifft sie neue Verbindungen der Formel I.

Die Integrine sind eine Gruppe von Adhäsionsrezeptoren, die bei Zell-Zellbindenden und Zell-Extrazelluläre Matrix-bindenden Prozessen eine wesentliche Rolle spielen. Sie weisen eine αβ-heterodimere Struktur auf und zeigen eine weite zelluläre Verbreitung und ein hohes Maß an evolutiver Konservierung. Zu den Integrinen gehört zum Beispiel der Fibrinogen-Rezeptor auf Thrombozyten, der vor allem mit der RGD-Sequenz des Fibrinogens interagiert, oder der Vitronectin-Rezeptor auf Osteoclasten, der vor allem mit der RGD-Sequenz des Vitronectins oder des Osteopontins interagiert. Man teilt die Integrine in drei Großgruppen ein, die β2-Unterfamilie mit den Vertetern LFA-1, Mac-1 und p150/95, die insbesondere für Zell-Zell-Interaktionen des Immunsystems verantwortlich sind, und die Unterfamilien β1 und β3, deren Vertreter hauptsächlich die Zellanheftung an Komponenten der extrazellulären Matrix vermitteln (Ruoslahti, Annu. Rev. Biochem. 1988, 57, 375). Die Integrine der β1-Unterfamilie, auch VLA-Proteine (very late (activation) antigen) genannt, umfassen mindestens sechs Rezeptoren, die spezifisch mit Fibronektin, Kollagen und/oder Laminin als Liganden interagieren. Innerhalb der VLA-Familie ist das Integrin VLA-4 (α4β1) insofern untypisch, als es hauptsächlich auf lymphoide und myeloide Zellen begrenzt ist und bei diesen verantwortlich ist für Zell-Zell-Interaktionen mit einer Vielzahl von anderen Zellen. VLA-4 vermittelt zum Beispiel die Interaktion von T- und B-Lymphozyten mit dem Heparin II-Bindungsfragment von humanem Plasmafibronektin (FN). Die Bindung von VLA-4 mit dem Heparin II-Bindungsfragment des Plasmafibronektins beruht vor allem auf einer Interaktion mit einer LDVP-Sequenz. Im Unterschied zum Fibrinogen- oder Vitronectin-Rezeptor ist VLA-4 kein typisches RGD-bindendes Integrin (Kilger und Holzmann, J. Mol. Meth. 1995, 73, 347).

Die im Blut zirkulierenden Leukozyten zeigen normalerweise nur eine geringe Affinität zu den vaskulären endothelialen Zellen, die die Blutgefäße auskleiden. Zytokine, die von entzündetem Gewebe abgegeben werden, bewirken die Aktivierung von Endothelzellen und damit die Expression einer Vielzahl von Zelloberflächenantigenen. Diese umfassen zum Beispiel die Adhäsionsmoleküle ELAM-1 (endothelial cell adhesion molecule-1; auch als E-Selektin bezeichnet), das unter anderem Neutrophile bindet, ICAM-1 (intercellular adhesion molecule-1), das mit LFA-1 (leucocyte function-associated antigen 1) auf Leukozyten interagiert, und VCAM-1 (vascular cell adhesion molecule-1), das verschiedene Leukozyten, unter anderem Lymphozyten, bindet (Osborn et al., Cell 1989, 59, 1203). VCAM-1 ist, wie ICAM-1, ein Mitglied der lmmunglobulin-Gen-Überfamilie. Identifiziert wurde VCAM-1 (zuerst bekannt als INCAM-110) als ein Adhäsionsmolekül, daß auf endothelialen Zellen durch Entzündungs-Zytokine wie TNF und IL-1 und Lipopolysaccharide (LPS) induziert wird. Elices et al. (Cell 1990, 60, 577) zeigten, daß VLA-4 und VCAM-1 ein Rezeptor-Ligand-Paar bilden, das die Anheftung von Lymphozyten an aktiviertes Endothel vermittelt. Die Bindung von VCAM-1 an VLA-4 erfolgt dabei nicht durch eine Interaktion des VLA-4 mit einer RGD-Sequenz, eine solche ist im VCAM-1-nicht enthalten (Bergelson et al., Current Biology 1995, 5, 615). VLA-4 tritt aber auch auf anderen Leukozyten auf, und über den VCAM-1/VLA-4-Adhäsionsmechanismus wird auch die Anheftung von anderen Leukozyten als Lymphozyten vermittelt. VLA-4 repräsentiert somit ein einzelnes Beispiel eines β1-Integrin-Rezeptors, der über die Liganden VCAM-1 bzw. Fibronektin sowohl bei Zell-Zell-Interaktionen als auch bei Zell-Extrazellulärer Matrix-Interaktionen eine wesentliche Rolle spielt.

Die Zytokin-induzierten Adhäsionsmoleküle spielen eine wichtige Rolle bei der Rekrutierung von Leukozyten in extravaskuläre Gewebebereiche. Leukozyten werden in entzündliche Gewebebereiche durch Zelladhäsionsmoleküle rekrutiert, die auf der Oberfläche von endothelialen Zellen exprimiert werden und als Liganden für Leukozyten-Zelloberflächen-Proteine oder -Proteinkomplexe (Rezeptoren) dienen (die Begriffe Ligand und Rezeptor können auch vice versa verwendet werden). Leukozyten aus dem Blut müssen zunächst an endotheliale Zellen anheften, bevor sie in das Synovium auswandern können. Da VCAM-1 an Zellen bindet, die das Integrin VLA-4 (α4β1) tragen, wie Eosinophile, T- und B-Lymphozyten, Monozyten oder auch Neutrophile, kommt ihm und dem VCAM-1/ VLA-4-Mechanismus die Funktion zu, derartige Zellen aus dem Blutstrom in Infektionsgebiete und Entzündungsherde zu rekrutieren (Elices et al., Cell 1990, 60, 577; Osborn, Cell 1990, 62, 3; Issekutz et al., J. Exp. Med. 1996, 183, 2175).

Der VCAM-1/VLA-4-Adhäsionsmechanismus wurde mit einer Reihe von physiologischen und pathologischen Prozessen in Verbindung gebracht. VCAM-1 wird außer von Zytokin-induziertem Endothel unter anderem noch von den folgenden Zellen exprimiert: Myoblasten, lymphoiden dendritischen Zellen und Gewebsmakrophagen, rheumatoidem Synovium, Zytokin-stimulierten Neuralzellen, parietalen Epithelzellen der Bowmans Kapsel, dem renalen Tubularepithel, entzündetem Gewebe bei Herz- und Nieren-Transplantat-Abstoßung und von Intestinalgewebe bei Graft versus host-Krankheit. VCAM-1 findet man auch exprimiert auf solchen Gewebearealen des arteriellen Endotheliums, die frühen arteriosklerotischen Plaques eines Kaninchenmodells entsprechen. Zusätzlich wird VCAM-1 auf follikulären dendritischen Zellen von humanen Lymphknoten exprimiert und findet sich auf Stromazellen des Knochenmarks, zum Beispiel in der Maus. Letzterer Befund weist auf eine Funktion von VCAM-1 in der B-Zell-Entwicklung hin. VLA-4 wird, außer auf Zellen haematopoetischen Ursprunges, auch zum Beispiel auf Melanoma-Zellinien gefunden, und der VCAM-1/VLA-4-Adhäsionsmechanismus wird mit der Metastasierung von solchen Tumoren in Verbindung gebracht (Rice et al., Science 1989, 246, 1303).

Die hauptsächliche Form, in der VCAM-1 in vivo auf endothelialen Zellen vorkommt und die die dominante Form in vivo ist, wird als VCAM-7D bezeichnet und trägt sieben Immunglobulin-Domänen. Die Domänen 4, 5 und 6 ähneln in ihren Aminosäuresequenzen den Domänen 1, 2 und 3. Die vierte Domäne ist bei einer weiteren, aus sechs Domänen bestehenden Form, hier als VCAM-6D bezeichnet, durch alternatives Splicing entfernt. Auch VCAM-6D kann VLA-4-exprimierende Zellen binden.

Weitere Angaben zu VLA-4, VCAM-1, Integrinen und Adhäsionsproteinen finden sich zum Beispiel in den Artikeln von Kilger und Holzmann, J. Mol. Meth. 1995, 73, 347; Elices, Cell Adhesion in Human Disease, Wiley, Chichester 1995, S. 79; Kuijpers, Springer Semin. Immunopathol. 1995, 16, 379.

Aufgrund der Rolle des VCAM-1/VLA-4-Mechanismus bei Zelladhäsionsprozessen, die von Bedeutung zum Beispiel bei Infektionen, Entzündungen oder Atherosklerose sind, wurde versucht, durch Eingriffe in diese Adhäsionsprozesse Krankheiten zu bekämpfen, insbesondere zum Beispiel Entzündungen (Osborn et al., Cell 1989, 59, 1203). Eine Methode hierzu ist die Verwendung von monoklonalen Antikörpern, die gegen VLA-4 gerichtet sind. Derartige monoklonale Antikörper (mAK), die als VLA-4-Antagonisten die Interaktion zwischen VCAM-1 und VLA-4 blockieren, sind bekannt. So inhibieren zum Beispiel die anti-VLA-4 mAK HP2/1 und HP1/3 die Anheftung von VLA-4 exprimierenden Ramos-Zellen (B-Zell-ähnlichen Zellen) an humane Nabelschnurendothelzellen und an VCAM-1-transfizierte COS-Zellen. Ebenso inhibiert der anti-VCAM-1 mAK 4B9 die Adhäsion von Ramos-Zellen, Jurkat-Zellen (T-Zell-ähnlichen Zellen) und HL60-Zellen (Granulozyten-ähnlichen Zellen) an COS-Zellen transfiziert mit genetischen Konstrukten, die veranlassen, daß VCAM-6D und VCAM-7D exprimiert werden. In vitro-Daten mit Antikörpern, die gegen die α4-Untereinheit von VLA-4 gerichtet sind, zeigen, daß die Anheftung von Lymphozyten an synoviale Endothelzellen blockiert wird, eine Adhäsion, die bei der rheumatoiden Arthritis eine Rolle spielt (van Dinther-Janssen et al., J. Immunol. 1991, 147, 4207).

In vivo-Versuche haben gezeigt, daß eine experimentelle autoimmune Enzephalomyelitis durch anti-α4 mAK gehemmt werden kann. Die Wanderung von Leukozyten in einen Entzündungsherd wird ebenfalls durch einen monoklonalen Antikörper gegen die α4-Kette von VLA-4 blockiert. Die Beeinflussung des VLA-4-abhängigen Adhäsionsmechanismus mit Antikörpern wurde auch in einem Asthma-Modell untersucht, um die Rolle von VLA-4 bei der Rekrutierung von Leukozyten in entzündetes Lungengewebe zu untersuchen (US-A-5871734; EP-A-626 861). Die Gabe von anti-VLA-4-Antikörpern inhibierte die Spätphasenreaktion und die Atemwegsüberreaktion in allergischen Schafen.

Der VLA-4 abhängige Zelladhäsionsmechanismus wurde ebenfalls in einem Primatenmodell der inflammatory bowel disease (IBD) untersucht. In diesem Modell, das der ulcerativen Colitis im Menschen entspricht, ergab die Gabe von anti-VLA-4-Antikörpern eine signifikante Reduktion der akuten Entzündung.

Darüber hinaus konnte gezeigt werden, daß die VLA-4-abhängige Zelladhäsion bei den folgenden klinischen Konditionen einschließlich der folgenden chronischen entzündlichen Prozesse eine Rolle spielt: Rheumatoide Arthritis (Cronstein und Weismann, Arthritis Rheum. 1993, 36, 147; Elices et al., J. Clin. Invest. 1994, 93, 405), Diabetes mellitus (Yang et al., Proc. Natl. Acad. Sci. USA 1993, 90, 10494), systemischer Lupus erythematosus (Takeuchi et al., J. Clin. Invest. 1993, 92, 3008), Allergien vom verzögerten Typ (Typ IV-Allergie) (Elices et al., Clin. Exp. Rheumatol. 1993, 11, S77), multiple Sklerose (Yednock et al., Nature 1992, 356, 63), Malaria (Ockenhouse et al., J. Exp. Med. 1992, 176, 1183), Arteriosklerose (Obrien et al., J. Clin. Invest. 1993, 92, 945), Transplantation (Isobe et al., Transplantation Proceedings 1994, 26, 867-868), verschiedene Malignitäten, zum Beispiel Melanom (Renkonen et al., Am. J. Pathol. 1992, 140, 763), Lymphom (Freedman et al., Blood 1992, 79, 206) und andere (Albelda et al., J. Cell Biol. 1991, 114, 1059).

Eine VLA-4-Blockierung durch geeignete Antagonisten bietet danach effektive therapeutische Möglichkeiten, insbesondere zum Beispiel verschiedene entzündliche Konditionen einschließlich Asthma und IBD zu behandeln. Die besondere Relevanz von VLA-4-Antagonisten für die Behandlung der rheumatoiden Arthritis ergibt sich dabei, wie bereits gesagt, aus der Tatsache, daß Leukozyten aus dem Blut zunächst an endotheliale Zellen anheften müssen, ehe sie in das Synovium auswandern können, und daß bei dieser Anheftung der VLA-4-Rezeptor eine Rolle spielt. Darauf, daß durch Entzündungsagenzien auf endothelialen Zellen VCAM-1 induziert wird (Osborn, Cell 1990, 62, 3; Stoolman, Cell 1989, 56, 907), und auf die Rekrutierung verschiedener Leukozyten in Infektionsgebiete und Entzündungsherde wurde bereits oben eingegangen. T-Zellen adherieren dabei an aktiviertes Endothel hauptsächlich über die LFA-1/ICAM-1- und VLA-4/VCAM-1-Adhäsionsmechanismen (Springer, Cell 1994, 76, 301). Auf den meisten synovialen T-Zellen ist die Bindungskapazität von VLA-4 für VCAM-1 bei der rheumatoiden Arthritis erhöht (Postigo et al., J. Clin. Invest. 1992, 89, 1445). Zusätzlich wurde eine verstärkte Anheftung von synovialen T-Zellen an Fibronektin beobachtet (Laffon et al., J. Clin. Invest. 1991, 88, 546; Morales-Ducret et al., J. Immunol. 1992, 149, 1424). VLA-4 ist also hochreguliert sowohl im Rahmen seiner Expression als auch hinsichtlich seiner Funktion auf T-Lymphozyten der rheumatoiden Synovialmembran. Die Blockierung der Bindung von VLA-4 an seine physiologischen Liganden VCAM-1 und Fibronektin ermöglicht eine effektive Verhinderung oder Linderung von artikulären Entzündungsprozessen. Dies wird auch durch Experimente mit dem Antikörper HP2/1 an Lewis-Ratten mit Adjuvanz-Arthritis bestätigt, bei denen eine effektive Krankheitsprävention beobachtet wurde (Barbadillo et al., Springer Semin. Immunopathol. 1995, 16, 427). VLA-4 stellt also ein wichtiges therapeutisches Zielmolekül dar.

Die oben erwähnten VLA-4-Antikörper und der Einsatz von Antikörpern als VLA-4-Antagonisten sind in den Patentanmeldungen WO-A-93/13798, WO-A-93/15764, WO-A-94/16094, WO-A-94/17828 und WO-A-95/19790 beschrieben. In den Patentanmeldungen WO-A-94/15958, WO-A-95/15973, WO-A-96/00581, WO-A-96/06108 und WO-A-96/20216 werden peptidische Verbindungen als VLA-4-Antagonisten beschrieben. Der Einsatz von Antikörpern und peptidischen Verbindungen als Arzneimitteln ist aber mit Nachteilen behaftet, zum Beispiel mangelnder oraler Verfügbarkeit, leichter Abbaubarkeit oder immunoger Wirkung bei längerfristiger Anwendung, und es besteht somit Bedarf nach VLA-4-Antagonisten mit einem günstigen Eigenschaftsprofil für einen Einsatz in der Therapie und Prophylaxe. Bestimmte nicht-peptidische Verbindungen mit VLA-4-inhibitorischer Wirkung werden in der WO-A-96/22966 und der EP-A-903353 beschrieben.

In der EP-A-580008 sind Phenylimidazolidin-Derivate und in der WO-A-95/14008 substituierte 5-Ring-Heterocyclen beschrieben, die am N-terminalen Ende des Moleküls eine Amino-, Amidino- oder Guanidinofunktion aufweisen und die thrombozytenaggregationshemmende Wirkungen zeigen. In der DE-A-19635522 sind weitere Heterocyclen beschrieben, die Inhibitoren der Knochenresorption sind. Hinweise, daß die von der DE-A-19635522 umfaßten Verbindungen VLA-4-Antagonisten sind oder die Leukozytenadhäsion hemmen, finden sich dort aber nicht. Überraschend wurde nun gefunden, daß von der DE-A-19635522 umfaßte Verbindungen auch die Leukozytenadhäsion hemmen und VLA-4-Antagonisten sind.

Die vorliegende Erfindung betrifft somit die Verwendung von Verbindungen der Formel I, 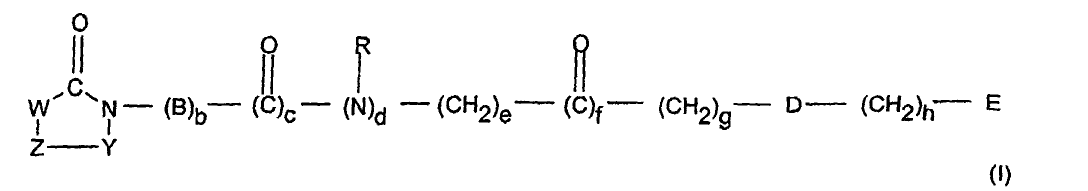 worin
- W: für R¹-A-C(R¹³) oder R¹-A-CH=C steht;
- Y: für eine Carbonyl-, Thiocarbonyl- oder Methylengruppe steht;
- Z: für N(R⁰), Sauerstoff, Schwefel oder eine Methylengruppe steht;
- A: einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)- Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
- B: einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet, wobei der zweiwertige (C₁-C₆)-Alkylen-Rest unsubstituiert oder durch einen Rest aus der Reihe (C₁-C₈)-Alkyl, (C₂-C₈)-Alkenyl, (C₂-C₈)-Alkinyl, (C₃-C₁₀)-Cycloalkyl, (C₃- C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert sein kann;
- D: für C(R²)(R³), N(R³) oder CH=C(R³) steht;
- E: Tetrazolyl, (R⁸O)₂P(O), HOS(O)₂, R⁹NHS(O)₂ oder R¹⁰CO bedeutet;
- R: Wasserstoff, (C₁-C₈)-Alkyl, (C₃-C₁₂)-Cycloalkyl, (C₃-C₁₂)-Cycloalkyl-(C₁-C₈)- alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁- C₈)-alkyl bedeuten, wobei Alkylreste durch Fluor ein- oder mehrfach substituiert sein können;
- R⁰: für Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₂)-Bicycloalkyl, (C₆-C₁₂)-Bicycloalkyl-(C₁-C₈)-alkyl, (C₆-C₁₂)- Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)- alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)- Bicycloalkyl-CO, (C₆-C₁₂)-Bicycloalkyl-(C₁-C₈)-alkyl-CO, (C₆-C₁₂)-Tricycloalkyl- CO, (C₆-C₁₂)-Tricycloalkyl-(C₁-C₈)-alkyl-CO, gegebenenfalls substituiertes (C₆- C₁₄)-Aryl-CO, im Arylrest gegebenenfalls substituiertes (C₆-C₁₄)-Aryl-(C₁-C₈)- alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)- Bicycloalkyl-S(O)ₙ, (C₆-C₁₂)-Bicycloalkyl-(C₁-C₈)-alkyl-S(O)ₙ, (C₆-C₁₂)- Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆- C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl- S(O)ₙ steht, wobei n für 1 oder 2 steht;
- R¹: Wasserstoff, (C₁-C₁₀)-Alkyl, das gegebenenfalls durch Fluor einfach oder mehrfach substituiert sein kann, (C₃-C₁₂)-Cycloalkyl, (C₃-C₁₂)-Cycloalkyl-(C₁- C₈)-alkyl, gegebenenfalls substituiertes R²¹-((C₆-C₁₄)-Aryl), im Arylrest gegebenenfalls substituiertes (R²¹-((C₆-C₁₄)-Aryl))-(C₁-C₈)-alkyl, den Rest Het-, Het-(C₁-C₈)-alkyl oder einen der Reste R²¹O-, R²²O-NH-, R²¹O-N(R²³)-, R²⁴NH-, R²⁵N(R²⁵)-, HO-((C₁-C₈)-Alkyl)-N(R²⁶)-, R²⁷C(O)-NH-, R²¹C(O)- N(R²³)-, R²¹C(O)-, R²¹O-C(O)-, R²⁸N(R²¹)-C(O)-, R²¹O-N=, O= und S= bedeutet;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)- Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)- Cycloalkyl, (C₂-C₈)-Alkenyl, (C₂-C₈)-Alkinyl, (C₂-C₈)-Alkenylcarbonyl, (C₂-C₈)- Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)- alkylaminocarbonyl, (C₁-C₁₈)-Alkylcarbonylamino-(C₁-C₃)-alkylphenyl-(C₁-C₃)- alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und den Rest R⁵ substituiert sein kann;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, Iminosäure-, gegebenenfalls N-(C₁- C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)- alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid- Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)- Cycloalkylaminocarbonyl, gegebenenfalls substituiertes (C₆-C₁₄)- Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: Wasserstoff, (C₁-C₁₈)-Alkyl, R¹²CO, R^{12a}CS, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵ bedeutet;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)- Aryloxy, den Rest R¹⁵, den Rest R¹⁵-O-, Amino, Mono- oder Di-((C₁-C₁₈)-alkyl)- amino, (C₂-C₈)-Alkenyl-amino, (C₂-C₈)-Alkinyl-amino, (C₃-C₁₂)-Cycloalkyl- amino, (C₃-C₁₂)-Cycloalkyl-(C₁-C₈)-alkyl-amino, den Rest R¹⁵-NH-, gegebenenfalls substituiertes (C₆-C₁₄)-Aryl-NH, (C₆-C₁₄)-Aryl(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl- (C₁-C₈)-alkyl-NH bedeutet;
- R^{12a}: Amino, Mono- oder Di-((C₁-C₁₈)-alkyl)-amino, (C₂-C₈)-Alkenyl-amino, (C₂-C₈)- Alkinyl-amino, (C₃-C₁₂)-Cycloalkyl-amino, (C₃-C₁₂)-Cycloalkyl-(C₁-C₈)-alkyl- amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl-(C₁-C₈)-alkyl-NH bedeutet;
- R¹³: Wasserstoff, (C₁-C₆)-Alkyl, im Arylrest gegebenenfalls substituiertes (C₆-C₁₄)- Aryl-(C₁-C₈)-alkyl oder (C₃-C₈)-Cycloalkyl bedeutet;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- R²¹: Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₄)-Aryl, (C₆-C₁₄)-Aryl-(C₁-C₈)-alkyl, den Rest Het- oder Het-(C₁- C₈)-alkyl bedeutet, wobei Alkylreste durch Fluor einfach oder mehrfach substituiert sein können und die Reste R²¹ bei mehrfachem Auftreten gleich oder verschieden sein können;
- R²²: (C₃-C₁₂)-Cycloalkyl, (C₃-C₁₂)-Cycloalkyl-(C₁-C₈)-alkyl, (C₆-C₁₄)-Aryl, den Rest Het- oder Het-(C₁-C₈)-alkyl bedeutet, wobei Alkylreste durch Fluor einfach oder mehrfach substituiert sein können;
- R²³: (C₁-C₈)-Alkyl, (C₃-C₁₂)-Cycloalkyl, (C₃-C₁₂)-Cycloalkyl-(C₁-C₈)-alkyl, (C₆-C₁₄)- Aryl, (C₆-C₁₄)-Aryl-(C₁-C₈)-alkyl, den Rest Het- oder Het-(C₁-C₈)-alkyl bedeutet, wobei Alkylreste durch Fluor einfach oder mehrfach substituiert sein können;
- R²⁴: (C₃-C₁₂)-Cycloalkyl, (C₃-C₁₂)-Cycloalkyl-(C₁-C₈)-alkyl, (C₆-C₁₄)-Aryl, (C₆-C₁₄)- Aryl-(C₁-C₈)-alkyl, den Rest Het- oder Het-(C₁-C₈)-alkyl bedeutet, wobei Alkylreste durch Fluor einfach oder mehrfach substituiert sein können;
- R²⁵: die Bedeutungen von R²³ hat, wobei die Reste R²⁵ gleich oder verschieden sein können;
- R²⁶: die Bedeutungen von R²¹ hat oder HO-((C₁-C₈)-Alkyl) bedeutet, wobei Alkylreste durch Fluor einfach oder mehrfach substituiert sein können;
- R²⁷: Wasserstoff, (C₃-C₁₂)-Cycloalkyl, (C₃-C₁₂)-Cycloalkyl-(C₁-C₈)-alkyl, (C₆-C₁₄)- Aryl-(C₁-C₈)-alkyl, den Rest Het- oder Het-(C₁-C₈)-alkyl bedeutet, wobei Alkylreste durch Fluor einfach oder mehrfach substituiert sein können;
- R²⁸: für einen der Reste R²¹-, R²¹O-, R²⁶N(R²⁶)-, R²¹C(O)-, R²¹O-C(O)-, ((C₁-C₁₈)- Alkyl-O-C(O)-((C₁-C₆)-alkyl)-O-C(O)-, R²¹N(R²¹)-C(O)-, R²¹N(R²¹)-C(=N(R²¹))- oder R²¹C(O)-N(R²¹)- steht;
- Het: für einen mono- oder polycyclischen, 4- bis 14-gliedrigen, aromatischen oder nicht aromatischen Ring steht, der 1, 2, 3 oder 4 gleiche oder verschiedene Heteroatome aus der Reihe N, O und S als Ringglieder enthält und gegebenenfalls durch einen oder mehrere, gleiche oder verschiedene Substituenten substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen sowie ihrer physiologisch verträglichen Salze zur Herstellung von Arzneimitteln zur Hemmung der Adhäsion und/oder Migration von Leukozyten oder zur Hemmung des VLA-4-Rezeptors, also von Arzneimitteln zur Behandlung oder Prophylaxe von Krankheiten, bei denen die Leukozytenadhäsion und/oder Leukozytenmigration ein unerwünschtes Ausmaß aufweist, oder von Krankheiten, bei denen VLA-4-abhängige Adhäsionsvorgänge eine Rolle spielen, zum Beispiel von Entzündungserkrankungen, sowie die Verwendung der Verbindungen der Formel I bei der Behandlung und Prophylaxe derartiger Krankheiten.

Alkylreste können geradkettig oder verzweigt sein. Dies gilt auch, wenn sie Substituenten tragen oder als Substituenten anderer Reste auftreten, beispielsweise in Alkoxy-, Alkoxycarbonyl- oder Aralkylresten. Entsprechendes gilt für Alkylenreste. Beispiele für geeignete (C₁-C₂₈)-Alkylreste sind: Methyl, Ethyl, Propyl, Butyl, Pentyl, Hexyl, Heptyl, Octyl, Decyl, Undecyl, Dodecyl, Tridecyl, Pentadecyl, Hexadecyl, Heptadecyl, Nonadecyl, Eicosyl, Docosyl, Tricosyl, Pentacosyl, Hexacosyl, Heptacosyl, Octacosyl, Isopropyl, Isobutyl, Isopentyl, Neopentyl, Isohexyl, 3-Methylpentyl, 2,3,5-Trimethylhexyl, sec-Butyl, tert-Butyl, tert-Pentyl. Bevorzugte Alkylreste sind Methyl, Ethyl, Propyl, Isopropyl, Butyl, Isobutyl, sec-Butyl und tert-Butyl. Beispiele für Alkylenreste sind Methylen, Ethylen, Tri-, Tetra-, Penta- und Hexamethylen oder durch einen Alkylrest substituiertes Methylen, zum Beispiel Methylen, das durch eine Methylgruppe, eine Ethylgruppe, eine Isopropylgruppe, eine Isobutylgruppe oder eine tert-Butylgruppe substituiert ist.

Auch Alkenyl- und Alkenylenrest sowie Alkinylreste können geradkettig oder verzweigt sein. Beispiele für Alkenylreste sind Vinyl, 1-Propenyl, Allyl, Butenyl, 3-Methyl-2-butenyl, für Alkenylenreste Vinylen oder Propenylen, für Alkinylreste Ethinyl, 1-Propinyl oder Propargyl.

Cycloalkylreste sind insbesondere Cyclopropyl, Cyclobutyl, Cyclopentyl, Cyclohexyl, Cycloheptyl, Cyclooctyl, Cyclononyl, Cyclodecyl, Cycloundecyl und Cyclododecyl, die aber auch durch beispielsweise durch (C₁-C₄)-Alkyl substituiert sein können. Als Beispiele für substituierte Cycloalkylreste seien 4-Methylcyclohexyl und 2,3-Dimethylcyclopentyl genannt. Analoges gilt für Cycloalkylenreste.

Die für R¹⁶ stehenden 6- bis 24-gliedrigen bicvclischen und tricyclischen Reste werden formal durch Abstraktion eines Wasserstoffatoms aus Bicyclen bzw. Tricyclen erhalten. Die zugrunde liegenden Bicyclen und Tricyclen können als Ringglieder nur Kohlenstoffatome enthalten, es kann sich also um Bicycloalkane oder Tricycloalkane handeln, sie können aber auch ein bis vier gleiche oder verschiedene Heteroatome aus der Reihe Stickstoff, Sauerstoff und Schwefel enthalten, es kann sich also um Aza-, Oxa- und Thiabicyclo- und -tricycloalkane handeln. Sind Heteroatome enthalten, so sind bevorzugt ein oder zwei Heteroatome, insbesondere Stickstoff- oder Sauerstoffatome, enthalten. Die Heteroatome können beliebige Positionen im bi- bzw. tricyclischen Gerüst einnehmen, sie können sich in den Brücken oder im Falle von Stickstoffatomen auch an den Brückenköpfen befinden. Sowohl die Bicyclo- und Tricycloalkane als auch ihre Hetero-Analoga können vollständig gesättigt sein oder eine oder mehrere Doppelbindungen enthalten; bevorzugt enthalten sie eine oder zwei Doppelbindungen oder sind insbesondere vollständig gesättigt. Sowohl die Bicyclound Tricycloalkane als auch die Hetero-Analoga und sowohl die gesättigten als auch die ungesättigten Vertreter können unsubstituiert sein oder in beliebigen geeigneten Positionen durch eine oder mehrere Oxogruppen und/oder eine oder mehrere gleiche oder verschiedene (C₁-C₄)-Alkylgruppen, zum Beispiel Methyloder Isopropylgruppen, bevorzugt Methylgruppen, substituiert sein. Die freie Bindung des bi- oder tricyclischen Restes kann sich in einer beliebigen Position des Moleküls befinden, der Rest kann also über ein Brückenkopfatom oder ein Atom in einer Brücke gebunden sein. Die freie Bindung kann sich auch in einer beliebigen stereochemischen Position befinden, beispielsweise in einer exo- oder einer endo-Position.

Beispiele für Grundkörper bicyclischer Ringsysteme, von denen sich ein bicyclischer Rest ableiten kann, sind das Norbornan (= Bicyclo[2.2.1]heptan), das Bicyclo[2.2.2]octan und das Bicyclo[3.2.1]octan, Beispiele für Heteroatome enthaltende Systeme, ungesättigte oder substituierte Systeme sind das 7-Azabicylo[2.2.1]heptan, das Bicyclo[2.2.2.]oct-5-en und der Campher (= 1,7,7-Trimethyl-2-oxobicyclo[2.2.1]heptan).

Beispiele für Systeme, von denen sich ein tricyclischer Rest ableiten kann, sind das Twistan (= Tricyclo[4.4.0.0^{3,8}]decan), das Adamantan (= Tricyclo[3.3.1.1^{3,7}]decan), das Noradamantan (= Tricyclo[3.3.1.0^{3,7}]nonan), das Tricyclo[2.2.1.0^{2,6}]heptan, das Tricyclo[5.3.2.0^{4,9}]dodecan, das Tricyclo[5.4.0.0^{2,9}]undecan oder das Tricyclo[5.5.1.0^{3,11}]tridecan.

Bevorzugt leiten sich bicyclische oder tricyclische Reste von verbrückten Bicyclen bzw. Tricyclen ab, also von Systemen, in denen Ringe zwei oder mehr als zwei Atome gemeinsam haben. Bevorzugt sind weiterhin auch bicyclische oder tricyclische Reste mit 6 bis 18 Ringgliedern, besonders bevorzugt solche mit 7 bis 12 Ringgliedern.

Im einzelnen besonders bevorzugte bi- und tricyclische Reste sind der 2-Norbornylrest, sowohl derjenige mit der freien Bindung in der exo-Position als auch derjenige mit der freien Bindung in der endo-Position, der 2-Bicyclo[3.2.1]octylrest, der 1-Adamantylrest, der 2-Adamantylrest und der Noradamantylrest, zum Beispiel der 3-Noradamantylrest. Darüber hinaus bevorzugt sind der 1- und der 2-Adamantylrest.

(C₆-C₁₄)-Arylgruppen sind beispielsweise Phenyl, Naphthyl, Biphenylyl, Anthryl oder Fluorenyl, wobei 1-Naphthyl, 2-Naphthyl und insbesondere Phenyl bevorzugt sind. Arylreste, insbesondere Phenylreste, können ein- oder mehrfach, bevorzugt ein-, zwei- oder dreifach, durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Methylendioxy, Ethylendioxy, Cyano, Hydroxycarbonyl, Aminocarbonyl, (C₁-C₄)-Alkoxycarbonyl, Phenyl, Phenoxy, Benzyl, Benzyloxy, (R⁸O)₂P(O), (R⁸O)₂P(O)-O-, Tetrazolyl substituiert sein. Entsprechendes gilt beispielsweise für Reste wie Aralkyl oder Arylcarbonyl. Aralkylreste sind insbesondere Benzyl sowie 1- und 2-Naphthylmethyl, 2-, 3- und 4-Biphenylylmethyl und 9-Fluorenylmethyl, die auch substituiert sein können. Substituierte Aralkylreste sind beispielsweise durch einen oder mehrere (C₁-C₈)-Alkylreste, insbesondere (C₁-C₄)-Alkylreste, im Arylteil substituiertes Benzyl und Naphthylmethyl, zum Beispiel 2-, 3- und 4-Methylbenzyl, 4-Isobutylbenzyl, 4-tert-Butylbenzyl, 4-Octylbenzyl, 3,5-Dimethylbenzyl, Pentamethylbenzyl, 2-, 3-, 4-, 5-, 6-, 7- und 8-Methyl-1-naphthylmethyl, 1-, 3-, 4-, 5-, 6-, 7- und 8-Methyl-2-naphthylmethyl, durch einen oder mehrere (C₁-C₈)-Alkoxyreste, insbesondere (C₁-C₄)-Alkoxyreste, im Arylteil substituiertes Benzyl und Naphthylmethyl, zum Beispiel 4-Methoxybenzyl, 4-Neopentyloxybenzyl, 3,5-Dimethoxybenzyl, 3,4-Methylendioxybenzyl, 2,3,4-Trimethoxybenzyl, weiter 2-, 3- und 4-Nitrobenzyl, Halobenzyl, zum Beispiel 2-, 3- und 4-Chlor- und 2-, 3-, und 4-Fluorbenzyl, 3,4-Dichlorbenzyl, Pentafluorbenzyl, Trifluormethylbenzyl, zum Beispiel 3- und 4-Trifluormethylbenzyl oder 3,5-Bis(trifluormethyl)benzyl. Substituierte Aralkylreste können aber auch unterschiedliche Substitutenten aufweisen. Beispiele für Pyridyl sind 2-Pyridyl, 3-Pyridyl und 4-Pyridyl.

In monosubstituierten Phenylresten kann sich der Substituent in der 2-, der 3- oder der 4-Position befinden, wobei die 3- und die 4-Position bevorzugt sind. Ist Phenyl zweifach substituiert, können die Substituenten in 1,2-, 1,3- oder 1,4-Position zueinander stehen. Zweifach substituiertes Phenyl kann also in der 2,3-Position, der 2,4-Position, der 2,5-Position, der 2,6-Position, der 3,4-Position oder der 3,5-Position, bezogen auf die Verknüpfungstelle, substituiert sein. Bevorzugt sind in zweifach substituierten Phenylresten die beiden Substituenten in der 3-Position und der 4-Position, bezogen auf die Verknüpfungsstelle, angeordnet. Entsprechendes gilt für Phenylenreste, die zum Beispiel als 1,4-Phenylen oder als 1,3-Phenylen vorliegen können.

Phenylen-(C₁-C₆)-alkyl ist insbesondere Phenylenmethyl (-C₆H₄-CH₂-) und Phenylenethyl, (C₁-C₆)-Alkylen-phenyl insbesondere Methylenphenyl (-CH₂-C₆H₄-). Phenylen-(C₂-C₆)-alkenyl ist insbesondere Phenylenethenyl und Phenylenpropenyl.

Heteroaryl steht für einen mono- oder polycyclischen aromatischen Rest mit 5 bis 14 Ringgliedern, der 1 bis 5 Heteroatome als Ringglieder enthält. Beispiele für Heteroatome sind N, O und S. Sind mehrere Heteroatome enthalten, können diese gleich oder verschieden sein. Heteroarylreste können ebenfalls ein- oder mehrfach, bevorzugt ein-, zwei- oder dreifach, durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Methylendioxy, Cyano, Hydroxycarbonyl, Aminocarbonyl, (C₁-C₄)-Alkoxycarbonyl, Phenyl, Phenoxy, Benzyl, Benzyloxy, (R⁸O)₂P(O), (R⁸O)₂P(O)-O-, Tetrazolyl substituiert sein. Bevorzugt steht Heteroaryl für einen mono- oder bicyclischen aromatischen Rest, der 1, 2, 3 oder 4, insbesondere 1 bis 3, gleiche oder verschiedene Heteroatome aus der Reihe N, O und S enthält und der durch 1, 2, 3 oder 4, insbesondere 1 bis 3, gleiche oder verschiedene Substituenten aus der Reihe (C₁-C₆)-Alkyl, (C₁-C₆)-Alkoxy, Fluor, Chlor, Nitro, Amino, Trifluormethyl, Hydroxy, (C₁-C₄)-Alkoxycarbonyl, Phenyl, Phenoxy, Benzyloxy und Benzyl substituiert sein kann. Besonders bevorzugt steht Heteroaryl für einen mono- oder bicyclischen aromatischen Rest mit 5 bis 10 Ringgliedern, insbesondere für einen entsprechenden 5- bis 6-gliedrigen monocyclischen aromatischen Rest, der 1 bis 3 Heteroatome aus der Reihe N, O und S enthält und durch 1 bis 2 Substituenten aus der Reihe (C₁-C₄)-Alkyl, (C₁-C₄)-Alkoxy, Phenyl, Phenoxy, Benzyloxy und Benzyl substituiert sein kann.

Het umfaßt zum einen aromatische Heterocyclen und damit auch die für Heteroaryl stehenden Gruppen, soweit diese hinsichtlich der Zahl der Ringglieder und Heteroatome unter die Definition von Het fallen. Het umfaßt aber zusätzlich auch nicht aromatische Heterocyclen, die vollständig gesättigt sind oder die eine oder mehrere Doppelbindungen im Ringsystem enthalten. Het kann an Stickstoffatomen und/oder Kohlenstoffatomen durch einen oder mehrere gleiche oder verschiedene Substituenten substituiert sein, beispielsweise durch (C₁-C₈)-Alkyl, insbesondere (C₁-C₄)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, Heteroaryl, Heteroaryl-(C₁-C₈)-alkyl, (C₁-C₈)-Alkoxy, insbesondere (C₁-C₄)-Alkoxy, gegebenenfalls substituiertes Phenoxy, Benzyloxy, Halogen, Nitro, Amino, (C₁-C₈)-Alkylamino, Di-((C₁-C₈)-Alkyl)-amino,

Trifluormethyl, Hydroxy, Methylendioxy, Ethylendioxy, Cyano, Hydroxycarbonyl, Aminocarbonyl, (C₁-C₄)-Alkoxycarbonyl und allgemein durch Estergruppen, Acylgruppen, (R⁸O)₂P(O), (R⁸O)₂P(O)-O-, Oxo, Thioxo, wobei Alkylreste durch Fluor einfach oder mehrfach substituiert sein können.

Heterocyclen, die für mono- oder bicyclische 5- bis 12-gliedrige heterocyclische Ringe stehen, können aromatisch oder teilweise oder vollständig gesättigt sein und können insbesondere an einem Stickstoffatom durch (C₁-C₇)-Alkyl, zum Beispiel Methyl oder Ethyl, Phenyl oder Phenyl-(C₁-C₄)-alkyl, zum Beispiel Benzyl, und/oder an einem oder mehreren Kohlenstoffatomen durch (C₁-C₄)-Alkyl, Halogen, Hydroxy, (C₁-C₄)-Alkoxy, zum Beispiel Methoxy, Phenyl-(C₁-C₄)-alkoxy, zum Beispiel Benzyloxy, oder Oxo substituiert sein.

Beispiele für Heterocyclen, die den Gruppen Heteroaryl, Het oder dem dem monooder bicyclischen 5- bis 12-gliedrigen heterocyclischen Ring zugrunde liegen können, sind Pyrrol, Furan, Thiophen, Imidazol, Pyrazol, Oxazol, Isoxazol, Thiazol, Isothiazol, Tetrazol, Pyridin, Pyrazin, Pyrimidin, Indol, Isoindol, Indazol, Phthalazin, Chinolin, Isochinolin, Chinoxalin, Chinazolin, Cinnolin, β-Carbolin oder benzanellierte, cyclopenta-, cyclohexa- oder cyclohepta-anellierte Derivate dieser Heterocyclen. Stickstoffheterocyclen können auch als N-Oxide vorliegen.

Reste, die für Heteroaryl, Het oder den Rest eines mono- oder bicyclischen 5- bis 12-gliedrigen heterocyclischen Rings stehen können, sind beispielsweise 2- oder 3-Pyrrolyl, Phenylpyrrolyl, zum Beispiel 4- oder 5-Phenyl-2-pyrrolyl, 2-Furyl, 2-Thienyl, 4-Imidazolyl, Methylimidazolyl, zum Beispiel 1-Methyl-2-, -4- oder -5-imidazolyl, 1,3-Thiazol-2-yl, 2-, 3- oder 4-Pyridyl, 2-, 3- oder 4-Pyridyl-N-oxid, 2-Pyrazinyl, 2-, 4-oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl oder, als Reste von teilhydrierten oder vollständig hydrierten heterocyclischen Ringen, beispielsweise auch Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl, Benzodioxolanyl.

Weitere Beispiele von Resten, die insbesondere für Het- und für den Substituenten R¹ stehen können, sind 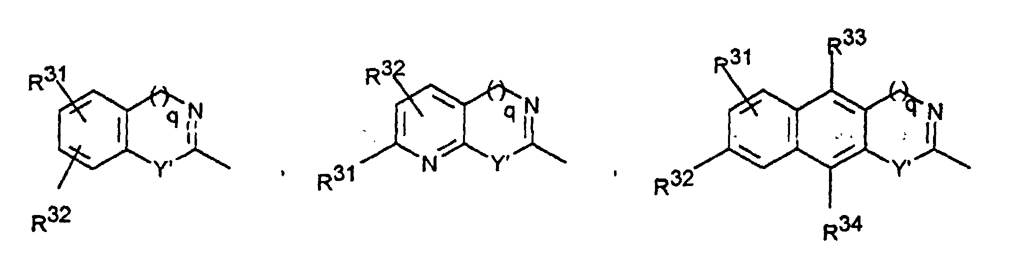 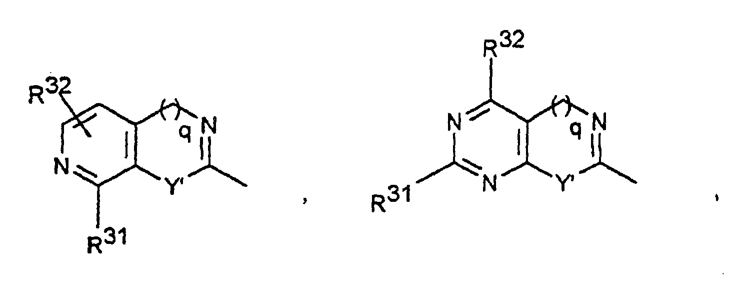 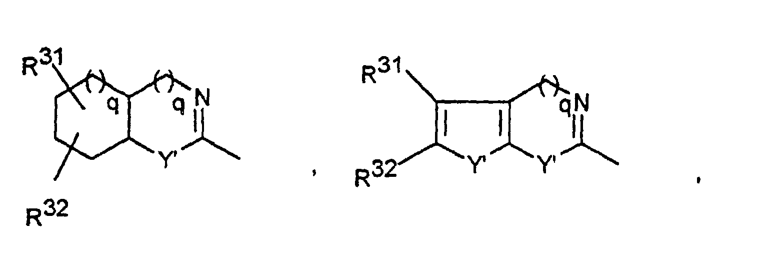 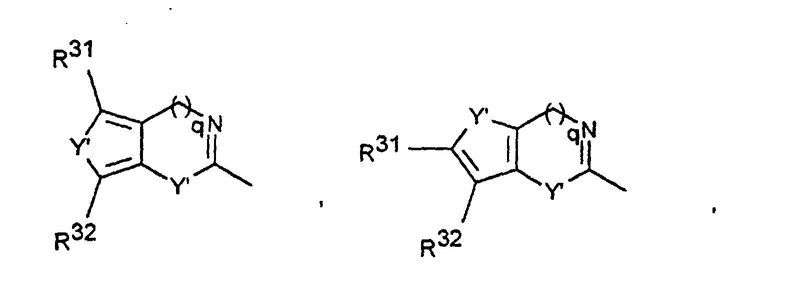 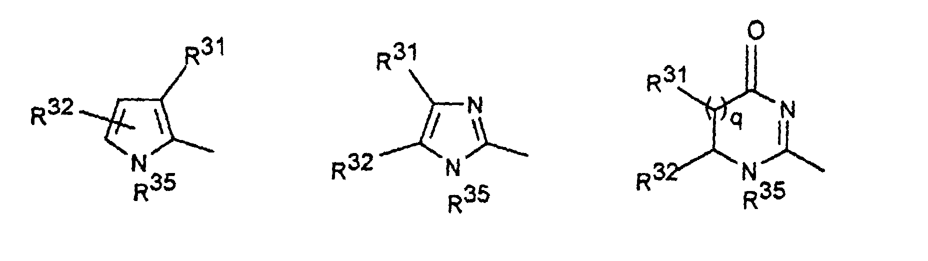 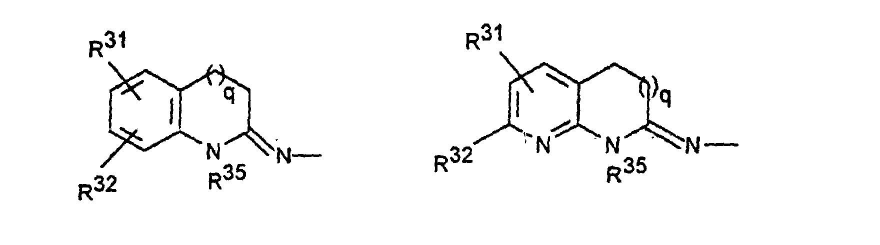 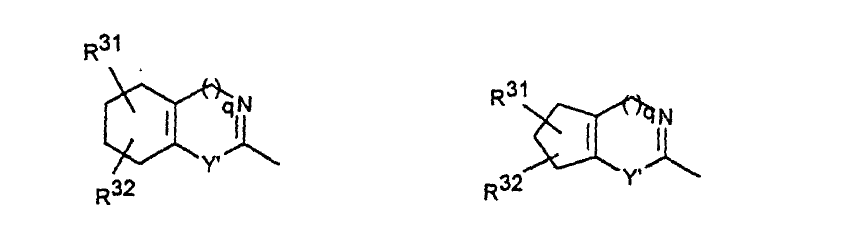 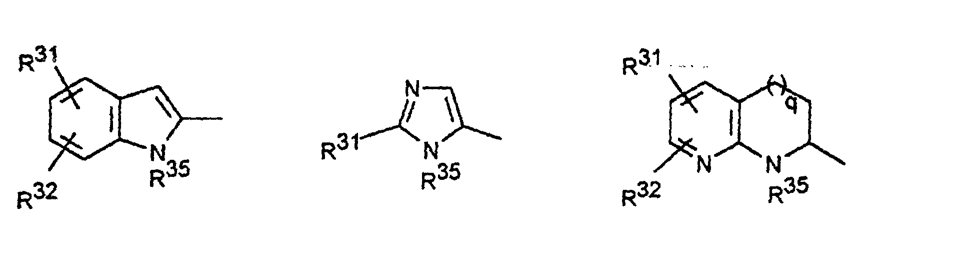 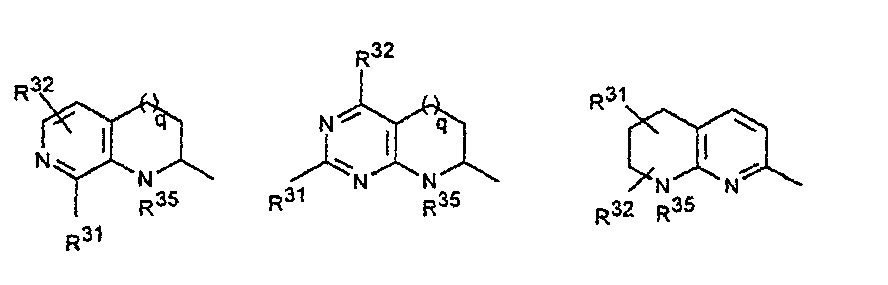 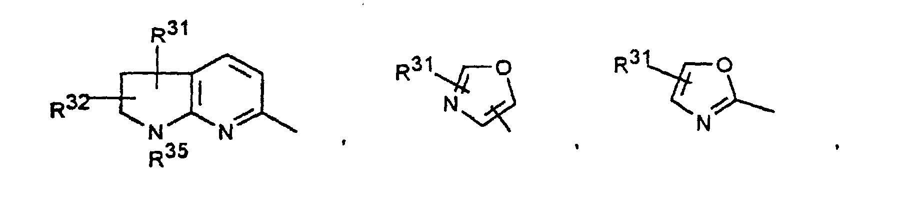 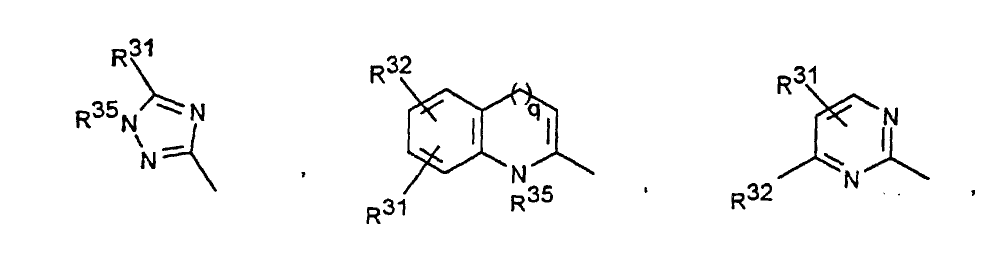 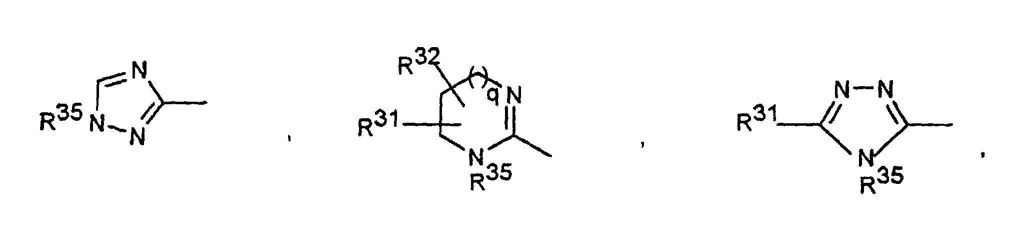 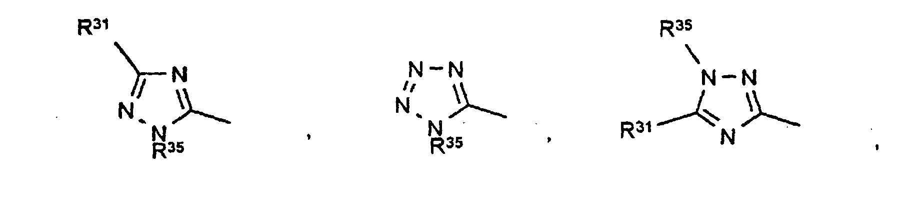 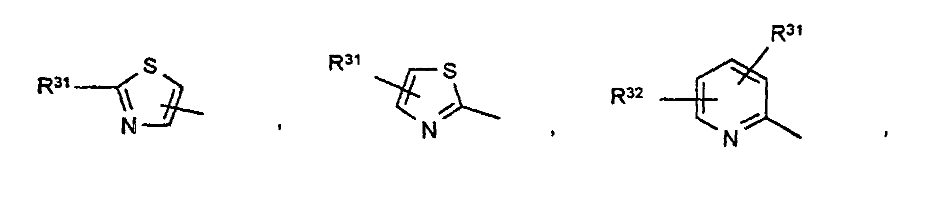 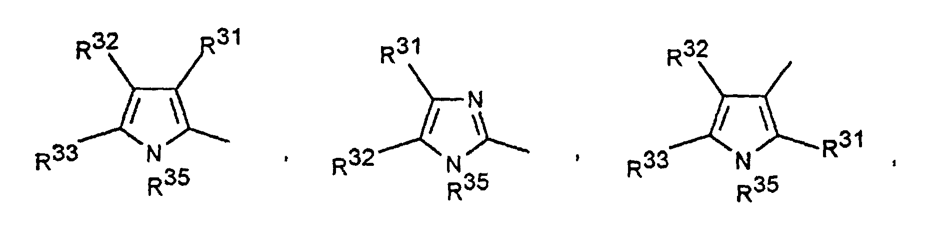 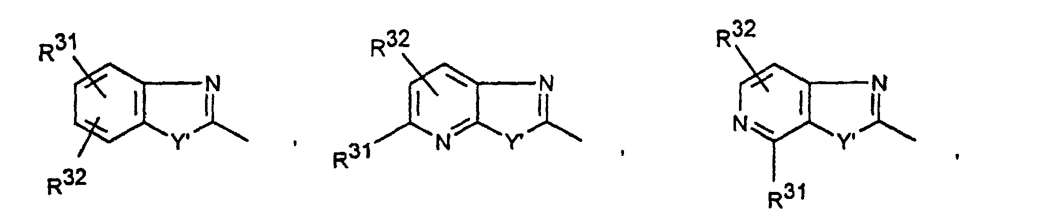 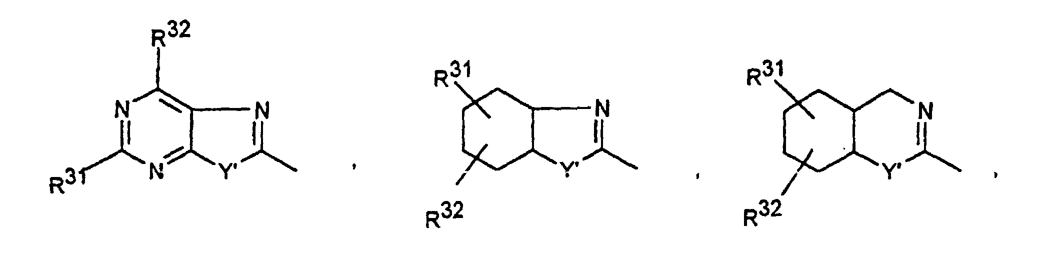 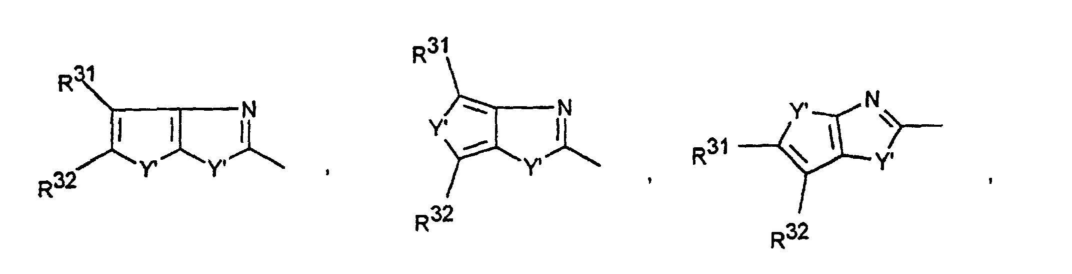 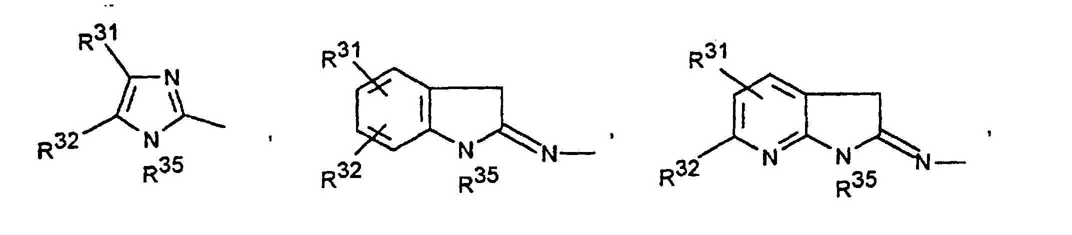 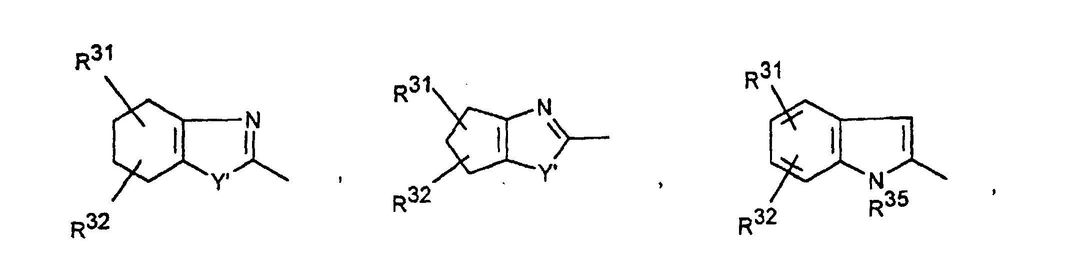 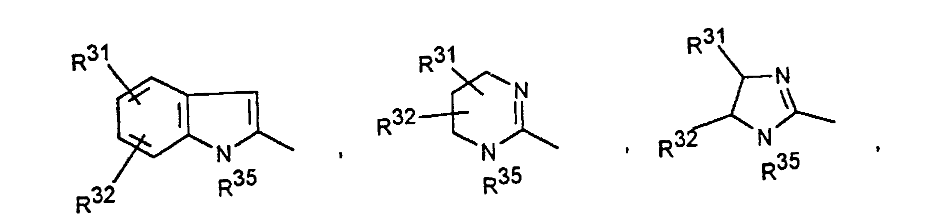 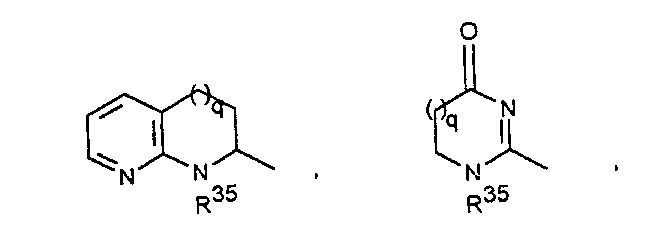 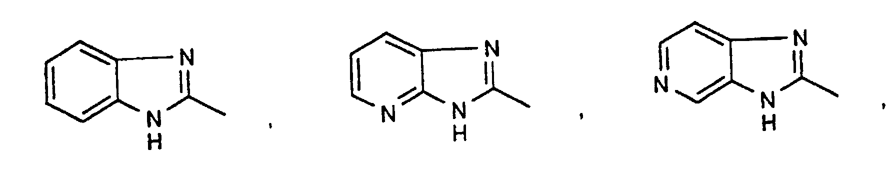 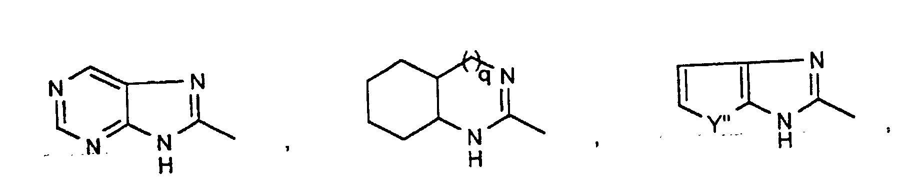 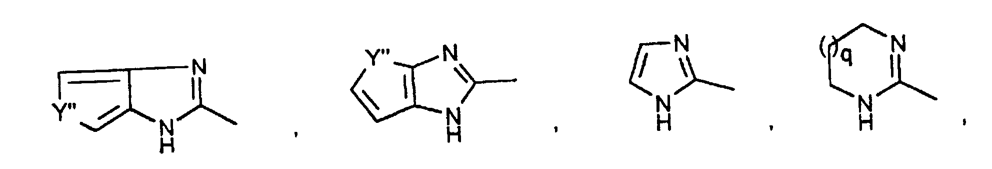 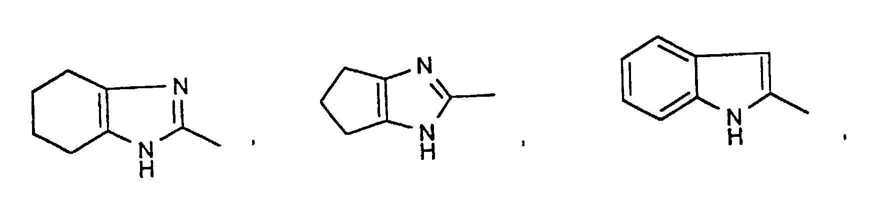 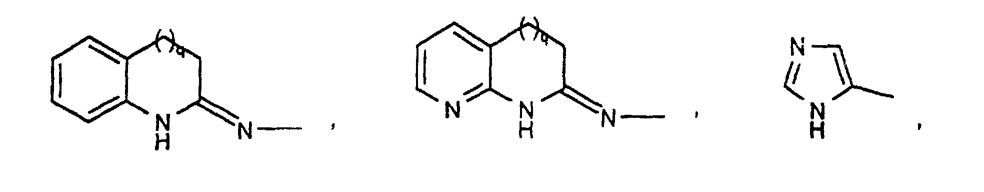 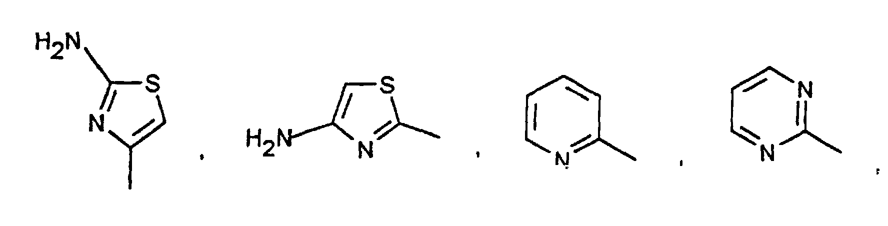 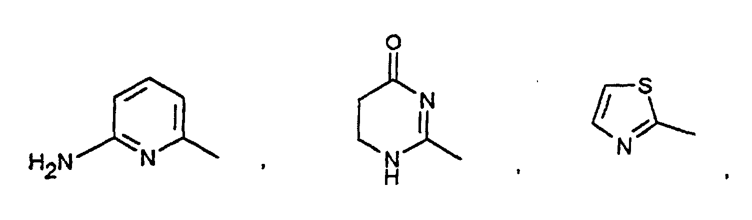 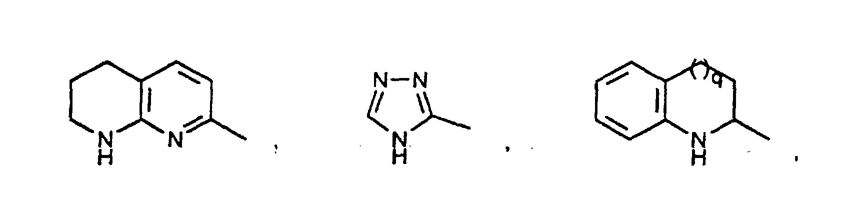 worin Y für NR³⁵, O oder S steht;
Y'' für NH, O oder S steht;
R³¹, R³², R³³ und R³⁴ unabhängig voneinander für Wasserstoff, (C₁-C₈)-Alkyl, insbesondere (C₁-C₄)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, Heteroaryl, Heteroaryl-(C₁-C₈)-alkyl, (C₁-C₈)-Alkoxy, insbesondere (C₁-C₄)-Alkoxy, Halogen, Nitro, Amino, (C₁-C₈)-Alkylamino, Di((C₁-C₈)-Alkyl)amino, Trifluormethyl, Hydroxy, Methylendioxy, Cyano, Hydroxycarbonyl, Aminocarbonyl, (C₁-C₄)-Alkoxycarbonyl, gegebenenfalls substituiertes Phenoxy, Benzyloxy, (R⁸O)₂P(O), (R⁸O)₂P(O)-O-, Oxo, Thioxo, stehen, wobei Alkylreste durch Fluor einfach oder mehrfach substituiert sein können;
R³⁵ für Wasserstoff, (C₁-C₁₀)-Alkyl, das gegebenenfalls durch Fluor einfach oder mehrfach substituiert sein kann, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, Heteroaryl, Heteroaryl-(C₁-C₈)-alkyl, Hydroxy, (C₁-C₈)-Alkoxy, Amino, Aminocarbonyl, Formyl, R³⁶C(O), R³⁶O-C(O) oder ((C₁-C₁₈)-Alkyl-O-C(O)-((C₁-C₆)-alkyl)-O-C(O)- steht;
R³⁶ für (C₁-C₁₀)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, Heteroaryl oder Heteroaryl-(C₁-C₈)-alkyl steht;
q für 0 oder 1 steht.

Halogen steht für Fluor, Chlor, Brom oder lod, insbesondere für Fluor oder Chlor.

Natürliche oder unnatürliche Aminosäuren können, falls chiral, in der D- oder L-Form vorliegen. Bevorzugt sind α-Aminosäuren. Beispielsweise seien genannt (vgl. Houben-Weyl, Methoden der organischen Chemie, Band 15/1 und 15/2, Georg Thieme Verlag, Stuttgart, 1974):

Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, tert-Butylglycin (Tbg), Neopentylglycin (Npg), Cyclohexylglycin (Chg), Cyclohexylalanin (Cha), 2-Thienylalanin (Thia), 2,2-Diphenylaminoessigsäure, 2-(p-Tolyl)-2-phenylaminoessigsäure, 2-(p-Chlorphenyl)-aminoessigsäure.

Unter Aminosäureseiten werden Seitenketten von natürlichen oder unnatürlichen Aminosäuren verstanden. Azaaminosäuren sind natürliche oder unnatürliche Aminosäuren, in denen der Zentralbaustein 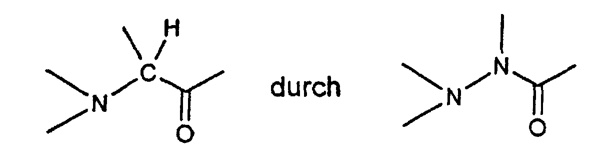 ersetzt ist.

Als Rest einer Iminosäure kommen insbesondere Reste von Heterocyclen aus der folgenden Gruppe in Betracht: Pyrrolidin-2-carbonsäure; Piperidin-2-carbonsäure; 1,2,3,4-Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Azabicyclo[2.2.2]octan-3-carbonsäure; 2-Azabicyclo[2.2.1]heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro(bicyclo[2.2.1]heptan)-2,3-pyrrolidin-5-carbonsäure; Spiro[bicyclo[2.2.2]octan)-2,3-pyrrolidin-5-carbonsäure; 2-Azatricyclo[4.3.0.1^{6,9}]decan-3-carbonsäure; Decahydrocyclohepta[b]pyrrol-2-carbonsäure; Decahydrocycloocta[c]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure, Hydroxypyrrolidin-2-carbonsäure, die alle gegebenenfalls substituiert sein können (siehe folgende Formeln):

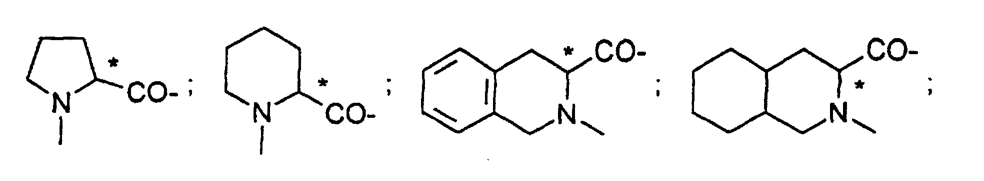

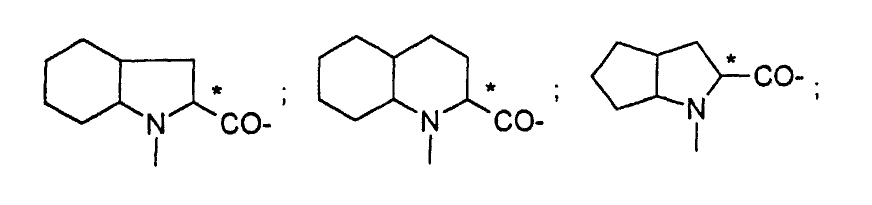

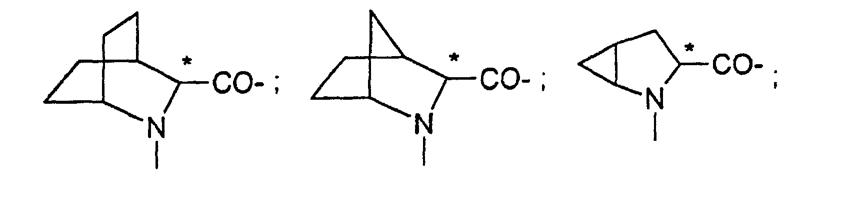

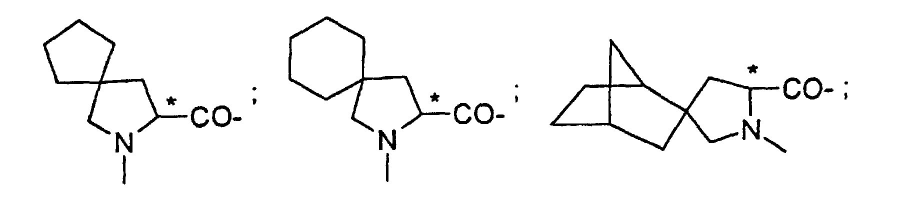

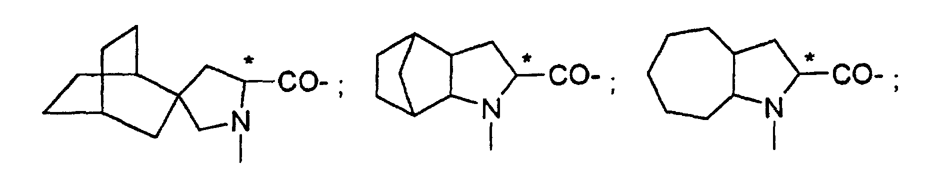

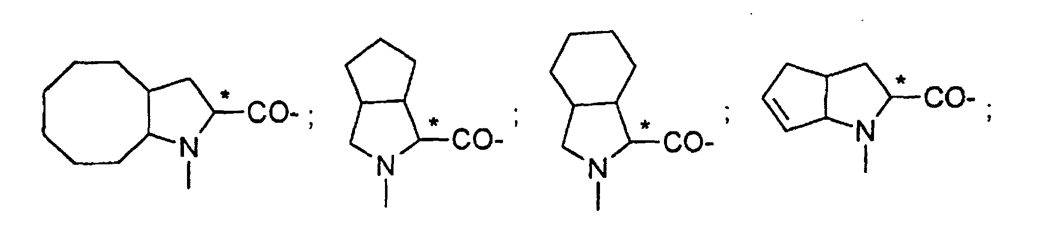

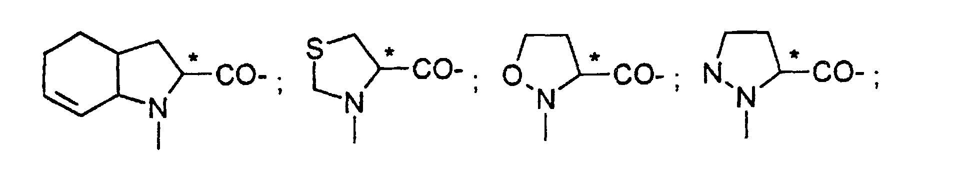

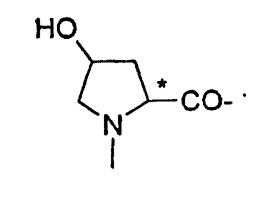

Die oben genannten Resten zugrundeliegenden Heterocyclen sind beispielsweise bekannt aus US-A-4,344,949; US-A 4,374,847; US-A 4,350,704; EP-A 29,488; EP-A 31,741; EP-A 46,953; EP-A 49,605; EP-A 49,658; EP-A 50,800; EP-A 51,020; EP-A 52,870; EP-A 79,022; EP-A 84,164; EP-A 89,637; EP-A 90,341; EP-A 90,362; EP-A 105,102; EP-A 109,020; EP-A 111,873; EP-A 271,865 und EP-A 344,682.

Dipeptide können als Bausteine natürliche oder unnatürliche Aminosäuren, Iminosäuren sowie Azaaminosäuren enthalten. Femer können die natürlichen oder unnatürlichen Aminosäuren, Iminosäuren, Azaaminosäuren und Dipeptide auch als Ester bzw. Amide vorliegen, wie zum Beispiel als Methylester, Ethylester, Isopropylester, Isobutylester, tert-Butylester, Benzylester, unsubstituiertes Amid, Ethylamid, Semicarbazid oder ω-Amino-(C₂-C₈)-alkylamid.

Funktionelle Gruppen der Aminosäuren, Iminosäuren und Dipeptide können geschützt vorliegen. Geeignete Schutzgruppen wie zum Beispiel Urethanschutzgruppen, Carboxylschutzgruppen und Seitenkettenschutzgruppen sind bei Hubbuch, Kontakte (Merck) 1979, Nr. 3, Seiten 14 bis 23, und bei Büllesbach, Kontakte (Merck) 1980, Nr. 1, Seiten 23 bis 35, beschrieben. Insbesondere seien genannt: Aloc, Pyoc, Fmoc, Tcboc, Z, Boc, Ddz, Bpoc, Adoc, Msc, Moc, Z(NO₂), Z(Halₙ), Bobz, Iboc, Adpoc, Mboc, Acm, tert-Butyl, OBzl, ONbzl, OMbzl, Bzl, Mob, Pic, Trt.

Physiologisch verträgliche Salze der Verbindungen der Formel I sind insbesondere pharmazeutisch verwendbare oder nicht-toxische Salze.

Solche Salze werden beispielsweise von Verbindungen der Formel I, welche saure Gruppen, zum Beispiel Carboxy, enthalten, mit Alkali- oder Erdalkalimetallen gebildet, wie zum Beispiel Na, K, Mg und Ca, sowie mit physiologisch verträglichen organischen Aminen, wie zum Beispiel Triethylamin, Ethanolamin oder Tris-(2-hydroxy-ethyl)-amin.

Verbindungen der Formel I, welche basische Gruppen, zum Beispiel eine Aminogruppe oder eine Guanidinogruppe enthalten, bilden mit anorganischen Säuren, wie zum Beispiel Salzsäure, Schwefelsäure oder Phosphorsäure, und mit organischen Carbon- oder Sulfonsäuren, wie zum Beispiel Essigsäure, Citronensäure, Benzoesäure, Maleinsäure, Fumarsäure, Weinsäure, Methansulfonsäure oder p-Toluolsulfonsäure Salze.

Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen.

Die Verbindungen der Formel I können in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, zum Beispiel Enantiomere und Diastereomere, und Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als links- als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Mischungen dieser Formen Gegenstand der Erfindung.

Die erfindungsgemäßen Verbindungen der Formel I können darüber hinaus bewegliche Wasserstoffatome enthalten, also in verschiedenen tautomeren Formen vorliegen. Auch diese alle Tautomeren sind Gegenstand der vorliegenden Erfindung. Die vorliegende Erfindung umfaßt weiterhin alle Solvate von Verbindungen der Formel I, zum Beispiel Hydrate oder Addukte mit Alkoholen, sowie Derivate der Verbindungen der Formel I, zum Beispiel Ester, Pro-Drugs und Metabolite, die wie die Verbindungen der Formel I wirken.

Die einzelnen Strukturelemente in der Formel I haben bevorzugt die folgenden Bedeutungen.
W steht bevorzugt für R¹-A-C(R¹³).
A steht bevorzugt für Methylen, Ethylen, Trimethylen, Tetramethylen, Pentamethylen, Cyclohexylen, Phenylen, Methylenphenyl, Methylenphenylmethyl, Phenylenmethyl oder Phenylenethyl.
Y steht bevorzugt für eine Carbonylgruppe.
Z steht bevorzugt für N(R⁰).
B steht bevorzugt für Methylen, Ethylen, Trimethylen, Tetramethylen, Vinylen, Phenylen oder für substituiertes Methylen oder Ethylen. Besonders bevorzugt steht B für einen zweiwertigen Methylenrest oder Ethylenrest (= 1,2-Ethylen), wobei jeder dieser Reste unsubstituiert oder substituiert sein kann, insbesondere für einen substituierten oder unsubstituierten Methylenrest. Ganz besonders bevorzugt sind diese beiden Reste substituiert. Ist ein für B stehender zweiwertiger Methylenrest oder Ethylenrest (= 1,2-Ethylen) substituiert, so ist er bevorzugt substituiert durch einen Rest aus der Reihe (C₁-C₈)-Alkyl, (C₂-C₆)-Alkenyl, (C₂-C₆)-Alkinyl, (C₃-C₈)-Cycloalkyl, insbesondere (C₅-C₆)-Cycloalkyl, (C₃-C₈)-Cycloalkyl-(C₁-C₄)-alkyl, insbesondere (C₅-C₆)-Cycloalkyl-(C₁-C₄)-alkyl, gegebenenfalls substituiertes (C₆-C₁₀)-Aryl, im Arylrest gegebenenfalls substituiertes (C₆-C₁₀)-Aryl-(C₁-C₄)-alkyl, gegebenenfalls substituiertes Heteroaryl oder im Heteroarylrest gegebenenfalls substituiertes Heteroaryl-(C₁-C₄)-alkyl, und ist er besonders bevorzugt substituiert durch (C₁-C₈)-Alkyl, also durch einen geradkettigen oder verzweigten Alkylrest mit 1, 2, 3, 4, 5, 6, 7 oder 8 Kohlenstoffatomen.
D steht bevorzugt für C(R²)(R³).
E steht bevorzugt für R¹⁰CO.
R steht bevorzugt für Wasserstoff, (C₁-C₆)-Alkyl oder Benzyl, insbesondere Wasserstoff, Methyl oder Ethyl.
R⁰ steht bevorzugt für (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl, besonders bevorzugt für (C₁-C₈)-Alkyl, (C₃-C₁₂)-Cycloalkyl, (C₃-C₁₂)-Cycloalkyl-(C₁-C₄)-alkyl, gegebenenfalls substituiertes (C₆-C₁₄)-Aryl, im Arylrest gegebenenfalls substituiertes (C₆-C₁₄)-Aryl-(C₁-C₄)-alkyl, gegebenenfalls substituiertes Heteroaryl oder im Heteroarylrest gegebenenfalls substituiertes Heteroaryl-(C₁-C₄)-alkyl, ganz besonders bevorzugt für gegebenenfalls substituiertes (C₆-C₁₄)-Aryl oder im Arylrest gegebenenfalls substituiertes (C₆-C₁₄)-Aryl-(C₁-C₈)-alkyl, darüber hinaus bevorzugt für im Arylrest gegebenenfalls substituiertes (C₆-C₁₄)-Aryl-(C₁-C₈)-alkyl. Speziell bevorzugt ist es, wenn R⁰ für unsubstituiertes oder im Arylrest einfach oder mehrfach substituiertes Biphenylylmethyl, Naphthylmethyl oder Benzyl steht.
R¹ steht bevorzugt für einen der Reste R²¹O-, R²⁴NH-, R²⁵N(R²⁵)-, HO-((C₁-C₈)-Alkyl)-N(R²⁶)-, R²¹O-C(O)- und R²⁸N(R²¹)-C(O)-.
R² steht bevorzugt für Wasserstoff oder (C₁-C₈)-Alkyl.
R³ steht bevorzugt für (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵, besonders bevorzugt für gegebenenfalls substituiertes (C₆-C₁₄)-Aryl, R¹¹NH, CON(CH₃)R⁴ oder CONHR⁴.
R⁴ steht bevorzugt für (C₁-C₈)-Alkyl, das gegebenenfalls wie in der Definition von R⁴ angegeben substituiert sein kann.
R¹¹ steht bevorzugt für Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵, wobei
R¹² hier für Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino, den Rest R¹⁵ oder den Rest R¹⁵-O- steht.
R¹³ steht bevorzugt für Wasserstoff und insbesondere für (C₁-C₆)-Alkyl, (C₃-C₈)-Cycloalkyl oder Benzyl, wobei ein ganz besonders bevorzugter Alkylrest, für den R¹³ steht, der Methylrest ist.
R¹⁵ steht bevorzugt für R¹⁶-(C₁-C₃)-alkyl oder für R¹⁶, besonders bevorzugt für R¹⁶-(C₁)-alkyl oder R¹⁶. Darüber hinaus bevorzugt steht R¹⁵ dann, wenn R³ für COOR¹⁵ steht, für den exo-2-Norbornylrest, den endo-2-Norbornylrest oder den Bicyclo[3.2.1]octylrest, und steht R¹⁵ dann, wenn R³ für CONHR¹⁵ steht, für den exo-2-Norbornylrest, den endo-2-Norbornylrest, den 3-Noradamantylrest und insbesondere den 1-Adamantylrest, den 2-Adamantylrest, den 1-Adamantylmethylrest oder den 2-Adamantylmethylrest.
R¹⁶ steht bevorzugt für einen 7- bis 12-gliedrigen verbrückten bicyclischen oder tricyclischen Rest, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann.
b, c und d stehen unabhängig voneinander für 1.
e, g und h stehen bevorzugt unabhängig voneinander für die Zahlen 0, 1, 2 oder 3.

Für die erfindungsgemäße Verwendung bevorzugte Verbindungen sind solche, in denen in der Formel I gleichzeitig
- W: für R¹-A-C(R¹³) oder R¹-A-CH=C steht;
- Y: für eine Carbonyl-, Thiocarbonyl- oder Methylengruppe steht;
- Z: für N(R⁰), Sauerstoff, Schwefel oder eine Methylengruppe steht;
- A: einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)- Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
- B: einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
- D: für C(R²)(R³), N(R³) oder CH=C(R³) steht;
- E: Tetrazolyl, (R⁸O)₂P(O), HOS(O)₂, R⁹NHS(O)₂ oder R¹⁰CO bedeutet;
- R und R⁰: unabhängig voneinander Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeuten, wobei Alkylreste durch Fluor ein- oder mehrfach substituiert sein können;
- R¹: Wasserstoff, (C₁-C₁₀)-Alkyl, das gegebenenfalls durch Fluor einfach oder mehrfach substituiert sein kann, (C₃-C₁₂)-Cycloalkyl, (C₃-C₁₂)-Cycloalkyl-(C₁- C₈)-alkyl, gegebenenfalls substituiertes R²¹-((C₆-C₁₄)-Aryl), im Arylrest gegebenenfalls substituiertes (R²¹-((C₆-C₁₄)-Aryl))-(C₁-C₈)-alkyl, den Rest Het-, Het-(C₁-C₈)-alkyl oder einen der Reste R²¹O-, R²²O-NH-, R²¹O-N(R²³)-, R²⁴NH-, R²⁵N(R²⁵)-, HO-((C₁-C₈)-Alkyl)-N(R²⁶)-, R²⁷C(O)-NH-, R²¹C(O)- N(R²³)-, R²¹C(O)-, R²¹O-C(O)-, R²⁸N(R²¹)-C(O)-, R²¹O-N=, O= und S= bedeutet;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)- Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)- Cycloalkyl, (C₂-C₈)-Alkenyl, (C₂-C₈)-Alkinyl, (C₂-C₈)-Alkenylcarbonyl, (C₂-C₈)- Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)- aminocarbonyl, Amino-(C₂-C₁₈)-alkylaminocarbonyl, Amino-(C₁-C₃)-alkyl- phenyl-(C₁-C₃)-alkylaminocarbonyl, (C₁-C₁₈)-Alkylcarbonylamino-(C₁-C₃)- alkylphenyl-(C₁-C₃)-alkylaminocarbonyl, (C₁-C₁₈)-Alkylcarbonylamino-(C₂-C₁₈)- alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)- Alkoxycarbonyl, gegebenenfalls substituiertes (C₃-C₈)-Cycloalkyl, HOS(O)₂-(C₁- C₃)-alkyl, R⁹NHS(O)₂-(C₁-C₃)-alkyl, (R⁸O)₂P(O)-(C₁-C₃)-alkyl, Tetrazolyl-(C₁- C₃)-alkyl, Halogen, Nitro, Trifluormethyl und den Rest R⁵ substituiert sein kann;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, Iminosäure-, gegebenenfalls N- (C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)- alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid- Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)- Cycloalkylaminocarbonyl, gegebenenfalls substituiertes (C₆-C₁₄)- Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: Wasserstoff, (C₁-C₁₈)-Alkyl, R¹²CO, gegebenenfalls substituiertes (C₆-C₁₄)- Aryl-S(O)₂, (C₁-C₁₈)-Alkyl-S(O)₂, im Arylrest gegebenenfalls substituiertes (C₆- C₁₄)-Aryl-(C₁-C₈)-alkyl, R⁹NHS(O)₂ oder den Rest R¹⁵ bedeutet;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)- Aryloxy, Amino oder Mono- oder Di-((C₁-C₁₈)-alkyl)-amino, den Rest R¹⁵ oder den Rest R¹⁵-O- bedeutet;
- R¹³: Wasserstoff, (C₁-C₆)-Alkyl, im Arylrest gegebenenfalls substituiertes (C₆-C₁₄)- Aryl-(C₁-C₈)-alkyl oder (C₃-C₈)-Cycloalkyl bedeutet;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- R²¹: Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₄)-Aryl, (C₆-C₁₄)-Aryl-(C₁-C₈)-alkyl, den Rest Het- oder Het-(C₁- C₈)-alkyl bedeutet, wobei Alkylreste durch Fluor einfach oder mehrfach substituiert sein können und die Reste R²¹ bei mehrfachem Auftreten gleich oder verschieden sein können;
- R²²: (C₃-C₁₂)-Cycloalkyl, (C₃-C₁₂)-Cycloalkyl-(C₁-C₈)-alkyl, (C₆-C₁₄)-Aryl, den Rest Het- oder Het-(C₁-C₈)-alkyl bedeutet, wobei Alkylreste durch Fluor einfach oder mehrfach substituiert sein können;
- R²³: (C₁-C₈)-Alkyl, (C₃-C₁₂)-Cycloalkyl, (C₃-C₁₂)-Cycloalkyl-(C₁-C₈)-alkyl, (C₆-C₁₄)- Aryl, (C₆-C₁₄)-Aryl-(C₁-C₈)-alkyl, den Rest Het- oder Het-(C₁-C₈)-alkyl bedeutet, wobei Alkylreste durch Fluor einfach oder mehrfach substituiert sein können;
- R²⁴: (C₃-C₁₂)-Cycloalkyl, (C₃-C₁₂)-Cycloalkyl-(C₁-C₈)-alkyl, (C₆-C₁₄)-Aryl, (C₆-C₁₄)- Aryl-(C₁-C₈)-alkyl, den Rest Het- oder Het-(C₁-C₈)-alkyl bedeutet, wobei Alkylreste durch Fluor einfach oder mehrfach substituiert sein können;
- R²⁵: die Bedeutungen von R²³ hat, wobei die Reste R²⁵ gleich oder verschieden sein können;
- R²⁶: die Bedeutungen von R²¹ hat oder HO-((C₁-C₈)-Alkyl) bedeutet, wobei Alkylreste durch Fluor einfach oder mehrfach substituiert sein können;
- R²⁷: Wasserstoff, (C₃-C₁₂)-Cycloalkyl, (C₃-C₁₂)-Cycloalkyl-(C₁-C₈)-alkyl, (C₆-C₁₄)- Aryl-(C₁-C₈)-alkyl, den Rest Het- oder Het-(C₁-C₈)-alkyl bedeutet, wobei Alkylreste durch Fluor einfach oder mehrfach substituiert sein können;
- R²⁸: für einen der Reste R²¹-, R²¹O-, R²⁶N(R²⁶)-, R²¹C(O)-, R²¹O-C(O)-, ((C₁-C₁₈)- Alkyl-O-C(O)-((C₁-C₆)-alkyl)-O-C(O)-, R²¹N(R²¹ )-C(O)-, R²¹N(R²¹)-C(=N(R²¹))- oder R²¹C(O)-N(R²¹)- steht;
- Het: für einen mono- oder polycyclischen, 4- bis 14-gliedrigen, aromatischen oder nicht aromatischen Ring steht, der 1, 2, 3 oder 4 gleiche oder verschiedene Heteroatome aus der Reihe N, O und S als Ringglieder enthält und gegebenenfalls durch einen oder mehrere, gleiche oder verschiedene Substituenten substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

Besonders bevorzugte Verbindungen der Formel I sind solche, worin gleichzeitig
- W: für R¹-A-CH=C und darin A für einen Phenylenrest oder einen Methylenphenylrest steht oder W für R¹-A-C(R¹³) und darin A für einen zweiwertigen Rest aus der Reihe Methylen, Ethylen, Trimethylen, Tetramethylen, Pentamethylen, Cyclohexylen, Phenylen, Phenylenmethyl, Methylenphenyl, Methylenphenylmethyl steht;
- B: für einen zweiwertigen Rest aus der Reihe Methylen, Ethylen, Trimethylen, Tetramethylen, Vinylen, Phenylen oder für substituiertes Methylen oder Ethylen steht;
- E: R¹⁰CO bedeutet;
- R: Wasserstoff, (C₁-C₆)-Alkyl oder Benzyl bedeutet;
- R⁰: für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
- R¹: für einen der Reste R²¹O-, R²⁴NH-, R²⁵N(R²⁵)-, HO-((C₁-C₈)-Alkyl)-N(R²⁶)-, R²¹O-C(O)- und R²⁸N(R²¹)-C(O)- steht;
- R²: für Wasserstoff oder (C₁-C₈)-Alkyl steht;
- R³: für (C₁-C₈)-Alkyl, gegebenenfalls substituiertes (C₆-C₁₄)-Aryl, (C₆-C₁₄)-Aryl-(C₁-C₈)-alkyl, (C₃-C₈)-Cycloalkyl, (C₂-C₈)-Alkenyl, (C₂-C₈)-Alkinyl, Pyridyl, R¹¹NH, R⁴CO, COOR⁴, CONHR⁴, CSNHR⁴, COOR¹⁵ und CONHR¹⁵ steht;
und e, g und h unabhängig voneinander für die Zahlen 0, 1, 2 oder 3 stehen; in allen ihren stereoisomeren Formen und Mischungen davon in allen Verhältnissen, sowie ihre physiologisch verträglichen Salze.

Ganz besonders bevorzugte Verbindungen der Formel I sind solche, worin W für R¹-A-C(R¹³) und R¹³ für (C₁-C₆)-Alkyl, im Arylrest gegebenenfalls substituiertes (C₆-C₁₄)-Aryl-(C₁-C₈)-alkyl oder (C₃-C₈)-Cycloalkyl steht,
in allen ihren stereoisomeren Formen und Mischungen davon in allen Verhältnissen, sowie ihre physiologisch verträglichen Salze.

Eine Reihe speziell bevorzugter Verbindungen der Formel I sind solche, worin R³ für gegebenenfalls substituiertes (C₆-C₁₄)-Aryl, für COOR⁴, für R¹¹NH oder für CONHR⁴ steht, wobei -NHR⁴ für den Rest einer α-Aminosäure, deren ω-Amino-(C₂-C₈)-alkylamid, deren (C₁-C₈)-Alkylester oder deren (C₆-C₁₄)-Aryl-(C₁-C₄)-alkylester steht, in allen ihren stereoisomeren Formen und Mischungen davon in allen Verhältnissen, sowie ihre physiologisch verträglichen Salze. Der für -NHR⁴ stehende Rest einer α-Aminosäure wird formal durch Abstraktion eines Wasserstoffatoms von der Aminogruppe der Aminosäure erhalten. Speziell bevorzugt ist es in dieser Reihe, wenn R³ für CONHR⁴ steht, wobei
-NHR⁴ für den Rest der α-Aminosäuren Valin, Lysin, Phenylglycin, Phenylalanin oder Tryptophan oder deren (C₁-C₈)-Alkylester oder (C₆-C₁₄)-Aryl-(C₁-C₄)-alkylester steht.

Darüber hinaus bevorzugte Verbindungen der Formel I in dieser Reihe sind solche, worin gleichzeitig
- W: für R¹-A-C(R¹³) steht;
- Y: für eine Carbonylgruppe steht;
- Z: für N(R⁰) steht;
- A: für Ethylen, Trimethylen, Tetramethylen, Pentamethylen, Cyclohexylen, Phenylen, Phenylenmethyl, Methylenphenyl oder Methylenphenylmethyl steht;
- B: für einen unsubstituierten oder substituierten Methylenrest steht;
- D: für C(R²)(R³) steht;
- E: für R¹⁰CO steht;
- R: für Wasserstoff oder (C₁-C₄)-Alkyl, insbesondere Wasserstoff, Methyl oder Ethyl, steht;
- R⁰: für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
- R¹: für den Rest R²⁸N(R²¹)-C(O)- steht;
- R²: für Wasserstoff steht;
- R³: für den Rest CONHR⁴ steht;
- R⁴: für Methyl steht, das durch Hydroxycarbonyl und einen Rest aus der Reihe (C₁-C₄)-Alkyl, Phenyl und Benzyl substituiert ist, oder für Methyl steht, das durch (C₁-C₈)-Alkoxycarbonyl, bevorzugt (C₁-C₄)-Alkoxycarbonyl, und einen Rest aus der Reihe (C₁-C₄)-Alkyl, Phenyl und Benzyl substituiert ist;
- R¹⁰: für Hydroxy oder (C₁-C₈)-Alkoxy, bevorzugt (C₁-C₄)-Alkoxy, steht;
- R¹³: für (C₁-C₆)-Alkyl, (C₃-C₇)-Cycloalkyl oder Benzyl, insbesondere Methyl, steht;
b, c und d für 1 stehen und e, f und g für 0 stehen;
h für 1 oder 2, bevorzugt für 1, steht;
in allen ihren stereoisomeren Formen und Mischungen davon in allen Verhältnissen, sowie ihre physiologisch verträglichen Salze.

Steht -NHR⁴ für einen (C₁-C₈)-Alkylester einer α-Aminosäure bzw. enthält R⁴ einen Alkoxycarbonylrest, so ist der Methyl-, Ethyl-, Isopropyl-, Isobutyl- oder tert-Butylester bevorzugt, steht -NHR⁴ für einen (C₆-C₁₄)-Aryl-(C₁-C₄)-alkylester einer α-Aminosäure, so ist der Benzylester bevorzugt.

Eine weitere Reihe speziell bevorzugter Verbindungen der Formel I bilden solche Verbindungen, worin gleichzeitig
- W: für R¹-A-CH=C und darin A für einen Phenylenrest oder einen Methylenphenylrest steht oder W für R¹-A-C(R¹³) und darin A für einen zweiwertigen Rest aus der Reihe Methylen, Ethylen, Trimethylen, Tetramethylen, Pentamethylen, Cyclohexylen, Phenylen, Phenylenmethyl, Methylenphenyl, Methylenphenylmethyl steht;
- B: für einen zweiwertigen Rest aus der Reihe Methylen, Ethylen, Trimethylen, Tetramethylen, Vinylen, Phenylen oder für substituiertes Methylen oder Ethylen steht;
- E: R¹⁰CO bedeutet;
- R: Wasserstoff oder (C₁-C₆)-Alkyl bedeutet;
- R⁰: für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
- R¹: für einen der Reste R²¹O-, R²¹O-N(R²³)-, R²⁴NH-, R²⁵N(R²⁵)-, HO-((C₁-C₈)-Alkyl)-N(R²⁶)-, R²¹O-C(O)- und R²⁸N(R²¹)-C(O)- steht;
- R²: für Wasserstoff oder (C₁-C₈)-Alkyl steht;
- R³: für CONHR¹⁵ oder CONHR⁴ steht, wobei R⁴ hierin für einen unsubstituierten oder durch einen oder mehrere (C₆-C₁₄)-Arylreste substituierten (C₁-C₈)-Alkylrest steht;
- R¹⁵: für R¹⁶-(C₁-C₆)-Alkyl oder R¹⁶ steht, wobei R¹⁶ für einen 7- bis 12-gliedrigen verbrückten bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann, und insbesondere R¹⁵ für einen Adamantylrest oder einen Adamantylmethylrest steht;
und e, g und h unabhängig voneinander für die Zahlen 0, 1, 2 oder 3 stehen und b, c und d für 1 stehen;
in allen ihren stereoisomeren Formen und Mischungen davon in allen Verhältnissen, sowie ihre physiologisch verträglichen Salze.

Darüber hinaus bevorzugte Verbindungen der Formel I in dieser Reihe sind solche, worin gleichzeitig
- W: für R¹-A-C(R¹³) steht;
- Y: für eine Carbonylgruppe steht;
- Z: für N(R⁰) steht;
- A: für Ethylen, Trimethylen, Tetramethylen, Pentamethylen, Cyclohexylen, Phenylen, Phenylenmethyl, Methylenphenyl oder Methylenphenylmethyl steht;
- B: für einen unsubstituierten oder substituierten Methylenrest steht;
- D: für C(R²)(R³) steht;
- E: für R¹⁰CO steht;
- R: für Wasserstoff oder (C₁-C₄)-Alkyl, insbesondere Wasserstoff, Methyl oder Ethyl, steht;
- R⁰: für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
- R¹: für den Rest R²⁸N(R²¹)-C(O)- steht;
- R²: für Wasserstoff steht;
- R³: für CONHR¹⁵ oder CONHR⁴ steht, wobei R⁴ hierin für einen unsubstituierten oder durch einen oder mehrere (C₆-C₁₀)-Arylreste substituierten (C₁-C₆)-Alkylrest steht;
- R¹⁰: für Hydroxy oder (C₁-C₈)-Alkoxy, bevorzugt (C₁-C₄)-Alkoxy, steht;
- R¹³: für (C₁-C₆)-Alkyl, (C₃-C₇)-Cycloalkyl oder Benzyl, insbesondere Methyl, steht;
- R¹⁵: für einen Adamantylrest oder einen Adamantylmethylrest steht;
b, c und d für 1 stehen und e, f und g für 0 stehen;
h für 1 oder 2, bevorzugt für 1, steht;
in allen ihren stereoisomeren Formen und Mischungen davon in allen Verhältnissen, sowie ihre physiologisch verträglichen Salze.

Weiterhin bilden eine Reihe speziell bevorzugter Verbindungen der Formel I solche Verbindungen, in denen gleichzeitig
- W: für R¹-A-C(R¹³) steht;
- Y: für eine Carbonylgruppe steht;
- Z: für N(R⁰) steht;
- A: für Ethylen, Trimethylen, Tetramethylen, Pentamethylen, Cyclohexylen, Phenylen, Phenylenmethyl, Methylenphenyl, Methylenphenylmethyl steht;
- B: für einen unsubstituierten oder substituierten Methylenrest oder Ethylenrest steht;
- D: für C(R²)(R³) steht;
- E: für R¹⁰CO steht;
- R: für Wasserstoff oder (C₁-C₄)-Alkyl, insbesondere Wasserstoff, Methyl oder Ethyl, steht;
- R⁰: für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
- R¹: für einen der Reste R²¹O-, R²⁴NH-, R²⁵N(R²⁵)-, HO-((C₁-C₈)-Alkyl)-N(R²⁶)-, R²¹O-C(O)- und R²⁸N(R²¹)-C(O)- steht;
- R²: für Wasserstoff steht;
- R³: für einen unsubstituierten Phenylrest oder Naphthylrest, einen durch einen, zwei oder drei gleiche oder verschiedene Reste aus der Reihe (C₁-C₄)-Alkyl, (C₁-C₄)-Alkoxy, Hydroxy, Halogen, Trifluormethyl, Nitro, Methylendioxy, Ethylendioxy, Hydroxycarbonyl, (C₁-C₄)-Alkoxycarbonyl, Aminocarbonyl, Cyan, Phenyl, Phenoxy und Benzyloxy substituierten Phenylrest oder Naphthylrest, einen Pyridylrest, einen (C₁-C₄)-Alkylrest, einen (C₂-C₄)-Alkenylrest, einen (C₂-C₄)-Alkinylrest oder einen (C₅-C₆)-Cycloalkylrest steht, und insbesondere R³ für einen unsubstituierten oder substituierten Phenylrest oder Naphthylrest steht;
- R¹⁰: für Hydroxy oder (C₁-C₈)-Alkoxy, insbesondere (C₁-C₄)-Alkoxy, steht und bevorzugt R¹⁰ für einen Rest aus der Reihe Hydroxy, Methoxy, Ethoxy, Propoxy und Isopropoxy steht;
- R¹³: für (C₁-C₆)-Alkyl, (C₃-C₇)-Cycloalkyl oder Benzyl, insbesondere Methyl, steht;
b, c und d für 1 stehen und e, f und g für 0 stehen;
h für 1 oder 2, bevorzugt für 1, steht;
in allen ihren stereoisomeren Formen und Mischungen davon in allen Verhältnissen, sowie ihre physiologisch verträglichen Salze.

Schließlich bilden eine Reihe speziell bevorzugter Verbindungen der Formel I solche Verbindungen, in denen gleichzeitig
- W: für R¹-A-C(R¹³) steht;
- Y: für eine Carbonylgruppe steht;
- Z: für N(R⁰) steht;
- A: für Ethylen, Trimethylen, Tetramethylen, Pentamethylen, Cyclohexylen, Phenylen, Phenylenmethyl, Methylenphenyl, Methylenphenylmethyl steht;
- B: für einen unsubstituierten oder substituierten Methylenrest oder Ethylenrest steht;
- D: für C(R²)(R³) steht;
- E: für R¹⁰CO steht;
- R: für Wasserstoff oder (C₁-C₄)-Alkyl, insbesondere Wasserstoff, Methyl oder Ethyl, steht;
- R⁰: für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
- R¹: für einen der Reste R²¹O-, R²⁴NH-, R²⁵N(R²⁵)-, HO-((C₁-C₈)-Alkyl)-N(R²⁶)-, R²¹O-C(O)- und R²⁸N(R²¹)-C(O)- steht;
- R²: für Wasserstoff steht;
- R³: für R¹¹NH steht;
- R¹⁰: für Hydroxy oder (C₁-C₈)-Alkoxy, insbesondere (C₁-C₄)-Alkoxy, steht und bevorzugt R¹⁰ für einen Rest aus der Reihe Hydroxy, Methoxy, Ethoxy, Propoxy und Isopropoxy steht;
- R¹³: für (C₁-C₆)-Alkyl, (C₃-C₇)-Cycloalkyl oder Benzyl, insbesondere Methyl, steht;
b, c, d und e für 1 stehen und f und g für 0 stehen;
h für 0 steht;
in allen ihren stereoisomeren Formen und Mischungen davon in allen Verhältnissen, sowie ihre physiologisch verträglichen Salze.

Ganz speziell bevorzugte Verbindungen der Formel I sind solche, worin ein für die Gruppe B stehender substituierter Methylenrest oder substituierter Ethylenrest als Substituenten einen Rest aus der Reihe (C₁-C₈)-Alkyl, (C₂-C₆)-Alkenyl, (C₂-C₆)-Alkinyl, (C₃-C₈)-Cycloalkyl, insbesondere (C₅-C₆)-Cycloalkyl, (C₃-C₈)-Cycloalkyl-(C₁-C₄)-alkyl, insbesondere (C₅-C₆)-Cycloalkyl-(C₁-C₄)-alkyl, gegebenenfalls substituiertes (C₆-C₁₀)-Aryl, im Arylrest gegebenenfalls substituiertes (C₆-C₁₀)-Aryl-(C₁-C₄)-alkyl, gegebenenfalls substituiertes Heteroaryl und im Heteroarylrest gegebenenfalls substituiertes Heteroaryl-(C₁-C₄)-alkyl trägt, in allen ihren stereoisomeren Formen und Mischungen davon in allen Verhältnissen, sowie ihre physiologisch verträglichen Salze. Noch spezieller bevorzugte Verbindungen der Formel I sind solche, worin B für einen unsubstituierten Methylenrest steht oder für einen Methylenrest steht, der durch einen (C₁-C₈)-Alkylrest, insbesondere durch einen (C₁-C₆)-Alkylrest, substituiert ist, in allen ihren stereoisomeren Formen und Mischungen davon in allen Verhältnissen, sowie ihre physiologisch verträglichen Salze.

Generell sind Verbindungen der Formel I bevorzugt, die an Chiralitätszentren, z. B an dem für D stehenden chiralen Kohlenstoffatom und an dem Zentrum W im 5-Ring-Heterocyclus in der Formel I, eine einheitliche Konfiguration aufweisen.

Die Verbindungen der Formel I können beispielsweise hergestellt werden durch Fragmentkondensation einer Verbindung der Formel II 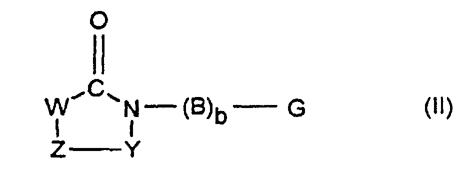 mit einer Verbindung der Formel III, 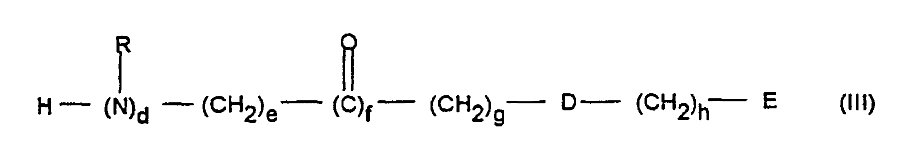 wobei W, Y, Z, B, D, E, R sowie b, d, e, f, g, und h wie oben angegeben definiert sind und G für Hydroxycarbonyl, (C₁-C₆)-Alkoxycarbonyl, aktivierte Carbonsäurederivate, wie Säurechloride oder Aktivester, oder Isocyanato steht.

Zur Kondensation der Verbindungen der Formel II mit denen der Formel III verwendet man vorteilhafterweise die an sich bekannten Kupplungsmethoden der Peptidchemie (siehe zum Beispiel Houben-Weyl, Methoden der Organischen Chemie, Band 15/1 und 15/2, Georg Thieme Verlag, Stuttgart, 1974). Dazu ist es in der Regel nötig, daß vorhandene, nicht reagierende Aminogruppen durch reversible Schutzgruppen während der Kondensation geschützt werden. Gleiches gilt für nicht an der Reaktion beteiligte Carboxylgruppen, die bevorzugt als (C₁-C₆)-Alkyl-, Benzyl- oder tert-Butylester vorliegen. Ein Aminogruppen-Schutz erübrigt sich, wenn die zu generierenden Aminogruppen noch als Nitro- oder Cyanogruppen vorliegen und erst nach der Kupplung zum Beispiel durch Hydrierung gebildet werden. Nach der Kupplung werden die vorhandenen Schutzgruppen in geeigneter Weise abgespalten. Beispielsweise können NO₂-Gruppen (Guanidinoschutz), Benzyloxycarbonylgruppen und Benzylester abhydriert werden. Die Schutzgruppen vom tert-Butyltyp werden sauer abgespalten, während der 9-Fluorenylmethyloxycarbonylrest durch sekundäre Amine entfernt wird. Die Herstellung der Verbindungen der Formel I kann beispielsweise auch erfolgen, indem man die Verbindungen nach üblichen Methoden schrittweise an einer Festphase aufbaut, wie dies weiter unten beispielhaft erläutert ist.

Verbindungen der Formel II, in der W für R¹-A-C(R¹³), Y für eine Carbonylgruppe und Z für NR⁰ steht, können beispielsweise hergestellt werden, indem man zunächst Verbindungen der Formel IV 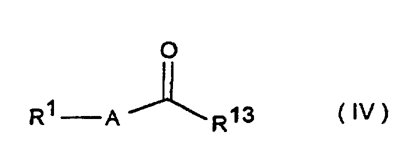 in einer Bucherer-Reaktion zu Verbindungen der Formel V, 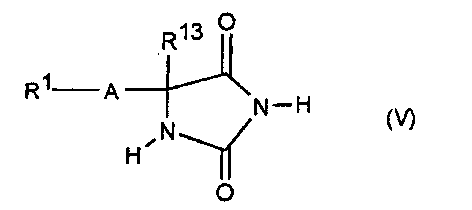 in der ebenso wie in der Formel IV R¹, R¹³ und A wie oben angegeben definiert sind, umsetzt (H. T. Bucherer, V. A. Lieb, J. Prakt. Chem. 141(1934), 5). Verbindungen der Formel VI, 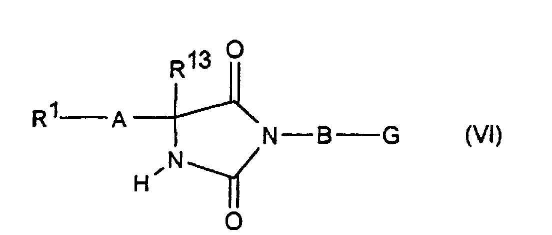 in der R¹, R¹³, A, B und G wie oben angegeben definiert sind, können dann erhalten werden, indem man die Verbindungen der Formel V beispielsweise zunächst mit einem alkylierenden Reagenz umsetzt, das den Rest -B-G in das Molekül einführt. Die Umsetzung von Verbindungen der Formel VI mit einem zweiten Reagenz der Formel R⁰-LG, in der R⁰ die oben angegebenen Bedeutungen hat und LG eine nucleophil substituierbare Abgangsgruppe, zum Beispiel Halogen, insbesondere Chlor oder Brom, (C₁-C₄)-Alkoxy, gegebenenfalls substituiertes Phenoxy oder eine heterocyclische Abgangsgruppe wie zum Beispiel Imidazolyl, darstellt, führt zu den entsprechenden Verbindungen der Formel II. Diese Umsetzungen können analog bekannten, dem Fachmann geläufigen Methoden durchgeführt werden. Je nach dem Einzelfall kann es hierbei, wie bei allen Schritten in der Synthese der Verbindungen der Formel I, angebracht sein, funktionelle Gruppen die zu Nebenreaktionen oder unerwünschten Reaktionen führen könnten, durch eine dem Syntheseproblem angepaßte Schutzgruppenstrategie temporär zu blockieren, was dem Fachmann bekannt ist.

Steht W für R¹-A-CH=C, so kann dieses Strukturelement beispielsweise eingeführt werden, indem analog bekannten Methoden ein Aldehyd mit einem 5-Ring-Heterocyclus kondensiert wird, der eine Methylengruppe in der der Gruppe W entsprechenden Position enthält.

Verbindungen der Formel I, in denen der 5-Ring-Heterocyclus einen Dioxo- oder Thioxo-oxo-substituierten Imidazolidinring darstellt, in dem W für R¹-A-C(R¹³) steht, können auch wie folgt erhalten werden:
Durch Reaktion von α-Aminosäuren oder N-substituierten α-Aminosäuren oder bevorzugt deren Ester, zum Beispiel der Methyl-, Ethyl-, tert-Butyl- oder Benzylester, beispielsweise einer Verbindung der Formel VII, 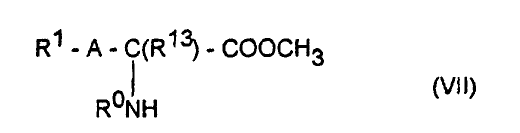 worin R⁰, R¹, R¹³ und A wie oben angegeben definiert sind, mit einem Isocyanat oder Isothiocyanat beispielsweise der Formel VIII, 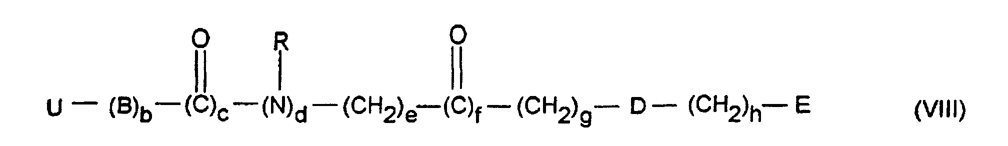 worin B, D, E und R sowie b, c, d, e, f, g und h wie oben angegeben definiert sind und U für Isocyanato oder Isothiocyanato steht, erhält man Harnstoff- oder Thioharnstoffderivate, beispielsweise der Formel IX, 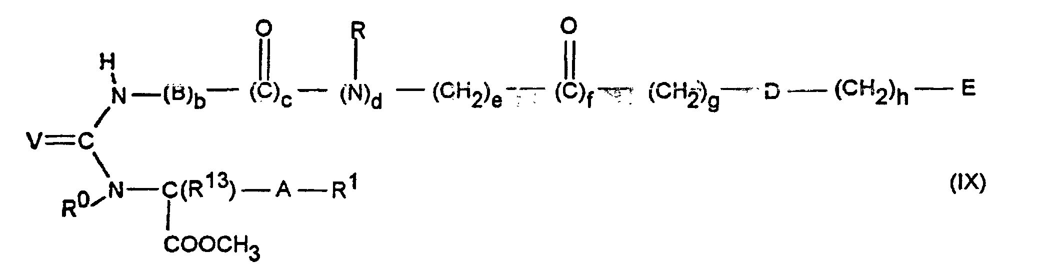 für die die oben angegebenen Definitionen gelten und in der V Sauerstoff oder Schwefel bedeutet, und die durch Erhitzen mit Säure unter Verseifung der Esterfunktionen zu Verbindungen der Formel Ia 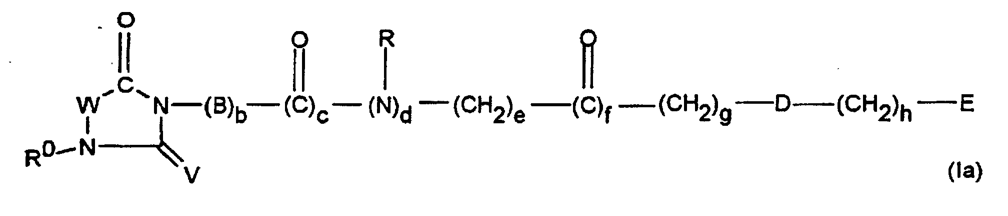 cyclisiert werden, in der V Sauerstoff oder Schwefel bedeutet, W für R¹-A-C(R¹³) steht und für die ansonsten die oben angegebenen Bedeutungen gelten. Die Cyclisierung der Verbindungen der Formel IX zu den Verbindungen der Formel Ia kann auch durch Behandlung mit Basen in inerten Lösungsmittel durchgeführt werden, zum Beispiel durch Behandlung mit Natriumhydrid in einem aprotischen Lösungsmittel wie Dimethylformamid.

Während der Cyclisierung können Guanidinogruppen durch Schutzgruppen, zum Beispiel NO₂, blockiert werden. Aminogruppen können in geschützer Form oder noch als NO₂- oder Cyanofunktion vorliegen, die später zur Aminogruppe reduziert oder im Falle der Cyanogruppe auch in die Formamidinogruppe umgewandelt werden kann.

Verbindungen der Formel I, in denen der 5-Ring-Heterocyclus einen Dioxo- oder Thioxo-oxo-substituierten Imidazolidinring darstellt, in dem W für R¹-A-C(R¹³) und c für 1 steht, können auch erhalten werden, indem man eine Verbindung der Formel VII mit einem Isocyanat oder Isothiocyanat der Formel X 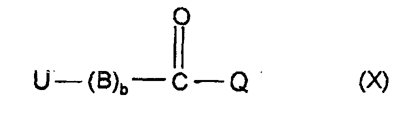 umsetzt, in der B, U und b wie oben für die Formel VIII angegeben definiert sind und Q eine Alkoxygruppe, zum Beispiel eine (C₁-C₄)-Alkoxygruppe wie Methoxy, Ethoxy oder tert-Butoxy, eine (C₆-C₁₄)-Aryloxygruppe, zum Beispiel Phenoxy, oder eine (C₆-C₁₄)-Aryl-(C₁-C₄)-alkoxygruppe, zum Beispiel Benzyloxy, bedeutet. Dabei wird eine Verbindung der Formel XI 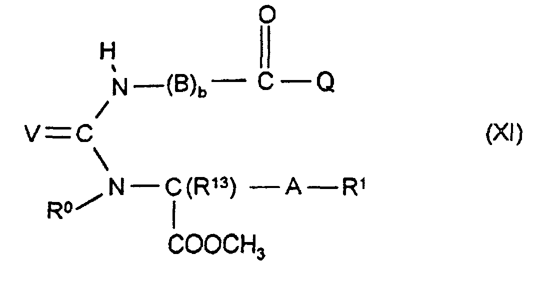 erhalten, in der A, B, V, Q, R⁰, R¹, R¹³ und b wie oben für die Formeln IX und X angegeben definiert sind, die dann unter dem Einfluß einer Säure oder einer Base, wie oben für die Cyclisierung der Verbindungen der Formel IX beschrieben, zu einer Verbindung der Formel XII, 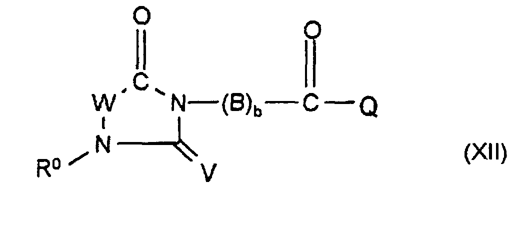 in der B, Q, V, W, R⁰ und b wie oben für die Formeln Ia und X angegeben definiert sind, cyclisiert wird. Aus der Verbindung der Formel XII wird dann durch Hydrolyse der Gruppe CO-Q zur Carbonsäure COOH und nachfolgende Kupplung mit einer Verbindung der Formel III, wie oben für die Kupplung der Verbindungen der Formeln II und III beschrieben, eine Verbindung der Formel Ia erhalten. Auch hier können während der Cyclisierung funktionelle Gruppen in geschützter Form vorliegen oder in Form von Vorstufen vorliegen, zum Beispiel Guanidinogruppen durch NO₂ blockiert werden oder Aminogruppen in geschützer Form oder noch als NO₂- oder Cyanofunktion vorliegen, die später zur Aminogruppe reduziert oder im Falle der Cyanogruppe auch in die Formamidinogruppe umgewandelt werden kann.

Eine weitere Methode zur Herstellung von Verbindungen der Formel Ia ist beispielsweise die Umsetzung von Verbindungen der Formel XIII, 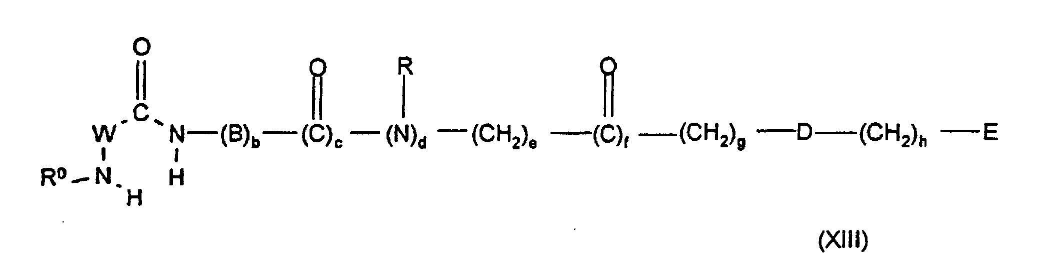 in der W für R¹-A-C(R¹³) steht und für die ansonsten die oben angegebenen Definitionen gelten, mit Phosgen, Thiophosgen oder entsprechenden Äquivalenten (analog S. Goldschmidt und M. Wick, Liebigs Ann. Chem. 575 (1952), 217-231 und C. Tropp, Chem. Ber. 61 (1928), 1431-1439).

Eine Überführung einer Aminofunktion in eine Guanidinofunktion kann mit folgenden Reagenzien durchgeführt werden:
1. O-Methylisoharnstoff (S.Weiss und H. Krommer, Chemiker Zeitung 98 (1974), 617-618)
2. S-Methylisothioharnstoff (R.F. Borne, M.L.Forrester und I.W. Waters, J. Med. Chem. 20 (1977), 771-776)
3. Nitro-S-methylisothioharnstoff (L.S. Hafner und R.E. Evans, J. Org. Chem. 24 (1959) 57)
4. Formamidinosulfonsäure (K. Kim, Y.-T. Lin und H.S. Mosher, Tetrah. Lett. 29 (1988), 3183-3186)
5. 3,5-Dimethyl-1-pyrazolyl-formamidinium-nitrat (F.L. Scott, D.G. O'Donovan und J. Reilly, J. Amer. Chem. Soc. 75 (1953), 4053-4054)
6. N,N'-Di-tert-butyloxycarbonyl-S-methyl-isothioharnstoff (R. J. Bergeron und J. S. McManis, J. Org. Chem. 52 (1987), 1700-1703)
7. N-Alkoxycarbonyl-, N,N'-Dialkoxycarbonyl-, N-Alkylcarbonyl- und N,N'dialkylcarbonyl-S-methyl-isothioharnstoff (H. Wollweber, H. Kölling, E. Niemers, A. Widdig, P. Andrews, H.-P. Schulz und H. Thomas, Arzneim. Forsch./Drug Res. 34 (1984), 531-542).

Amide können beispielsweise durch partielle Hydrolyse unter sauren oder basischen Bedingungen gemäß literaturbekannten Verfahren aus den entsprechenden Nitrilen (Cyano-Verbindungen) erhalten werden.

Hinsichtlich der Herstellung der Verbindungen der Formel I wird weiterhin vollinhaltlich Bezug genommen auf die WO-A-95/14008 und die DE-A-19635522 und die ihr entsprechenden Patentschriften, zum Beispiei die EP-A-796855 und die US-A-6218415, sowie die WO-A-96/33976.

Die Verbindungen der Formel I sind Antagonisten des Adhäsionsrezeptors VLA-4. Sie haben die Fähigkeit, Zell-Zell- und Zell-Matrix-Interaktionsprozesse zu inhibieren, bei denen Wechselwirkungen zwischen VLA-4 mit seinen Liganden eine Rolle spielen. Die Wirksamkeit der Verbindungen der Formel I kann beispielsweise in einem Assay nachgewiesen werden, in dem die Bindung von Zellen, die den VLA-4-Rezeptor aufweisen, zum Beispiel von Leukozyten, an Liganden dieses Rezeptors gemessen wird, zum Beispiel an VCAM-1, das dafür vorteilhafterweise auch gentechnisch hergestellt werden kann. Einzelheiten eines solchen Assay sind weiter unten beschrieben. Insbesondere vermögen die Verbindungen der Formel I die Adhäsion und die Migration von Leukozyten inhibieren, etwa die Anheftung von Leukozyten an endotheliale Zellen, die - wie oben erläutert - über den VCAM-1/VLA-4-Adhäsionsmechanismus gesteuert wird.

Die Verbindungen der Formel I und ihre physiologisch verträglichen Salze eignen sich daher zur Behandlung und Prophylaxe von Krankheiten, die auf der Wechselwirkung zwischen dem VLA-4-Rezeptor und seinen Liganden beruhen oder durch eine Hemmung dieser Wechselwirkung beeinflußt werden können, und insbesondere eignen sie sich für die Behandlung und Prophylaxe von Krankheiten, die zumindest teilweise durch ein unerwünschtes Ausmaß an Leukozytenadhäsion und/oder Leukozytenmigration verursacht werden oder damit verbunden sind, und zu deren Vorbeugung, Linderung oder Heilung die Adhäsion und/oder Migration von Leukozyten verringert werden soll. Sie können somit zum Beispiel bei entzündlichen Erscheinungen unterschiedlichster Ursache als Entzündungshemmer eingesetzt werden. Anwendung finden die Verbindungen der Formel I gemäß der vorliegenden Erfindung beispielsweise zur Behandlung oder Prophylaxe der rheumatoiden Arthritis, der inflammatorischen bowel disease (ulcerativen Colitis), des systemischen Lupus erythematosus oder zur Behandlung oder Prophylaxe von inflammatorischen Erkrankungen des zentralen Nervensystems, wie zum Beispiel der multiplen Sklerose, zur Behandlung oder Prophylaxe von Asthma oder von Allergien, z. B Allergien vom verzögerten Typ (Typ IV-Allergie), weiterhin zur Behandlung oder Prophylaxe von cardiovaskulären Erkrankungen, der Arteriosklerose, von Restenosen, zur Behandlung oder Prophylaxe von Diabetes, zur Verhinderung der Schädigung von Organtransplantaten, zur Hemmung von Tumorwachstum oder Tumormetastasierung bei verschiedenen Malignitäten, zur Therapie der Malaria, sowie von weiteren Krankheiten, bei denen eine Blockierung des Integrins VLA-4 und/oder eine Beeinflussung der Leukozytenaktivität zur Vorbeugung, Linderung oder Heilung angebracht erscheint. Die Verbindungen der Formel I und ihre Salze können weiterhin für diagnostische Zwecke, zum Beispiel bei in vitro-Diagnosen, und als Hilfsmittel in biochemischen Untersuchungen eingesetzt werden, bei denen eine VLA-4-Blockierung oder eine Beeinflussung von Zell-Zell- oder Zell-Matrix-Interaktionen angestrebt wird.

Die Verbindungen der Formel I und ihre physiologisch verträglichen Salze können erfindungsgemäß am Tier, bevorzugt am Säugetier, und insbesondere am Menschen als Arzneimittel zur Therapie oder Prophylaxe verabreicht werden. Sie können für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I und/oder ihrer physiologisch verträglichen Salze neben üblichen pharmazeutisch einwandfreien Trägerstoffen und/oder Zusatzstoffen enthalten. Gegenstand der vorliegenden Erfindung ist auch die Verwendung von pharmazeutischen Zubereitungen, die eine oder mehrere Verbindungen der Formel I und/oder ihre physiologisch verträglichen Salze enthalten, für die oben genannten erfindungsgemäßen Verwendungen der Verbindungen der Formel I. Die pharmazeutischen Zubereitungen enthalten normalerweise etwa 0,5 bis 90 Gew.-% der therapeutisch wirksamen Verbindungen der Formel I und/oder ihrer physiologisch verträglichen Salze.

Die Arzneimittel können oral, zum Beispiel in Form von Pillen, Tabletten, Filmtabletten, Dragees, Granulaten, Hart- und Weichgelatinekapseln, Lösungen, Sirupen, Emulsionen oder Suspensionen verabreicht werden. Die Verabreichung kann aber auch rektal, zum Beispiel in Form von Suppositorien, oder parenteral, zum Beispiel in Form von Injektions- oder infusionslösungen, Mikrokapseln oder Rods, oder perkutan, zum Beispiel in Form von Salben oder Tinkturen, oder auf anderem Wege, zum Beispiel in Form von Nasalsprays oder Aerosolmischungen, erfolgen.

Die Herstellung der erfindungsgemäß einzusetzenden pharmazeutischen Präparate erfolgt in an sich bekannter Weise, wobei neben der oder den Verbindungen der Formel I und/oder ihren physiologisch verträglichen Salzen pharmazeutisch inerte anorganische oder organische Trägerstoffe verwendet werden. Für die Herstellung von Pillen, Tabletten, Dragees und Hartgelatinekapseln kann man zum Beispiel Lactose, Maisstärke oder Derivate davon, Talk, Stearinsäure oder deren Salze etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich zum Beispiel Wasser, Alkohole, Glycerin, Polyole, Saccharose, Invertzucker, Glukose, pflanzliche Öle etc. Als Trägerstoffe für Mikrokapseln, Implantate oder Rods eignen sich zum Beispiel Mischpolymerisate aus Glykolsäure und Milchsäure.

Die pharmazeutischen Präparate können neben den Wirk- und Trägerstoffen noch Zusatzstoffe, wie zum Beispiel Füllstoffe, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I und/oder deren physiologisch verträgliche Salze enthalten. Ferner können sie neben mindestens einer Verbindung der Formel I und/oder ihren physiologisch verträglichen Salzen noch einen oder mehrere andere therapeutisch oder prophylaktisch wirksame Stoffe enthalten, zum Beispiel Stoffe mit entzündungshemmender Wirkung.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,01 bis 100 mg/kg, vorzugsweise 0,1 bis 10 mg/kg, insbesondere 0,3 bis 2 mg/kg Körpergewicht zur Erzielung wirksamer Ergebnisse angemessen. Bei intravenöser Applikation beträgt die Tagesdosis im allgemeinen etwa 0,01 bis 50 mg/kg, vorzugsweise 0,01 bis 10 mg/kg Körpergewicht. Die Tagesdosis kann, insbesondere bei der Applikation größerer Mengen, in mehrere, zum Beispiel 2, 3, oder 4, Teilverabreichungen aufgeteilt werden. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 500 mg, vorzugsweise 1 bis 100 mg Wirkstoff der Formel I und/oder dessen physiologisch verträgliche Salze pro Dosis.

Bestimmte Verbindungen der Formel I sind im Stand der Technik nicht explizit offenbart und stellen eine Auswahl aus der von der deutschen Patentanmeldung 19635522.2 und den ihr entsprechenden Patentanmeldungen umfaßten Vielfalt von Verbindungen dar. Gegenstand der vorliegenden Erfindung sind auch solche neuen Verbindungen per se. Die vorliegende Erfindung betrifft somit auch Verbindungen der Formel Ib per se, 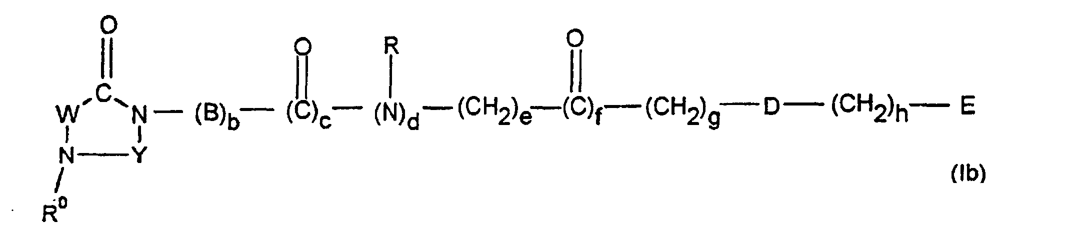 worin
- W: für R¹-A-C(R¹³) oder R¹-A-CH=C steht;
- Y: für eine Carbonyl-, Thiocarbonyl- oder Methylengruppe steht;
- A: einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)- Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
- B: einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
- D: für C(R²)(R³), N(R³) oder CH=C(R³) steht;
- E: Tetrazolyl, (R⁸O)₂P(O), HOS(O)₂, R⁹NHS(O)₂ oder R¹⁰CO bedeutet;
- R: und R⁰ unabhängig voneinander Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeuten, wobei Alkylreste durch Fluor ein- oder mehrfach substituiert sein können;
- R¹: den Rest R²⁸N(R²¹)-C(O)- bedeutet;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)- Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)- Cycloalkyl, (C₂-C₈)-Alkenyl, (C₂-C₈)-Alkinyl, (C₂-C₈)-Alkenylcarbonyl, (C₂-C₈)- Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)- aminocarbonyl, Amino-(C₂-C₁₈)-alkylaminocarbonyl, Amino-(C₁-C₃)-alkyl- phenyl-(C₁-C₃)-alkylaminocarbonyl, (C₁-C₁₈)-Alkylcarbonylamino-(C₁-C₃)- alkylphenyl-(C₁-C₃)-alkylaminocarbonyl, (C₁-C₁₈)-Alkylcarbonylamino-(C₂-C₁₈)- alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)- Alkoxycarbonyl, gegebenenfalls substituiertes (C₃-C₈)-Cycloalkyl, HOS(O)₂-(C₁- C₃)-alkyl, R⁹NHS(O)₂-(C₁-C₃)-alkyl, (R⁸O)₂P(O)-(C₁-C₃)-alkyl, Tetrazolyl-(C₁- C₃)-alkyl, Halogen, Nitro, Trifluormethyl und den Rest R⁵ substituiert sein kann;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, Iminosäure-, gegebenenfalls N- (C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)- alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid- Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)- Cycloalkylaminocarbonyl, gegebenenfalls substituiertes (C₆-C₁₄)- Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: Wasserstoff, (C₁-C₁₈)-Alkyl, R¹²CO, R^{12a}CS, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵ bedeutet;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)- Aryloxy, den Rest R¹⁵, den Rest R¹⁵-O-, Amino, Mono- oder Di-((C₁-C₁₈)-alkyl)- amino, (C₂-C₈)-Alkenyl-amino, (C₂-C₈)-Alkinyl-amino, (C₃-C₁₂)-Cycloalkyl- amino, (C₃-C₁₂)-Cycloalkyl-(C₁-C₈)-alkyl-amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann,gegebenenfalls substituiertes Heteroaryl-NH oder im Heteroarylrest gegebenenfalls substituiertes Heteroaryl- (C₁-C₈)-alkyl-NH bedeutet;
- R^{12a}: Amino, Mono- oder Di-((C₁-C₁₈)-alkyl)-amino, (C₂-C₈)-Alkenyl-amino, (C₂-C₈)- Alkinyl-amino, (C₃-C₁₂)-Cycloalkyl-amino, (C₃-C₁₂)-Cycloalkyl-(C₁-C₈)-alkyl- amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl-(C₁-C₈)-alkyl-NH bedeutet;
- R¹³: Wasserstoff, (C₁-C₆)-Alkyl, im Arylrest gegebenenfalls substituiertes (C₆-C₁₄)- Aryl-(C₁-C₈)-alkyl oder (C₃-C₈)-Cycloalkyl bedeutet;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- R²¹: Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₄)-Aryl, (C₆-C₁₄)-Aryl-(C₁-C₈)-alkyl, den Rest Het- oder Het-(C₁- C₈)-alkyl bedeutet, wobei Alkylreste durch Fluor einfach oder mehrfach substituiert sein können und die Reste R²¹ bei mehrfachem Auftreten gleich oder verschieden sein können;
- R²⁶: die Bedeutungen von R²¹ hat oder HO-((C₁-C₈)-Alkyl) bedeutet, wobei Alkylreste durch Fluor einfach oder mehrfach substituiert sein können;
- R²⁸: für einen der Reste R²¹-, R²¹O-, R²⁶N(R²⁶)-, R²¹C(O)-, R²¹O-C(O)-, ((C₁-C₁₈)- Alkyl-O-C(O)-((C₁-C₆)-alkyl)-O-C(O)-, R²¹N(R²¹)-C(O)-, R²¹N(R²¹)-C(=N(R²¹))- oder R²¹C(O)-N(R²¹)- steht;
- Het: für einen mono- oder polycyclischen, 4- bis 14-gliedrigen, aromatischen oder nicht aromatischen Ring steht, der 1, 2, 3 oder 4 gleiche oder verschiedene Heteroatome aus der Reihe N, O und S als Ringglieder enthält und gegebenenfalls durch einen oder mehrere, gleiche oder verschiedene Substituenten substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

Alle obigen Erläuterungen zur Formel I, zum Beispiel hinsichtlich Alkylresten, Arylresten, usw. gelten für die Verbindungen der Formel Ib entsprechend. Die obigen bevorzugten Bedeutungen gelten hier entsprechend. Besonders bevorzugt stehen in den Verbindungen der Formel Ib zudem unabhängig voneinander b für 1, c für 1, d für 1, f für 0 und g für 0. e und h stehen besonders bevorzugt unabhängig voneinander für 0 oder 1. Besonders bevorzugt ist es auch, wenn Y für eine Carbonylgruppe steht. R¹¹ steht besonders bevorzugt für Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵, wobei R¹² hier Wasserstoff, (C₁-C₁₈)-Alkyl, (C₂-C₃)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino, den Rest R¹⁵ oder den Rest R¹⁵-O- bedeutet.

Die obigen Erläuterungen zur Herstellung der Verbindungen der Formel I und deren Verwendung gelten ebenso auch für die Verbindungen der Formel Ib. Diese Verbindungen sind naturgemäß ebenfalls Inhibitoren der Leukozytenadhäsion und/oder Antagonisten des VLA-4-Rezeptors sind und zur Behandlung und Prophylaxe von Krankheiten geeignet sind, die durch ein unerwünschtes Ausmaß an Leukozytenadhäsion und/oder Leukozytenmigration verursacht werden oder damit verbunden sind oder bei denen Zell-Zell- oder Zell-Matrix-Interaktionen eine Rolle spielen, die auf Wechselwirkungen von VLA-4-Rezeptoren mit ihren Liganden beruhen, beispielsweise von Entzündungsprozessen. Gegenstand der vorliegenden Erfindung sind weiterhin die Verbindungen der Formel Ib zur Verwendung als Arzneimittel sowie pharmazeutische Präparate, die eine oder mehrere Verbindungen der Formel Ib und/oder ihre physiologisch verträglichen Salze neben pharmazeutisch einwandfreien Trägerstoffen und/oder Zusatzstoffen enthalten.

Nicht offenbart im Stand der Technik sind bestimmte Verbindungen der Formel I, in denen die in der Gruppe W enthaltene Gruppe R¹-A keinen Ring enthält, sondern nur acyclische Strukturelemente enthält. Gegenstand der vorliegenden Erfindung sind auch solche neuen Verbindungen per se. Die vorliegende Erfindung betrifft somit Verbindungen der Formel Ic per se, 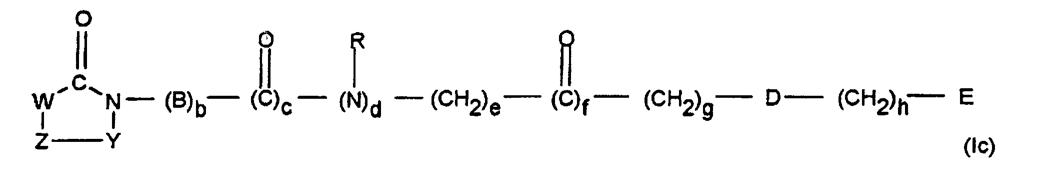 worin
- W: für R¹-A-C(R¹³) oder R¹-A-CH=C steht;
- Y: für eine Carbonyl-, Thiocarbonyl- oder Methylengruppe steht;
- Z: für N(R⁰), Sauerstoff, Schwefel oder eine Methylengruppe steht;
- A: für einen zweiwertigen (C₁-C₆)-Alkylen-Rest steht;
- B: einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
- D: für C(R²)(R³), N(R³) oder CH=C(R³) steht;
- E: Tetrazolyl, (R⁸O)₂P(O), HOS(O)₂, R⁹NHS(O)₂ oder R¹⁰CO bedeutet;
- R: und R⁰ unabhängig voneinander Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeuten, wobei Alkylreste durch Fluor ein- oder mehrfach substituiert sein können;
- R¹: Wasserstoff, (C₁-C₁₀)-Alkyl, das gegebenenfalls durch Fluor einfach oder mehrfach substituiert sein kann, oder einen der Reste R⁴¹O-, R⁴¹O-N(R⁴²)-, R⁴²N(R⁴²)-, HO-((C₁-C₈)-Alkyl)-N(R⁴³)-, HC(O)-NH-, R⁴¹C(O)-N(R⁴²)-, R⁴¹C(O)-, R⁴¹O-C(O)-, R⁴⁴N(R⁴¹)-C(O)-, R⁴¹O-N=, O= und S= bedeutet;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)- Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)- Cycloalkyl, (C₂-C₈)-Alkenyl, (C₂-C₈)-Alkinyl, (C₂-C₈)-Alkenylcarbonyl, (C₂-C₈)- Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)- alkylaminocarbonyl, (C₁-C₁₈)-Alkylcarbonylamino-(C₁-C₃)-alkylphenyl-(C₁-C₃)- alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und den Rest R⁵ substituiert sein kann;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, Iminosäure-, gegebenenfalls N-(C₁- C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)- alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid- Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)- Cycloalkylaminocarbonyl, gegebenenfalls substituiertes (C₆-C₁₄)- Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: Wasserstoff, (C₁-C₁₈)-Alkyl, R¹²CO, R^{12a}CS, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵ bedeutet;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)- Aryloxy, den Rest R¹⁵, den Rest R¹⁵-O-, Amino, Mono- oder Di-((C₁-C₁₈)-alkyl)- amino, (C₂-C₈)-Alkenyl-amino, (C₂-C₈)-Alkinyl-amino, (C₃-C₁₂)-Cycloalkyl- amino, (C₃-C₁₂)-Cycloalkyl-(C₁-C₈)-alkyl-amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl- (C₁-C₈)-alkyl-NH bedeutet;
- R^{12a}: Amino, Mono- oder Di-((C₁-C₁₈)-alkyl)-amino, (C₂-C₈)-Alkenyl-amino, (C₂-C₈)- Alkinyl-amino, (C₃-C₁₂)-Cycloalkyl-amino, (C₃-C₁₂)-Cycloalkyl-(C₁-C₈)-alkyl- amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl-(C₁-C₈)-alkyl-NH bedeutet;
- R¹³: Wasserstoff, (C₁-C₆)-Alkyl, im Arylrest gegebenenfalls substituiertes (C₆-C₁₄)- Aryl-(C₁-C₈)-alkyl oder (C₃-C₈)-Cycloalkyl bedeutet;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- R⁴¹: Wasserstoff oder (C₁-C₈)-Alkyl bedeutet, wobei Alkylreste durch Fluor einfach oder mehrfach substituiert sein können und die Reste R⁴¹ bei mehrfachem Auftreten gleich oder verschieden sein können;
- R⁴²: (C₁-C₈)-Alkyl bedeutet, wobei Alkylreste durch Fluor einfach oder mehrfach substituiert sein können und die Reste R⁴² bei mehrfachem Auftreten gleich oder verschieden sein können;
- R⁴³: die Bedeutungen von R⁴¹ hat oder HO-((C₁-C₈)-Alkyl) bedeutet, wobei Alkylreste durch Fluor einfach oder mehrfach substituiert sein können;
- R⁴⁴: für einen der Reste R⁴¹-, R⁴¹O-, R⁴³N(R⁴³)-, R⁴¹C(O)-, R⁴¹O-C(O)-, ((C₁-C₁₈)- Alkyl-O-C(O)-((C₁-C₆)-alkyl)-O-C(O)-, R⁴¹N(R⁴¹)-C(O)-, R⁴¹N(R⁴¹)-C(=N(R⁴¹))- oder R⁴¹C(O)-N(R⁴¹)- steht;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

Alle obigen Erläuterungen zur Formel I, zum Beispiel hinsichtlich Alkylresten, Arylresten, usw. gelten für die Verbindungen der Formel Ic entsprechend. Die obigen bevorzugten Bedeutungen gelten hier entsprechend. Besonders bevorzugt stehen in den Verbindungen der Formel Ic zudem unabhängig voneinander b für 1, c für 1, d für 1, f für 0 und g für 0. e und h stehen besonders bevorzugt unabhängig voneinander für 0 oder 1. Besonders bevorzugt ist es auch, wenn Y für eine Carbonylgruppe steht. R¹¹ steht bevorzugt für Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵, wobei R¹² hier Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino, den Rest R¹⁵ oder den Rest R¹⁵-O- bedeutet.

Die obigen Erläuterungen zur Herstellung der Verbindungen der Formel I und deren Verwendung gelten ebenso auch für die Verbindungen der Formel Ic. Diese Verbindungen sind naturgemäß ebenfalls Inhibitoren der Leukozytenadhäsion und/oder Antagonisten des VLA-4-Rezeptors sind und zur Behandlung und Prophylaxe von Krankheiten geeignet sind, die durch ein unerwünschtes Ausmaß an Leukozytenadhäsion und/oder Leukozytenmigration verursacht werden oder damit verbunden sind oder bei denen Zell-Zell- oder Zell-Matrix-Interaktionen eine Rolle spielen, die auf Wechselwirkungen von VLA-4-Rezeptoren mit ihren Liganden beruhen, beispielsweise von Entzündungsprozessen. Gegenstand der vorliegenden Erfindung sind weiterhin die Verbindungen der Formel Ic zur Verwendung als Arzneimittel sowie pharmazeutische Präparate, die eine oder mehrere Verbindungen der Formel Ic und/oder ihre physiologisch verträglichen Salze neben pharmazeutisch einwandfreien Trägerstoffen und/oder Zusatzstoffen enthalten.

Weiterhin sind im Stand der Technik, abgesehen von der EP-A-903353, noch keine Verbindungen der Formel I offenbart, in der b für 1 steht und B für einen substituierten Alkylenrest steht. Gegenstand der vorliegenden Erfindung sind somit auch solche Verbindungen der Formel Id per se, 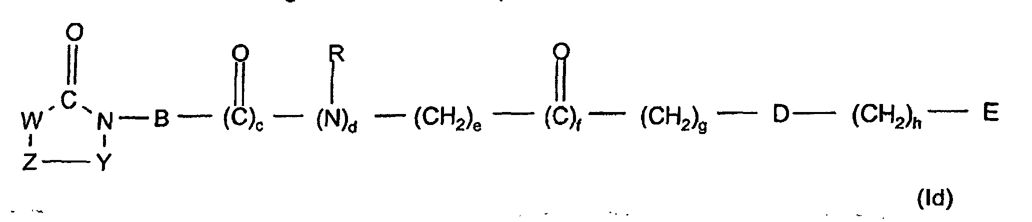 worin
- W: für R¹-A-C(R¹³) oder R¹-A-CH=C steht;
- Y: für eine Carbonyl-, Thiocarbonyl- oder Methylengruppe steht;
- Z: für N(R⁰), Sauerstoff, Schwefel oder eine Methylengruppe steht;
- A: einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)- Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
- B: einen zweiwertigen (C₁-C₆)-Alkylen-Rest bedeutet, der durch einen Rest aus der Reihe (C₁-C₈)-Alkyl, (C₂-C₈)-Alkenyl, (C₂-C₈)-Alkinyl, (C₃-C₁₀)-Cycloalkyl, (C₃- C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert ist;
- D: für C(R²)(R³), N(R³) oder CH=C(R³) steht;
- E: Tetrazolyl, (R⁸O)₂P(O), HOS(O)₂, R⁹NHS(O)₂ oder R¹⁰CO bedeutet;
- R: Wasserstoff, (C₁-C₈)-Alkyl, (C₃-C₁₂)-Cycloalkyl, (C₃-C₁₂)-Cycloalkyl-(C₁-C₈)- alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁- C₈)-alkyl bedeuten, wobei Alkylreste durch Fluor ein- oder mehrfach substituiert sein können;
- R⁰: für Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₂)-Bicycloalkyl, (C₆-C₁₂)-Bicycloalkyl-(C₁-C₈)-alkyl, (C₆-C₁₂)- Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)- alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)- Bicycloalkyl-CO, (C₆-C₁₂)-Bicycloalkyl-(C₁-C₈)-alkyl-CO, (C₆-C₁₂)-Tricycloalkyl- CO, (C₆-C₁₂)-Tricycloalkyl-(C₁-C₈)-alkyl-CO, gegebenenfalls substituiertes (C₆- C₁₄)-Aryl-CO, im Arylrest gegebenenfalls substituiertes (C₆-C₁₄)-Aryl-(C₁-C₈)- alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)- Bicycloalkyl-S(O)ₙ, (C₆-C₁₂)-Bicycloalkyl-(C₁-C₈)-alkyl-S(O)ₙ, (C₆-C₁₂)- Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆- C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl- S(O)ₙ steht, wobei n für 1 oder 2 steht;
- R¹: Wasserstoff, (C₁-C₁₀)-Alkyl, das gegebenenfalls durch Fluor einfach oder mehrfach substituiert sein kann, (C₃-C₁₂)-Cycloalkyl, (C₃-C₁₂)-Cycloalkyl-(C₁- C₈)-alkyl, gegebenenfalls substituiertes R²¹-((C₆-C₁₄)-Aryl), im Arylrest gegebenenfalls substituiertes (R²¹-((C₆-C₁₄)-Aryl))-(C₁-C₈)-alkyl, den Rest Het-, Het-(C₁-C₈)-alkyl oder einen der Reste R²¹O-, R²²O-NH-, R²¹O-N(R²³)-, R²⁴NH-, R²⁵N(R²⁵)-, HO-((C₁-C₈)-Alkyl)-N(R²⁶)-, R²⁷C(O)-NH-, R²¹C(O)- N(R²³)-, R²¹C(O)-, R²¹O-C(O)-, R²⁸N(R²¹)-C(O)-, R²¹O-N=, O= und S= bedeutet;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)- Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)- Cycloalkyl, (C₂-C₈)-Alkenyl, (C₂-C₈)-Alkinyl, (C₂-C₈)-Alkenylcarbonyl, (C₂-C₈)- Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)- alkylaminocarbonyl, (C₁-C₁₈)-Alkylcarbonylamino-(C₁-C₃)-alkylphenyl-(C₁-C₃)- alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und den Rest R⁵ substituiert sein kann;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, Iminosäure-, gegebenenfalls N-(C₁- C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)- alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid- Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)- Cycloalkylaminocarbonyl, gegebenenfalls substituiertes (C₆-C₁₄)- Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: Wasserstoff, (C₁-C₁₈)-Alkyl, R¹²CO, R^{12a}CS, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵ bedeutet;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)- Aryloxy, den Rest R¹⁵, den Rest R¹⁵-O-, Amino, Mono- oder Di-((C₁-C₁₈)-alkyl)- amino, (C₂-C₈)-Alkenyl-amino, (C₂-C₈)-Alkinyl-amino, (C₃-C₁₂)-Cycloalkyl- amino, (C₃-C₁₂)-Cycloalkyl-(C₁-C₈)-alkyl-amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl- (C₁-C₈)-alkyl-NH bedeutet;
- R^{12a}: Amino, Mono- oder Di-((C₁-C₁₈)-alkyl)-amino, (C₂-C₈)-Alkenyl-amino, (C₂-C₈)- Alkinyl-amino, (C₃-C₁₂)-Cycloalkyl-amino, (C₃-C₁₂)-Cycloalkyl-(C₁-C₈)-alkyl- amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl-(C₁-C₈)-alkyl-NH bedeutet;
- R¹³: Wasserstoff, (C₁-C₆)-Alkyl, im Arylrest gegebenenfalls substituiertes (C₆-C₁₄)- Aryl-(C₁-C₈)-alkyl oder (C₃-C₈)-Cycloalkyl bedeutet;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- R²¹: Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₄)-Aryl, (C₆-C₁₄)-Aryl-(C₁-C₈)-alkyl, den Rest Het- oder Het-(C₁- C₈)-alkyl bedeutet, wobei Alkylreste durch Fluor einfach oder mehrfach substituiert sein können und die Reste R²¹ bei mehrfachem Auftreten gleich oder verschieden sein können;
- R²²: (C₃-C₁₂)-Cycloalkyl, (C₃-C₁₂)-Cycloalkyl-(C₁-C₈)-alkyl, (C₆-C₁₄)-Aryl, den Rest Het- oder Het-(C₁-C₈)-alkyl bedeutet, wobei Alkylreste durch Fluor einfach oder mehrfach substituiert sein können;
- R²³: (C₁-C₈)-Alkyl, (C₃-C₁₂)-Cycloalkyl, (C₃-C₁₂)-Cycloalkyl-(C₁-C₈)-alkyl, (C₆-C₁₄)- Aryl, (C₆-C₁₄)-Aryl-(C₁-C₈)-alkyl, den Rest Het- oder Het-(C₁-C₈)-alkyl bedeutet, wobei Alkylreste durch Fluor einfach oder mehrfach substituiert sein können;
- R²⁴: (C₃-C₁₂)-Cycloalkyl, (C₃-C₁₂)-Cycloalkyl-(C₁-C₈)-alkyl, (C₆-C₁₄)-Aryl, (C₆-C₁₄)- Aryl-(C₁-C₈)-alkyl, den Rest Het- oder Het-(C₁-C₈)-alkyl bedeutet, wobei Alkylreste durch Fluor einfach oder mehrfach substituiert sein können;
- R²⁵: die Bedeutungen von R²³ hat, wobei die Reste R²⁵ gleich oder verschieden sein können;
- R²⁶: die Bedeutungen von R²¹ hat oder HO-((C₁-C₈)-Alkyl) bedeutet, wobei Alkylreste durch Fluor einfach oder mehrfach substituiert sein können;
- R²⁷: Wasserstoff, (C₃-C₁₂)-Cycloalkyl, (C₃-C₁₂)Cycloalkyl-(C₁-C₈)-alkyl, (C₆-C₁₄)- Aryl-(C₁-C₈)-alkyl, den Rest Het- oder Het-(C₁-C₈)-alkyl bedeutet, wobei Alkylreste durch Fluor einfach oder mehrfach substituiert sein können;
- R²⁸: für einen der Reste R²¹-, R²¹O-, R²⁶N(R²⁶)-, R²¹C(O)-, R²¹O-C(O)-, ((C₁-C₁₈)- Alkyl-O-C(O)-((C₁-C₆)-alkyl)-O-C(O)-, R²¹N(R²¹)-C(O)-, R²¹N(R²¹)-C(=N(R²¹))- oder R²¹C(O)-N(R²¹)- steht;
- Het: für einen mono- oder polycyclischen, 4- bis 14-gliedrigen, aromatischen oder nicht aromatischen Ring steht, der 1, 2, 3 oder 4 gleiche oder verschiedene Heteroatome aus der Reihe N, O und S als Ringglieder enthält und gegebenenfalls durch einen oder mehrere, gleiche oder verschiedene Substituenten substituiert sein kann;
c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;

in allen ihren stereoisomeren Formen und Mischungen davon in allen Verhältnissen, sowie ihre physiologisch verträglichen Salze;

wobei die Gruppe R¹-A- nicht für Phenyl, Pyrrolyl, Imidazolyl oder Pyridyl stehen kann, wobei diese Reste durch (C₁-C₈)-Alkyl, (C₁-C₈)-Alkoxy, Trifluormethyl, Hydroxy, Hydroxy-(C₁-C₄)-alkyl, Formyl, Hydroxycarbonyl, Aminocarbonyl, (C₁-C₄)-Alkoxycarbonyl, Phenyl, Phenoxy, Benzyloxy, Tetrazolyl, Phenyl-(C₁-C₄)-alkyl und (C₁-C₄)-Alkyl-CO substituiert sein können, und die Gruppe R¹-A- nicht für den Rest R^{X}-(C₁-C₂)-alkyl stehen kann, worin R^{X} für Phenyl, Furyl, Thienyl, Pyrrolyl, Imidazolyl oder Pyridyl steht, wobei diese Reste auch benzanelliert sein können und durch (C₁-C₈)-Alkyl, (C₁-C₈)-Alkoxy, Halogen, Nitro, Amino, Trifluormethyl, Hydroxy, Hydroxy-(C₁-C₄)-alkyl, Methylendioxy, Ethylendioxy, Cyan, Formyl, Hydroxycarbonyl, Aminocarbonyl, (C₁-C₄)-Alkoxycarbonyl, Phenyl, Phenoxy, Benzyloxy, Tetrazolyl, Phenyl-(C₁-C₄)-alkyl und (C₁-C₄)-Alkyl-CO substituiert sein können.

Alle obigen Erläuterungen zur Formel I, zum Beispiel hinsichtlich Alkylresten, Arylresten, usw. gelten auch für die Verbindungen der Formel Id entsprechend. Auch die obigen bevorzugten Bedeutungen gelten hier entsprechend. Besonders bevorzugt stehen in den Verbindungen der Formel Id zudem unabhängig voneinander c für 1, d für 1, f für 0 und g für 0. e und h stehen besonders bevorzugt unabhängig voneinander für 0 oder 1. Besonders bevorzugt ist es auch, wenn Y für eine Carbonylgruppe steht. Hinsichtlich der Gruppe B gilt für die Verbindungen der Formel Id zudem folgendes.

Der für die Gruppe B stehende (C₁-C₆)-Alkylenrest ist in den Verbindungen der Formel Id bevorzugt ein (C₁-C₄)-Alkylenrest, besonders bevorzugt ein Methylenrest oder ein Ethylenrest (= 1,2-Ethylen), ganz besonders bevorzugt ein Methylenrest. Der Substituent an der Gruppe B kann zum einen einen Cyclus enthalten, wenn es sich um einen Substituenten aus der Reihe (C₃-C₁₀)-Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆) handelt, und kann zum anderen acyclisch sein, wenn es sich um einen Substituenten aus der Reihe (C₁-C₈)-Alkyl, (C₂-C₈)-Alkenyl und (C₂-C₈)-Alkinyl handelt. Die acyclischen Substituenten können 2, 3, 4, 5, 6, 7 oder 8 Kohlenstoffatome oder im Falle des gesättigten Alkylrests auch 1 Kohlenstoffatom enthalten. Im Falle der Alkenylreste und Alkinylreste kann sich die Doppelbindung oder Dreifachbindung in einer beliebigen Position befinden und im Falle der Doppelbindung cis-Konfiguration oder trans-Konfiguration aufweisen. Wie oben erläutert, können diese Alkylreste, Alkenylreste und Alkinylreste geradkettig oder verzweigt sein.

Als Beispiele für Substituenten, die der für B stehende (C₁-C₆)-Alkylenrest tragen kann, seien genannt Methyl, Ethyl, n-Propyl, n-Butyl, n-Pentyl, n-Hexyl, n-Heptyl, n-Octyl, Isopropyl, Isobutyl, Isopentyl, Isohexyl, sec-Butyl, tert-Butyl, tert-Pentyl, Neopentyl, Neohexyl, 3-Methylpentyl, 2-Ethylbutyl, Vinyl, Allyl, 1-Propenyl, 2-Butenyl, 3-Butenyl, 3-Methyl-2-butenyl, Ethinyl, 1-Propinyl, 2-Propinyl, 6-Hexinyl, Phenyl, Benzyl, 1-Phenylethyl, 2-Phenylethyl, 3-Phenylpropyl, 4-Biphenylylmethyl, Cyclopropyl, Cyclopropylmethyl, Cyclopentyl, Cyclohexyl, Cyclohexylmethyl, 2-Cyclohexylethyl, 3-Cyclooctylpropyl, 2-Pyridyl, 3-Pyridyl, 4-Pyridyl, 4-Pyridylmethyl, 2-(4-Pyridyl)ethyl, 2-Furylmethyl, 2-Thienylmethyl, 3-Thienylmethyl oder 2-(3-lndolyl)ethyl. Bevorzugt ist der Substituent, den der für B stehenden (C₁-C₆)-Alkylenrest trägt, ein (C₁-C₈)-Alkylrest.

Bevorzugte Verbindungend der Formel Id sind solche, worin gleichzeitig
- W: für R¹-A-C(R¹³) steht;
- Y: für eine Carbonylgruppe steht;
- Z: für N(R⁰) steht;
- A: einen zweiwertigen Rest aus der Reihe (C₃-C₇)-Cycloalkylen, Phenylen, Phenylen-(C₁-C₆)-alkyl, (C₁-C₆)-Alkylen-phenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
- B: einen zweiwertigen Methylenrest oder Ethylenrest bedeutet, der durch einen Rest aus der Reihe (C₁-C₈)-Alkyl, (C₂-C₈)-Alkenyl, (C₂-C₈)-Alkinyl, (C₃-C₁₀)- Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)- alkyl, gegebenenfalls substituiertes Heteroaryl und im Heteroarylrest gegebenenfalls substituiertes Heteroaryl-(C₁-C₆)-alkyl substituiert ist;
- D: für C(R²)(R³) steht;
- E: Tetrazolyl oder R¹⁰CO bedeutet;
- R: Wasserstoff oder (C₁-C₈)-Alkyl bedeutet;
- R⁰: für Wasserstoff, (C₁-C₈)-Alkyl, (C₃-C₁₂)-Cycloalkyl, (C₃-C₁₂)-Cycloalkyl-(C₁- C₈)-alkyl, (C₆-C₁₂)-Bicycloalkyl, (C₆-C₁₂)-Bicycloalkyl-(C₁-C₈)-alkyl, (C₆-C₁₂)- Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)- alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)- Bicycloalkyl-CO, (C₆-C₁₂)-Bicycloalkyl-(C₁-C₈)-alkyl-CO, (C₆-C₁₂)-Tricycloalkyl- CO, (C₆-C₁₂)-Tricycloalkyl-(C₁-C₈)-alkyl-CO, gegebenenfalls substituiertes (C₆- C₁₄)-Aryl-CO, im Arylrest gegebenenfalls substituiertes (C₆-C₁₄)-Aryl-(C₁-C₈)- alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)- Bicycloalkyl-S(O)ₙ, (C₆-C₁₂)-Bicycloalkyl-(C₁-C₈)-alkyl-S(O)ₙ, (C₆-C₁₂)- Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl- S(O)ₙ oder im Heteroarylrest gegebenenfalls substituiertes Heteroaryl-(C₁-C₈)- alkyl-S(O)ₙ steht, wobei n für 1 oder 2 steht;
- R¹: für einen der Reste R²¹O-, R²⁴NH-, R²⁵N(R²⁵)-, HO-((C₁-C₈)-Alkyl)-N(R²⁶)-, R²¹O-C(O)- und R²⁸N(R²¹)-C(O)- steht;
- R²: Wasserstoff oder (C₁-C₈)-Alkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)- Cycloalkyl, (C₂-C₈)-Alkenyl, (C₂-C₈)-Alkinyl, (C₂-C₈)-Alkenylcarbonyl, (C₂-C₈)- Alkinylcarbonyl, Pyridyl, R¹¹NH, CON(CH₃)R⁴, CONHR⁴, CON(CH₃)R¹⁵ oder CONHR¹⁵ bedeutet;
- R⁴: (C₁-C₁₀)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, (C₆-C₁₄)-Aryl- (C₁-C₈)-alkoxycarbonyl, das im Arylrest auch substituiert sein kann, (C₁-C₈)- Alkoxy, (C₁-C₈)-Alkoxycarbonyl, gegebenenfalls substituiertes (C₃-C₈)- Cycloalkyl, Tetrazolyl-(C₁-C₃)-alkyl, Trifluormethyl und R⁵ substituiert sein kann;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, oder einen Rest R⁶CO- bedeutet, wobei der Aryl- und unabhängig davon der Heterocyclus-Rest ein- oder mehrfach durch gleiche oder verschiedene Reste aus der Reihe (C₁-C₈)-Alkyl, (C₁-C₈)-Alkoxy, Halogen, Nitro, Amino oder Trifluormethyl substituiert sein können;
- R⁶: für einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert sein kann, sowie deren Ester und Amide bedeutet, wobei freie funktionelle Gruppen durch in der Peptidchemie übliche Schutzgruppen geschützt sein können;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: für R¹²CO, gegebenenfalls substituiertes (C₆-C₁₄)-Aryl-S(O)₂ oder (C₁-C₁₈)- Alky(-S(O)₂ steht;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)- Aryloxy, den Rest R¹⁵ oder den Rest R¹⁵-O- bedeutet;
- R¹³: Wasserstoff oder (C₁-C₄)-Alkyl bedeutet;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
c und d für 1 stehen und f für 0 steht;
e und h unabhängig voneinander für 0 oder 1 stehen und g für 0 steht;
in allen ihren stereoisomeren Formen und Mischungen davon in allen Verhältnissen, sowie ihre physiologisch verträglichen Salze.

Besonders bevorzugte Verbindungen der Formel Id sind zum einen solche, worin der Rest, durch den die Gruppe B substituiert ist, ein (C₁-C₈)-Alkylrest ist, in allen ihren stereoisomeren Formen und Mischungen davon in allen Verhältnissen, sowie ihre physiologisch verträglichen Salze. Zum anderen sind besonders bevorzugte Verbindungen der Formel Id solche, worin der Rest R¹ für R²⁸N(R²¹)-C(O)- steht, in allen ihren stereoisomeren Formen und Mischungen davon in allen Verhältnissen, sowie ihre physiologisch verträglichen Salze.

Die obigen Erläuterungen zur Herstellung der Verbindungen der Formel I und deren Verwendung gelten ebenso auch für die Verbindungen der Formel Id. Diese Verbindungen sind naturgemäß ebenfalls Inhibitoren der Leukozytenadhäsion und/oder Antagonisten des VLA-4-Rezeptors sind und zur Behandlung und Prophylaxe von Krankheiten geeignet sind, die durch ein unerwünschtes Ausmaß an Leukozytenadhäsion und/oder Leukozytenmigration verursacht werden oder damit verbunden sind oder bei denen Zell-Zell- oder Zell-Matrix-Interaktionen eine Rolle spielen, die auf Wechselwirkungen von VLA-4-Rezeptoren mit ihren Liganden beruhen, beispielsweise von Entzündungsprozessen. Gegenstand der vorliegenden

Erfindung sind weiterhin die Verbindungen der Formel Id zur Verwendung als Arzneimittel sowie pharmazeutische Präparate, die eine oder mehrere Verbindungen der Formel Id und/oder ihre physiologisch verträglichen Salze neben pharmazeutisch einwandfreien Trägerstoffen und/oder Zusatzstoffen enthalten, wobei auch für diese pharmazeutischen Präparate wiederum die obigen Erläuterungen gelten.

### Beispiele

Die Produkte wurden über Massenspektren (MS) und/oder NMR-Spektren identifiziert. Verbindungen, die durch Chromatographie unter Verwendung eines Laufmittels gereinigt wurden, das beispielsweise Essigsäure oder Trifluoressigsäure enthielt, und anschließend gefriergetrocknet wurden, enthielten zum Teil je nach Durchführung der Gefriertrocknung noch die aus dem Laufmittel stammende Säure, sind also teilweise oder vollständig in Form eines Salzes der verwendeten Säure, zum Beispiel in Form des Essigsäuresalzes oder Trifluoressigsäuresalzes, angefallen.

Es bedeuten:
- DMF: N,N-Dimethylformamid
- THF: Tetrahydrofuran
- DCC: N,N'-Dicyclohexylcarbodiimid
- HOBt: 1-Hydroxybenzotriazol

### Beispiel 1

### ((R,S)-4-(4-(Aminocarbonyl)-phenyl)-3-benzyl-4-methyl-2,5-dioxoimidazolidin-1-yl)-acetyl-L-aspartyl-L-phenylglycin

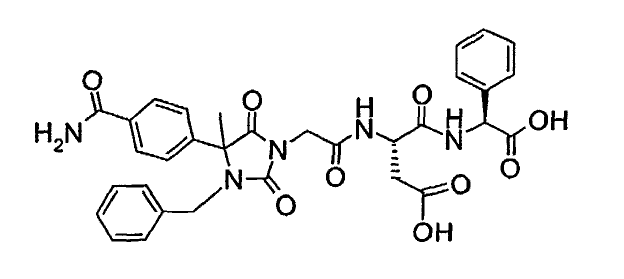

### 1a) (R,S)-4-(4-Cyanophenyl)-4-methyl-2,5-dioxoimidazolidin

20 g (138 mmol) p-Acetylbenzonitril, 115,6 g Ammoniumcarbonat (1,21 mol) und 11,6 g Kaliumcyanid (178 mmol) wurden in 600 ml einer Mischung aus 50 % Ethanol und 50 % Wasser gelöst. Das Gemisch wurde 5 Stunden bei 55 °C gerührt und über Nacht bei Raumtemperatur stehen gelassen. Die Lösung wurde mit 6 N HCI auf pH = 6,3 eingestellt und anschließend zwei Stunden bei Raumtemperatur gerührt. Der Niederschlag wurde abgesaugt, mit Wasser gewaschen und über Phosphorpentoxid im Hochvakuum getrocknet. Ausbeute: 22,33 g (75 %).
FAB-MS: 216,1 (M + H)⁺

### 1b) ((R,S)-4-(4-Cyanophenyl)-4-methyl-2,5-dioxoimidazolidin-1-yl)-essigsäuremethylester

1,068 g Natrium (46,47 mmol) wurden unter Stickstoff in 110 ml abs. Methanol gelöst. Die klare Lösung wurde mit 10 g (R,S)-4-(4-Cyanophenyl)-4-methyl-2,5-dioxoimidazolidin (46,47 mmol) versetzt und das Gemisch wurde 2 h unter Rückfluß gekocht. Man gab 7,75 g (46,68 mmol) Kaliumiodid zu und tropfte innerhalb einer Stunde eine Lösung von 4,53 ml Chloressigsäuremethylester (51,3 mmol) in 5 ml Methanol zu. Es wurde 6 Stunden zum Sieden erhitzt, über Nacht bei Raumtemperatur stehen gelassen und eingeengt. Der ölige Rückstand wurde über Kieselgel mit Methylenchlorid/Essigester (9/1) chromatographiert. Ausbeute: 8,81 g (66 %).
FAB-MS: 288 (M + H)⁺

### 1c) ((R,S)-4-(4-Cyanophenyl)-3-benzyl-4-methyl-2,5-dioxoimidazolidin-1-yl)-essigsäure-methylester

5 g ((R,S)-4-(4-Cyanophenyl)-4-methyl-2,5-dioxoimidazolidin-1-yl)-essigsäuremethylester (17,4 mmol) wurden unter Argon in 20 ml wasserfreiem DMF gelöst. Im Argon-Gegenstrom wurden 920 mg einer Natriumhydrid-Dispersion in Mineralöl (19,14 mmol) zugegeben. Das Reaktionsgemisch wurde 15 Minuten bei Raumtemperatur gerührt. Anschließend setzte man eine Lösung von 3,27 g Benzylbromid (19,14 mmol) in 10 ml wasserfreiem DMF zu. Es wurde 4 Stunden bei Raumtemperatur gerührt und dann über Nacht bei Raumtemperatur stehen gelassen. Die Lösung wurde konzentriert. Zur Reinigung wurde die Substanz über Kieselgel mit Methylenchlorid/Essigester (9,75/0,25) chromatographiert. Die Fraktionen mit der reinen Substanz wurden eingeengt. Ausbeute: 4,28 g (72 %).
FAB-MS: 378 (M + H)⁺

### 1d) ((R,S)-4-(4-Aminocarbonylphenyl)-3-benzyl-4-methyl-2,5-dioxoimidazolidin-1-yl)-essigsäure

2,77 g ((R,S)-4-(4-Cyanophenyl)-3-benzyl-4-methyl-2,5-dioxoimidazolidin-1-yl)-essigsäure-methylester (7,34 mmol) wurden in 50 ml konz. HCl 5 Stunden bei 40 °C gerührt. Das Reaktionsgemisch wurde mit Wasser verdünnt und mit Methylenchlorid extrahiert. Die organische Phase wurde über Natriumsulfat getrocknet und nach Filtration im Vakuum eingeengt. Der Rückstand wurde mit Methylenchlorid, dann Methylenchlorid/Essigester (9,9/0,2), dann mit Methylenchlorid/Essigester (9/1) über Kieselgel chromatographiert. Man erhielt 460 mg eines Gemisches aus ((R,S)-4-(4-Aminocarbonylphenyl)-3-benzyl-4-methyl-2,5-dioxoimidazolidin-1-yl)-essigsäure und ((R, S)-4-(4-Aminocarbonylphenyl)-3-benzyl-4-methyl-2, 5-dioxoimidazolidin-1-yl)-essigsäure-methylester, das mit 120 ml eines Triethylamin/Wasser/Dioxan-Gemisches (1/1/1) versetzt und über Nacht bei Raumtemperatur gerührt wurde. Das Lösungsmittel wurde im Vakuum entfernt und der Rückstand mehrmals mit verdünnter Essigsäure gefriergetrocknet. Ausbeute: 407 mg (16,8 %).
ESI-MS: 382,1 (M + H)⁺

### 1e) ((R,S)-4-(4-Aminocarbonylphenyl)-3-benzyl-4-methyl-2,5-dioxoimidazolidin-1-yl)-acetyl-L-aspartyl-L-phenylglycin-di-tert-butylester

Zu einer Lösung von 200 mg ((R,S)-4-(4-Aminocarbonylphenyl)-3-benzyl-4-methyl-2,5-dioxoimidazolidin-1-yl)-essigsäure (0,52 mmol), 215,8 mg H-Asp(OBu^{t})-Phg-OBu^{t}-Hydrochlorid (0,52 mmol) und 71 mg HOBt (0,52 mmol) in 5 ml DMF gab man bei 0 °C 114 mg DCC (0,52 mmol). Man ließ eine Stunde bei 0 °C und 3 Stunden bei Raumtemperatur rühren. Anschließend ließ man den Ansatz über Nacht bei Raumtemperatur stehen, saugte den Niederschlag ab und engte das Filtrat ein. Zur Reinigung wurde die Substanz über Kieselgel mit Methylenchlorid/Methanol/Eisessig/Wasser (9/1/0,1/0,1) chromatographiert.
Ausbeute: 90 mg (23,3 %).
ESI-MS: 742,4 (M + H)⁺

### 1f) ((R,S)-4-(4-Aminocarbonylphenyl)-3-benzyl-4-methyl-2,5-dioxoimidazolidin-1-yl)-acetyl-L-aspartyl-L-phenylglycin

90 mg ((R,S)-4-(4-Aminocarbonylphenyl)-3-benzyl-4-methyl-2,5-dioxoimidazolidin-1-yl)-acetyl-L-aspartyl-L-phenylglycin-di-tert-butylester (0,12 mmol) wurden in einer Mischung aus 2,25 ml Trifluoressigsäure und 0,25 ml Wasser gelöst. Man ließ eine Stunde bei Raumtemperatur stehen und engte im Wasserstrahlvakuum ein. Zur Reinigung wurde die Substanz über Kieselgel mit Methylenchlorid/Methanol/Eisessig/Wasser (7/3/0,3/0,3) und anschließend mit Methylenchlorid/Methanol/Eisessig/Wasser (8,5/1,5/0,15/0,15) chromatographiert. Der erhaltene Rückstand wurde gefriergetrocknet. Ausbeute: 17 mg eines farblosen Feststoffes (22,5 %).
FAB-MS: 630,2 (M + H)⁺

### Beispiel 2

### ((R,S)-4-(4-(4,5-Dihydro-2-imidazolyl)-phenyl)-3-(2-naphthyl-methyl)-4-methyl-2,5-dioxoimidazolidin-1-yl)-acetyl-L-aspartyl-L-phenylglycin

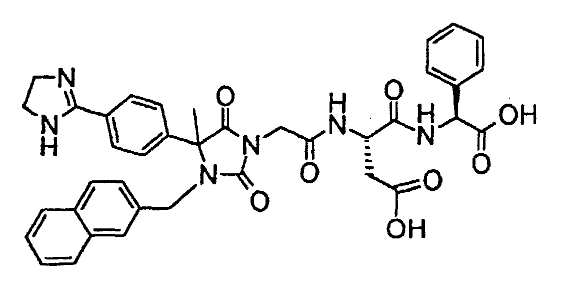

### 2a) ((R,S)-4-(4-(Ethoxy-imino-methyl)-phenyl)-3-(2-naphthyl-methyl)-4-methyl-2,5-dioxoimidazolidin-1-yl)-essigsäure-methylester-hydrochlorid

Eine Suspension von 4 g (9,37 mmol) ((R,S)-4-(4-Cyano-phenyl)-3-(2-naphthylmethyl)-4-methyl-2,5-dioxoimidazolidin-1-yl)-essigsäure-methylester (hergestellt durch Umsetzung von ((R,S)-4-(4-Cyanophenyl)-4-methyl-2,5-dioxoimidazolidin-1-yl)-essigsäure-methylester mit 2-Brommethyl-naphthalin analog Beispiel 1c) in 40 ml absolutem Ethanol wurde auf 0 °C gekühlt. In die Suspension wurde trockenes HCI-Gas eingeleitet, wobei die Temperatur stets unter 10 °C gehalten wurde, bis im IR-Spektrum die Nitrilbande nicht mehr vorlag. Die ethanolische Lösung wurde bis zur Trübung mit Diethylether versetzt und über Nacht bei 4 °C stehen lassen. Der Niederschlag wurde abgesaugt. Man erhielt 4,2 g (88 %) der Titelverbindung als farblosen Feststoff.

### 2b) ((R,S)-4-(4-(4,5-Dihydro-2-imidazolyl)-phenyl)-3-(2-naphthyl-methyl)-4-methyl-2,5-dioxoimidazolidin-1-yl)-essigsäure-hydrochlorid

Man gab zu einer Suspension von 2,2 g (4,3 mmol) an ((R,S)-4-(4-(Ethoxy-iminomethyl)-phenyl)-3-((2-naphthyl)-methyl)-4-methyl-2,5-dioxoimidazolidin-1-yl)-essigsäure-methylester-hydrochlorid in 20 ml Isopropanol 4,75 ml Ethylendiamin und ließ das Gemisch über Nacht bei Raumtemperatur rühren.Das Lösungsmittel wurde im Vakum entfernt und der Rückstand mit Diethylether verrieben. Der Diethylether wurde abdekantiert, der Rückstand im Vakuum getrocknet und anschließend mit 6 N Salzsäure 2 h am Rückfluß erhitzt. Der Niederschlag wurde abgesaugt und mit Wasser gewaschen. Man erhielt 1,5 g (71 %) der Titelverbindung als farblosen Feststoff.

### 2c) ((R,S)-4-(4-(4,5-Dihydro-2-imidazolyl)-phenyl)-3-(2-naphthyl-methyl)-4-methyl 2,5-dioxoimidazolidin-1-yl)-acetyl-L-aspartyl-L-phenylglycin-di-tert-butyl-esterhydrochlorid

Eine Lösung von 493 mg (1 mmol) ((R,S)-4-(4-(4,5-Dihydro-2-imidazolyl)-phenyl)-3-(2-naphthyl-methyl)-4-methyl-2,5-dioxoimidazolidin-1-yl)-essigsäure-hydrochlorid in 10 ml absolutem DMF wurde mit 135 mg (1 mmol) HOBt und anschließend bei 0 °C mit 220 mg (1 mmol) DCC versetzt. Nach 60 minütigem Rühren bei 0 °C und 60 minütigem bei Raumtemperatur gab man 414 mg (1 mmol) H-Asp(O^{t}Bu)-Phg-(O^{t}Bu) x HCl und anschließend 0,1 ml N-Ethylmorpholin zu und ließ das Gemisch über Nacht bei Raumtemperatur stehen. Das Lösungsmittel wurde im Vakuum entfernt und der Rückstand zwischen Essigester und Wasser verteilt. Die Phasen wurden getrennt und die organische Phase nacheinander mit gesättigter NaHCO₃-Lösung, KHSO₄/K₂SO₄-Lösung und Wasser gewaschen und über Natriumsulfat getrocknet. Das Natriumsulfat wurde abfiltriert, das Filtrat im Vakuum eingeengt und der Rückstand mit Dichlormethan/Methanol/Essigsäure/Wasser (9/1/0,1/0,1) über Kieselgel chromatographiert. Nach Einengen der Produktfraktionen erhielt man 430 mg (50 %) der Titelverbindung.

### 2d) ((R, S)-4-(4-(4,5-Dihydro-2-imidazolyl)-phenyl)-3-(2-naphthyl-methyl)-4-methyl-2,5-dioxoimidazolidin-1-yl)-acetyl-L-aspartyl-L-phenylglycin

430 mg (0,5 mmol) an ((R,S)-4-(4-(4,5-Dihydro-2-imidazolyl)-phenyl)-3-(2-naphthylmethyl)-4-methyl-2,5-dioxoimidazolidin-1-yl)-acetyl-L-aspartyl-L-phenylglycin-di-tertbutyl-ester-hydrochlorid wurden mit 5 ml 90 %-iger Trifluoressigsäure versetzt. Nach 1 h bei Raumtemperatur wurde das Reaktionsgemisch eingeengt und der Rückstand mit Wasser/Butanol/Essigsäure (43/4,3/3,5) über Sephadex LH20 chromatographiert. Nach Gefriertrocknen der Produktfraktionen erhielt man 92 mg (26 %) der Titelverbindung.
ES(+)-MS: 705,2 (M+H)⁺

### Beispiel 3

### (S)-3-(((R,S)-4-(4-(4,5-Dihydro-2-imidazolyl)-phenyl)-3-(2-naphthyl-methyl)-4-methyl-2,5-dioxoimidazolidin-1 -yl)-acetylamino)-2-(1-adamantylmethyloxycarbonylamino)-propionsäure

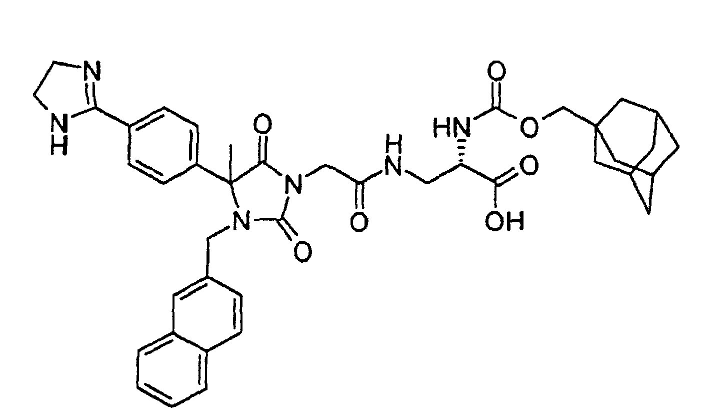

### 3a) (S)-3-Amino-2-benzyloxycarbonylaminopropionsäure-tert-butylester

10 g (42 mmol) (S)-3-Amino-2-benzyloxycarbonylamino-propionsäure wurden in einem Gemisch aus 100 ml Dioxan, 100 ml Isobutylen und 8 ml konz. H₂SO₄ 3 Tage bei 20 atm N₂-Druck im Autoklaven geschüttelt. Überschüssiges Isobutylen wurde abgeblasen und zur verbleibenden Lösung wurden 150 ml Diethylether und 150 ml gesättigte NaHCO₃-Lösung gegeben. Die Phasen wurden getrennt und die wäßrige Phase wurde 2 x mit je 100 ml Diethylether extrahiert. Die vereinigten organischen Phasen wurden mit 2 x 100 ml Wasser gewaschen und über Na₂SO₄ getrocknet. Nach Entfernen des Lösungsmittels im Vakuum erhielt man 9,58 g (78 %) der Titelverbindung als blaßgelbes Öl.

### 3b) (S)-2-Amino-3-tert-butoxycarbonylamino-propionsäure-tert-butylesterhydrochlorid

Man gab zu einer Lösung von 10 g (34 mmol) an (S)-3-Amino-2-benzyloxycarbonylaminopropionsäure-tert-butylester in 600 ml THF/Wasser (2/1) bei 0 °C 8,9 g (40,8 mmol) Di-tert-butyl-dicarbonat und anschließend portionsweise 1 N NaOH, so daß der pH-Wert der Lösung zwischen 9 und 10 lag (Verbrauch an 1 N NaOH: 32 ml). Nach 3 h Rühren bei Raumtemperatur gab man 1 I Wasser zu und extrahierte dreimal mit Diethylether. Nach Trocknen über Natriumsulfat, Filtration und Entfernen des Lösungsmittels im Vakuum wurde der Rückstand mit Dichlormethan/Methanol (20/1) über Kieselgel chromatographiert. Man erhielt 13,19 g (98 %) an (S)-2-Benzyloxycarbonylamino-3-tert-butoxycarbonylaminopropionsäure-tert-butylester. 13,1 g des (S)-2-Benzyloxycarbonylamino-3-tertbutoxycarbonylamino-propionsäure-tert-butylesters wurden in Methanol/HCl über 10 % Pd/C hydriert. Nach 1,5 h wurde filtriert und das Filtrat im Vakuum eingeengt. Man erhielt 9,77 g (99 %) der Titelverbindung als farblosen Feststoff.

### 3c) (S)-2-(1-Adamantylmethyloxycarbonylamino)-3-tert-butoxycarbonylaminopropionsäure-tert-butylester

Eine Lösung von 10,9 g (65,4 mmol) (1-Hydroxymethyl)-adamantan und 10,6 g (65,4 mmol) Carbonyldiimidazol in 60 ml THF wurde 1,5 h bei 50 °C gerührt. Man gab 9,7 g (32,7 mmol) (S)-2-Amino-3-tert-butoxycarbonylamino-propionsäure-tert-butylesterhydrochlorid in 25 ml THF und 5,6 ml (32,7 mmol) Diisopropyl-ethylamin zu, rührte 4 h bei 60 °C und ließ über Nacht bei Raumtemperatur stehen. Das Lösungsmittel wurde im Vakuum entfernt und der Rückstand mit Heptan/Essigester (7/3) über Kieselgel chromatographiert. Man erhielt 8,7 g (59 %) der Titelverbindung als farbloses Öl.

### 3d) (S)-2-(1-Adamantylmethyloxycarbonylamino)-3-amino-propionsäure-tertbutylester

Eine Lösung von 8,7 g (19,22 mmol) (S)-2-(1-Adamantylmethyloxycarbonylamino)-3-tert-butoxycarbonylamino-propionsäure-tert-butylester in 180 ml Trifluoressigsäure/Dichlormethan (1/1) wurde nach 1 min in 1,5 I eiskalte NaHCO₃-Lösung gegeben, das Gemisch dreimal mit Dichlormethan extrahiert und die Dichlormethan-Phase anschließend über Natriumsulfat getrocknet. Nach Filtration und Entfernen des Lösungsmittels im Vakuum erhielt man 6,35 g (94 %) der Titelverbindung als farblosen Feststoff.

### 3e) (S)-3-(((R,S)-4-(4-(4,5-Dihydro-2-imidazolyl)-phenyl)-3-(2-naphthyl-methyl)-4-methyl-2,5-dioxoimidazolidin-1-yl)-acetylamino)-2-(1-adamantylmethyloxycarbonylamino)-propionsäure

Die Synthese erfolgte analog Beispiel 2 unter Verwendung von (S)-2-(1-Adamantylmethyloxycarbonylamino)-3-amino-propionsäure-tert-butylester an Stelle von H-Asp(O^{t}Bu)-Phg-(O^{t}Bu) x HCI. Nach Spaltung des tert-Butylesters mit 90 %iger Trifluoressigsäure wurde das Rohprodukt durch Chromatographie über Kieselgel mit Dichlormethan/Methanol/Essigsäure/Wasser (9/1/0,1/0,1) gereinigt. ES(+)-MS: 735,3 (M+H)⁺

Die Verbindungen der Beispiele 5 bis 17 wurden durch Festphasensynthese nach der in Beispiel 4 angegebenen allgemeinen Vorschrift hergestellt.

### Beispiel 4

### Festphasensynthese (Allgemeine Vorschrift)

### Allgemeines

Die Synthesen am polymeren Träger wurden nach der Synthesesequenz durchgeführt, die im Schema 1 dargestellt ist. Die Reste R⁵⁰ bis R⁵⁵ im Schema 1 haben die Bedeutung der Reste, die sich in der Formel I in der betreffenden Position im Molekül befinden, oder sie können funktionelle Gruppen in geschützter Form oder in Form von Vorstufen enthalten. R⁵⁰ entspricht dem Rest R. R⁵¹ entspricht den Resten R⁴ und R¹⁵, wobei in diesen Resten vorhandene funktionelle Gruppen in geschützter Form oder in Form von Vorstufen vorliegen können (der Rest -NHR⁵¹ kann also beispielsweise für den Rest einer Aminosäure stehen, der formal durch Entfernen eines Wasserstoffatoms von der Aminogruppe erhalten wird). R⁵² entspricht zusammen mit der CH-Gruppe, an die dieser Rest gebunden ist, der Gruppe B (R⁵² entspricht also einem Substituenten an einer für B stehenden Methylengruppe). R⁵³ entspricht R¹³. R⁵⁴ entspricht der Gruppe R¹-A, wobei darin vorhandene funktionelle Gruppen in geschützter Form oder in Form von Vorstufen vorliegen können. R⁵⁵ entspricht der Gruppe R⁰.

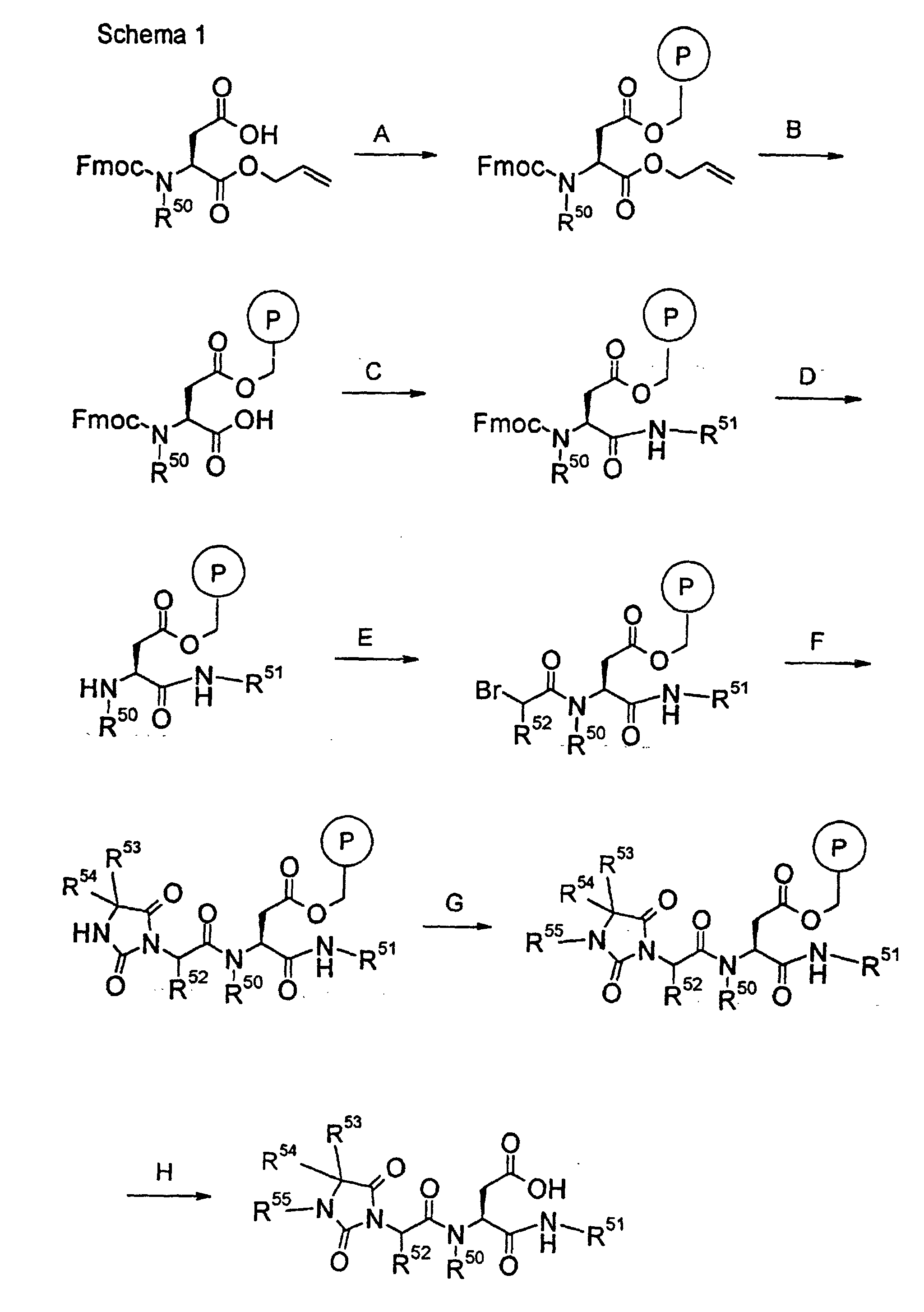

Die Synthese von Zwischenstufen in größerem Maßstab wurde in speziellen Reaktionsgefäßen mit eingelassenen Fritten am Boden des Reaktionsgefäßes durchgeführt, die Synthese der Verbindungen der Formel I wurde in Spritzen oder Reaktionsblöcken durchgeführt (Act 496, MultiSynTech). Die Synthesen am Harz wurden durch on bead-Analytik (FT-IR mit ATR-Einheit und MAS-NMR) und Abspaltung einer analytischen Probe vom Harz (HPLC, MS, NMR) verfolgt.

### Darstellung des Asparaginsäurebausteines FmocAsp(OH)OAllyl

FmocAsp(OtBu)OAllyl (40.g, 88,7 mmol) wurde mit 25 ml Trifluoressigsäure versetzt und 30 min bei Raumtemperatur gerührt. Das Lösungsmittel wurde am Rotationsverdampfer abgezogen. Der Rückstand wurde im Vakuum getrocknet. Man erhielt FmocAsp(OH)OAllyl als gelbes Öl (33,9 g, 97 %). ES(+)-MS: 395,2 (M+H)⁺

### Anknüpfung an den polymeren Träger (Schritt A in Schema 1)

40 g Wang-Polystyrrolharz (1,1 mmol/g; Bachem) wurden 5 min mit 20 ml DMF bei Raumtemperatur vorgequollen. Nach Zugabe einer Lösung von 26,0 g (1,5 Äquivalente) FmocAsp(OH)OAllyl und 34,3 g (1,5 Äquivalente) 1-Benzotriazolyloxytripyrrolidinophosphonium-hexafluorophosphat (PyBOP) und 9,3 ml (1,5 Äquivalente) Diisopropyl-ethylamin in 120 ml DMF wurde das Gemisch 10 h bei 40 °C geschüttelt. Nach beendeter Reaktion wurde die Lösung abgesaugt und das Harz mit DMF gewaschen (5 x 20 ml). Nach Zugabe einer Lösung von Acetanhydrid (10 ml) und Diisopropyl-ethylamin (9,3 ml, 1,5 Äquivalente) in 40 ml DMF wurde das Gemisch erneut für 30 min bei Raumtemperatur geschüttelt. Die Lösung wurde abgesaugt und das Harz nacheinander jeweils dreimal mit 40 ml DMF, Methanol und Dichlormethan gewaschen. Das Harz wurde anschließend im Vakuum getrocknet. Die Bestimmung der Beladung nach der Fmoc-Methode ergab eine Beladung von 0,6 mmol/g.

### Abspaltung der Allylgruppe am polymeren Träger (Schritt B)

Das Harz wurde unter Argon 5 min in DMF bei Raumtemperatur vorgequollen. Nach Zugabe von Tetrakis(triphenylphosphin)palladium und N-Methylpyrrolidin (10 Äquivalente) wurde das Gemisch unter Argon 6 h bei 40 °C geschüttelt. Nach beendeter Reaktion wurde die Lösung abgesaugt und das Harz nacheinander jeweils dreimal mit DMF, Methanol, Toluol und Dichlormethan gewaschen und anschließend getrocknet.

### Kupplung mit Aminoverbindungen am polymeren Träger (Schritt C)

Das beladene Harz mit freier Carboxylfunktion wurde 5 min in DMF bei Raumtemperatur vorgequollen. Nach Zugabe einer Lösung von HOBt (1,2 Äquivalente), TOTU (1,2 Äquivalente) und Diisopropyl-ethylamin (1,2 Äquivalente) in DMF wurde das Gemisch 30 min bei Raumtemperatur geschüttelt. Die Aminoverbindung (1,2 Äquivalente) wurde gelöst in DMF zugegeben. Die Suspension wurde bei Raumtemperatur bis zur vollständigen Umsetzung geschüttelt (HPLC-Kontrolle). Nach beendeter Reaktion wurde die Lösung abgesaugt und das Harz nacheinander jeweils dreimal mit DMF, Methanol, Toluol und Dichlormethan gewaschen und anschließend getrocknet.

### Abspaltung der Fmoc-Schutzgruppe (Schritt D)

Zur Abspaltung der Fmoc-Schutzgruppe wurde das Harz 5 min in DMF bei Raumtemperatur vorgequollen. Nach Zugabe einer Lösung von DMF/Piperidin (1/1) wurde 20 min bei Raumtemperatur geschüttelt. Die Lösung wurde abgesaugt und der Vorgang wiederholt. Die Abspaltung einer analytischen Probe ergab vollständige Umsetzung nach HPLC/MS Untersuchung. Nach vollständiger Umsetzung wurde das Harz dreimal mit Dichlormethan gewaschen und direkt in die Kupplung eingesetzt.

### Kupplung mit α-Halogencarbonsäuren (Schritt E)

Das Harz wurde 5 min mit Dichlormethan bei Raumtemperatur vorgequollen. Die α-Halogencarbonsäurehalogenide (1,5 Äquivalente) wurden gelöst in Dichlormethan zugegeben. Nach Zugabe einer katalytischen Menge 4-Dimethylaminopyridin und von Diisopropyl-ethylamin (1 Äquivalent) wurde das Gemisch für 8 h bei Raumtemperatur geschüttelt. Nach beendeter Reaktion wurde die Lösung abgesaugt und das Harz nacheinander jeweils dreimal mit DMF, Toluol und Dichlormethan gewaschen und anschließend sofort weiter umgesetzt.

Anstatt unter Verwendung der Säurehalogenide kann die Kupplung auch mit den Säuren mit Hilfe von Diisopropylcarbodiimid (DIC) durchgeführt werden. Dazu werden aus α-Halogencarbonsäuren (5 Äquivalente) durch 30-minütige Reaktion mit DIC (2,4 Äquivalente) in Dichlormethan die symmetrischen Anhydride gebildet. Nach dieser Zeit werden 2 Äquivalente Diisopropyl-ethylamin zugegeben. Das Gemisch wird zu dem Harz gegeben und 12 h bei Raumtemperatur geschüttelt. Nach beendeter Reaktion wird die Lösung abgesaugt und das Harz nacheinander jeweils dreimal mit DMF, Toluol und Dichlormethan gewaschen und anschließend sofort weiter umgesetzt.

### Kupplung der α-Halogenacylverbindungen mit Hydantoinen (Schritt F)

Die 4,4-disubstituierten Hydantoine (2 Äquivalente) wurden in DMF mit Diazabicycloundecen (DBU) (2 Äquivalente) bei Raumtemperatur aktiviert. Die aktivierte Lösung wurde nach 15 min zu dem in DMF für 5 min vorgequollenen Harz gegeben. Das Gemisch wurde 8 h bei Raumtemperatur geschüttelt. Nach beendeter Reaktion wurde die Lösung abgesaugt und das Harz nacheinander jeweils dreimal mit DMF, Methanol, Toluol und Dichlormethan gewaschen und anschließend getrocknet.

### N-Alkylierung des Hydantoins am polymeren Träger (Schritt G)

Das Harz wurde 5 min in DMF bei Raumtemperatur vorgequollen. Nach Zugabe von N'''-tert-Butyl-N,N,N',N',N",N"-hexamethylphosphorimidsäuretriamid (Phosphazen-Base P1-t-Bu) (3 Äquivalente) wurde 30 min bei Raumtemperatur geschüttelt. Nach Zugabe des Alkylierungsmittels (Bromid oder lodid) wurde 4 h bei Raumtemperatur geschüttelt. Nach beendeter Reaktion wurde die Lösung abgesaugt und das Harz nacheinander jeweils dreimal mit DMF, Toluol und Dichlormethan gewaschen und anschließend getrocknet.

Anstatt unter Verwendung von Phospazenen kann die Alkylierung auch mit Caesiumcarbonat durchgeführt werden. Dazu wird das Harz 5 min in DMF bei Raumtemperatur vorgequollen. Nach Zugabe von Caesiumcarbonat (3 Äquivalente) wird 30 min bei Raumtemperatur geschüttelt. Nach Zugabe des Alkylierungsmittels (Bromid oder lodid) wird 6 h bei 50 °C geschüttelt. Nach beendeter Reaktion wird die Lösung abgesaugt und das Harz nacheinander jeweils dreimal mit DMF, Methanol/Wasser/DMF(1,5/1,5/7), DMF, Toluol und Dichlormethan gewaschen und anschließend getrocknet.

### Abspaltung vom Harz (Schritt H)

Zur Abspaltung der Verbindung vom Harz wurde ein Gemisch von Trifluoressigsäure/Dichlormethan (1/1) zum Harz zugegeben. Die Suspension wurde 1 h geschüttelt. Das Harz wurde abfiltriert. Die verbleibende Lösung wurde im Vakuum eingeengt. Der Rückstand wurde durch Kieselgelchromatographie gereinigt (Dichlormethan und Essigester).

Nach der im Beispiel 4 beschriebenen allgemeinen Methode wurden die Verbindungen der Beispiele 5 bis 17 hergestellt, die die in der Formel If angegebene Struktur aufweisen. Die Bedeutungen der Reste in den einzelnen Verbindungen sind in der Tabelle 1 angegeben.

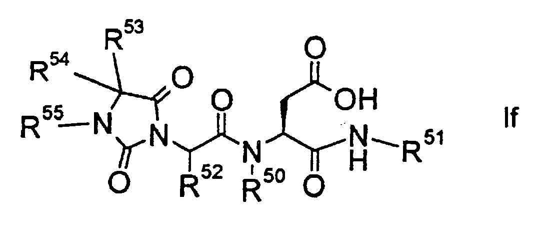

In der Tabelle 1 bedeuten:

| | | | |
|---|---|---|---|
| Bn | = Benzyl | 2-Py | = 2-Pyridylmethyl |
| 3-Py | = 3-Pyridylmethyl | 4-Py | = 4-Pyridylmethyl |
| H | = Wasserstoff | Me | = Methyl |
| Et | = Ethyl | nBu | = n-Butyl |
| iBu | = Isobutyl | | |

Phg steht für den L-Phenylglycyl-Rest, also den Rest der Aminosäure L-Phenylglycin, der für den Rest -NH-R⁵¹ in der Formel If steht und der formal durch Abstraktion eines Wasserstoffatoms von der Aminogruppe der Phenylglycins erhalten wird.

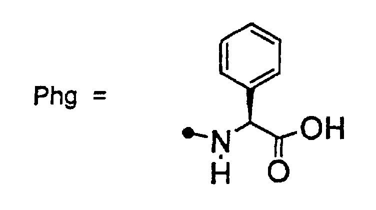

**Tabelle 1**

| Beispiel | R⁵⁰ | -NH-R⁵¹ | R⁵² | R⁵³ | R⁵⁴ | R⁵⁵ | ES-(+)-MS |
|---|---|---|---|---|---|---|---|
| 5 | H | Phg | H | Me | Me | Bn | 525,5 |
| 6 | H | Phg | nBu | Me | Me | Bn | 581,6 |
| 7 | H | Phg | nBu | Me | Me | 2-Py | 582,6 |
| 8 | H | Phg | nBu | Me | Me | 3-Py | 582,6 |
| 9 | H | Phg | nBu | Me | Me | 4-Py | 582,6 |
| 10 | H | Phg | iBu | Me | Me | Bn | 581,6 |
| 11 | H | Phg | iBu | Me | Me | 2-Py | 582,6 |
| 12 | H | Phg | iBu | Me | Me | 3-Py | 582,6 |
| 13 | H | Phg | iBu | Me | Me | 4-Py | 582,6 |
| 14 | H | Phg | H | Me | Me | 2-Py | 526,5 |
| 15 | H | Phg | H | Me | Me | 3-Py | 526,5 |
| 16 | H | Phg | H | Me | Me | 4-Py | 526,5 |
| 17 | H | Phg | Et | Me | Me | Bn | 553,6 |

### Untersuchung der biologischen Aktivität

Als Testmethode für die Wirksamkeit der Verbindungen der Formel I auf die Interaktion zwischen VCAM-1 und VLA-4 wird ein Assay verwendet, der für diese Interaktion spezifisch ist. Die zellulären Bindungspartner, d. h. die VLA-4-Integrine, werden in ihrer natürlichen Form als Oberflächenmoleküle auf humanen U937-Zellen (ATCC CRL 1593), die zur Gruppe der Leukozyten gehören, angeboten. Als spezifische Bindungspartner werden gentechnisch hergestellte rekombinante lösliche Fusionsproteine, bestehend aus der extrazytoplasmatischen Domäne von humanen VCAM-1 und der konstanten Region eines humanen Immunglobulins der Subklasse IgG1, verwendet.

### Testmethode

### Assay zur Messung der Adhäsion von U937-Zellen (ATCC CRL 1593) an hVCAM-1(1-3)-IgG

1. Herstellung von humanem VCAM-1(1-3)-IgG und humanem CD4-lgG
   Eingesetzt wurde ein genetisches Konstrukt zur Expression der extrazellulären Domäne des humanen VCAM-1, verbunden mit der genetischen Sequenz der schweren Kette des humanen Immunglobulins IgG1 (Hinge, CH2 und CH3 Regionen), von Dr. Brian Seed, Massachusetts General Hospital, Boston, USA. Das lösliche Fusionsprotein hVCAM-1(1-3)-IgG enthielt die drei aminoterminalen extrazellulären Immunglobulin-ähnlichen Domänen des humanen VCAM-1 (Damle und Aruffo, Proc. Natl. Acad. Sci. USA 1991, 88, 6403). CD4-IgG (Zettlmeissl et al., DNA and Cell Biology 1990, 9, 347) diente als Fusionsprotein für negative Kontrollen. Die rekombinanten Proteine wurden als lösliche Proteine nach DEAE/Dextran-vermittelter DNA-Transfektion in COS-Zellen (ATCC CRL1651) gemäß Standardprozeduren exprimiert (Ausubel et al., Current protocols in molecular biology, John Wiley & Sons, Inc., 1994).
2. Assay zur Messung der Adhäsion von U937-Zellen an hVCAM-1 (1-3)-IgG
   2.1 96 well-Mikrotitertestplatten (Nunc Maxisorb) wurden mit 100 µl/well einer Ziege-anti-human-lgG-Antikörperlösung (10 µg/ml in 50 mM Tris, pH 9,5) 1 Stunde bei Raumtemperatur inkubiert. Nach Entfernen der Antikörperlösung wurde einmal mit PBS gewaschen.
   2.2 150 µl/well eines Blockierungspuffers (1% BSA in PBS) wurde 0,5 Stunden bei Raumtemperatur auf den Platten inkubiert. Nach Entfernen des Blockierungspuffers wurde einmal mit PBS gewaschen.
   2.3 100 µl pro well eines Zellkulturüberstandes von transfektierten COS-Zellen wurde für 1,5 Stunden bei Raumtemperatur auf den Platten inkubiert. Die COS-Zellen waren mit einem Plasmid transfiziert, welches für die drei N-terminalen Immunglobulin-ähnlichen Domänen des VCAM-1, gekoppelt an den Fc-Teil von humanem IgG₁ (hVCAM-1(1-3)-IgG), codiert. Der Gehalt an hVCAM-1(1-3)-IgG betrug ca. 0,5 - 1 µg/ml. Nach Entfernen des Kulturüberstandes wurde einmal mit PBS gewaschen.
   2.4 Die Platten wurden mit 100 µl/well Fc-Rezeptor-Blockpuffer (1 mg/ml γ-Globulin, 100 mM NaCl, 100 µM MgCl₂, 100 µM MnCl₂, 100 µM CaCl₂, 1 mg/ml BSA in 50 mM HEPES, pH 7,5) für 20 Minuten bei Raumtemperatur inkubiert. Nach Entfernen des Fc-Rezeptor-Blockpuffers wurde einmal mit PBS gewaschen.
   2.5 20 µl Bindungspuffer (100 mM NaCI, 100 µM MgCl₂, 100 µM MnCl₂, 100 µM CaCl₂, 1 mg/ml BSA in 50 mM HEPES, pH 7,5) wurden vorgelegt, die zu testenden Substanzen in 10 µl Bindungspuffer zugegeben und für 20 Minuten inkubiert. Als Kontrollen dienten Antikörper gegen VCAM-1 (BBT, Nr. BBA6) und gegen VLA-4 (Immunotech, Nr. 0764).
   2.6 U937-Zellen wurden 20 Minuten in Fc-Rezeptor-Blockpuffer inkubiert und anschließend in einer Konzentration von 1 x 10⁶/ml und in einer Menge von 100 µl pro well zupipettiert (Endvolumen 125µl/well).
   2.7 Die Platten wurden in einem 45°-Winkel in Stoppuffer (100 mM NaCl, 100 µM MgCl₂, 100 µM MnCl₂, 100 µM CaCl₂ in 25 mM Tris, pH 7,5) langsam eingetaucht und ausgeschlagen. Der Vorgang wurde wiederholt.
   2.8 Anschließend wurden 50 µl/well einer Färbelösung (16,7 µg/ml Hoechst Farbstoff 33258, 4 % Formaldehyd, 0,5 % Triton-X-100 in PBS) 15 Minuten auf den Platten inkubiert.
   2.9 Die Platten wurden ausgeschlagen, in einem 45°-Winkel in Stoppuffer (100 mM NaCl, 100 µM MgCl₂, 100 µM MnCl₂, 100 µM CaCl₂ in 25 mM Tris, pH 7,5) langsam eingetaucht. Der Vorgang wurde wiederholt. Anschließend wurde mit der Flüssigkeit in einem Cytofluorimeter (Millipore) gemessen (Sensitivität: 5, Filter: Anregungswellenlänge: 360 nm, Emissionswellenlänge: 460 nm).

Die Intensität des von den angefärbten U937-Zellen emittierten Lichts ist ein Maß für die Zahl der an der Platte verbliebenen, an das hVCAM-1(1-3)-IgG adhärierten U937-Zellen und somit ein Maß für die Fähigkeit der zugesetzten Testsubstanz, diese Adhäsion zu hemmen. Aus der Hemmung der Adhäsion bei verschiedenen Konzentrationen der Testsubstanz wurde die Konzentration IC₅₀ berechnet, die zu einer Hemmung der Adhäsion um 50 % führt.

Es wurden die folgenden Testergebnisse erhalten:

| Beispiel | U937/VCAM-1 Zelladhäsionstest IC₅₀ (µM) |
|---|---|
| 1 | 7,5 |
| 2 | 14,5 |
| 3 | 40 |

## Patentansprüche

1. Verwendung von Verbindungen der Formel I, 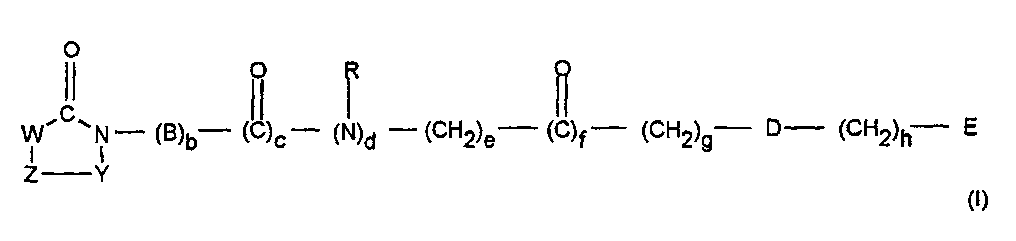 worin
W für R¹-A-C(R¹³) oder R¹-A-CH=C steht;
Y für eine Carbonyl-, Thiocarbonyl- oder Methylengruppe steht;
Z für N(R⁰), Sauerstoff, Schwefel oder eine Methylengruppe steht;
A einen zweiwertigen Rest aus der Reihe (C₁-C₆)-Alkylen, (C₃-C₁₂)- Cycloalkylen, (C₁-C₆)-Alkylen-(C₃-C₁₂)-cycloalkyl, Phenylen, Phenylen-(C₁- C₆)-alkyl, (C₁-C₆)-Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6- gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
B einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet, wobei der zweiwertige (C₁-C₆)-Alkylen-Rest unsubstituiert oder durch einen Rest aus der Reihe (C₁-C₈)-Alkyl, (C₂-C₈)-Alkenyl, (C₂-C₈)-Alkinyl, (C₃-C₁₀)-Cycloalkyl, (C₃- C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert sein kann;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R Wasserstoff, (C₁-C₈)-Alkyl, (C₃-C₁₂)-Cycloalkyl, (C₃-C₁₂)-Cycloalkyl-(C₁-C₈)- alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl- (C₁-C₈)-alkyl bedeuten, wobei Alkylreste durch Fluor ein- oder mehrfach substituiert sein können;
R⁰ für Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₂)-Bicycloalkyl, (C₆-C₁₂)-Bicycloalkyl-(C₁-C₈)-alkyl, (C₆-C₁₂)- Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)- alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)- Bicycloalkyl-CO, (C₆-C₁₂)-Bicycloalkyl-(C₁-C₈)-alkyl-CO, (C₆-C₁₂)-Tricycloalkyl- CO, (C₆-C₁₂)-Tricycloalkyl-(C₁-C₈)-alkyl-CO, gegebenenfalls substituiertes (C₆- C₁₄)-Aryl-CO, im Arylrest gegebenenfalls substituiertes (C₆-C₁₄)-Aryl-(C₁-C₈)- alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)- Bicycloalkyl-S(O)ₙ, (C₆-C₁₂)-Bicycloalkyl-(C₁-C₈)-alkyl-S(O)ₙ, (C₆-C₁₂)- Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆- C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl- S(O)ₙ steht, wobei n für 1 oder 2 steht;
R¹ Wasserstoff, (C₁-C₁₀)-Alkyl, das gegebenenfalls durch Fluor einfach oder mehrfach substituiert sein kann, (C₃-C₁₂)-Cycloalkyl, (C₃-C₁₂)-Cycloalkyl-(C₁- C₈)-alkyl, gegebenenfalls substituiertes R²¹-((C₆-C₁₄)-Aryl), im Arylrest gegebenenfalls substituiertes (R²¹-((C₆-C₁₄)-Aryl))-(C₁-C₈)-alkyl, den Rest Het-, Het-(C₁-C₈)-alkyl oder einen der Reste R²¹O-, R²²O-NH-, R²¹O-N(R²³)-, R²⁴NH-, R²⁵N(R²⁵)-, HO-((C₁-C₈)-Alkyl)-N(R²⁶)-, R²⁷C(O)-NH-, R²¹C(O)- N(R²³)-, R²¹C(O)-, R²¹O-C(O)-, R²⁸N(R²¹)-C(O)-, R²¹O-N=, O= und S= bedeutet;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)- Cycloalkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)- Cycloalkyl, (C₂-C₈)-Alkenyl, (C₂-C₈)-Alkinyl, (C₂-C₈)-Alkenylcarbonyl, (C₂-C₈)- Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)- alkylaminocarbonyl, (C₁-C₁₈)-Alkylcarbonylamino-(C₁-C₃)-alkylphenyl-(C₁-C₃)- alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und den Rest R⁵ substituiert sein kann;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N-(C₁- C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)- alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid- Rest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)- Cycloalkylaminocarbonyl, gegebenenfalls substituiertes (C₆-C₁₄)- Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ Wasserstoff, (C₁-C₁₈)-Alkyl, R¹²CO, R^{12a}CS, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵ bedeutet;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)- Aryloxy, den Rest R¹⁵, den Rest R¹⁵-O-, Amino, Mono- oder Di-((C₁-C₁₈)-alkyl)- amino, (C₂-C₈)-Alkenyl-amino, (C₂-C₈)-Alkinyl-amino, (C₃-C₁₂)-Cycloalkyl- amino, (C₃-C₁₂)-Cycloalkyl-(C₁-C₈)-alkyl-amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl- (C₁-C₈)-alkyl-NH bedeutet;
R^{12a} Amino, Mono- oder Di-((C₁-C₁₈)-alkyl)-amino, (C₂-C₈)-Alkenyl-amino, (C₂-C₈)- Alkinyl-amino, (C₃-C₁₂)-Cycloalkyl-amino, (C₃-C₁₂)-Cycloalkyl-(C₁-C₈)-alkyl- amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl-(C₁-C₈)-alkyl-NH bedeutet;
R¹³ Wasserstoff, (C₁-C₆)-Alkyl, im Arylrest gegebenenfalls substituiertes (C₆-C₁₄)- Aryl-(C₁-C₈)-alkyl oder (C₃-C₈)-Cycloalkyl bedeutet;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
R²¹ Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₄)-Aryl, (C₆-C₁₄)-Aryl-(C₁-C₈)-alkyl, den Rest Het- oder Het-(C₁- C₈)-alkyl bedeutet, wobei Alkylreste durch Fluor einfach oder mehrfach substituiert sein können und die Reste R²¹ bei mehrfachem Auftreten gleich oder verschieden sein können;
R²² (C₃-C₁₂)-Cycloalkyl, (C₃-C₁₂)-Cycloalkyl-(C₁-C₈)-alkyl, (C₆-C₁₄)-Aryl, den Rest Het- oder Het-(C₁-C₈)-alkyl bedeutet, wobei Alkylreste durch Fluor einfach oder mehrfach substituiert sein können;
R²³ (C₁-C₈)-Alkyl, (C₃-C₁₂)-Cycloalkyl, (C₃-C₁₂)-Cycloalkyl-(C₁-C₈)-alkyl, (C₆-C₁₄)- Aryl, (C₆-C₁₄)-Aryl-(C₁-C₈)-alkyl, den Rest Het- oder Het-(C₁-C₈)-alkyl bedeutet, wobei Alkylreste durch Fluor einfach oder mehrfach substituiert sein können;
R²⁴ (C₃-C₁₂)-Cycloalkyl, (C₃-C₁₂)-Cycloalkyl-(C₁-C₈)-alkyl, (C₆-C₁₄)-Aryl, (C₆-C₁₄)- Aryl-(C₁-C₈)-alkyl, den Rest Het- oder Het-(C₁-C₈)-alkyl bedeutet, wobei Alkylreste durch Fluor einfach oder mehrfach substituiert sein können;
R²⁵ die Bedeutungen von R²³ hat, wobei die Reste R²⁵ gleich oder verschieden sein können;
R²⁶ die Bedeutungen von R²¹ hat oder HO-((C₁-C₈)-Alkyl) bedeutet, wobei Alkylreste durch Fluor einfach oder mehrfach substituiert sein können;
R²⁷ Wasserstoff, (C₃-C₁₂)-Cycloalkyl, (C₃-C₁₂)Cycloalkyl-(C₁-C₈)-alkyl, (C₆-C₁₄)- Aryl-(C₁-C₈)-alkyl, den Rest Het- oder Het-(C₁-C₈)-alkyl bedeutet, wobei Alkylreste durch Fluor einfach oder mehrfach substituiert sein können;
R²⁸ für einen der Reste R²¹-, R²¹O-, R²⁶N(R²⁶)-, R²¹C(O)-, R²¹O-C(O)-, ((C₁-C₁₈)- Alkyl-O-C(O)-((C₁-C₆)-alkyl)-O-C(O)-, R²¹N(R²¹)-C(O)-, R²¹N(R²¹)-C(=N(R²¹))- oder R²¹C(O)-N(R²¹)- steht;
Het für einen mono- oder polycyclischen, 4- bis 14-gliedrigen, aromatischen oder nicht aromatischen Ring steht, der 1, 2, 3 oder 4 gleiche oder verschiedene Heteroatome aus der Reihe N, O und S als Ringglieder enthält und gegebenenfalls durch einen oder mehrere, gleiche oder verschiedene Substituenten substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen sowie ihrer physiologisch verträglichen Salze zur Herstellung von Arzneimitteln zur Hemmung der Adhäsion und/oder Migration von Leukozyten oder zur Hemmung des VLA-4-Rezeptors.

2. Verwendung von Verbindungen der Formel I gemäß Anspruch 1 in allen ihren stereoisomeren Formen und Mischungen davon in allen Verhältnissen sowie von ihren physiologisch verträglichen Salzen, wobei in der Formel I
W für R¹-A-C(R¹³) oder R¹-A-CH=C steht;
Y für eine Carbonyl-, Thiocarbonyl- oder Methylengruppe steht;
Z für N(R⁰), Sauerstoff, Schwefel oder eine Methylengruppe steht;
A einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)- Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
B einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R und R⁰ unabhängig voneinander Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeuten, wobei Alkylreste durch Fluor ein- oder mehrfach substituiert sein können;
R¹ Wasserstoff, (C₁-C₁₀)-Alkyl, das gegebenenfalls durch Fluor einfach oder mehrfach substituiert sein kann, (C₃-C₁₂)-Cycloalkyl, (C₃-C₁₂)-Cycloalkyl-(C₁- C₈)-alkyl, gegebenenfalls substituiertes R²¹-((C₆-C₁₄)-Aryl), im Arylrest gegebenenfalls substituiertes (R²¹-((C₆-C₁₄)-Aryl))-(C₁-C₈)-alkyl, den Rest Het-, Het-(C₁-C₈)-alkyl oder einen der Reste R²¹O-, R²²O-NH-, R²¹O-N(R²³)-, R²⁴NH-, R²⁵N(R²⁵)-, HO-((C₁-C₈)-Alkyl)-N(R²⁶)-, R²⁷C(O)-NH-, R²¹C(O)- N(R²³)-, R²¹C(O)-, R²¹O-C(O)-, R²⁸N(R²¹)-C(O)-, R²¹O-N=, O= und S= bedeutet;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)- Cycloalkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)- Cycloalkyl, (C₂-C₈)-Alkenyl, (C₂-C₈)-Alkinyl, (C₂-C₈)-Alkenylcarbonyl, (C₂-C₈)- Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)- aminocarbonyl, Amino-(C₂-C₁₈)-alkylaminocarbonyl, Amino-(C₁-C₃)-alkyl- phenyl-(C₁-C₃)-alkylaminocarbonyl, (C₁-C₁₈)-Alkylcarbonylamino-(C₁-C₃)- alkylphenyl-(C₁-C₃)-alkylaminocarbonyl, (C₁-C₁₈)-Alkylcarbonylamino-(C₂-C₁₈)- alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)- Alkoxycarbonyl, gegebenenfalls substituiertes (C₃-C₈)-Cycloalkyl, HOS(O)₂-(C₁- C₃)-alkyl, R⁹NHS(O)₂-(C₁-C₃)-alkyl, (R⁸O)₂P(O)-(C₁-C₃)-alkyl, Tetrazolyl-(C₁- C₃)-alkyl, Halogen, Nitro, Trifluormethyl und den Rest R⁵ substituiert sein kann;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N- (C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)- alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid- Rest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)- Cycloalkylaminocarbonyl, gegebenenfalls substituiertes (C₆-C₁₄)- Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ Wasserstoff, (C₁-C₁₈)-Alkyl, R¹²CO, gegebenenfalls substituiertes (C₆-C₁₄)- Aryl-S(O)₂, (C₁-C₁₈)-Alkyl-S(O)₂, im Arylrest gegebenenfalls substituiertes (C₆- C₁₄)-Aryl-(C₁-C₈)-alkyl, R⁹NHS(O)₂ oder den Rest R¹⁵ bedeutet;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)- Aryloxy, Amino oder Mono- oder Di-((C₁-C₁₈)-alkyl)-amino, den Rest R¹⁵ oder den Rest R¹⁵-O- bedeutet;
R¹³ Wasserstoff, (C₁-C₆)-Alkyl, im Arylrest gegebenenfalls substituiertes (C₆-C₁₄)- Aryl-(C₁-C₈)-alkyl oder (C₃-C₈)-Cycloalkyl bedeutet;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
R²¹ Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₄)-Aryl, (C₆-C₁₄)-Aryl-(C₁-C₈)-alkyl, den Rest Het- oder Het-(C₁- C₈)-alkyl bedeutet, wobei Alkylreste durch Fluor einfach oder mehrfach substituiert sein können und die Reste R²¹ bei mehrfachem Auftreten gleich oder verschieden sein können;
R²² (C₃-C₁₂)-Cycloalkyl, (C₃-C₁₂)-Cycloalkyl-(C₁-C₈)-alkyl, (C₆-C₁₄)-Aryl, den Rest Het- oder Het-(C₁-C₈)-alkyl bedeutet, wobei Alkylreste durch Fluor einfach oder mehrfach substituiert sein können;
R²³ (C₁-C₈)-Alkyl, (C₃-C₁₂)-Cycloalkyl, (C₃-C₁₂)-Cycloalkyl-(C₁-C₈)-alkyl, (C₆-C₁₄)- Aryl, (C₆-C₁₄)-Aryl-(C₁-C₈)-alkyl, den Rest Het- oder Het-(C₁-C₈)-alkyl bedeutet, wobei Alkylreste durch Fluor einfach oder mehrfach substituiert sein können;
R²⁴ (C₃-C₁₂)-Cycloalkyl, (C₃-C₁₂)-Cycloalkyl-(C₁-C₈)-alkyl, (C₆-C₁₄)-Aryl, (C₆-C₁₄)- Aryl-(C₁-C₈)-alkyl, den Rest Het- oder Het-(C₁-C₈)-alkyl bedeutet, wobei Alkylreste durch Fluor einfach oder mehrfach substituiert sein können;
R²⁵ die Bedeutungen von R²³ hat, wobei die Reste R²⁵ gleich oder verschieden sein können;
R²⁶ die Bedeutungen von R²¹ hat oder HO-((C₁-C₈)-Alkyl) bedeutet, wobei Alkylreste durch Fluor einfach oder mehrfach substituiert sein können;
R²⁷ Wasserstoff, (C₃-C₁₂)-Cycloalkyl, (C₃-C₁₂)Cycloalkyl-(C₁-C₈)-alkyl, (C₆-C₁₄)- Aryl-(C₁-C₈)-alkyl, den Rest Het- oder Het-(C₁-C₈)-alkyl bedeutet, wobei Alkylreste durch Fluor einfach oder mehrfach substituiert sein können;
R²⁸ für einen der Reste R²¹-, R²¹O-, R²⁶N(R²⁶)-, R²¹C(O)-, R²¹O-C(O)-, ((C₁-C₁₈)- Alkyl-O-C(O)-((C₁-C₆)-alkyl)-O-C(O)-, R²¹N(R²¹)-C(O)-, R²¹N(R²¹)-C(=N(R²¹))- oder R²¹C(O)-N(R²¹)- steht;
Het für einen mono- oder polycyclischen, 4- bis 14-gliedrigen, aromatischen oder nicht aromatischen Ring steht, der 1, 2, 3 oder 4 gleiche oder verschiedene Heteroatome aus der Reihe N, O und S als Ringglieder enthält und gegebenenfalls durch einen oder mehrere, gleiche oder verschiedene Substituenten substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen.

3. Verwendung von Verbindungen der Formel I gemäß Anspruch 1 und/oder 2 in allen ihren stereoisomeren Formen und Mischungen davon in allen Verhältnissen sowie von ihren physiologisch verträglichen Salzen, wobei in der Formel I R⁰ für (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl, bevorzugt für unsubstituiertes oder im Arylrest einfach oder mehrfach substituiertes Biphenylylmethyl, Naphthylmethyl oder Benzyl steht.

4. Verwendung von Verbindungen der Formel I gemäß Anspruch 1 und/oder 3 in allen ihren stereoisomeren Formen und Mischungen davon in allen Verhältnissen sowie von ihren physiologisch verträglichen Salzen, wobei in der Formel I gleichzeitig
W für R¹-A-CH=C und darin A für einen Phenylenrest oder einen Methylenphenylrest steht oder W für R¹-A-C(R¹³) und darin A für einen zweiwertigen Rest aus der Reihe Methylen, Ethylen, Trimethylen, Tetramethylen, Pentamethylen, Cyclohexylen, Phenylen, Phenylenmethyl, Methylenphenyl, Methylenphenylmethyl steht;
B für einen zweiwertigen Rest aus der Reihe Methylen, Ethylen, Trimethylen, Tetramethylen, Vinylen, Phenylen oder für substituiertes Methylen oder Ethylen steht;
E R¹⁰CO bedeutet;
R Wasserstoff, (C₁-C₆)-Alkyl oder Benzyl bedeutet;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste R²¹O-, R²⁴NH-, R²⁵N(R²⁵)-, HO-((C₁-C₈)-Alkyl)-N(R²⁶)-, R²¹O-C(O)- und R²⁸N(R²¹)-C(O)- steht;
R² für Wasserstoff oder (C₁-C₈)-Alkyl steht;
R³ für (C₁-C₈)-Alkyl, gegebenenfalls substituiertes (C₆-C₁₄)-Aryl, (C₆-C₁₄)-Aryl-(C₁-C₈)-alkyl, (C₃-C₈)-Cycloalkyl, (C₂-C₈)-Alkenyl, (C₂-C₈)-Alkinyl, Pyridyl, R¹¹NH, R⁴CO, COOR⁴, CONHR⁴, CSNHR⁴, COOR¹⁵ und CONHR¹⁵ steht;
und e, g und h unabhängig voneinander für die Zahlen 0, 1, 2 oder 3 stehen.

5. Verwendung von Verbindungen der Formel I gemäß einem oder mehreren der Ansprüche 1 bis 4 in allen ihren stereoisomeren Formen und Mischungen davon in allen Verhältnissen sowie von ihren physiologisch verträglichen Salzen, wobei in der Formel I W für R¹-A-C(R¹³) und R¹³ für (C₁-C₆)-Alkyl, im Arylrest gegebenenfalls substituiertes (C₆-C₁₄)-Aryl-(C₁-C₈)-alkyl oder (C₃-C₈)-Cycloalkyl steht.

6. Verwendung von Verbindungen der Formel I gemäß einem oder mehreren der Ansprüche 1 bis 5 in allen ihren stereoisomeren Formen und Mischungen davon in allen Verhältnissen sowie von ihren physiologisch verträglichen Salzen, wobei in der Formel I R³ für gegebenenfalls substituiertes (C₆-C₁₄)-Aryl, für COOR⁴, für R¹¹NH oder für CONHR⁴ steht, wobei -NHR⁴ für den Rest einer α-Aminosäure, deren ω-Amino-(C₂-C₈)-alkylamid, deren (C₁-C₈)-Alkylester oder deren (C₆-C₁₄)-Aryl-(C₁-C₄)-alkylester, bevorzugt für den Rest der α-Aminosäuren Valin, Lysin, Phenylglycin, Phenylalanin oder Tryptophan oder deren (C₁-C₈)-Alkylester oder (C₆-C₁₄)-Aryl-(C₁-C₄)-alkylester, steht.

7. Verwendung von Verbindungen der Formel I gemäß einem oder mehreren der Ansprüche 1 und 3 bis 6 in allen ihren stereoisomeren Formen und Mischungen davon in allen Verhältnissen sowie von ihren physiologisch verträglichen Salzen, wobei in der Formel I gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl, Methylenphenyl oder Methylenphenylmethyl steht;
B für einen unsubstituierten oder substituierten Methylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für den Rest R²⁸N(R²¹)-C(O)- steht;
R² für Wasserstoff steht;
R³ für den Rest CONHR⁴ steht;
R⁴ für Methyl steht, das durch Hydroxycarbonyl und einen Rest aus der Reihe (C₁-C₄)-Alkyl, Phenyl und Benzyl substituiert ist, oder für Methyl steht, das durch (C₁-C₈)-Alkoxycarbonyl, bevorzugt (C₁-C₄)-Alkoxycarbonyl, und einen Rest aus der Reihe (C₁-C₄)-Alkyl, Phenyl und Benzyl substituiert ist;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
b, c und d für 1 stehen und e, f und g für 0 stehen;
h für 1 oder 2, bevorzugt für 1, steht.

8. Verwendung von Verbindungen der Formel I gemäß einem oder mehreren der Ansprüche 1, 3 und 4 in allen ihren stereoisomeren Formen und Mischungen davon in allen Verhältnissen sowie von ihren physiologisch verträglichen Salzen, wobei in der Formel I gleichzeitig
W für R¹-A-CH=C und darin A für einen Phenylenrest oder einen Methylenphenylrest steht oder W für R¹-A-C(R¹³) und darin A für einen zweiwertigen Rest aus der Reihe Methylen, Ethylen, Trimethylen, Tetramethylen, Pentamethylen, Cyclohexylen, Phenylen, Phenylenmethyl, Methylenphenyl, Methylenphenylmethyl steht;
B für einen zweiwertigen Rest aus der Reihe Methylen, Ethylen, Trimethylen, Tetramethylen, Vinylen, Phenylen oder für substituiertes Methylen oder Ethylen steht;
E R¹⁰CO bedeutet;
R Wasserstoff oder (C₁-C₆)-Alkyl bedeutet;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste R²¹O-, R²¹O-N(R²³)-, R²⁴NH-, R²⁵N(R²⁵)-, HO-((C₁-C₈)-Alkyl)-N(R²⁶)-, R²¹O-C(O)- und R²⁸N(R²¹)-C(O)- steht;
R² für Wasserstoff oder (C₁-C₈)-Alkyl steht;
R³ für CONHR¹⁵ oder CONHR⁴ steht, wobei R⁴ hierin für einen unsubstituierten oder durch einen oder mehrere (C₆-C₁₄)-Arylreste substituierten (C₁-C₈)-Alkylrest steht;
R¹⁵ für R¹⁶-(C₁-C₆)-Alkyl oder R¹⁶ steht, wobei R¹⁶ für einen 7- bis 12-gliedrigen verbrückten bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann, und insbesondere R¹⁵ für einen Adamantylrest oder einen Adamantylmethylrest steht;
und e, g und h unabhängig voneinander für die Zahlen 0, 1, 2 oder 3 stehen und b, c und d für 1 stehen.

9. Verwendung von Verbindungen der Formel I gemäß einem oder mehreren der Ansprüche 1, 3, 4, 5 und 8 in allen ihren stereoisomeren Formen und Mischungen davon in allen Verhältnissen sowie von ihren physiologisch verträglichen Salzen, wobei in der Formel I gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl, Methylenphenyl oder Methylenphenylmethyl steht;
B für einen unsubstituierten oder substituierten Methylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für den Rest R²⁸N(R²¹)-C(O)- steht;
R² für Wasserstoff steht;
R³ für CONHR¹⁵ oder CONHR⁴ steht, wobei R⁴ hierin für einen unsubstituierten oder durch einen oder mehrere (C₆-C₁₀)-Arylreste substituierten (C₁-C₆)-Alkylrest steht;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
R¹⁵ für einen Adamantylrest oder einen Adamantylmethylrest steht;
b, c und d für 1 stehen und e, f und g für 0 stehen;
h für 1 oder 2, bevorzugt für 1, steht.

10. Verwendung von Verbindungen der Formel I gemäß einem oder mehreren der Ansprüche 1, 3, 4 und 5 in allen ihren stereoisomeren Formen und Mischungen davon in allen Verhältnissen sowie von ihren physiologisch verträglichen Salzen, wobei in der Formel I gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl, Methylenphenyl, Methylenphenylmethyl steht;
B für einen unsubstituierten oder substituierten Methylenrest oder Ethylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste R²¹O-, R²⁴NH-, R²⁵N(R²⁵)-, HO-((C₁-C₈)-Alkyl)-N(R²⁶)-, R²¹O-C(O)- und R²⁸N(R²¹)-C(O)- steht;
R² für Wasserstoff steht;
R³ für einen unsubstituierten Phenylrest oder Naphthylrest, einen durch einen, zwei oder drei gleiche oder verschiedene Reste aus der Reihe (C₁-C₄)-Alkyl, (C₁-C₄)-Alkoxy, Hydroxy, Halogen, Trifluormethyl, Nitro, Methylendioxy, Ethylendioxy, Hydroxycarbonyl, (C₁-C₄)-Alkoxycarbonyl, Aminocarbonyl, Cyan, Phenyl, Phenoxy und Benzyloxy substituierten Phenylrest oder Naphthylrest, einen Pyridylrest, einen (C₁-C₄)-Alkylrest, einen (C₂-C₄)-Alkenylrest, einen (C₂-C₄)-Alkinylrest oder einen (C₅-C₆)-Cycloalkylrest steht, und insbesondere R³ für einen unsubstituierten oder substituierten Phenylrest oder Naphthylrest steht;
R¹⁰ für Hydroxy oder (C₁-C₈)-Alkoxy, insbesondere (C₁-C₄)-Alkoxy, steht und bevorzugt R¹⁰ für einen Rest aus der Reihe Hydroxy, Methoxy, Ethoxy, Propoxy und Isopropoxy steht;
R¹³ für (C₁-C₆)-Alkyl, (C₃-C₇)-Cycloalkyl oder Benzyl, insbesondere Methyl, steht;
b, c und d für 1 stehen und e, f und g für 0 stehen;
h für 1 oder 2, bevorzugt für 1, steht.

11. Verwendung von Verbindungen der Formel I gemäß einem oder mehreren der Ansprüche 1, 3, 4 und 5 in allen ihren stereoisomeren Formen und Mischungen davon in allen Verhältnissen sowie von ihren physiologisch verträglichen Salzen, wobei in der Formel I gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl, Methylenphenyl, Methylenphenylmethyl steht;
B für einen unsubstituierten oder substituierten Methylenrest oder Ethylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste R²¹O-, R²⁴NH-, R²⁵N(R²⁵)-, HO-((C₁-C₈)-Alkyl)-N(R²⁶)-, R²¹O-C(O)- und R²⁸N(R²¹)-C(O)- steht;
R² für Wasserstoff steht;
R³ für R¹¹NH steht;
R¹⁰ für Hydroxy oder (C₁-C₈)-Alkoxy, insbesondere (C₁-C₄)-Alkoxy, steht und bevorzugt R¹⁰ für einen Rest aus der Reihe Hydroxy, Methoxy, Ethoxy, Propoxy und Isopropoxy steht;
R¹³ für (C₁-C₆)-Alkyl, (C₃-C₇)-Cycloalkyl oder Benzyl, insbesondere Methyl, steht;
b, c, d und e für 1 stehen und f und g für 0 stehen;
h für 0 steht.

12. Verwendung von Verbindungen der Formel I gemäß einem oder mehreren der Ansprüche 1 und 3 bis 11 in allen ihren stereoisomeren Formen und Mischungen davon in allen Verhältnissen sowie von ihren physiologisch verträglichen Salzen, worin ein für die Gruppe B stehender substituierter Methylenrest oder substituierter Ethylenrest als Substituenten einen Rest aus der Reihe (C₁-C₈)-Alkyl, (C₂-C₆)-Alkenyl, (C₂-C₆)-Alkinyl, (C₃-C₈)-Cycloalkyl, insbesondere (C₅-C₆)-Cycloalkyl, (C₃-C₈)-Cycloalkyl-(C₁-C₄)-alkyl, insbesondere (C₅-C₆)-Cycloalkyl-(C₁-C₄)-alkyl, gegebenenfalls substituiertes (C₆-C₁₀)-Aryl, im Arylrest gegebenenfalls substituiertes (C₆-C₁₀)-Aryl-(C₁-C₄)-alkyl, gegebenenfalls substituiertes Heteroaryl und im Heteroarylrest gegebenenfalls substituiertes Heteroaryl-(C₁-C₄)-alkyl trägt.

13. Verwendung von Verbindungen der Formel I gemäß einem oder mehreren der Ansprüche 1 und 3 bis 12 in allen ihren stereoisomeren Formen und Mischungen davon in allen Verhältnissen sowie von ihren physiologisch verträglichen Salzen, worin B für einen unsubstituierten Methylenrest steht oder für einen Methylenrest steht, der durch einen (C₁-C₈)-Alkylrest substituiert ist.

14. Verwendung von Verbindungen der Formel I gemäß einem oder mehreren der Ansprüche 1 bis 13 und/oder ihren physiologisch verträglichen Salzen zur Herstellung von Arzneimitteln zur Entzündungshemmung.

15. Verwendung von Verbindungen der Formel I gemäß einem oder mehreren der Ansprüche 1 bis 13 und/oder ihren physiologisch verträglichen Salzen zur Herstellung von Arzneimitteln zur Behandlung oder Prophylaxe der rheumatoiden Arthritis, der inflammatorischen bowel disease, des systemischen Lupus erythematosus oder von inflammatorischen Erkrankungen des zentralen Nervensystems.

16. Verwendung von Verbindungen der Formel I gemäß einem oder mehreren der Ansprüche 1 bis 13 und/oder ihren physiologisch verträglichen Salzen zur Herstellung von Arzneimitteln zur Behandlung oder Prophylaxe von Asthma oder Allergien.

17. Verwendung von Verbindungen der Formel I gemäß einem oder mehreren der Ansprüche 1 bis 13 und/oder ihren physiologisch verträglichen Salzen zur Herstellung von Arzneimitteln zur Behandlung oder Prophylaxe von cardiovaskulären Erkrankungen, der Arteriosklerose, von Restenosen oder von Diabetes, zur Verhinderung der Schädigung von Organtransplantaten, zur Hemmung von Tumorwachstum oder Tumormetastasierung oder zur Therapie der Malaria.

18. Verwendung von Verbindungen der Formel I gemäß einem oder mehreren der Ansprüche 1 bis 13 und/oder ihren physiologisch verträglichen Salzen zur Hemmung der Adhäsion und/oder Migration von Leukozyten oder zur Hemmung des VLA-4-Rezeptors.

19. Verwendung von Verbindungen der Formel I gemäß einem oder mehreren der Ansprüche 1 bis 13 und/oder ihren physiologisch verträglichen Salzen zur Behandlung oder Prophylaxe von Krankheiten, bei denen die Leukozytenadhäsion und/oder Leukozytenmigration ein unerwünschtes Ausmaß aufweist, oder von Krankheiten, bei denen VLA-4-abhängige Adhäsionsvorgänge eine Rolle spielen, zur Behandlung oder Prophylaxe der rheumatoiden Arthritis, der inflammatorischen bowel disease, des systemischen Lupus erythematosus, von inflammatorischen Erkrankungen des zentralen Nervensystems, von Asthma, von Allergien, von cardiovaskulären Erkrankungen, von Arteriosklerose, von Restenosen, von Diabetes, zur Verhinderung der Schädigung von Organtransplantaten, zur Hemmung von Tumorwachstum oder Tumormetastasierung, zur Therapie der Malaria, oder als Entzündungshemmer.

20. Verbindungen der Formel Ib, 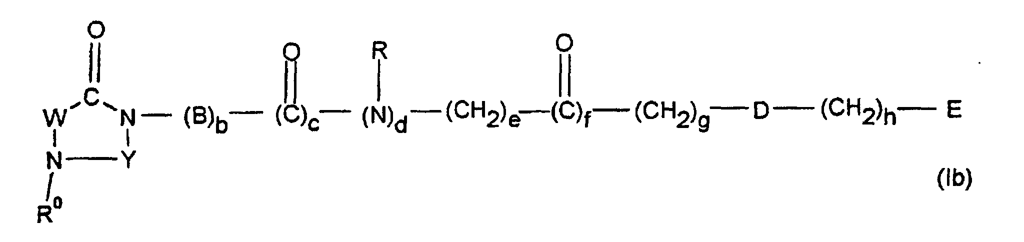 worin
W für R¹-A-C(R¹³) oder R¹-A-CH=C steht;
Y für eine Carbonyl-, Thiocarbonyl- oder Methylengruppe steht;
A einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)- Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
B einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R und R⁰ unabhängig voneinander Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeuten, wobei Alkylreste durch Fluor ein- oder mehrfach substituiert sein können;
R¹ den Rest R²⁸N(R²¹)-C(O)- bedeutet;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)- Cycloalkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)- Cycloalkyl, (C₂-C₈)-Alkenyl, (C₂-C₈)-Alkinyl, (C₂-C₈)-Alkenylcarbonyl, (C₂-C₈)- Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)- aminocarbonyl, Amino-(C₂-C₁₈)-alkylaminocarbonyl, Amino-(C₁-C₃)-alkyl- phenyl-(C₁-C₃)-alkylaminocarbonyl, (C₁-C₁₈)-Alkylcarbonylamino-(C₁-C₃)- alkylphenyl-(C₁-C₃)-alkylaminocarbonyl, (C₁-C₁₈)-Alkylcarbonylamino-(C₂-C₁₈)- alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)- Alkoxycarbonyl, gegebenenfalls substituiertes (C₃-C₈)-Cycloalkyl, HOS(O)₂-(C₁- C₃)-alkyl, R⁹NHS(O)₂-(C₁-C₃)-alkyl, (R⁸O)₂P(O)-(C₁-C₃)-alkyl, Tetrazolyl-(C₁- C₃)-alkyl, Halogen, Nitro, Trifluormethyl und den Rest R⁵ substituiert sein kann;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N- (C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)- alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid- Rest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)- Cycloalkylaminocarbonyl, gegebenenfalls substituiertes (C₆-C₁₄)- Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ Wasserstoff, (C₁-C₁₈)-Alkyl, R¹²CO, R^{12a}CS, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵ bedeutet;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)- Aryloxy, den Rest R¹⁵, den Rest R¹⁵-O-, Amino, Mono- oder Di-((C₁-C₁₈)-alkyl)- amino, (C₂-C₈)-Alkenyl-amino, (C₂-C₈)-Alkinyl-amino, (C₃-C₁₂)-Cycloalkyl- amino, (C₃-C₁₂)-Cycloalkyl-(C₁-C₈)-alkyl-amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl- (C₁-C₈)-alkyl-NH bedeutet;
R^{12a} Amino, Mono- oder Di-((C₁-C₁₈)-alkyl)-amino, (C₂-C₈)-Alkenyl-amino, (C₂-C₈)- Alkinyl-amino, (C₃-C₁₂)-Cycloalkyl-amino, (C₃-C₁₂)-Cycloalkyl-(C₁-C₈)-alkyl- amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl-(C₁-C₈)-alkyl-NH bedeutet;
R¹³ Wasserstoff, (C₁-C₆)-Alkyl, im Arylrest gegebenenfalls substituiertes (C₆-C₁₄)- Aryl-(C₁-C₈)-alkyl oder (C₃-C₈)-Cycloalkyl bedeutet;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
R²¹ Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₄)-Aryl, (C₆-C₁₄)-Aryl-(C₁-C₈)-alkyl, den Rest Het- oder Het-(C₁- C₈)-alkyl bedeutet, wobei Alkylreste durch Fluor einfach oder mehrfach substituiert sein können und die Reste R²¹ bei mehrfachem Auftreten gleich oder verschieden sein können;
R²⁶ die Bedeutungen von R²¹ hat oder HO-((C₁-C₈)-Alkyl) bedeutet, wobei Alkylreste durch Fluor einfach oder mehrfach substituiert sein können;
R²⁸ für einen der Reste R²¹-, R²¹O-, R²⁶N(R²⁶)-, R²¹C(O)-, R²¹O-C(O)-, ((C₁-C₁₈)- Alkyl-O-C(O)-((C₁-C₆)-alkyl)-O-C(O)-, R²¹N(R²¹)-C(O)-, R²¹N(R²¹)-C(=N(R²¹))- oder R²¹C(O)-N(R²¹)- steht;
Het für einen mono- oder polycyclischen, 4- bis 14-gliedrigen, aromatischen oder nicht aromatischen Ring steht, der 1, 2, 3 oder 4 gleiche oder verschiedene Heteroatome aus der Reihe N, O und S als Ringglieder enthält und gegebenenfalls durch einen oder mehrere, gleiche oder verschiedene Substituenten substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

21. Verbindungen der Formel Ib gemäß Anspruch 20 und/oder ihre physiologisch verträglichen Salze zur Verwendung als Arzneimittel.

22. Pharmazeutisches Präparat, **dadurch gekennzeichnet, daß** es eine oder mehrere Verbindungen der Formel Ib gemäß Anspruch 20 und/oder ihre physiologisch verträglichen Salze neben pharmazeutisch einwandfreien Trägerstoffen und/oder Zusatzstoffen enthält.

23. Verbindungen der Formel Ic, 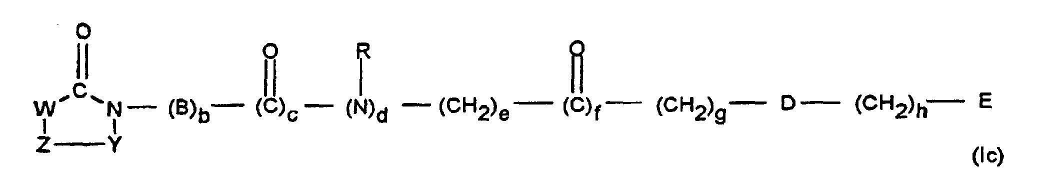 in der
W für R¹-A-C(R¹³) oder R¹-A-CH=C steht;
Y für eine Carbonyl-, Thiocarbonyl- oder Methylengruppe steht;
Z für N(R⁰), Sauerstoff, Schwefel oder eine Methylengruppe steht;
A für einen zweiwertigen (C₁-C₆)-Alkylen-Rest steht;
B einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R und R⁰ unabhängig voneinander Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₈-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeuten, wobei Alkylreste durch Fluor ein- oder mehrfach substituiert sein können;
R¹ Wasserstoff, (C₁-C₁₀)-Alkyl, das gegebenenfalls durch Fluor einfach oder mehrfach substituiert sein kann, oder einen der Reste R⁴¹O-, R⁴¹O-N(R⁴²)-, R⁴²N(R⁴²)-, HO-((C₁-C₈)-Alkyl)-N(R⁴³)-, HC(O)-NH-, R⁴¹C(O)-N(R⁴²)-, R⁴¹C(O)-, R⁴¹O-C(O)-, R⁴⁴N(R⁴¹)-C(O)-, R⁴¹O-N=, O= und S= bedeutet;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)- Cycloalkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)- Cycloalkyl, (C₂-C₈)-Alkenyl, (C₂-C₈)-Alkinyl, (C₂-C₈)-Alkenylcarbonyl, (C₂-C₈)- Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)- alkylaminocarbonyl, (C₁-C₁₈)-Alkylcarbonylamino-(C₁-C₃)-alkylphenyl-(C₁-C₃)- alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und den Rest R⁵ substituiert sein kann;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N-(C₁- C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)- alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid- Rest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)- Cycloalkylaminocarbonyl, gegebenenfalls substituiertes (C₆-C₁₄)- Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ Wasserstoff, (C₁-C₁₈)-Alkyl, R¹²CO, R^{12a}CS, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵ bedeutet;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)- Aryloxy, den Rest R¹⁵, den Rest R¹⁵-O-, Amino, Mono- oder Di-((C₁-C₁₈)-alkyl)- amino, (C₂-C₈)-Alkenyl-amino, (C₂-C₈)-Alkenyl-amino, (C₃-C₁₂)-Cycloalkyl- amino, (C₃-C₁₂)-Cycloalkyl-(C₁-C₈)-alkyl-amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl- (C₁-C₈)-alkyl-NH bedeutet;
R^{12a} Amino, Mono- oder Di-((C₁-C₁₈)-alkyl)-amino, (C₂-C₈)-Alkenyl-amino, (C₂-C₈)- Alkinyl-amino, (C₃-C₁₂)-Cycloalkyl-amino, (C₃-C₁₂)-Cycloalkyl-(C₁-C₈)-alkyl- amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl-(C₁-C₈)-alkyl-NH bedeutet;
R¹³ Wasserstoff, (C₁-C₆)-Alkyl, im Arylrest gegebenenfalls substituiertes (C₆-C₁₄)- Aryl-(C₁-C₈)-alkyl oder (C₃-C₈)-Cycloalkyl bedeutet;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
R⁴¹ Wasserstoff oder (C₁-C₈)-Alkyl bedeutet, wobei Alkylreste durch Fluor einfach oder mehrfach substituiert sein können und die Reste R⁴¹ bei mehrfachem Auftreten gleich oder verschieden sein können;
R⁴² (C₁-C₈)-Alkyl bedeutet, wobei Alkylreste durch Fluor einfach oder mehrfach substituiert sein können und die Reste R⁴² bei mehrfachem Auftreten gleich oder verschieden sein können;
R⁴³ die Bedeutungen von R⁴¹ hat oder HO-((C₁-C₈)-Alkyl) bedeutet, wobei Alkylreste durch Fluor einfach oder mehrfach substituiert sein können;
R⁴⁴ für einen der Reste R⁴¹-, R⁴¹O-, R⁴³N(R⁴³)-, R⁴¹C(O)-, R⁴¹O-C(O)-, ((C₁-C₁₈)- Alkyl-O-C(O)-((C₁-C₆)-alkyl)-O-C(O)-, R⁴¹N(R⁴¹)-C(O)-, R⁴¹N(R⁴¹)-C(=N(R⁴¹))- oder R⁴¹C(O)-N(R⁴¹)- steht;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

24. Verbindungen der Formel lc gemäß Anspruch 23 und/oder ihre physiologisch verträglichen Salze zur Verwendung als Arzneimittel.

25. Pharmazeutisches Präparat, **dadurch gekennzeichnet, daß** es eine oder mehrere Verbindungen der Formel Ic gemäß Anspruch 23 und/oder ihre physiologisch verträglichen Salze neben pharmazeutisch einwandfreien Trägerstoffen und/oder Zusatzstoffen enthält.

26. Verbindungen der Formel Id, 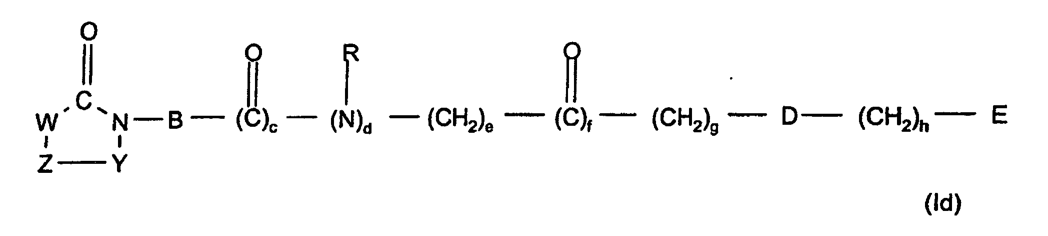 worin
W für R¹-A-C(R¹³) oder R¹-A-CH=C steht;
Y für eine Carbonyl-, Thiocarbonyl- oder Methylengruppe steht;
Z für N(R⁰), Sauerstoff, Schwefel oder eine Methylengruppe steht;
A einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)- Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
B einen zweiwertigen (C₁-C₆)-Alkylen-Rest bedeutet, der durch einen Rest aus der Reihe (C₁-C₈)-Alkyl, (C₂-C₈)-Alkenyl, (C₂-C₈)-Alkinyl, (C₃-C₁₀)-Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert ist;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R Wasserstoff, (C₁-C₈)-Alkyl, (C₃-C₁₂)-Cycloalkyl, (C₃-C₁₂)-Cycloalkyl-(C₁-C₈)- alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl- (C₁-C₈)-alkyl bedeuten, wobei Alkylreste durch Fluor ein- oder mehrfach substituiert sein können;
R⁰ für Wasserstoff, (C₁-C₈)-Alkyl, (C₃-C₁₂)-Cycloalkyl, (C₃-C₁₂)-Cycloalkyl-(C₁-C₈)- alkyl, (C₆-C₁₂)-Bicycloalkyl, (C₆-C₁₂)-Bicycloalkyl-(C₁-C₈)-alkyl, (C₆-C₁₂)- Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)- Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)-Tricycloalkyl-CO, (C₆-C₁₂)- Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)- Cycloalkyl-S(O)ₙ, (C₃-C₁₂)-Cycloalkyl-(C₁-C₈)-alkyl-S(O)ₙ, (C₆-C₁₂)-Bicycloalkyl- S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆- C₁₂)-Tricycloalkyl-(C₁-C₈)-alkyl-S(O)ₙ, gegebenenfalls substituiertes (C₈-C₁₄)-Aryl- S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
R¹ Wasserstoff, (C₁-C₁₀)-Alkyl, das gegebenenfalls durch Fluor einfach oder mehrfach substituiert sein kann, (C₃-C₁₂)-Cycloalkyl, (C₃-C₁₂)-Cycloalkyl-(C₁-C₈)- alkyl, gegebenenfalls substituiertes R²¹-((C₆-C₁₄)-Aryl), im Arylrest gegebenenfalls substituiertes (R²¹-((C₆-C₁₄)-Aryl))-(C₁-C₈)-alkyl, den Rest Het-, Het-(C₁-C₈)-alkyl oder einen der Reste R²¹O-, R²²O-NH-, R²¹O-N(R²³)-, R²⁴NH-, R²⁵N(R²⁵)-, HO-((C₁-C₈)-Alkyl)-N(R²⁶)-, R²⁷C(O)-NH-, R²¹C(O)-N(R²³)-, R²¹C(O)-, R²¹O-C(O)-, R²⁸N(R²¹)-C(O)-, R²¹O-N=, O= und S= bedeutet;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂- C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)- alkylaminocarbonyl, (C₁-C₁₈)-Alkylcarbonylamino-(C₁-C₃)-alkylphenyl-(C₁-C₃)- alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und den Rest R⁵ substituiert sein kann;
R⁵ gegebenenfalls substituiertes (C₈-C₁₄)-Aryl, im Arylrest gegebenenfalls substituiertes (C₆-C₁₄)-Aryl-(C₁-C₈)-alkyl, einen mono- oder bicyclischen 5- bis 12- gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N-(C₁- C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl, (C₁-C₁₈)-Alkylcarbonyl, (C₁- C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid- Rest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆- C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)- Cycloalkylaminocarbonyl, gegebenenfalls substituiertes (C₆-C₁₄)- Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ Wasserstoff, (C₁-C₁₈)-Alkyl, R¹²CO, R^{12a}CS, gegebenenfalls substituiertes (C₆- C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵ bedeutet;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)- Aryloxy, den Rest R¹⁵, den Rest R¹⁵-O-, Amino, Mono- oder Di-((C₁-C₁₈)-alkyl)- amino, (C₂-C₈)-Alkenyl-amino, (C₂-C₈)-Alkinyl-amino, (C₃-C₁₂)-Cycloalkyl-amino, (C₃-C₁₂)-Cycloalkyl-(C₁-C₈)-alkyl-amino, den Rest R¹⁵-NH-, gegebenenfalls substituiertes (C₆-C₁₄)-Aryl-NH, (C₆-C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl-(C₁-C₈)-alkyl-NH bedeutet;
R^{12a} Amino, Mono- oder Di-((C₁-C₁₈)-alkyl)-amino, (C₂-C₈)-Alkenyl-amino, (C₂-C₈)- Alkinyl-amino, (C₃-C₁₂)-Cycloalkyl-amino, (C₃-C₁₂)-Cycloalkyl-(C₁-C₈)-alkyl- amino, den Rest R¹⁵-NH-, gegebenenfalls substituiertes (C₆-C₁₄)-Aryl-NH, (C₆- C₁₄)-Aryl-(C₁-C₈)-alkyl-NH, das im Arylrest auch substituiert sein kann, gegebenenfalls substituiertes Heteroaryl-NH oder im Heteroarylrest gegebenenfalls substituiertes Heteroaryl-(C₁-C₈)-alkyl-NH bedeutet;
R¹³ Wasserstoff, (C₁-C₆)-Alkyl, im Arylrest gegebenenfalls substituiertes (C₆-C₁₄)- Aryl-(C₁-C₈)-alkyl oder (C₃-C₈)-Cycloalkyl bedeutet;
R¹⁵ für R¹⁶-(C₁-C₆)-alkyl oder für R¹⁶ steht;
R¹⁶ für einen 6- bis 24-gliedrigen bicyclischen oder tricydischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
R²¹ Wasserstoff, (C₁-C₈)-Alkyl, (C₃-C₁₂)-Cycloalkyl, (C₃-C₁₂)-Cycloalkyl-(C₁-C₈)-alkyl, (C₆-C₁₄)-Aryl, (C₆-C₁₄)-Aryl-(C₁-C₈)-alkyl, den Rest Het- oder Het-(C₁-C₈)-alkyl bedeutet, wobei Alkylreste durch Fluor einfach oder mehrfach substituiert sein können und die Reste R²¹ bei mehrfachem Auftreten gleich oder verschieden sein können;
R²² (C₃-C₁₂)-Cycloalkyl, (C₃-C₁₂)-Cycloalkyl-(C₁-C₈)-alkyl, (C₆-C₁₄)-Aryl, den Rest Het- oder Het-(C₁-C₈)-alkyl bedeutet, wobei Alkylreste durch Fluor einfach oder mehrfach substituiert sein können;
R²³ (C₁-C₈)-Alkyl, (C₃-C₁₂)-Cycloalkyl, (C₃-C₁₂)-Cycloalkyl-(C₁-C₈)-alkyl, (C₆-C₁₄)-Aryl, (C₆-C₁₄)-Aryl-(C₁-C₈)-alkyl, den Rest Het- oder Het-(C₁-C₈)-alkyl bedeutet, wobei Alkylreste durch Fluor einfach oder mehrfach substituiert sein können;
R²⁴ (C₃-C₁₂)-Cycloalkyl, (C₃-C₁₂)-Cycloalkyl-(C₁-C₈)-alkyl, (C₆-C₁₄)-Aryl, (C₆-C₁₄)-Aryl- (C₁-C₈)-alkyl, den Rest Het- oder Het-(C₁-C₈)-alkyl bedeutet, wobei Alkylreste durch Fluor einfach oder mehrfach substituiert sein können;
R²⁵ die Bedeutungen von R²³ hat, wobei die Reste R²⁵ gleich oder verschieden sein können;
R²⁶ die Bedeutungen von R²¹ hat oder HO-((C₁-C₈)-Alkyl) bedeutet, wobei Alkylreste durch Fluor einfach oder mehrfach substituiert sein können;
R²⁷ Wasserstoff, (C₃-C₁₂)-Cycloalkyl, (C₃-C₁₂)-Cycloalkyl-(C₁-C₈)-alkyl, (C₆-C₁₄)-Aryl- (C₁-C₈)-alkyl, den Rest Het- oder Het-(C₁-C₈)-alkyl bedeutet, wobei Alkylreste durch Fluor einfach oder mehrfach substituiert sein können;
R²⁸ für einen der Reste R²¹-, R²¹O-, R²⁶N(R²⁶)-, R²¹C(O)-, R²¹O-C(O)-, ((C₁-C₁₈)- Alkyl-O-C(O)-((C₁-C₆)-alkyl)-O-C(O)-, R²¹N(R²¹)-C(O)-, R²¹N(R²¹)-C(=N(R²¹))- oder R²¹C(O)-N(R²¹)- steht;
Het für einen mono- oder polycyclischen, 4- bis 14-gliedrigen, aromatischen oder nicht aromatischen Ring steht, der 1, 2, 3 oder 4 gleiche oder verschiedene Heteroatome aus der Reihe N, O und S als Ringglieder enthält und gegebenenfalls durch einen oder mehrere, gleiche oder verschiedene Substituenten substituiert sein kann;
c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze;
wobei die Gruppe R¹-A- nicht für Phenyl, Pyrrolyl, Imidazolyl oder Pyridyl stehen kann, wobei diese Reste durch (C₁-C₈)-Alkyl, (C₁-C₈)-Alkoxy, Trifluormethyl, Hydroxy, Hydroxy-(C₁-C₄)-alkyl, Formyl, Hydroxycarbonyl, Aminocarbonyl, (C₁-C₄)-Alkoxycarbonyl, Phenyl, Phenoxy, Benzyloxy, Tetrazolyl, Phenyl-(C₁-C₄)-alkyl und (C₁-C₄)-Alkyl-CO substituiert sein können, und die Gruppe R¹-A- nicht für den Rest R^{x}-(C₁-C₂)-alkyl stehen kann, worin R^{x} für Phenyl, Furyl, Thienyl, Pyrrolyl, Imidazolyl oder Pyridyl steht, wobei diese Reste auch benzanelliert sein können und durch (C₁-C₈)-Alkyl, (C₁-C₈)-Alkoxy, Halogen, Nitro, Amino, Trifluormethyl, Hydroxy, Hydroxy-(C₁-C₄)-alkyl, Methylendioxy, Ethylendioxy, Cyan, Formyl, Hydroxycarbonyl, Aminocarbonyt, (C₁-C₄)-Alkoxycarbonyl, Phenyl, Phenoxy, Benzyloxy, Tetrazolyl, Phenyl-(C₁-C₄)-alkyl und (C₁-C₄)-Alkyl-CO substituiert sein können.

27. Verbindungen der Formel Id gemäß Anspruch 26, in der gleichzeitig
W für R¹-A-C(R¹³) steht;
Y für eine Carbonylgruppe steht;
Z für N(R⁰) steht;
A einen zweiwertigen Rest aus der Reihe (C₃-C₇)-Cycloalkylen, Phenylen, Phenylen-(C₁-C₆)-alkyl, (C₁-C₆)-Alkylen-phenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
B einen zweiwertigen Methylenrest oder Ethylenrest bedeutet, der durch einen Rest aus der Reihe (C₁-C₈)-Alkyl, (C₂-C₈)-Alkenyl, (C₂-C₈)-Alkinyl, (C₃-C₁₀)- Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)- alkyl, gegebenenfalls substituiertes Heteroaryl und im Heteroarylrest gegebenenfalls substituiertes Heteroaryl-(C₁-C₆)-alkyl substituiert ist;
D für C(R²)(R³) steht;
E Tetrazolyl oder R¹⁰CO bedeutet;
R Wasserstoff oder (C₁-C₈)-Alkyl bedeutet;
R⁰ für Wasserstoff, (C₁-C₈)-Alkyl, (C₃-C₁₂)-Cycloalkyl, (C₃-C₁₂)-Cycloalkyl-(C₁- C₈)-alkyl, (C₆-C₁₂)-Bicycloalkyl, (C₆-C₁₂)-Bicycloalkyl-(C₁-C₈)-alkyl, (C₆-C₁₂)- Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)- alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)- Bicycloalkyl-CO, (C₆-C₁₂)-Bicycloalkyl-(C₁-C₈)-alkyl-CO, (C₆-C₁₂)-Tricycloalkyl- CO, (C₆-C₁₂)-Tricycloalkyl-(C₁-C₈)-alkyl-CO, gegebenenfalls substituiertes (C₆- C₁₄)-Aryl-CO, im Arylrest gegebenenfalls substituiertes (C₆-C₁₄)-Aryl-(C₁-C₈)- alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)- Bicycloalkyl-S(O)ₙ, (C₆-C₁₂)-Bicycloalkyl-(C₁-C₈)-alkyl-S(O)ₙ, (C₆-C₁₂)- Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl- S(O)ₙ oder im Heteroarylrest gegebenenfalls substituiertes Heteroaryl-(C₁-C₈)- alkyl-S(O)ₙ steht, wobei n für 1 oder 2 steht;
R¹ für einen der Reste R²¹O-, R²⁴NH-, R²⁵N(R²⁵)-, HO-((C₁-C₈)-Alkyl)-N(R²⁶)-, R²¹O-C(O)- und R²⁸N(R²¹)-C(O)- steht;
R² Wasserstoff oder (C₁-C₈)-Alkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)- Cycloalkyl, (C₂-C₈)-Alkenyl, (C₂-C₈)-Alkinyl, (C₂-C₈)-Alkenylcarbonyl, (C₂-C₈)- Alkinylcarbonyl, Pyridyl, R¹¹NH, CON(CH₃)R⁴, CONHR⁴, CON(CH₃)R¹⁵ oder CONHR¹⁵ bedeutet;
R⁴ (C₁-C₁₀)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, (C₆-C₁₄)-Aryl- (C₁-C₈)-alkoxycarbonyl, das im Arylrest auch substituiert sein kann, (C₁-C₈)- Alkoxy, (C₁-C₈)-Alkoxycarbonyl, gegebenenfalls substituiertes (C₃-C₈)- Cycloalkyl, Tetrazolyl-(C₁-C₃)-alkyl, Trifluormethyl und R⁵ substituiert sein kann;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, oder einen Rest R⁶CO- bedeutet, wobei der Aryl- und unabhängig davon der Heterocyclus-Rest ein- oder mehrfach durch gleiche oder verschiedene Reste aus der Reihe (C₁-C₈)-Alkyl, (C₁-C₈)-Alkoxy, Halogen, Nitro, Amino oder Trifluormethyl substituiert sein können;
R⁶ für einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert sein kann, sowie deren Ester und Amide bedeutet, wobei freie funktionelle Gruppen durch in der Peptidchemie übliche Schutzgruppen geschützt sein können;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ für R¹²CO, gegebenenfalls substituiertes (C₆-C₁₄)-Aryl-S(O)₂ oder (C₁-C₁₈)- Alkyl-S(O)₂ steht;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)- Aryloxy, den Rest R¹⁵ oder den Rest R¹⁵-O- bedeutet;
R¹³ Wasserstoff oder (C₁-C₄)-Alkyl bedeutet;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
c und d für 1 stehen und f für 0 steht;
e und h unabhängig voneinander für 0 oder 1 stehen und g für 0 steht;
in allen ihren stereoisomeren Formen und Mischungen davon in allen Verhältnissen, sowie ihre physiologisch verträglichen Salze.

28. Verbindungen der Formel Id gemäß Anspruch 26 und/oder 27, worin der Rest, durch den die Gruppe B substituiert ist, ein (C₁-C₈)-Alkylrest ist, in allen ihren stereoisomeren Formen und Mischungen davon in allen Verhältnissen, sowie ihre physiologisch verträglichen Salze.

29. Verbindungen der Formel Id gemäß einem oder mehreren der Ansprüche Anspruch 26 bis 28, worin der Rest R¹ für R²⁸N(R²¹)-C(O)- steht, in allen ihren stereoisomeren Formen und Mischungen davon in allen Verhältnissen, sowie ihre physiologisch verträglichen Salze.

30. Verbindungen der Formel Id gemäß einem oder mehreren der Ansprüche 26 bis 29 und/oder ihre physiologisch verträglichen Salze zur Verwendung als Arzneimittel.

31. Pharmazeutisches Präparat, **dadurch gekennzeichnet, daß** es eine oder mehrere Verbindungen der Formel Ib gemäß einem oder mehreren der Ansprüche 26 bis 29 und/oder ihre physiologisch verträglichen Salze neben pharmazeutisch einwandfreien Trägerstoffen und/oder Zusatzstoffen enthält.

## Claims

1. The use of compounds of the formula I 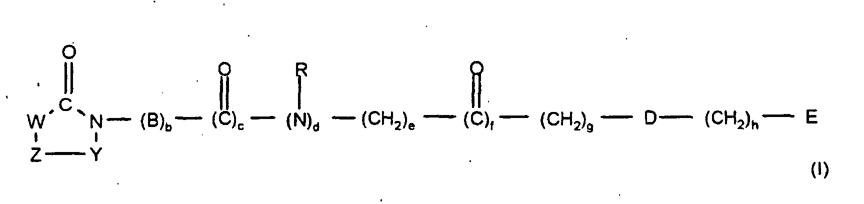 in which
W is R¹-A-C(R¹³) or R¹-A-CH=C;
Y is a carbonyl, thiocarbonyl or methylene group;
Z is N(R⁰), oxygen, sulfur or a methylene group;
A is a bivalent radical from the group consisting of (C₁-C₆)-alkylene, (C₃-C₁₂)-cycloalkylene, (C₁-C₆)-alkylene-(C₃-C₁₂)-cycloalkyl, phenylene, phenylene-(C₁-C₆)-alkyl, (C₁-C₆)-alkylenephenyl, (C₁- C₆)-alkylenephenyl-(C₁-C₆)-alkyl, phenylene-(C₂-C₆)-alkenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur;
B is a bivalent radical from the group consisting of (C₁-C₆)-alkylene, (C₂-C₆)-alkenylene, phenylene, phenylene-(C₁-C₃)-alkyl, (C₁-C₃)- alkylenephenyl, where the bivalent (C₁-C₆)-alkylene radical can be unsubstituted or substituted by a radical from the group consisting of (C₁-C₈)-alkyl, (C₂-C₈)-alkenyl, (C₂-C₈)-alkynyl, (C₃-C₁₀)-cycloalkyl, (C₃-C₁₀)-cycloalkyl-(C₁-C₆)-alkyl, optionally substituted (C₆-C₁₄)-aryl, (C₆-C₁₄)-aryl-(C₁-C₆)-alkyl optionally substituted in the aryl radical, optionally substituted heteroaryl and heteroaryl-(C₁-C₆)-alkyl optionally substituted in the heteroaryl radical;
D is C(R²)(R³), N(R³) or CH=C(R³);
E is tetrazolyl, (R⁸O)₂P(O), HOS(O)₂, R⁹NHS(O)₂ or R¹⁰CO;
R is hydrogen, (C₁-C₈)-alkyl, (C₃-C₁₂)cycloalkyl, (C₃-C₁₂)-cycloalkyl- (C₁-C₈)-alkyl, optionally substituted (C₆-C₁₄)-aryl, (C₆-C₁₄)-aryl- (C₁-C₈)-alkyl optionally substituted in the aryl radical, optionally substituted heteroaryl or heteroaryl-(C₁-C₈)-alkyl optionally substituted in the heteroaryl radical, where alkyl radicals can be mono- or polysubstituted by fluorine;
R⁰ is hydrogen, (C₁-C₈)-alkyl, (C₃-C₁₂)-cycloalkyl, (C₃-C₁₂)-cycloalkyl- (C₁-C₈)-alkyl, (C₆-C₁₂)-bicycloalkyl, (C₆-C₁₂)-bicycloalkyl-(C₁-C₈)- alkyl, (C₆-C₁₂)-tricycloalkyl, (C₆-C₁₂)-tricycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl, heteroaryl-(C₁-C₈)-alkyl optionally substituted in the heteroaryl radical, CHO, (C₁-C₈)-alkyl-CO, (C₃-C₁₂)-cycloalkyl-CO, (C₃-C₁₂)-cycloalkyl-(C₁-C₈)-alkyl-CO, (C₆-C₁₂)-bicycloalkyl-CO, (C₆- C₁₂)-bicycloalkyl-(C₁-C₈)-alkyl-CO, (C₆-C₁₂)-tricycloalkyl-CO, (C₆- C₁₂)-tricycloalkyl-(C₁-C₈)-alkyl-CO, optionally substituted (C₆-C₁₄)- aryl-CO, (C₆-C₁₄)-aryl-(C₁-C₈)-alkyl-CO optionally substituted in the aryl radical, optionally substituted heteroaryl-CO, heteroaryl-(C₁-C₈)- alkyl-CO optionally substituted in the heteroaryl radical, (C₁-C₈)- alkyl-S(O)ₙ, (C₃-C₁₂)-cycloalkyl-S(O)ₙ, (C₃-C₁₂)-cycloalkyl-(C₁-C₈)- alkyl-S(O)ₙ, (C₆-C₁₂)-bicycloalkyl-S(O)ₙ, (C₆-C₁₂)-bicycloalkyl- (C₁-C₈)-alkyl-S(O)ₙ, (C₆-C₁₂)-tricycloalkyl-S(O)ₙ, (C₆-C₁₂)- tricycloalkyl-(C₁-C₈)-alkyl-S(O)ₙ, optionally substituted (C₆-C₁₄)-aryl- S(O)ₙ, (C₆-C₁₄)-aryl-(C₁-C₈)-alkyl-S(O)ₙ optionally substituted in the aryl radical, optionally substituted heteroaryl-S(O)ₙ or heteroaryt-(C₁- C₈)-alkyl-S(O)ₙ optionally substituted in the heteroaryl radical, where n is 1 or 2;
R¹ is hydrogen, (C₁-C₁₀)-alkyl, which can optionally be monosubstituted or polysubstituted by fluorine, (C₃-C₁₂)-cycloalkyl, (C₃-C₁₂)- cycloalkyl-(C₁-C₈)-alkyl, optionally substituted R²¹-((C₆-C₁₄)-aryl), (R²¹-((C₆-C₁₄)-aryl))-(C₁-C₈)-alkyl optionally substituted in the aryl radical, the radical Het-, Het-(C₁-C₈)-alkyl or one of the radicals R²¹O-, R²²O-NH-, R²¹O-N(R²³)-, R²⁴NH-, R²⁵N(R²⁵)-, HO-((C₁-C₈)- alkyl)-N(R²⁶)-, R²⁷C(O)-NH-, R²¹C(O)-N(R²³)-, R²¹C(O)-, R²¹O-C(O)-, R²⁸N(R²¹)-C(O)-, R²¹O-N=, O= and S=;
R² is hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, R⁴CO, COOR⁴, CON(CH₃)R⁴, CONHR⁴, CSNHR⁴, COOR¹⁵, CON(CH₃)R¹⁵ or CONHR¹⁵;
R⁴ is hydrogen or (C₁-C₂₈)-alkyl which can optionally be mono- or polysubstituted by identical or different radicals from the group consisting of hydroxyl, hydroxycarbonyl, aminocarbonyl, mono- or di- ((C₁-C₁₈)-alkyl)-aminocarbonyl, amino-(C₂-C₁₈)-alkylaminocarbonyl, amino-(C₁-C₃)-alkylphenyl-(C₁-C₃)-alkylaminocarbonyl, (C₁-C₁₈)-alkylcarbonylamino-(C₁-C₃)-alkylphenyl- (C₁-C₃)-alkylaminocarbonyl, (C₁-C₁₈)-alkylcarbonylamino- (C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, HOS(O)₂-(C₁-C₃)-alkyl, R⁹NHS(O)₂-(C₁-C₃)-alkyl, (R⁸O)₂P(O)-(C₁-C₃)-alkyl, tetrazolyl-(C₁-C₃)-alkyl, halogen, nitro, trifluoromethyl or the radical R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino and trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or reduced in the peptide bond to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkylcarbonyl, (C₁-C₁₈)-alkoxycarbonyl, (C₆-C₁₄)- arylcarbonyl, (C₆-C₁₄)-aryl-(C₁-C₈)-alkylcarbonyl or (C₆-C₁₄)-aryl-(C₁- C₁₈)-alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)- alkoxy, halogen, nitro, amino and trifluoromethyl, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or reduced in the peptide bond to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkylaminocarbonyl, (C₃-C₈)-cycloalkylaminocarbonyl, optionally substituted (C₆-C₁₄)-arylaminocarbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹¹ is hydrogen, (C₁-C₁₈)-alkyl, R¹²CO, R^{12a}CS, optionally substituted (C₆-C₁₄)-aryl-S(O)₂, (C₁-C₁₈)-alkyl-S(O)₂, (C₆-C₁₄)-aryl-(C₁-C₈)-alkyl optionally substituted in the aryl radical, R⁹NHS(O)₂ or the radical R¹⁵;
R¹² is hydrogen, (C₁-C₁₈)-alkyl, (C₂-C₈)-alkenyl, (C₂-C₈)-alkynyl, optionally substituted (C₆-C₁₄)-aryl, (C₁-C₁₈)-alkoxy, (C₆-C₁₄)-aryl- (C₁-C₈)-alkoxy which can also be substituted in the aryl radical, optionally substituted (C₆-C₁₄)-aryloxy, the radical R¹⁵, the radical R¹⁵-O-, amino, mono- or di-((C₁-C₁₈)-alkyl)amino, (C₂-C₈)- alkenylamino, (C₂-C₈)-alkynylamino, (C₃-C₁₂)-cycloalkylamino, (C₃-C₁₂)-cycloalkyl-(C₁-C₈)-alkylamino, the radical R¹⁵-NH-, optionally substituted (C₆-C₁₄)-aryl-NH, (C₆-C₁₄)-aryl-(C₁-C₈)-alkyl- NH, which can also be substituted in the aryl radical, optionally substituted heteroaryl-NH or heteroaryl-(C₁-C₈)-alkyl-NH optionally substituted in the heteroaryl radical;
R^{12a} is amino, mono- or di-((C₁-C₁₈)-alkyl)-amino, (C₂-C₈)-alkenylamino, (C₂-C₈)-alkynylamino, (C₃-C₁₂)-cycloalkylamino, (C₃-C₁₂)-cycloalkyl- (C₁-C₈)-alkylamino, the radical R¹⁵-NH-, optionally substituted (C₆-C₁₄)-aryl-NH, (C₆-C₁₄)-aryl-(C₁-C₈)-alkyl-NH, which can also be substituted in the aryl radical, optionally substituted heteroaryl-NH or heteroaryl-(C₁-C₈)-alkyl-NH optionally substituted in the heteroaryl radical;
R¹³ is hydrogen, (C₁-C₆)-alkyl, (C₆-C₁₄)-aryl-(C₁-C₈)-alkyl optionally substituted in the aryl radical or (C₃-C₈)-cycloalkyl;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
R²¹ is hydrogen, (C₁-C₈)-alkyl, (C₃-C₁₂)-cycloalkyl, (C₃-C₁₂)-cycloalkyl- (C₁-C₈)-alkyl, (C₆-C₁₄)-aryl, (C₆-C₁₄)-aryl-(C₁-C₈)-alkyl, the radical Het- or Het-(C₁-C₈)-alkyl, where alkyl radicals can be monosubstituted or polysubstituted by fluorine and the radicals R²¹ can be identical or different if they occur several times;
R²² is (C₃-C₁₂)-cycloalkyl, (C₃-C₁₂)-cycloalkyl-(C₁-C₈)-alkyl, (C₆-C₁₄)-aryl, the radical Het- or Het-(C₁-C₈)-alkyl, where alkyl radicals can be monosubstituted or polysubstituted by fluorine;
R²³ is (C₁-C₈)-alkyl, (C₃-C₁₂)-cycloalkyl, (C₃-C₁₂)-cycloalkyl-(C₁-C₈)-alkyl, (C₆-C₁₄)-aryl, (C₆-C₁₄)-aryl-(C₁-C₈)-alkyl, the radical Het- or Het- (C₁-C₈)-alkyl, where alkyl radicals can be monosubstituted or polysubstituted by fluorine;
R²⁴ (C₃-C₁₂)-cycloalkyl, (C₃-C₁₂)-cycloalkyl-(C₁-C₈)-alkyl, (C₆-C₁₄)-aryl, (C₆-C₁₄)-aryl-(C₁-C₈)-alkyl, the radical Het- or Het-(C₁-C₈)-alkyl, where alkyl radicals can be monosubstituted or polysubstituted by fluorine;
R²⁵ has the meanings of R²³, where the radicals R²⁵ can be identical or different;
R²⁶ has the meanings of R²¹ or HO-((C₁-C₈)-alkyl), where alkyl radicals can be monosubstituted or polysubstituted by fluorine;
R²⁷ is hydrogen, (C₃-C₁₂)-cycloalkyl, (C₃-C₁₂)-cycloalkyl-(C₁-C₈)-alkyl, (C₆-C₁₄)-aryl-(C₁-C₈)-alkyl, the radical Het- or Het-(C₁-C₈)-alkyl, where alkyl radicals can be monosubstituted or polysubstituted by fluorine;
R²⁸ is one of the radicals R²¹-, R²¹O-, R²⁶N(R²⁶)-, R²¹C(O)-, R²¹O-C(O)-, ((C₁-C₁₈)-alkyl-O-C(O)-((C₁-C₆)-alkyl)-O-C(O)-, R²¹N(R²¹)-C(O)-, R²¹N(R²¹)-C(=N(R²¹))- or R²¹C(O)-N(R²¹)-;
Het is a mono- or polycyclic, 4- to 14-membered, aromatic or nonaromatic ring which contains 1, 2, 3 or 4 identical or different heteroatoms from the group consisting of N, O and S as ring members and can optionally be substituted by one or more, identical or different substituents;
b, c, d and f independently of one another are 0 or 1, but cannot all simultaneously be 0;
e, g and h independently of one another are 0, 1, 2, 3, 4, 5 or 6;
in all their stereoisomeric forms and mixtures thereof in all ratios, and their physiologically tolerable salts for the production of pharmaceuticals for inhibition of the adhesion and/or migration of leucocytes or for inhibition of the VLA-4 receptor.

2. The use of compounds of the formula I as claimed in claim 1 in all their stereoisomeric forms and mixtures thereof in all ratios, and of their physiologically tolerable salts, where in the formula I
W is R¹-A-C(R¹³) or R¹-A-CH=C;
Y is a carbonyl, thiocarbonyl or methylene group;.
Z is N(R⁰), oxygen, sulfur or a methylene group;
A is a bivalent radical from the group consisting of (C₁-C₆)-alkylene, (C₃-C₁₂)-cycloalkylene, (C₁-C₆)-alkylene-(C₃-C₁₂)-cycloalkyl, phenylene, phenylene-(C₁-C₆)-alkyl, (C₁-C₆)-alkylenephenyl, (C₁-C₆)-alkylenephenyl-(C₁-C₆)-alkyl, phenylene-(C₂-C₆)-alkenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur;
B is a bivalent radical from the group consisting of (C₁-C₆)-alkylene, (C₂-C₆)-alkenylene, phenylene, phenylene-(C₁-C₃)-alkyl, (C₁-C₃)- alkylene-phenyl;
D is C(R²)(R³), N(R³) or CH=C(R³);
E is tetrazolyl, (R⁸O)₂P(O), HOS(O)₂, R⁹NHS(O)₂ or R¹⁰CO;
R and R⁰ independently of one another are hydrogen, (C₁-C₈)-alkyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical, where alkyl radicals can be mono- or polysubstituted by fluorine;
R¹ is hydrogen, (C₁-C₁₀)-alkyl, which can optionally be monosubstituted or polysubstituted by fluorine, (C₃-C₁₂)-cycloalkyl, (C₃-C₁₂)- cycloalkyl-(C₁-C₈)-alkyl, optionally substituted R²¹-((C₆-C₁₄-aryl), (R²¹-((C₆-C₁₄)-aryl))-(C₁-C₈)-alkyl optionally substituted in the aryl radical, the radical Het-, Het-(C₁-C₈)-alkyl or one of the radicals R²¹O-, R²²O-NH-, R²¹O-N(R²³)-, R²⁴NH-, R²⁵N(R²⁵)-, HO-((C₁-C₈)- alkyl)-N(R²⁶)-, R²⁷C(O)-NH-, R²¹C(O)-N(R²³)-, R²¹C(O)-, R²¹O-C(O)-, R²⁸N(R²¹)-C(O)-, R²¹O-N=, O= and S=;
R² is hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl, optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, R⁴CO, COOR⁴, CON(CH₃)R⁴, CONHR⁴, CSNHR⁴, COOR¹⁵, CON(CH₃)R¹⁵ or CONHR¹⁵;
R⁴ is hydrogen or (C₁-C₂₈)-alkyl which can optionally be mono- or polysubstituted by identical or different radicals from the group consisting of hydroxyl, hydroxycarbonyl, aminocarbonyl, mono- or di- ((C₁-C₁₈)-alkyl)-aminocarbonyl, amino-(C₂-C₁₈)-alkylaminocarbonyl, amino-(C₁-C₃)-alkylphenyl-(C₁-C₃)-alkylaminocarbonyl, (C₁-C₁₈)- alkylcarbonylamino-(C₁-C₃)-alkylphenyl-(C₁-C₃)-alkylaminocarbonyl, (C₁-C₁₈)-alkylcarbonylamino-(C₂-C₁₈)-alkylaminocarbonyl, (C₆-C₁₄)- aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, HOS(O)₂-(C₁-C₃)-alkyl, R⁹NHS(O)₂-(C₁-C₃)-alkyl, (R⁸O)₂P(O)-(C₁-C₃)-alkyl, tetrazolyl- (C₁-C₃)-alkyl, halogen, nitro, trifluoromethyl and the radical R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12- membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino or trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkylcarbonyl, (C₁-C₁₈)-alkoxycarbonyl, (C₆-C₁₄)- arylcarbonyl, (C₆-C₁₄)-aryl-(C₁-C₈)-alkylcarbonyl or (C₆-C₁₄)-aryl- (C₁-C₁₈)-alkyloxy carbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)-alkoxy, halogen, nitro, amino and trifluoromethyl, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkylaminocarbonyl, (C₃-C₈)-cycloalkylaminocarbonyl, optionally substituted (C₆-C₁₄)-arylaminocarbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹¹ is hydrogen, (C₁-C₁₈)-alkyl, R¹²CO, optionally substituted (C₆-C₁₄)- aryl-S(O)₂, (C₁-C₁₈)-alkyl-S(O)₂, (C₆-C₁₄)-aryl-(C₁-C₈)-alkyl optionally substituted in the aryl radical, R⁹NHS(O)₂ or the radical R¹⁵;
R¹² is hydrogen, (C₁-C₁₈)-alkyl, (C₂-C₈)-alkenyl, (C₂-C₈)-alkynyl, optionally substituted (C₆-C₁₄)-aryl, (C₁-C₁₈)-alkoxy, (C₆-C₁₄)-aryl- (C₁-C₈)-alkoxy which can also be substituted in the aryl radical, optionally substituted (C₆-C₁₄)-aryloxy, amino or mono- or di-((C₁- C₁₈)-alkyl)amino, the radical R¹⁵ or the radical R¹⁵-O-;
R¹³ is hydrogen, (C₁-C₆)-alkyl, (C₆-C₁₄)-aryl-(C₁-C₈)-alkyl optionally substituted in the aryl radical or (C₃-C₈)-cycloalkyl;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
R²¹ is hydrogen, (C₁-C₈)-alkyl, (C₃-C₁₂)-cycloalkyl, (C₃-C₁₂)-cycloalkyl- (C₁-C₈)-alkyl, (C₆-C₁₄)-aryl, (C₆-C₁₄)-aryl-(C₁-C₈)-alkyl, the radical Het- or Het-(C₁-C₈)-alkyl, where alkyl radicals can be monosubstituted or polysubstituted by fluorine and the radicals R²¹ can be identical or different if they occur several times;
R²² is (C₃-C₁₂)-cycloalkyl, (C₃-C₁₂)-cycloalkyl-(C₁-C₈)-alkyl, (C₆-C₁₄)-aryl, the radical Het- or Het-(C₁-C₈)-alkyl, where alkyl radicals can be monosubstituted or polysubstituted by fluorine;
R²³ is (C₁-C₈)-alkyl, (C₃-C₁₂)-cycloalkyl, (C₃-C₁₂)-cycloalkyl-(C₁-C₈)-alkyl, (C₆-C₁₄)-aryl, (C₆-C₁₄)-aryl-(C₁-C₈)-alkyl, the radical Het- or Het- (C₁-C₈)-alkyl, where alkyl radicals can be monosubstituted or polysubstituted by fluorine;
R²⁴ (C₃-C₁₂)-cycloalkyl, (C₃-C₁₂)-cycloalkyl-(C₁-C₈)-alkyl, (C₆-C₁₄)-aryl, (C₆-C₁₄)-aryl-(C₁-C₈)-alkyl, the radical Het- or Het-(C₁-C₈)-alkyl, where alkyl radicals can be monosubstituted or polysubstituted by fluorine;
R²⁵ has the meanings of R²³, where the radicals R²⁵ can be identical or different;
R²⁶ has the meanings of R²¹ or HO-((C₁-C₈)-alkyl), where alkyl radicals can be monosubstituted or polysubstituted by fluorine;
R²⁷ is hydrogen, (C₃-C₁₂)-cycloalkyl, (C₃-C₁₂)-cycloalkyl-(C₁-C₈)-alkyl, (C₆-C₁₄)-aryl-(C₁-C₈)-alkyl, the radical Het- or Het-(C₁-C₈)-alkyl, where alkyl radicals can be monosubstituted or polysubstituted by fluorine;
R²⁸ is one of the radicals R²¹-, R²¹O-, R²⁶N(R²⁶)-, R²¹C(O)-, R²¹O-C(O)-, ((C₁-C₁₈)-alkyl-O-C(O)-((C₁-C₆)-alkyl)-O-C(O)-, R²¹N(R²¹)-C(O)-, R²¹N(R²¹)-C(=N(R²¹))- or R²¹C(O)-N(R²¹)-;
Het is a mono- or polycyclic, 4- to 14-membered, aromatic or nonaromatic ring which contains 1, 2, 3 or 4 identical or different heteroatoms from the group consisting of N, O and S as ring members and can optionally be substituted by one or more, identical or different substituents;
b, c, d and f independently of one another are 0 or 1, but cannot all simultaneously be 0;
e, g and h independently of one another are 0, 1, 2, 3, 4, 5 or 6.

3. The use of compounds of the formula I as claimed in claim 1 and/or 2 in all their stereoisomeric forms and mixtures thereof in all ratios, and of their physiologically tolerable salts, where in the formula I R⁰ is (C₁-C₈)-alkyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical, preferably biphenylylmethyl, naphthylmethyl or benzyl, each of which is unsubstituted or monosubstituted or polysubstituted in the aryl radical.

4. The use of compounds of the formula I as claimed in claim 1 and/or 3 in all their stereoisomeric forms and mixtures thereof in all ratios, and of their physiologically tolerable salts, where in the formula I simultaneously W is R¹-A-CH=C and therein A is a phenylene radical or a methylenephenyl radical or W is R¹-A-C(R¹³) and therein A is a bivalent radical from the group consisting of methylene, ethylene, trimethylene, tetramethylene, pentamethylene, cyclohexylene, phenylene, phenylenemethyl, methylenephenyl, methylenephenylmethyl;
B is a bivalent radical from the group consisting of methylene, ethylene, trimethylene, tetramethylene, vinylene, phenylene, or substituted methylene or ethylene;
E is R¹⁰CO;
R is hydrogen, (C₁-C₆)-alkyl or benzyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is one of the radicals R²¹O-, R²⁴NH-, R²⁵N(R²⁵)-, HO-((C₁-C₈)-alkyl- N(R²⁶)-, R²¹O-C(O)- and R²⁸N(R²¹)-C(O)-;
R² is hydrogen or (C₁-C₈)-alkyl;
R³ is (C₁-C₈)-alkyl, optionally substituted (C₆-C₁₄)-aryl, (C₆-C₁₄)-aryl-(C₁-C₈)-alkyl, (C₃-C₈)-cycloalkyl, (C₂-C₈)-alkenyl, (C₂-C₈)-alkynyl, pyridyl,
R¹¹NH, R⁴CO, COOR⁴, CONHR⁴, CSNHR⁴, COOR¹⁵ and CONHR¹⁵;
and e, g and h independently of one another are the numbers 0, 1, 2 or 3.

5. The use of compounds of the formula I as claimed in one or more of claims 1 to 4 in all their stereoisomeric forms and mixtures thereof in all ratios, and of their physiologically tolerable salts, where in the formula I W is R¹-A-C(R¹³) and R¹³ is (C₁-C₆)-alkyl, (C₆-C₁₄)-aryl-(C₁-C₈)-alkyl optionally substituted in the aryl radical or (C₃-C₈)-cycloalkyl.

6. The use of compounds of the formula I as claimed in one or more of claims 1 to 5 in all their stereoisomeric forms and mixtures thereof in all ratios, and of their physiologically tolerable salts, where in the formula I R³ is optionally substituted (C₆-C₁₄)-aryl, COOR⁴, R¹¹NH or CONHR⁴, where -NHR⁴ is the radical of an α-amino acid, its ω-amino-(C₂-C₈)-alkylamide, its (C₁-C₈)-alkyl ester or its (C₆-C₁₄)-aryl-(C₁-C₄)-alkyl ester, where -NHR⁴ is preferably the radical of the α-amino acids valine, lysine, phenylglycine, phenylalanine or tryptophan or their (C₁-C₈)-alkyl esters or (C₆-C₁₄)-aryl-(C₁-C₄)-alkyl esters.

7. The use of compounds of the formula I as claimed in one or more of claims 1 and 3 to 6 in all their stereoisomeric forms and mixtures thereof in all ratios, and of their physiologically tolerable salts, where in the formula I simultaneously
W is R¹-A-C(R¹³);
Y is a carbonyl group;
Z is N(R⁰);
A is ethylene, trimethylene, tetramethylene, pentamethylene, cyclohexylene, phenylene, phenylenemethyl, methylenephenyl or methylenephenylmethyl;
B is an unsubstituted or substituted methylene radical;
D is C(R²)(R³);
E is R¹⁰CO;
R is hydrogen or (C₁-C₄)-alkyl, in particular hydrogen, methyl or ethyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is the radical R²⁸N(R²¹)-C(O)-;
R² is hydrogen;
R³ is the radical CONHR⁴;
R⁴ is methyl which is substituted by hydroxycarbonyl and a radical from the group consisting of (C₁-C₄)-alkyl, phenyl and benzyl, or is methyl which is substituted by (C₁-C₈)-alkoxycarbonyl, preferably (C₁-C₄)-alkoxycarbonyl, and a radical from the group consisting of (C₁-C₄)-alkyl, phenyl and benzyl;
R¹⁰ is hydroxyl or (C₁-C₈)-alkoxy, preferably (C₁-C₄)-alkoxy;
R¹³ is (C₁-C₆)-alkyl, (C₃-C₇)-cycloalkyl or benzyl, in particular methyl;
b, c and d are 1 and e, f and g are 0;
h is 1 or 2; preferably 1.

8. The use of compounds of the formula I as claimed in one or more of claims 1, 3 and 4 in all their stereoisomeric forms and mixtures thereof in all ratios, and of their physiologically tolerable salts, where in the formula I simultaneously
W is R¹-A-CH=C and therein A is a phenylene radical or a methylenephenyl radical or W is R¹-A-C(R¹³) and therein A is a bivalent radical from the group consisting of methylene, ethylene, trimethylene, tetramethylene, pentamethylene, cyclohexylene, phenylene, phenylenemethyl, methylenephenyl, methylenephenylmethyl;
B is a bivalent radical from the group consisting of methylene, ethylene, trimethylene, tetramethylene, vinylene, phenylene or substituted methylene or ethylene;
E is R¹⁰ CO;
R is hydrogen or (C₁-C₆)-alkyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is one of the radicals R²¹O-, R²¹O-N(R²³)-, R²⁴NH-, R²⁵N(R²⁵)-, HO-((C₁-C₈)-alkyl-N(R²⁶)-, R²¹O-C(O)- and R²⁸N(R²¹)-C(O)-;
R² is hydrogen or (C₁-C₈)-alkyl;
R³ is CONHR¹⁵ or CONHR⁴ where R⁴ herein is a (C₁-C₈)-alkyl radical which is unsubstituted or substituted by one or more (C₆-C₁₄)-aryl radicals;
R¹⁵ is R¹⁶-(C₁-C₆)-alkyl or R¹⁶, where R¹⁶ is a 7- to 12-membered bridged bicyclic or tricyclic radical which is saturated or partially unsaturated and
which can also contain one to four identical or different heteroatoms from the group consisting of nitrogen, oxygen and sulfur and which can also be substituted by one or more identical or different substituents from the group consisting of (C₁-C₄)-alkyl and oxo, and in particular R¹⁵ is an adamantyl radical or an adamantylmethyl radical;
and e, g and h independently of one another are the numbers 0, 1, 2 or 3 and b, c and d are 1.

9. The use of compounds of the formula I as claimed in one or more of claims 1, 3, 4, 5 and 8 in all their stereoisomeric forms and mixtures thereof in all ratios, and of their physiologically tolerable salts, where in the formula I simultaneously
W is R¹-A-C(R¹³);
Y is a carbonyl group;
Z is N(R⁰);
A is ethylene, trimethylene, tetramethylene, pentamethylene, cyclohexylene, phenylene, phenylenemethyl, methylenephenyl or methylenephenylmethyl;
B is an unsubstituted or substituted methylene radical;
D is C(R²)(R³);
E is R¹⁰CO;
R is hydrogen or (C₁-C₄)-alkyl, in particular hydrogen, methyl or ethyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is the radical R²⁸N(R²¹)-C(O)-;
R² is hydrogen;
R³ is CONHR¹⁵ or CONHR⁴ where R⁴ herein is a (C₁-C₆)-alkyl radical which is unsubstituted or substituted by one or more (C₆-C₁₀)-aryl radicals;
R¹⁰ is hydroxyl or (C₁-C₈)-alkoxyl, preferably (C₁-C₄)-alkoxy;
R¹³ is (C₁-C₆)-alkyl, (C₃-C₇)-cycloalkyl or benzyl, in particular methyl;
R¹⁵ is an adamantyl radical or an adamantylmethyl radical;
b, c and d are 1 and e, f and g are 0;
h is 1 or 2, preferably 1.

10. The use of compounds of the formula I as claimed in one or more of claims 1, 3, 4 and 5 in all their stereoisomeric forms and mixtures thereof in all ratios, and of their physiologically tolerable salts, where in the formula I simultaneously
W is R¹-A-C(R¹³);
Y is a carbonyl group;
Z is N(R⁰);
A is ethylene, trimethylene, tetramethylene, pentamethylene, cyclohexylene, phenylene, phenylenemethyl, methylenephenyl, methylenephenylmethyl;
B is an unsubstituted or substituted methylene radical or ethylene radical;
D is C(R²)(R³);
E is R¹⁰CO;
R is hydrogen or (C₁-C₄)-alkyl, in particular hydrogen, methyl or ethyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl which is optionally substituted in the aryl radical;
R¹ is one of the radicals R²¹O-, R²⁴NH-, R²⁵N(R²⁵)-, HO-((C₁-C₈)-alkyl)-N(R²⁶)-, R²¹O-C(O)- and R²⁸N(R²¹)-C(O)-;
R² is hydrogen;
R³ is an unsubstituted phenyl or naphthyl radical, a phenyl radical or naphthyl radical substituted by one, two or three identical or different radicals from the group consisting of (C₁-C₄)-alkyl, (C₁-C₄)-alkoxy, hydroxyl, halogen, trifluoromethyl, nitro, methylenedioxy, ethylenedioxy, hydroxycarbonyl, (C₁-C₄)-alkoxycarbonyl, aminocarbonyl, cyano, phenyl, phenoxy and benzyloxy, a pyridyl radical, a (C₁-C₄)-alkyl radical, a (C₂-C₄)-alkenyl radical, a (C₂-C₄)-alkynyl radical or a (C₅-C₆)-cycloalkyl radical, and in particular R³ is an unsubstituted or substituted phenyl radical or naphthyl radical;
R¹⁰ is hydroxyl or (C₁-C₈)-alkoxy, in particular (C₁-C₄)-alkoxy, and preferably R¹⁰ is a radical from the group consisting of hydroxyl, methoxy, ethoxy, propoxy and isopropoxy;
R¹³ is (C₁-C₆)-alkyl, (C₃-C₇)-cycloalkyl or benzyl, in particular methyl;
b, c and d are 1 and e, f and g are 0;
h is 1 or 2, preferably 1.

11. The use of compounds of the formula I as claimed in one or more of claims 1, 3, 4 and 5 in all their stereoisomeric forms and mixtures thereof in all ratios, and of their physiologically tolerable salts, where in the formula I simultaneously
W is R¹-A-C(R¹³);
Y is a carbonyl group;
Z is N(R⁰);
A is ethylene, trimethylene, tetramethylene, pentamethylene, cyclohexylene, phenylene, phenylenemethyl, methylenephenyl, methylenephenylmethyl;
B is an unsubstituted or substituted methylene radical or ethylene radical;
D is C(R²)(R³);
E is R¹⁰CO;
R is hydrogen or (C₁-C₄)-alkyl, in particular hydrogen, methyl or ethyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is one of the radicals R²¹O-, R²⁴NH-, R²⁵N(R²⁵)-, HO-((C₁-C₈)-alkyl)-N(R²⁶)-, R²¹O-C(O)- and R²⁸N(R²¹)-C(O)-;
R² is hydrogen;
R³ is R¹¹NH;
R¹⁰ is hydroxyl or (C₁-C₈)-alkoxy, in particular (C₁-C₄)-alkoxy, and preferably R¹⁰ is a radical from the group consisting of hydroxyl, methoxy, ethoxy, propoxy and isopropoxy;
R¹³ is (C₁-C₆)-alkyl, (C₃-C₇)-cycloalkyl or benzyl, in particular methyl;
b, c, d and e are 1 and f and g are 0;
h is 0.

12. The use of compounds of the formula I as claimed in one or more of claims 1 and 3 to 11 in all their stereoisomeric forms and mixtures thereof in all ratios, and of their physiologically tolerable salts, in which a substituted methylene radical or substituted ethylene radical representing the group B carries as a substituent a radical from the group consisting of (C₁-C₈)-alkyl, (C₂-C₆)-alkenyl, (C₂-C₆)-alkynyl, (C₃-C₈)-cycloalkyl, in particular (C₅-C₆)-cycloalkyl, (C₃-C₈)-cycloalkyl-(C₁-C₄)-alkyl, in particular (C₅-C₆)-cycloalkyl-(C₁-C₄)-alkyl, optionally substituted (C₆-C₁₀)-aryl, (C₆-C₁₀)-aryl-(C₁-C₄)-alkyl optionally substituted in the aryl radical, optionally substituted heteroaryl and heteroaryl- (C₁-C₄)-alkyl optionally substituted in the heteroaryl radical.

13. The use of compounds of the formula I as claimed in one or more of claims 1 and 3 to 12 in all their stereoisomeric forms and mixtures thereof in all ratios, and of their physiologically tolerable salts, in which B is an unsubstituted methylene radical or a methylene radical which is substituted by a (C₁-C₈)-alkyl radical.

14. The use of compounds of the formula I as claimed in one or more of claims 1 to 13 and/or of their physiologically tolerable salts for the production of pharmaceuticals for the suppression of inflammation.

15. The use of compounds of the formula I as claimed in one or more of claims 1 to 13 and/or of their physiologically tolerable salts for the production of pharmaceuticals for the treatment or prophylaxis of rheumatoid arthritis, inflammatory bowel disease, systemic lupus erythematosus or inflammatory disorders of the central nervous system.

16. The use of compounds of the formula I as claimed in one or more of claims 1 to 13 and/or of their physiologically tolerable salts for the production of pharmaceuticals for the treatment or prophylaxis of asthma or allergies.

17. The use of compounds of the formula I as claimed in one or more of claims 1 to 13 and/or their physiologically tolerable salts for the production of pharmaceuticals for the treatment or prophylaxis of cardiovascular disorders, arteriosclerosis, restenoses or diabetes, for the prevention of damage to organ transplants, for the inhibition of tumor growth or tumor metastasis or for the therapy of malaria.

18. The use of compounds of the formula I as claimed in one or more of claims 1 to 13 and/or their physiologically tolerable salts for inhibition of the adhesion and/or migration of leucocytes or for inhibition of the VLA-4 receptor.

19. The use of compounds of the formula I as claimed in one or more of claims 1 to 13 and/or their physiologically tolerable salts for the treatment or prophylaxis of illnesses in which leucocyte adhesion and/or leucocyte migration has an undesired extent, or of illnesses in which VLA-4-dependent adhesion processes play a part, for the treatment or prophylaxis of rheumatoid arthritis, inflammatory bowel disease, systemic lupus erythematosus, inflammatory disorders of the central nervous system, asthma, allergies, cardiovascular disorders, arteriosclerosis, restenoses, diabetes, for the prevention of damage to organ transplants, for inhibition of tumor growth or tumor metastasis, for the therapy of malaria, or as antiinflammatory agents.

20. A compound of the formula Ib 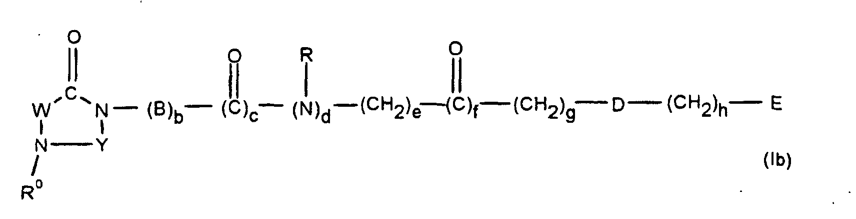 in which
W is R¹-A-C(R¹³) or R¹-A-CH=C;
Y is a carbonyl, thiocarbonyl or methylene group;
A is a bivalent radical from the group consisting of (C₁-C₆)-alkylene, (C₃-C₁₂)-cycloalkylene, (C₁-C₆)-alkylene-(C₃-C₁₂)-cycloalkyl, phenylene, phenylene-(C₁-C₆)-alkyl, (C₁-C₆)-alkylenephenyl, (C₁-C₆)-alkylenephenyl-(C₁-C₆)-alkyl, phenylene-(C₂-C₆)-alkenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur;
B is a bivalent radical from the group consisting of (C₁-C₆)-alkylene, (C₂-C₆)-alkenylene, phenylene, phenylene-(C₁-C₃)-alkyl, (C₁-C₃)-alkylenephenyl;
D is C(R²)(R³), N(R³) or CH=C(R³);
E is tetrazolyl, (R⁸O)₂P(O), HOS(O)₂, R⁹NHS(O)₂ or R¹⁰CO;
R and R⁰ independently of one another are hydrogen, (C₁-C₈)-alkyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical where alkyl radicals can be mono- or polysubstituted by fluorine;
R¹ is the radical R²⁸N(R²¹)-C(O)-;
R² is hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)- alkenylcarbonyl, (C₂-C₈)-alkynylcarbonyl, pyridyl, R¹¹NH, R⁴CO, COOR⁴, CON(CH₃)R⁴, CONHR⁴, CSNHR⁴, COOR¹⁵, CON(CH₃)R¹⁵ or CONHR¹⁵;
R⁴ is hydrogen or (C₁-C₂₈)-alkyl which can optionally be mono- or polysubstituted by identical or different radicals from the group consisting of hydroxyl, hydroxycarbonyl, aminocarbonyl, mono- or di- ((C₁-C₁₈)-alkyl)aminocarbonyl, amino-(C₂-C₁₈)-alkylaminocarbonyl, amino-(C₁-C₃)-alkylphenyl-(C₁-C₃)-alkylaminocarbonyl, (C₁-C₁₈)-alkylcarbonylamino-(C₁-C₃)-alkylphenyl- (C₁-C₃)-alkylaminocarbonyl, (C₁-C₁₈)-alkylcarbonylamino- (C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, HOS(O)₂-(C₁-C₃)-alkyl, R⁹NHS(O)₂-(C₁-C₃)-alkyl, (R⁸O)₂P(O)-(C₁-C₃)-alkyl, tetrazolyl-(C₁-C₃)-alkyl, halogen, nitro, trifluoromethyl and the radical R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino and trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkylcarbonyl, (C₁-C₁₈)-alkoxycarbonyl, (C₆-C₁₄)- arylcarbonyl, (C₆-C₁₄)-aryl-(C₁-C₈)-alkylcarbonyl or (C₆-C₁₄)-aryl- (C₁-C₁₈)-alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)-alkoxy, halogen, nitro, amino and trifluoromethyl, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkylaminocarbonyl, (C₃-C₈)-cycloalkylaminocarbonyl, optionally substituted (C₆-C₁₄)-arylaminocarbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹¹ is hydrogen, (C₁-C₁₈)-alkyl, R¹²CO, R^{12a}CS, optionally substituted (C₆-C₁₄)-aryl-S(O)₂, (C₁-C₁₈)-alkyl-S(O)₂, (C₆-C₁₄)-aryl-(C₁-C₈)-alkyl optionally substituted in the aryl radical, R⁹NHS(O)₂ or the radical R¹⁵;
R¹² is hydrogen, (C₁-C₁₈)-alkyl, (C₂-C₈)-alkenyl, (C₂-C₈)-alkynyl, optionally substituted (C₆-C₁₄)-aryl, (C₁-C₁₈)-alkoxy, (C₆-C₁₄)-aryl- (C₁-C₈)-alkoxy, which can also be substituted in the aryl radical, optionally substituted (C₆-C₁₄)-aryloxy, the radical R¹⁵, the radical R¹⁵-O-, amino, mono- or di-((C₁-C₁₈)-alkyl)amino, (C₂-C₈)- alkenylamino, (C₂-C₈)-alkynylamino, (C₃-C₁₂)-cycloalkylamino, (C₃-C₁₂)-cycloalkyl-(C₁-C₈)-alkylamino, the radical R¹⁵-NH-, optionally substituted (C₆-C₁₄)-aryl-NH, (C₆-C₁₄)-aryl-(C₁-C₈)-alkyl- NH, which can also be substituted in the aryl radical, optionally substituted heteroaryl-NH or heteroaryl-(C₁-C₈)-alkyl-NH optionally substituted in the heteroaryl radical;
R^{12a} is amino, mono- or di-((C₁-C₁₈)-alkyl)amino, (C₂-C₈)-alkenylamino, (C₂-C₈)-alkynylamino, (C₃-C₁₂)-cycloalkylamino, (C₃-C₁₂)-cycloalkyl- (C₁-C₈)-alkylamino, the radical R¹⁵-NH-, optionally substituted (C₆-C₁₄)aryl-NH, (C₆-C₁₄)-aryl-(C₁-C₈)-alkyl-NH, which can also be substituted in the aryl radical, optionally substituted heteroaryl-NH or heteroaryl-(C₁-C₈)-alkyl-NH optionally substituted in the heteroaryl radical;
R¹³ is hydrogen, (C₁-C₆)-alkyl, (C₆-C₁₄)-aryl-(C₁-C₈)-alkyl optionally substituted in the aryl radical or (C₃-C₈)-cycloalkyl;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
R²¹ is hydrogen, (C₁-C₈)-alkyl, (C₃-C₁₂)-cycloalkyl, (C₃-C₁₂)-cycloalkyl- (C₁-C₈)-alkyl, (C₆-C₁₄)-aryl, (C₆-C₁₄)-aryl-(C₁-C₈)-alkyl, the radical Het- or Het-(C₁-C₈)-alkyl, where alkyl radicals can be monosubstituted or polysubstituted by fluorine and the radicals R²¹ can be identical or different if they occur several times;
R²⁶ has the meanings of R²¹ or HO-((C₁-C₈)-alkyl), where alkyl radicals can be monosubstituted or polysubstituted by fluorine;
R²⁸ is one of the radicals R²¹-, R²¹O-, R²⁶N(R²⁶)-, R²¹C(O)-, R²¹O-C(O)-, ((C₁-C₁₈)-alkyl-O-C(O)-((C₁-C₆)-alkyl)-O-C(O)-, R²¹N(R²¹)-C(O)-, R²¹N(R²¹)-C(=N(R²¹))- or R²¹C(O)-N(R²¹)-;
Het is a mono- or polycyclic, 4- to 14-membered, aromatic or nonaromatic ring which contains 1, 2, 3 or 4 identical or different heteroatoms from the group consisting of N, O and S as ring members and can optionally be substituted by one or more, identical or different substituents:
b, c, d and f independently of one another can be 0 or 1, but cannot all simultaneously be 0;
e, g and h independently of one another are the numbers 0, 1, 2, 3, 4, 5 or 6;
in all its stereoisomeric forms and mixtures thereof in all ratios, or its physiologically tolerable salts.

21. A compound of the formula Ib as claimed in claim 20 and/or its physiologically tolerable salts for use as a pharmaceutical.

22. A pharmaceutical preparation, which comprises one or more compounds of the formula lb as claimed in claim 20 and/or their physiologically tolerable salts in addition to pharmaceutically innocuous excipients and/or additives.

23. A compound of the formula Ic 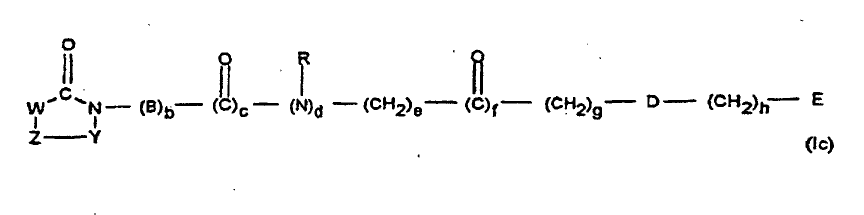 in which
W is R¹-A-C(R¹³) or R¹-A-CH=C;
Y is a carbonyl, thiocarbonyl or methylene group;
Z is N(R⁰), oxygen, sulfur or a methylene group;
A is a bivalent (C₁-C₆)-alkylene radical;
B is a bivalent radical from the group consisting of (C₁-C₆)-alkylene, (C₂-C₆)-alkenylene, phenylene, phenylene-(C₁-C₃)-alkyl, (C₁-C₃)-alkylenephenyl;
D is C(R²)(R³), N(R³) or CH=C(R³);
E is tetrazolyl, (R⁸O)₂P(O), HOS(O)₂, R⁹NHS(O)₂ or R¹⁰CO;
R and R⁰ independently of one another are hydrogen, (C₁-C₈)-alkyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical where alkyl radicals can be mono- or polysubstituted by fluorine;
R¹ is hydrogen, (C₁-C₁₀)-alkyl, which can optionally be monosubstituted or polysubstituted by fluorine, or one of the radicals R⁴¹O-, R⁴¹O- N(R⁴²)-, R⁴²N(R⁴²)-, HO-((C₁-C₈)-alkyl)-N(R⁴³)-, HC(O)-NH-, R⁴¹C(O)- N(R⁴²)-, R⁴¹C(O)-, R⁴¹O-C(O)-, R⁴⁴N(R⁴¹)-C(O)-, R⁴¹O-N=, O= and S=;
R² is hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, R⁴CO, COOR⁴, CON(CH₃)R⁴, CONHR⁴, CSNHR⁴, COOR¹⁵, CON(CH₃)R¹⁵ or CONHR¹⁵;
R⁴ is hydrogen or (C₁-C₂₈)-alkyl which can optionally be mono- or polysubstituted by identical or different radicals from the group consisting of hydroxyl, hydroxycarbonyl, aminocarbonyl, mono- or di- ((C₁-C₁₈)-alkyl)aminocarbonyl, amino-(C₂-C₁₈)-alkylaminocarbonyl, amino-(C₁-C₃)-alkylphenyl-(C₁-C₃)-alkylaminocarbonyl, (C₁-C₁₈)-alkylcarbonylamino-(C₁-C₃)-alkylphenyl- (C₁-C₃)-alkylaminocarbonyl, (C₁-C₁₈)-alkylcarbonylamino- (C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, HOS(O)₂-(C₁-C₃)-alkyl, R⁹NHS(O)₂-(C₁-C₃)-alkyl, (R⁸O)₂P(O)-(C₁-C₃)-alkyl, tetrazolyl-(C₁-C₃)-alkyl, halogen, nitro, trifluoromethyl and the radical R⁵ ;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12- membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino or trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkylcarbonyl, (C₁-C₁₈)-alkoxycarbonyl, (C₆-C₁₄)- arylcarbonyl, (C₆-C₁₄)-aryl-(C₁-C₈)-alkylcarbonyl or (C₆-C₁₄)-aryl- (C₁-C₁₈)-alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)-alkoxy, halogen, nitro, amino and trifluoromethyl, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkylaminocarbonyl, (C₃-C₈)-cycloalkylaminocarbonyl, optionally substituted (C₆-C₁₄)-arylaminocarbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹¹ is hydrogen, (C₁-C₁₈)-alkyl, R¹²CO, R^{12a}CS, optionally substituted (C₆-C₁₄)-aryl-S(O)₂, (C₁-C₁₈)-alkyl-S(O)₂, (C₆-C₁₄)-aryl-(C₁-C₈)-alkyl optionally substituted in the aryl radical, R⁹NHS(O)₂ or the radical R¹⁵;
R¹² is hydrogen, (C₁-C₁₈)-alkyl, (C₂-C₈)-alkenyl, (C₂-C₈)-alkynyl, optionally substituted (C₆-C₁₄)-aryl, (C₁-C₁₈)-alkoxy, (C₆-C₁₄)-aryl- (C₁-C₈)-alkoxy which can also be substituted in the aryl radical, optionally substituted (C₆-C₁₄)-aryloxy, the radical R¹⁵, the radical R¹⁵-O-, amino, mono- or di-((C₁-C₁₈)-alkyl)amino, (C₂-C₈)- alkenylamino, (C₂-C₈)-alkynylamino, (C₃-C₁₂)-cycloalkylamino, (C₃-C₁₂)-cycloalkyl-(C₁-C₈)-alkylamino, the radical R¹⁵-NH-, optionally substituted (C₆-C₁₄)-aryl-NH, (C₆-C₁₄)-aryl-(C₁-C₈)-alkyl- NH which can also be substituted in the aryl radical, optionally substituted heteroaryl-NH or heteroaryl-(C₁-C₈)-alkyl-NH optionally substituted in the heteroaryl radical;
R^{12a} is amino, mono- or di-((C₁-C₁₈)-alkyl)amino, (C₂-C₈)-alkenylamino, (C₂-C₈)-alkynylamino, (C₃-C₁₂)-cycloalkylamino, (C₃-C₁₂)-cycloalkyl- (C₁-C₈)-alkylamino, the radical R¹⁵-NH-, optionally substituted (C₆-C₁₄)-aryl-NH, (C₆-C₁₄)-aryl-(C₁-C₈)-alkyl-NH, which can also be substituted in the aryl radical, optionally substituted heteroaryl-NH or heteroaryl-(C₁-C₈)-alkyl-NH optionally substituted in the heteroaryl radical;
R¹³ is hydrogen, (C₁-C₆)-alkyl, (C₆-C₁₄)-aryl-(C₁-C₈)-alkyl optionally substituted in the aryl radical or (C₃-C₈)-cycloalkyl;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
R⁴¹ is hydrogen or (C₁-C₈)-alkyl, where alkyl radicals can be monosubstituted or polysubstituted by fluorine and the radicals R⁴¹ can be identical or different if they occur several times;
R⁴² is (C₁-C₈)-alkyl, where alkyl radicals can be monosubstituted or polysubstituted by fluorine and the radicals R⁴² can be identical or different if they occur several times;
R⁴³ has the meanings of R⁴¹ or is HO-((C₁-C₈)-alkyl), where alkyl radicals can be monosubstituted or polysubstituted by fluorine;
R⁴⁴ is one of the radicals R⁴¹-, R⁴¹O-, R⁴³N(R⁴³)-, R⁴¹C(O)-, R⁴¹O-C(O)-, ((C₁-C₁₈)-alkyl-O-C(O)-((C₁-C₆)-alkyl)-O-C(O)-, R⁴¹N(R⁴¹)-C(O)-, R⁴¹N(R⁴¹)-C(=N(R⁴¹))- or R⁴¹C(O)-N(R⁴¹)-;
b, c, d and f independently of one another are 0 or 1, but cannot all simultaneously be 0;
e, g and h independently of one another are 0, 1, 2, 3, 4, 5 or 6;
in all its stereoisomeric forms and mixtures thereof in all ratios, or its physiologically tolerable salts.

24. A compound of the formula Ic as claimed in claim 23 and/or its physiologically tolerable salts for use as a pharmaceutical.

25. A pharmaceutical preparation, which comprises one or more compounds of the formula Ic as claimed in claim 23 and/or its physiologically tolerable salts in addition to pharmaceutically innocuous excipients and/or additives.

26. A compound of the formula Id 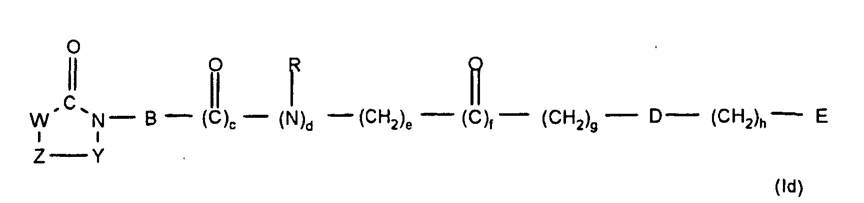 in which
W is R¹-A-C(R¹³) or R¹-A-CH=C;
Y is a carbonyl, thiocarbonyl or methylene group;
Z is N(R⁰), oxygen, sulfur or a methylene group;
A is a bivalent radical from the group consisting of (C₁-C₆)-alkylene, (C₃-C₁₂)-cycloalkylene, (C₁-C₆)-alkylene-(C₃-C₁₂)-cycloalkyl, phenylene, phenylene-(C₁-C₆)-alkyl, (C₁-C₆)-alkylenephenyl, (C₁-C₆)-alkylenephenyl-(C₁-C₆)-alkyl, phenylene-(C₂-C₆)-alkenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur;
B is a bivalent (C₁-C₆)-alkylene radical which is substituted by a radical from the group consisting of (C₁-C₈)-alkyl, (C₂-C₈)-alkenyl, (C₂-C₈)-alkynyl, (C₃-C₁₀)-cycloalkyl, (C₃-C₁₀)-cycloalkyl-(C₁-C₆)-alkyl, optionally substituted (C₆-C₁₄)-aryl, (C₆-C₁₄)-aryl-(C₁-C₆)-alkyl optionally substituted in the aryl radical, optionally substituted heteroaryl and heteroaryl-(C₁-C₆)-alkyl optionally substituted in the heteroaryl radical;
D is C(R²)(R³), N(R³) or CH=C(R³);
E is tetrazolyl, (R⁸O)₂P(O), HOS(O)₂, R⁹NHS(O)₂ or R¹⁰CO;
R is hydrogen, (C₁-C₈)-alkyl, (C₃-C₁₂)-cycloalkyl, (C₃-C₁₂)-cycloalkyl- (C₁-C₈)-alkyl, optionally substituted (C₆-C₁₄)-aryl, (C₆-C₁₄)-aryl-(C₁- C₈)-alkyl optionally substituted in the aryl radical, optionally substituted heteroaryl or heteroaryl-(C₁-C₈)-alkyl optionally substituted in the heteroaryl radical, where alkyl radicals can be mono- or polysubstituted by fluorine;
R⁰ is hydrogen,(C₁-C₈)-alkyl, (C₃-C₁₂)-cycloalkyl, (C₃-C₁₂)-cycloalkyl- (C₁-C₈)-alkyl, (C₆-C₁₂)-bicycloalkyl, (C₆-C₁₂)-bicycloalkyl-(C₁-C₈)- alkyl, (C₆-C₁₂)-tricycloalkyl, (C₆-C₁₂)-tricycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl, heteroaryl-(C₁-C₈)-alkyl optionally substituted in the heteroaryl radical, CHO, (C₁-C₈)-alkyl-CO, (C₃-C₁₂)-cycloalkyl-CO, (C₃-C₁₂)-cycloalkyl-(C₁-C₈)-alkyl-CO, (C₆-C₁₂)-bicycloalkyl-CO, (C₆-C₁₂)-bicycloalkyl-(C₁-C₈)-alkyl-CO, (C₆-C₁₂)-tricycloalkyl-CO, (C₆-C₁₂)-tricycloalkyl-(C₁-C₈)-alkyl-CO, optionally substituted (C₆-C₁₄)-aryl-CO, (C₆-C₁₄)-aryl-(C₁-C₈)-alkyl-CO optionally substituted in the aryl radical, optionally substituted heteroaryl-CO, heteroaryl-(C₁-C₈)-alkyl-CO optionally substituted in the heteroaryl radical, (C₁-C₈)-alkyl-S(O)ₙ, (C₃-C₁₂)-cycloalkyl-S(O)ₙ, (C₃-C₁₂)- cycloalkyl-(C₁-C₈)-alkyl-S(O)ₙ, (C₆-C₁₂)-bicycloalkyl-S(O)ₙ, (C₆-C₁₂)- bicycloalkyl-(C₁-C₈)-alkyl-S(O)ₙ, (C₆-C₁₂)-tricycloalkyl-S(O)ₙ, (C₆- C₁₂)-tricycloalkyl-(C₁-C₈)-alkyl-S(O)ₙ, optionally substituted (C₆-C₁₄)- aryl-S(O)ₙ, (C₆-C₁₄)-aryl-(C₁-C₈)-alkyl-S(O)ₙ optionally substituted in the aryl radical, optionally substituted heteroaryl- S(O)ₙ or heteroaryl-(C₁-C₈)-alkyl-S(O)ₙ optionally substituted in the heteroaryl radical, where n is 1 or 2;
R¹ is hydrogen, (C₁-C₁₀)-alkyl, which can optionally be monosubstituted or polysubstituted by fluorine, (C₃-C₁₂)-cycloalkyl, (C₃-C₁₂)- cycloalkyl-(C₁-C₈)-alkyl, optionally substituted R²¹-((C₆-C₁₄)-aryl), (R²¹-((C₆-C₁₄)-aryl))-(C₁-C₈)-alkyl optionally substituted in the aryl radical, the radical Het-, Het-(C₁-C₈)-alkyl or one of the radicals R²¹O-, R²²O-NH-, R²¹O-N(R²³)-, R²⁴NH-, R²⁵N(R²⁵)-, HO-((C₁-C₈)- alkyl)-N(R²⁶)-, R²⁷C(O)-NH-, R²¹C(O)-N(R²³)-, R²¹C(O)-, R²¹O-C(O)-, R²⁸N(R²¹)-C(O)-, R²¹O-N=, O= and S=;
R² is hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, R⁴CO, COOR⁴, CON(CH₃)R⁴, CONHR⁴, CSNHR⁴, COOR¹⁵, CON(CH₃)R¹⁵ or CONHR¹⁵;
R⁴ is hydrogen or (C₁-C₂₈)-alkyl which can optionally be mono- or polysubstituted by identical or different radicals from the group consisting of hydroxyl, hydroxycarbonyl, aminocarbonyl, mono- or di- ((C₁-C₁₈)-alkyl)aminocarbonyl, amino-(C₂-C₁₈)-alkylaminocarbonyl, amino-(C₁-C₃)-alkylphenyl-(C₁-C₃)-alkylaminocarbonyl, (C₁-C₁₈)-alkylcarbonylamino-(C₁-C₃)-alkylphenyl- (C₁-C₃)-alkylaminocarbonyl, (C₁-C₁₈)-alkylcarbonylamino- (C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, HOS(O)₂-(C₁-C₃)-alkyl, R⁹NHS(O)₂-(C₁-C₃)-alkyl, (R⁸O)₂P(O)-(C₁-C₃)-alkyl, tetrazolyl-(C₁-C₃)-alkyl, halogen, nitro, trifluoromethyl and the radical R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino and trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkylcarbonyl, (C₁-C₁₈)-alkoxycarbonyl, (C₆-C₁₄)- arylcarbonyl, (C₆-C₁₄)-Aryl-(C₁-C₈)-alkylcarbonyl or (C₆-C₁₄)-aryl- (C₁-C₁₈)-alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)-alkoxy, halogen, nitro, amino and trifluoromethyl, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkylaminocarbonyl, (C₃-C₈)-cycloalkylaminocarbonyl, optionally substituted (C₆-C₁₄)-arylaminocarbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹¹ is hydrogen, (C₁-C₁₈)-alkyl, R¹²CO, R^{12a}CS, optionally substituted (C₆-C₁₄)-aryl-S(O)₂, (C₁-C₁₈)-alkyl-S(O)₂, (C₆-C₁₄)-aryl-(C₁-C₈)-alkyl optionally substituted in the aryl radical, R⁹NHS(O)₂ or the radical R¹⁵;
R¹² is hydrogen, (C₁-C₁₈)-alkyl, (C₂-C₈)-alkenyl, (C₂-C₈)-alkynyl, optionally substituted (C₆-C₁₄)-aryl, (C₁-C₁₈)-alkoxy, (C₆-C₁₄)-aryl- (C₁-C₈)-alkoxy which can also be substituted in the aryl radical, optionally substituted (C₆-C₁₄)-aryloxy, the radical R¹⁵, the radical R¹⁵-O-, amino or mono- or di-((C₁-C₁₈)-alkyl)amino, (C₂-C₈)- alkenylamino, (C₂-C₈)-alkynylamino, (C₃-C₁₂)-cycloalkylamino, (C₃-C₁₂)-cycloalkyl-(C₁-C₈)-alkylamino, the radical R¹⁵-NH-, optionally substituted (C₆-C₁₄)-aryl-NH, (C₆-C₁₄)-aryl-(C₁-C₈)-alkyl- NH, which can also be substituted in the aryl radical, optionally substituted heteroaryl-NH or heteroaryl-(C₁-C₈)-alkyl-NH optionally substituted in the heteroaryl radical;
R^{12a} is amino, mono- or di-((C₁-C₁₈)-alkyl)amino, (C₂-C₈)-alkenylamino, (C₂-C₈)-alkynylamino, (C₃-C₁₂)-cycloalkylamino, (C₃-C₁₂)-cycloalkyl- (C₁-C₈)-alkylamino, the radical R¹⁵-NH-, optionally substituted (C₆-C₁₄)-aryl-NH, (C₆-C₁₄)-aryl-(C₁-C₈)-alkyl-NH, which can also be substituted in the aryl radical, optionally substituted heteroaryl-NH or heteroaryl-(C₁-C₈)-alkyl-NH optionally substituted in the heteroaryl radical;
R¹³ is hydrogen, (C₁-C₆)-alkyl, (C₆-C₁₄)-aryl-(C₁-C₈)-alkyl optionally substituted in the aryl radical or (C₃-C₈)-cycloalkyl;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
R²¹ is hydrogen, (C₁-C₈)-alkyl, (C₃-C₁₂)-cycloalkyl, (C₃-C₁₂)-cycloalkyl- (C₁-C₈)-alkyl, (C₆-C₁₄)-aryl, (C₆-C₁₄)-aryl-(C₁-C₈)-alkyl, the radical Het- or Het-(C₁-C₈)-alkyl, where alkyl radicals can be monosubstituted or polysubstituted by fluorine and the radicals R²¹ can be identical or different if they occur several times;
R²² is (C₃-C₁₂)-cycloalkyl, (C₃-C₁₂)-cycloalkyl-(C₁-C₈)-alkyl, (C₆-C₁₄)-aryl, the radical Het- or Het-(C₁-C₈)-alkyl, where alkyl radicals can be monosubstituted or polysubstituted by fluorine;
R²³ is (C₁-C₈)-alkyl, (C₃-C₁₂)-cycloalkyl, (C₃-C₁₂)-cycloalkyl-(C₁-C₈)-alkyl, (C₆-C₁₄)-aryl, (C₆-C₁₄)-aryl-(C₁-C₈)-alkyl, the radical Het- or Het- (C₁-C₈)-alkyl, where alkyl radicals can be monosubstituted or polysubstituted by fluorine;
R²⁴ (C₃-C₁₂)-cycloalkyl, (C₃-C₁₂)-cycloalkyl-(C₁-C₈)-alkyl, (C₆-C₁₄)-aryl, (C₆-C₁₄)-aryl-(C₁-C₈)-alkyl, the radical Het- or Het-(C₁-C₈)-alkyl, where alkyl radicals can be monosubstituted or polysubstituted by fluorine;
R²⁵ has the meanings of R²³, where the radicals R²⁵ can be identical or different;
R²⁶ has the meanings of R²¹ or HO-((C₁-C₈)-alkyl), where alkyl radicals can be monosubstituted or polysubstituted by fluorine;
R²⁷ is hydrogen, (C₃-C₁₂)-cycloalkyl, (C₃-C₁₂)-cycloalkyl-(C₁-C₈)-alkyl, (C₆-C₁₄)-aryl-(C₁-C₈)-alkyl, the radical Het- or Het-(C₁-C₈)-alkyl, where alkyl radicals can be monosubstituted or polysubstituted by fluorine;
R²⁸ is one of the radicals R²¹-, R²¹O-, R²⁶N(R²⁶)-, R²¹C(O)-, R²¹O-C(O)-, ((C₁-C₁₈)-alkyl-O-C(O)-((C₁-C₆)-alkyl)-O-C(O)-, R²¹N(R²¹)-C(O)-, R²¹N(R²¹)-C(=N(R²¹))- or R²¹C(O)-N(R²¹)-;
Het is a mono- or polycyclic, 4- to 14-membered, aromatic or nonaromatic ring which contains 1, 2, 3 or 4 identical or different heteroatoms from the group consisting of N, O and S as ring members and can optionally be substituted by one or more, identical or different substituents;
c, d and f independently of one another are 0 or 1, but cannot all simultaneously be 0;
e, g and h independently of one another are 0, 1, 2, 3, 4, 5 or 6;
in all its stereoisomeric forms and mixtures thereof in all ratios, or its physiologically tolerable salts;
subject to the proviso that the group R¹-A- may not be phenyl, pyrrolyl, imidazolyl or pyridyl, each of which radicals may be substituted by (C₁-C₈)-alkyl, (C₁-C₈)-alkoxy, trifluoromethyl, hydroxyl, hydroxy-(C₁-C₄)-alkyl, formyl, hydroxycarbonyl, aminocarbonyl, (C₁-C₄)-alkoxycarbonyl, phenyl, phenoxy, benzyloxy, tetrazolyl, phenyl-(C₁-C₄)-alkyl and (C₁-C₄)-alkyl-CO, and the group R¹-A- may not be the radical R^{x}-(C₁-C₂)-alkyl, wherein R^{x} is phenyl, furyl, thienyl, pyrrolyl, imidazolyl or pyridyl, each of which radicals may also be benzofused and be substituted by (C₁-C₈)-alkyl, (C₁-C₈)-alkoxy, halogen, nitro, amino, trifluoromethyl, hydroxyl, hydroxy-(C₁-C₄)-alkyl, methylenedioxy, ethylenedioxy, cyano, formyl, hydroxycarbonyl, aminocarbonyl, (C₁-C₄)-alkoxycarbonyl, phenyl, phenoxy, benzyloxy, tetrazolyl, phenyl-(C₁-C₄)-alkyl and (C₁-C₄)-alkyl-CO.

27. A compound of the formula Id as claimed in claim 26, in which simultaneously
W is R¹-A-C(R¹³);
Y is a carbonyl group;
Z is N(R⁰);
A is a bivalent radical from the group consisting of (C₃-C₇)- cycloalkylene, phenylene, phenylene-(C₁-C₆)-alkyl, (C₁-C₆)-alkylene phenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur;
B is a bivalent methylene radical or ethylene radical which is substituted by a radical from the group consisting of (C₁-C₈)-alkyl, (C₂-C₈)-alkenyl, (C₂-C₈)-alkynyl, (C₃-C₁₀)-cycloalkyl, (C₃-C₁₀)- cycloalkyl-(C₁-C₆)-alkyl, optionally substituted (C₆-C₁₄)-aryl, (C₆- C₁₄)-aryl-(C₁-C₆)-alkyl optionally substituted in the aryl radical, optionally substituted heteroaryl and heteroaryl-(C₁-C₆)-alkyl optionally substituted in the heteroaryl radical;
D is C(R²)(R³);
E is tetrazolyl or R¹⁰CO;
R is hydrogen or (C₁-C₈)-alkyl;
R⁰ is hydrogen, (C₁-C₈)-alkyl, (C₃-C₁₂)-cycloalkyl, (C₃-C₁₂)-cycloalkyl- (C₁-C₈)-alkyl, (C₆-C₁₂)-bicycloalkyl, (C₆-C₁₂)-bicycloalkyl-(C₁-C₈)- alkyl, (C₆-C₁₂)-tricycloalkyl, (C₆-C₁₂)-tricycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl, heteroaryl-(C₁-C₈)-alkyl optionally substituted in the heteroaryl radical, CHO, (C₁-C₈)-alkyl-CO, (C₃-C₁₂)-cycloalkyl-CO, (C₃-C₁₂)-cycloalkyl-(C₁-C₈)-alkyl-CO, (C₆-C₁₂)-bicycloalkyl-CO, (C₆- C₁₂)-bicycloalkyl-(C₁-C₈)-alkyl-CO, (C₆-C₁₂)-tricycloalkyl-CO, (C₆- C₁₂)-tricycloalkyl-(C₁-C₈)-alkyl-CO, optionally substituted (C₆-C₁₄)- aryl-CO, (C₆-C₁₄)-aryl-(C₁-C₈)-alkyl-CO optionally substituted in the aryl radical, optionally substituted heteroaryl-CO, heteroaryl-(C₁-C₈)- alkyl-CO optionally substituted in the heteroaryl radical, (C₁-C₈)- alkyl-S(O)ₙ, (C₃-C₁₂)-cycloalkyl-S(O)ₙ, (C₃-C₁₂)-cycloalkyl-(C₁-C₈)- alkyl-S(O)ₙ, (C₆-C₁₂)-bicycloalkyl-S(O)ₙ, (C₆-C₁₂)-bicycloalkyl-(C₁- C₈)-alkyl-S(O)ₙ, (C₆-C₁₂)-tricycloalkyl-S(O)ₙ, (C₆-C₁₂)-tricycloalkyl- (C₁-C₈)-alkyl-S(O)ₙ, optionally substituted (C₆-C₁₄)-aryl-S(O)ₙ, (C₆- C₁₄)-aryl-(C₁-C₈)-alkyl-S(O)ₙ optionally substituted in the aryl radical, optionally substituted heteroaryl S(O)ₙ or heteroaryl-(C₁-C₈)-alkyl- S(O)ₙ optionally substituted in the heteroaryl radical, where n is 1 or 2;
R¹ is one of the radicals R²¹O-, R²⁴NH-, R²⁵N(R²⁵)-, HO-((C₁-C₈)-alkyl)- N(R²⁶)-, R²¹O-C(O)- and R²⁸N(R²¹)-C(O)-;
R² is hydrogen or (C₁-C₈)-alkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, CON(CH₃)R⁴, CONHR⁴, CON(CH₃)R¹⁵ or CONHR¹⁵;
R⁴ is (C₁-C₁₀)-alkyl, which can optionally be mono- or polysubstituted by identical or different radicals from the group consisting of hydroxyl, hydroxylcarbonyl, aminocarbonyl, mono- or di-((C₁-C₁₈)- alkyl)aminocarbonyl, (C₆-C₁₄)-aryl-(C₁-C₈)-alkoxycarbonyl, which can also be substituted in the aryl radical, (C₁-C₈)-alkoxy, (C₁-C₈)- alkoxycarbonyl, optionally substituted (C₃-C₈)-cycloalkyl, tetrazolyl- (C₁-C₃)-alkyl, trifluoromethyl and R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12- membered heterocyclic ring which can be aromatic, partially hydrogenated or completely hydrogenated and which can contain one, two or three identical or different heteroatoms from the group consisting of nitrogen, oxygen and sulfur, or a radical R⁶CO-, where the aryl and, independently thereof, the heterocyclic radical, can be mono- or polysubstituted by identical or different radicals from the group consisting of (C₁-C₈)-alkyl, (C₁-C₈)-alkoxy, halogen, nitro, amino or trifluoromethyl;
R⁶ is a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical, and their esters and amides, where free functional groups can be protected by protective groups customary in peptide chemistry;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹¹ is R¹²CO, optionally substituted (C₆-C₁₄)-aryl-S(O)₂ or (C₁-C₁₈)-alkyl- S(O)₂;
R¹² is hydrogen, (C₁-C₁₈)-alkyl, (C₂-C₈)-alkenyl, (C₂-C₈)-alkynyl, optionally substituted (C₆-C₁₄)-aryl, (C₁-C₁₈)-alkoxy, (C₆-C₁₄)-aryl- (C₁-C₈)-alkoxy which can also be substituted in the aryl radical, optionally substituted (C₆-C₁₄)-aryloxy, the radical R¹⁵ or the radical R¹⁵-O-;
R¹³ is hydrogen or (C₁-C₄)-alkyl;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
c and d are 1 and f is 0;
e and h independently of one another are 0 or 1 and g is 0;
in all its stereoisomeric forms and mixtures thereof in all ratios, or its physiologically tolerable salts.

28. A compound of the formula Id as claimed in claim 26 and/or 27, in which the radical by which the group B is substituted is a (C₁-C₈)-alkyl radical, in all its stereoisomeric forms and mixtures thereof in all ratios, or its physiologically tolerable salts.

29. A compound of the formula Id as claimed in one or more of claims 26 to 28, in which the radical R¹ is R²⁸N(R²¹)-C(O)-, in all its stereoisomeric forms and mixtures thereof in all ratios, or its physiologically tolerable salts.

30. A compound of the formula Id as claimed in one or more of claims 26 to 29 and/or its physiologically tolerable salts for use as a pharmaceutical.

31. A pharmaceutical preparation which comprises one or more compounds of the formula Ib as claimed in one or more of claims 26 to 29 and/or its physiologically tolerable salts in addition to pharmaceutically innocuous excipients and/or additives.

## Revendications

1. Utilisation des composés de formule I, 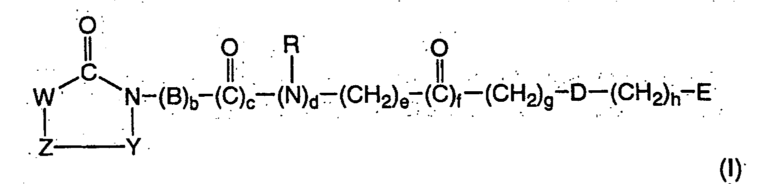 dans laquelle,
W est R¹-A-C(R¹³) ou R¹-A-CH=C ;
Y est un groupe carbonyle, thiocarbonyle ou méthylène ;
Z est N(R⁰), l'oxygène, le soufre ou un groupe méthylène ;
A est un radical bivalent de la sérié (C₁-C₆)-alkylène, (C₃-C₁₂)- cycloalkylène ; (C₁-C₆)-alkylène-(C₃-C₁₂)-cycloalkyle, phénylène, phényléne-(C₁-C₆)-alkyle, (C₁-C₆)-alkylène-phényle, (C₁-C₆)- alkylène-phényl-(C₁-C₆)-alkyle, phénylène-(C₂-C₆)-alcényle ou un radical bivalent d'un cycle saturé ou insaturé à 5 ou 6 membres, qui contient 1 ou 2 atomes d'azote et qui peut être substitué une ou deux fois par un groupe (C₁-C₆)-alkyle ou l'oxygène ou le soufre lié deux fois ;
B est un radical bivalent de la série (C₁-C₆)-alkylène, (C₂-C₆)- alcénylène, phénylène, phénylène-(C₁-C₃)-alkyle, (C₁-C₃)-alkylène- phényle, où le radical (C₁-C₆)-alkylène est non substitué ou peut être substitué par un radical de la série (C₁-C₈)-alkyle, (C₂-C₈)- alcényle, (C₂-C₈)-alcynyle, (C₃-C₁₀)-cycloalkyle, (C₃-C₁₀)-cycloalkyl- (C₁-C₆)-alkyle, (C₆-C₁₄)-aryle éventuellement substitué, (C₆-C₁₄)- aryl-(C₁-C₆)-alkyle éventuellement substitué dans le radical aryle, hétéroaryle éventuellement substitué et hétéroaryl-(C₁-C₆)-alkyle éventuellement substitué dans le radical hétéroaryle ;
D est C(R²)(R³), N(R³) ou CH=C(R³) ;
E signifie tétrazolyle, (R⁸O)₂P(O), HOS(O)₂, R⁹NHS(O)₂ ou R¹⁰CO ;
R signifie l'hydrogène, (C₁-C₈)-alkyle, (C₃-C₁₂)-cycloalkyle, (C₃-C₁₂)- cycloalkyl-(C₁-C₈)-alkyle, (C₆-C₁₄)-aryle éventuellement substitué, (C₆-C₁₄)-aryl-(C₁-C₈)-alkyle éventuellement substitué dans le radical aryle, hétéroaryle éventuellement substitué ou hétéroaryl-(C₁-C₈)- alkyle éventuellement substitué dans le radical hétéroaryle, où les radicaux alkyle peuvent être substitués une ou plusieurs fois par le fluor ;
R⁰ est l'hydrogène, (C₁-C₈)-alkyle, (C₃-C₁₂)-cycloalkyle, (C₃-C₁₂)- cycloalkyl-(C₁-C₈)-alkyle, (C₆-C₁₂)-bicycloalkyle, (C₆-C₁₂)- bicycloalkyl-(C₁-C₈)-alkyle, (C₆-C₁₂)-tricycloalkyle, (C₆-C₁₂)- tricycloalkyl-(C₁-C₈)-alkyle, (C₆-C₁₄)-aryle éventuellement substitué, (C₆-C₁₄)-aryl-(C₁-C₈)-alkyle éventuellement substitué dans le radical aryle, hétéroaryle éventuellement substitué, hétéroaryl-(C₁-C₈)- alkyle éventuellement susbstitué dans le radical hétéroaryle, CHO, (C₁-C₈)-alkyle-CO, (C₃-C₁₂)-cycloalkyl-CO,(C₃-C₁₂)-cycloalkyl-(C₁- C₈)-alkyl-CO, (C₆-C₁₂)-bicycloalkyl-CO,(C₆-C₁₂)-bicycloalkyl-(C₁-C₈)- alkyl-CO, (C₆-C₁₂)-tricycloalkyl-CO,(C₆-C₁₂)-tricycloalkyl-(C₁-C₈)- alkyl-CO, (C₆-C₁₄)-aryl-CO éventuellement substitué, (C₆-C₁₄)-aryl- (C₁-C₈)-alkyl-CO éventuellement substitué dans le radical aryle, hétéroaryl-CO éventuellement substitué, hétéroaryl-(C₁-C₈)-alkyl- CO éventuellement substitué dans le radical hétéroaryle, (C₁-C₈)- alkyl-S(O)ₙ, (C₃-C₁₂)-cycloalkyl-S(O)ₙ, (C₃-C₁₂)-cycloalkyl-(C₁-C₈)- alkyl-S(O)ₙ, (C₆-C₁₂)-bicycloalkyl-S(O)ₙ, (C₆-C₁₂)-bicycloalkyl-(C₁- C₈)-alkyl-S(O)ₙ, (C₆-C₁₂)-tricycloalkyl-S(O)n, (C₆-C₁₂)-tricycloalkyl- (C₁-C₈)-alkyl-S(O)ₙ, (C₆-C₁₄)-aryl-S(O)ₙ éventuellement substitué, (C₆-C₁₄)-aryl-(C₁-C₈)-alkyl-S(O)ₙ éventuellement substitué dans le radical aryle, hétéroaryl-S(O)ₙ éventuellement substitué ou hétéroaryl-(C₁-C₈)-alkyl-S(O)ₙ éventuellement substitué dans le radical hétéroaryle, n vaut 1 ou 2 ;
R¹ signifie l'hydrogène, (C₁-C₁₀)-alkyle qui peut être éventuellement substitué une ou plusieurs fois par le fluor, (C₃-C₁₂)-cycloalkyle, (C₃-C₁₂)-cycloalkyl-(C₁-C₈)-alkyle, R²¹-((C₆-C₁₄)-aryle) éventuellement substitué, (R²¹-((C₆-C₁₄)-aryl))-(C₁-C₈)-alkyle éventuellement substitué dans le radical aryle, le radical hét, hét- (C₁-C₈)-alkyle ou un des radicaux R²¹O-, R²²O-NH-, R²¹O-N(R²³)-, R²⁴NH-, R²⁵N(R²⁵)-, HO-((C₁-C₈)-alkyl)-N(R²⁶)-, R²⁷C(O)-NH-, R²¹C(O)-N(R²³)-, R²¹C(O)-, R²¹O-C(O)-, R²⁸N(R²¹)-C(O)-, R²¹O-N=, O= et S= ;
R² signifie l'hydrogène, (C₁-C₈)-alkyle, (C₆-C₁₄)-aryle éventuellement substitué, (C₆-C₁₄)-aryl-(C₁-C₈)-alkyle éventuellement substitué dans le radical aryle ou (C₃-C₈)-cycloalkyle;
R³ signifie l'hydrogène, (C₁-C₈)-alkyle, (C₆-C₁₄)-aryle éventuellement substitué, (C₆-C₁₄)-aryl-(C₁-C₈)-alkyle éventuellement substitué dans le radical aryle, (C₃-C₈)-cycloalkyle, (C₂-C₈)-alcényle, (C₂-C₈)- alcynyle, (C₂-C₈)-alcénylcarbonyle, (C₂-C₈)-alcynylcarbonyle, pyridyle, R¹¹NH, R⁴CO, COOR⁴, CON(CH₃)R⁴, CONHR⁴, CSNHR⁴, COOR¹⁵, CON(CH₃)R¹⁵ ou CONHR¹⁵ ;
R⁴ signifie l'hydrogène ou (C₁-C₂₈)-alkyle, qui peut être substitué éventuellement une ou plusieurs fois par des radicaux identiques ou différents de la série hydroxy, hydroxycarbonyle, aminocarbonyle, mono- ou di-((C₁-C₁₈)-alkyl)-aminocarbonyle, amino-(C₂-C₁₈)- alkylaminocarbonyle, amino-(C₁-C₃)-alkyl-phényl-(C₁-C₃)-alkyl- aminocarbonyle, (C₁-C₁₈)-alkylcarbonylamino-(C₁-C₃)-alkylphényl- (C₁-C₃)-alkylaminocarbonyle, (C₁-C₁₈)-alkylcarbonylamino-(C₂-C₁₈)- alkyl- aminocarbonyle, (C₆-C₁₄)-aryl-(C₁-C₈)-alcoxycarbonyle, qui peut être substitué également dans le radical aryle, amino, mercapto, (C₁-C₁₈)-alcoxy, (C₁-C₁₈)-alcoxycarbonyle, (C₃-C₈)- cycloalkyle éventuellement substitué, HOS(O)₂-(C₁-C₃)-alkyle, R⁹NHS(O)₂-(C₁-C₃)-alkyle, (R⁸O)₂P(O)-(C₁-C₃)-alkyle, tétrazolyl-(C₁- C₃)-alkyle, halogène, nitro, trifluorométhyle et par le radical R⁵ ;
R⁵ signifie (C₆-C₁₄)-aryle éventuellement substitué, (C₆-C₁₄)-aryle-(C₁- C₈)-alkyle éventuellement substitué dans le radical aryle, un cycle hétérocyclique mono- ou bicyclique de 5 à 12 membres,qui peut être aromatique, en partie hydrogéné ou complètement hydrogéné et qui peut contenir un, deux ou trois hétéroatomes identiques ou différents de la série de l'azote, l'oxygène et le soufre, un radical R⁶ ou un radial R⁶CO-, dans lequel le radical aryle et le radical hétérocyclique indépendamment de celui-ci peut être substitué une ou plusieurs fois par des radicaux identiques ou différents de la série (C₁-C₁₈)-alkyle, (C₁-C₁₈)-alcoxy, halogène, nitro, amino ou trifluorométhyle ;
R⁶ est R⁷R⁸N, R⁷O ou R⁷S ou une chaîne latérale acide aminé, un radical acide aminé naturel ou non naturel, acide imino, acide azaamino éventuellement N-(C₁-C₈)-alkylé ou N-((C₆-C₁₄)-aryl-(C₁- C₈)-alkylé) ou un radical dipeptidique, qui peut être également substitué dans le radical aryle et/ou pour lequel la liaison peptide est réduite en -NH-CH₂-, ainsi que leur esters et amides, où à la place des groupes fonctionnels libres peut signifier éventuellement l'hydrogène ou un hydroxyméthyle et/ou dans lequel des groupes fonctionnels libres peuvent être protégés par des groupes protecteurs classiques dans la chimie des peptides;
R⁷ signifie l'hydrogène, (C₁-C₁₈)-alkyle, (C₆-C₁₄)-aryl-(C₁-C₈)-alkyle, (C₁-C₁₈)-alkylcarbonyle, (C₁-C₁₈)-alcoxycarbonyle, (C₆-C₁₄)- arylcarbonyle, (C₆-C₁₄)-aryl-(C₁-C₈)-alkylcarbonyle ou (C₆-C₁₄)-aryl- (C₁-C₁₈)-alkyloxycarbonyle, où les groupes alkyle peuvent être éventuellement substitués par un groupe amino et/ou les restes aryle peuvent être éventuellement substitués une ou plusieurs fois, de préférence une fois, par des radicaux identiques ou différents de la série (C₁-C₈)-alkyle, (C₁-C₈)-alcoxy, halogène, nitro, amino et trifluorométhyle, un radical acide aminé naturel ou non naturel, acide imino, acide azaamino éventuellement N-(C₁-C₈)-alkylé ou N- ((C₆-C₁₄)-aryl-(C₁-C₈)-alkylé) ou un radical dipeptidique, qui peut être également substitué dans le radical aryle et/ou dans lequel la liaison peptide est réduite en -NH-CH₂- ;
R⁸ signifie l'hydrogène, (C₁-C₁₈)-alkyle, (C₆-C₁₄)-aryle éventuellement substitué ou (C₆-C₁₄)-aryl-(C₁-C₈)-alkyle, qui peut être également substitué dans le radical aryle ;
R⁹ signifie l'hydrogène, aminocarbonyle, (C₁-C₁₈)-alkylaminocarbonyle, (C₃-C₈)-cycloalkylaminocarbonyle, (C₆-C₁₄)-arylaminocarbonyle éventuellement substitué, (C₁-C₁₈)-alkyle, (C₆-C₁₄)-aryle éventuellement substitué ou (C₃-C₈)-cycloalkyle ;
R¹⁰ signifie hydroxy, (C₁-C₁₈)-alcoxy, (C₆-C₁₄)-aryl-(C₁-C₈)-alcoxy, qui peut également être substitué dans le radical aryle, (C₆-C₁₄)-aryloxy éventuellement substitué, amino ou mono- ou di-((C₁-C₁₈)-alkyl)- amino ;
R¹¹ signifie l'hydrogène, (C₁-C₁₈)-alkyle, R¹²CO, R^{12a}CS, (C₆-C₁₄)-aryl- S(O)₂ éventuellement substitué, (C₁-C₁₈)-alkyl-S(O)₂, (C₆-C₁₄)-aryl- (C₁-C₈)-alkyle éventuellement substitué dans le radical aryle, R⁹NHS(O)₂ ou le radical R¹⁵ ;
R¹² signifie l'hydrogène, (C₁-C₁₈)-alkyle, (C₂-C₈)-alcényle, (C₂-C₈)- alcynyle, (C₆-C₁₄)-aryle éventuellement substitué, (C₁-C₁₈)-alcoxy, (C₆-C₁₄)-aryl-(C₁-C₈)-alcoxy, qui peut également être substitué dans le radical aryle, (C₆-C₁₄)-aryloxy éventuellement substitué, le radical R¹⁵, le radical R¹⁵-O-, amino, mono- ou di-((C₁-C₁₈)-alkyl)-amino, (C₂-C₈)-alcényl-amino, (C₂-C₈)-alcynyl-amino, (C₃-C₁₂)-cycloalkyl- amino, (C₃-C₁₂)-cycloalkyl-(C₁-C₈)-alkyl-amino, le radical R¹⁵-NH-, (C₆-C₁₄)-aryl-NH éventuellement substitué, (C₆-C₁₄)-aryl-(C₁-C₈)- alkyl-NH, qui peut également être substitué dans le radical aryle, hétéroaryl-NH éventuellement substitué ou hétéroaryl-(C₁-C₈)-alkyl- NH éventuellement substitué dans le radical hétéroaryle ;
R^{12a} signifie amino, mono- ou di-((C₁-C₁₈)-alkyl)-amino, (C₂-C₈)-alcényl- amino, (C₂-C₈)-alcynyl-amino, (C₃-C₁₂)-cycloalkyl-amino, (C₃-C₁₂)- cycloalkyl-(C₁-C₈)-alkyl-amino, le radical R¹⁵-NH-, (C₆-C₁₄)-aryl-NH éventuellement substitué, (C₆-C₁₄)-aryl-(C₁-C₈)-alkyl-NH, qui peut être également substitué dans le radical aryle, hétéroaryl-NH éventuellement substitué ou hétéroaryl-(C₁-C₈)-alkyl-NH éventuellement substitué dans le radical hétéroaryle;
R¹³ signifie l'hydrogène, (C₁-C₆)-alkyle, (C₆-C₁₄)-aryl-(C₁-C₈)-alkyle éventuellement substitué dans le radical aryle ou (C₃-C₈)- cycloalkyle;
R¹⁵ est R¹⁶-(C₁-C₆)-alkyle ou R¹⁶ ;
R¹⁶ est un radical bicyclique ou tricyclique de 6 à 24 membres, qui est saturé ou partiellement insaturé et qui également peut contenir un à quatre hétéroatomes identiques ou différents de la série de l'azote, l'oxygène et le soufre et qui peut être également substitué par un ou plusieurs substituants identiques ou différents de le série de (C₁- C₄)-alkyle et oxo ;
R²¹ est l'hydrogène, (C₁-C₈)-alkyle, (C₃-C₁₂)-cycloalkyle, (C₃-C₁₂)- cycloalkyl-(C₁-C₈)-alkyle, (C₆-C₁₄)-aryle, (C₆-C₁₄)-aryl-(C₁-C₈)-alkyle, le radical hét- ou hét-(C₁-C₈)-alkyle, où les radicaux alkyle peuvent être substitués une fois ou plusieurs fois par le fluor où le radical R²¹ peut être identique ou différent pour des apparitions multiples ;
R²² signifie (C₃-C₁₂)-cycloalkyle, (C₃-C₁₂)-cycloalkyl-(C₁-C₈)-alkyle, (C₆- C₁₄)-aryle, le radical hét- ou hét-(C₁-C₈)-alkyle, où les radicaux alkyle peuvent être substitués une ou plusieurs fois par le fluor ;
R²³ signifie (C₁-C₈)-alkyle, (C₃-C₁₂)-cycloalkyle, (C₃-C₁₂)-cycloalkyl-(C₁- C₈)-alkyle, (C₆-C₁₄)-aryle, (C₆-C₁₄)-aryl-(C₁-C₈)-alkyle, le radical hét- ou hét-(C₁-C₈)-alkyle, où les radicaux alkyle peuvent être substitués une ou plusieurs fois par le fluor ;
R²⁴ signifie (C₃-C₁₂)-cycloalkyle, (C₃-C₁₂)-cycloalkyl-(C₁-C₈)-alkyle, (C₆- C₁₄)-aryle, (C₆-C₁₄)-aryl-(C₁-C₈)-alkyle, le radical hét- ou hét-(C₁- C₈)-alkyle, où les radicaux alkyle peuvent être substitués une ou plusieurs fois par le fluor ;
R²⁵ a les significations de R²³, où le radical R²⁵ peut être identique ou différent ;
R²⁶ a les significations de R²¹ ou signifie HO-((C₁-C₈)-alkyle), où le radical alkyle peut être substitué une ou plusieurs fois par le fluor ;
R²⁷ signifie l'hydrogène, (C₃-C₁₂)-cycloalkyle, (C₃-C₁₂)-cycloalky)-(C₁- C₈)-alkyle, (C₆-C₁₄)-aryl-(C₁-C₈)-alkyle; le radical hét- ou hét-(C₁- C₈)-alkyle, où les radicaux alkyle peuvent être substitués une ou plusieurs fois par le fluor ;
R²⁸ est le radical R²¹-, R²¹O-, R²⁶N(R²⁶)-, R²¹C(O)-, R²¹O-C(O)-,((C₁- C₁₈)-alkyl-O-C(O)-((C₁-C₆)-alkyl)-O-C(O)-, R²¹N(R²¹)-C(O), R²¹N(R²¹)-C(=N(R²¹))- ou R²¹C(O)-N(R²¹)- ;
Hét est un cycle aromatique ou non aromatique, mono ou polycyclique, de 4 à 14 membres, qui contient 1, 2, 3 ou 4 hétéroatomes identiques ou différents de la série de N, O et S comme membres cycliques et peut être éventuellement substitué par un ou plusieurs substituants identiques ou différents ;
b, c, d, et f sont indépendamment les uns des autres 0 ou 1, mais ne peuvent pas tous être en même temps 0 ;
e, g et h sont indépendamment les uns des autres 0,1, 2, 3, 4, 5 ou 6 ;
dans toutes leurs formes stéréoisomères et leurs mélanges dans tous rapports ainsi que leurs sels compatibles physiologiquement pour la préparation de médicaments pour l'inhibition de l'adhésion et/ou de la migrations des leucocytes ou pour l'inhibition du récepteur VLA-4.

2. Utilisation des composés de formule I selon la revendication 1 dans toutes leurs formes stéréoisomères et leurs mélanges dans tous rapports ainsi que leurs sels compatibles physiologiquement, où dans la formule I
W est R¹-A-C(R¹³) ou R¹-A-CH=C ;
Y est un groupe carbonyle, thiocarbonyle ou méthylène ;
Z est N(R⁰), l'oxygène, le soufre ou un groupe méthylène ;
A signifie un radical bivalent de la série (C₁-C₆)-alkylène, (C₃-C₁₂)- cycloalkylène ; (C₁-C₆)-alkylène-(C₃-C₁₂)-cycloalkyle, phénylène, phénylène-(C₁-C₆)-alkyle, (C₁-C₆)-alkylène-phényle, (C₁-C₆)- alkylène-phényl-(C₁-C₆)-alkyle, phénylène-(C₂-C₆)-alcényle ou un radical bivalent d'un cycle saturé ou insaturé à 5 ou 6 membres, qui contient 1 ou 2 atomes d'azote et qui peut être substitué une ou deux fois par un groupe (C₁-C₆)-alkyle ou l'oxygène ou le soufre lié deux fois;
B est un radical bivalent de la série (C₁-C₆)-alkylène, (C₂-C₆)- alcénylène, phénylène, phénylène-(C₁-C₃)-alkyle, (C₁-C₃)-alkylène- phényle ;
D est C(R²)(R³), N(R³) ou CH=C(R³) ;
E signifie tétrazolyle, (R⁸O)₂P(O), HOS(O)₂, R⁹NHS(O)₂ ou R¹⁰CO ;
R et R⁰ indépendamment l'un de l'autre signifient l'hydrogène, (C₁-C₈)- alkyle, (C₃-C₁₂)-cycloalkyle, (C₃-C₁₂)-cycloalkyl-(C₁-C₈)-alkyle, (C₆- C₁₄)-aryle éventuellement substitué, (C₆-C₁₄)-aryl-(C₁-C₈)-alkyle éventuellement substitué dans le radical aryle, hétéroaryle éventuellement substitué ou hétéroaryl-(C₁-C₈)-alkyle éventuellement substitué dans le radical hétéroaryle, où les radicaux alkyle peuvent être substitués une ou plusieurs fois par le fluor ;
R¹ signifie l'hydrogène, (C₁-C₁₀)-alkyle qui peut être éventuellement substitué une ou plusieurs fois par le fluor, (C₃-C₁₂)-cycloalkyle, (C₃-C₁₂)-cycloalkyl-(C₁-C₈)-alkyle, R²¹-((C₆-C₁₄-aryle) éventuellement substitué, (R²¹-((C₆-C₁₄)-aryl))-(C₁-C₈)-alkyle éventuellement substitué dans le radical aryle, le radical hét, hét- (C₁-C₈)-alkyle ou un des radicaux R²¹O-, R²²O-NH-, R²¹O-N(R²³)-, R²⁴NH-, R²⁵N(R²⁵)-, HO-((C₁-C₈)-alkyl)-N(R²⁶)-, R²⁷C(O)-NH-, R²¹C(O)-N(R²³)-, R²¹C(O)-, R²¹O-C(O)-, R²⁸N(R²¹)-C(O)-, R²¹O-N=, O= et S= ;
R² signifie l'hydrogène, (C₁-C₈)-alkyle, (C₆-C₁₄)-aryle éventuellement substitué, (C₆-C₁₄)-aryl-(C₁-C₈)-alkyle éventuellement substitué dans le radical aryle ou (C₃-C₈)-cycloalkyle;
R³ signifie l'hydrogène, (C₁-C₈)-alkyle, (C₆-C₁₄)-aryle éventuellement substitué, (C₆-C₁₄)-aryl-(C₁-C₈)-alkyle éventuellement substitué dans le radical aryle, (C₃-C₈)-cycloalkyle, (C₂-C₈)-alcényle, (C₂-C₈)- alcynyle, (C₂-C₈)-alcénylcarbonyle, (C₂-C₈)-alcynylcarbonyle, pyridyle, R¹¹NH, R⁴CO, COOR⁴, CON(CH₃)R⁴, CONHR⁴, CSNHR⁴, COOR¹⁵, CON(CH₃)R¹⁵ ou CONHR¹⁵ ;
R⁴ signifie l'hydrogène ou (C₁-C₂₈)-alkyle, qui peut être substitué éventuellement une ou plusieurs fois par des radicaux identiques ou différents de la série hydroxy, hydroxycarbonyle, aminocarbonyle, mono-, ou di-((C₁-C₁₈)-alkyl)-aminocarbonyle, amino-(C₂-C₁₈)- alkylaminocarbonyle, amino-(C₁-C₃)-alkyl-phényl-(C₁-C₃)- alkylaminocarbonyle, (C₁-C₁₈)-alkylcarbonylamino-(C₁-C₃)- alkylphényl-(C₁-C₃)-alkylaminocarbonyle, (C₁-C₁₈)- alkylcarbonylamino-(C₂-C₁₈)-alkylaminocarbonyle, (C₆-C₁₄)-aryl-(C₁- C₈)-alcoxycarbonyle, qui peut être substitué également dans le radical aryle, amino, mercapto, (C₁-C₁₈)-alcoxy, (C₁-C₁₈)- alcoxycarbonyle, (C₃-C₈)-cycloalkyle éventuellement substitué, HOS(O)₂-(C₁-C₃)-alkyle, R⁹NHS(O)₂-(C₁-C₃)-alkyle, (R⁸O)₂P(O)-(C₁- C₃)-alkyle, tétrazolyl-(C₁-C₃)-alkyle, halogène, nitro, trifluorométhyle et par le radical R⁵ ;
R⁵ signifie (C₆-C₁₄)-aryle éventuellement substitué, (C₆-C₁₄)-aryl-(C₁- C₈)-alkyle éventuellement substitué dans le radical aryle; un cycle hétérocyclique mono- ou bicyclique de 5 à 12 membres, qui peut être aromatique, en partie hydrogéné ou complètement hydrogéné et qui peut contenir un, deux ou trois hétéroatomes identiques ou différents de la série de l'azote, l'oxygène et le soufre, un radical R⁶ ou un radial R⁶CO-, dans lequel le radical aryle et le radical hétérocyclique indépendamment de celui-ci peut être substitué une ou plusieurs fois par des radicaux identiques ou différents de la série (C₁-C₁₈)-alkyle, (C₁-C₁₈)-alcoxy, halogène, nitro, amino ou trifluorométhyle ;
R⁶ est R⁷R⁸N, R⁷O ou R⁷S ou une chaîne latérale acide aminé, un radical acide aminé naturel ou non naturel, acide imino, acide azaamino éventuellement N-(C₁-C₈)-alkylé ou N-((C₆-C₁₄)-aryl-(C₁- C₈)-alkylé) ou un radical dipeptidique, qui peut être également substitué dans le radical aryle et/ou pour lequel la liaison peptide est réduite en -NH-CH₂-, ainsi que leur esters et amides, où à la place des groupes fonctionnels libres peut signifier éventuellement l'hydrogène ou un hydroxyméthyle et/ou dans lequel des groupes fonctionnels libres peuvent être protégés par des groupes protecteurs classiques dans la chimie des peptides ;
R⁷ signifie l'hydrogène, (C₁-C₁₈)-alkyle, (C₆-C₁₄)-aryl-(C₁-C₈)-alkyle, (C₁-C₁₈)-alkylcarbonyle, (C₁-C₁₈)-alcoxycarbonyle, (C₆-C₁₄)-aryl- carbonyle, (C₆-C₁₄)-aryl-(C₁-C₈)-alkylcarbonyle ou (C₆-C₁₄)-aryl-(C₁- C₁₈)-alkyloxycarbonyle, où les groupes alkyle peuvent être éventuellement substitués par un groupe amino et/ou les restes aryle peuvent être éventuellement substitués une ou plusieurs fois, de préférence une fois, par des radicaux identiques ou différents de la série (C₁-C₈)-alkyle, (C₁-C₈)-alcoxy, halogène, nitro, amino et trifluorométhyle, un radical acide aminé naturel ou non naturel, acide imino, acide azaamino éventuellement N-(C₁-C₈)-alkylé ou N- ((C₆-C₁₄)-aryl-(C₁-C₈)-alkylé) ou un radical dipeptidique, qui peut être également substitué dans le radical aryle et/ou dans lequel la liaison peptide est réduite en -NH-CH₂- ;
R⁸ signifie l'hydrogène, (C₁-C₁₈)-alkyle, (C₆-C₁₄)-aryle éventuellement substitué ou (C₆-C₁₄)-aryl-(C₁-C₈)-alkyle, qui peut être également substitué dans le radical aryle ;
R⁹ signifie l'hydrogène, aminocarbonyle, (C₁-C₁₈)-alkylaminocarbonyle, (C₃-C₈)-cycloalkylaminocarbonyle, (C₆-C₁₄)-arylaminocarbonyle éventuellement substitué, (C₁-C₁₈)-alkyle, (C₆-C₁₄)-aryle ou (C₃-C₈)- cycloalkyle éventuellement substitué ;
R¹⁰ signifie hydroxy, (C₁-C₁₈)-alcoxy, (C₆-C₁₄)-aryl-(C₁-C₈)-alcoxy, qui peut également être substitué dans le radical aryle, (C₆-C₁₄)-aryloxy éventuellement substitué, amino où mono- ou di-((C₁-C₁₈)-alkyl)- amino ;
R¹¹ signifie l'hydrogène, (C₁-C₁₈)-alkyle, R¹²CO, (C₆-C₁₄)-aryl-S(O)₂ éventuellement substitué, (C₁-C₁₈)-alkyl-S(O)₂, (C₆-C₁₄)-aryl-(C₁- C₈)-alkyle éventuellement substitué dans le radical aryle, R⁹NHS(O)₂ ou le radical R¹⁵;
R¹² signifie l'hydrogène, (C₁-C₁₈)-alkyle, (C₂-C₈)-alcényle, (C₂-C₈)- alcynyle, (C₆-C₁₄)-aryle éventuellement substitué, (C₁-C₁₈)-alcoxy, (C₆-C₁₄)-aryl-(C₁-C₈)-alcoxy, qui peut également être substitué dans le radical aryle, (C₆-C₁₄)-aryloxy éventuellement substitué, amino, mono- ou di-((C₁-C₁₈)-alkyl)-amino, le radical R¹⁵ ou le radical R¹⁵-O- ;
R¹³ signifie l'hydrogène, (C₁-C₆)-alkyle, (C₆-C₁₄)-aryl-(C₁-C₈)-alkyle éventuellement substitué dans le radical aryle ou (C₃-C₈)- cycloalkyle ;
R¹⁵ est R¹⁶-(C₁-C₆₎-alkyle ou R¹⁶ ;
R¹⁶ est un radical bicyclique ou tricyclique de 6 à 24 membres, qui est saturé ou partiellement insaturé et qui également peut contenir un à quatre hétéroatomes identiques ou différents de la série de l'azote, l'oxygène et le soufre et qui peut être également substitué par un ou plusieurs substituants identiques ou différents de le série de (C₁- C₄)-alkyle et oxo ;
R²¹ signifie l'hydrogène, (C₁-C₈)-alkyle, (C₃-C₁₂)-cycloalkyle, (C₃-C₁₂)- cycloalkyl-(C₁-C₈)-alkyle, (C₆-C₁₄)-aryle, (C₆-C₁₄)-aryl-(C₁-C₈)-alkyle, le radical hét- ou hét-(C₁-C₈)-alkyle, où les radicaux alkyle peuvent être substitués une fois ou plusieurs fois par le fluor où le radical R²¹ peut être identique ou différent pour des apparitions multiples ;
R²² signifie (C₃-C₁₂)-cycloalkyle, (C₃-C₁₂)-cycloalkyl-(C₁-C₈)-alkyle, (C₆- C₁₄)-aryle, le radical hét- ou hét-(C₁-C₈)-alkyle, où les radicaux alkyle peuvent être substitués une ou plusieurs fois par le fluor ;
R²³ signifie (C₁-C₈)-alkyle, (C₃-C₁₂)-cycloalkyle, (C₃-C₁₂)-cycloalkyl-(C₁- C₈)-alkyle, (C₆-C₁₄)-aryle, (C₆-C₁₄)-aryl-(C₁-C₈)-alkyle, le radical hét- ou' hét-(C₁-C₈)-alkyle, où les radicaux alkyle peuvent être substitués une ou plusieurs fois par le fluor ;
R²⁴ signifie (C₃-C₁₂)-cycloalkyle, (C₃-C₁₂)-cycloalkyl-(C₁-C₈)-alkyle, (C₆- C₁₄)-aryle, (C₆-C₁₄)-aryl-(C₁-C₈)-alkyle, le radical hét- ou hét-(C₁- C₈)-alkyle, où les radicaux alkyle peuvent être substitués une ou plusieurs fois par le fluor ;
R²⁵ a les significations de R²³, où les radicaux R²⁵ peuvent être identiques ou différents ;
R²⁶ a les significations de R²¹ ou signifie HO-((C₁-C₈)-alkyle), où les radicaux alkyle peuvent être substitués une ou plusieurs fois par le fluor ;
R²⁷ signifie l'hydrogène, (C₃-C₁₂)-cycloalkyle, (C₃-C₁₂)-cycloalkyl-(C₁- C₈)-alkyle, (C₆-C₁₄)-aryl-(C₁-C₈)-alkyle, le radical hét- ou hét-(C₁- C₈)-alkyle, où les radicaux alkyle peuvent être substitués une ou plusieurs fois par le fluor ;
R²⁸ est un des radicaux R²¹-, R²¹O-, R²⁶N(R²⁶)-, R²¹C(O)-, R²¹O-C(O)-, ((C₁-C₁₈)-alkyl-O-C(O)-((C₁-C₆)-alkyl)-O-C(O)-, R²¹N(R²¹)-C(O)-, R²¹N(R²¹)-C(=N(R²¹))- ou R²¹C(O)-N(R²¹)- ;
hét est un cycle aromatique ou non aromatique, mono ou polycyclique, de 4 à 14 membres, qui contient 1, 2, 3 ou 4 hétéroatomes identiques ou différents de la série de N, O et S comme membres cycliques et peut être éventuellement substitué par un ou plusieurs substituants identiques ou différents ;
b, c, d, et f sont indépendamment les uns des autres 0 ou 1, mais ne peuvent pas tous être en même temps 0 ;
e, g et h sont indépendamment les uns des autres 0, 1, 2, 3, 4, 5 ou 6.

3. Utilisation des composés de formule I selon la revendication 1 et/ou 2 dans toutes leurs formes stéréoisomères et leurs mélanges dans tous rapports ainsi que leurs sels compatibles physiologiquement, où dans la formule I R⁰ est (C₁-C₈)-alkyle, (C₃-C₁₂)-cycloalkyle, (C₃-C₁₂)-cycloalkyl-(C₁-C₈)-alkyle, (C₆-C₁₄)-aryle éventuellement substitué, (C₆-C₁₄)-aryl-(C₁-C₈)-alkyle éventuellement substitué dans le radical aryle, hétéroaryle éventuellement substitué ou hétéroaryl-(C₁-C₈)-alkyle éventuellement substitué dans le radical hétéroaryle, de préférence biphénylméthyle, naphtylmèthyle ou benzyle non substitué ou substitué une ou plusieurs fois dans le radical aryle.

4. Utilisation des composés de formule I selon la revendication 1 et/ou 3 dans toutes leurs formes stéréoisomères et leurs mélanges dans tous rapports ainsi que leurs sels compatibles physiologiquement, où dans la formule I en même temps
W est R¹-A-CH=C et dans lequel A est un radical phényle ou un radical méthylènephényle ou W est R¹-A-C(R¹³) et dans lequel A est un radical bivalent de la série du méthylène, éthylène, triméthylène, tétraméthylène, pentaméthylène, cyclohexylène, phénylène, phénylèneméthyle, méthylènephényle, méthylène- phénylméthyle ;
B est un radical bivalent de la série du méthylène, éthylène, triméthylène, tétraméthylène, vinylène, phénylène ou méthylène ou éthylène substitué ;
E signifie R¹⁰CO ;
R signifie l'hydrogène, (C₁-C₆)-alkyle ou benzyle ;
R⁰ est (C₁-C₈)-alkyle, (C₃-C₈)-cycloalkyle, (C₆-C₁₄)-aryle éventuellement substitué, ou (C₆-C₁₄)-aryl-(C₁-C₈)-alkyle éventuellement substitué dans le radical aryle ;
R¹ est un des radicaux R²¹O-, R²⁴NH-1 R²⁵N(R²⁵)-, HO-((C₁-C₈)-alkyl)- N(R²⁶)-, R²¹O-C(O)- et R²⁸N(R²¹)-C(O)- ;
R² est l'hydrogène ou un radical (C₁-C₈)alkyle ;
R³ est (C₁-C₈)alkyle, (C₆-C₁₄)-aryle éventuellement substitué, (C₆- C₁₄)-aryl-(C₁-C₈)-alkyle, (C₃-C₈)-cycloalkyle, (C₂-C₈)-alcényle, (C₂- C₈)-alcynyle, pyridyle, R¹¹NH, R⁴CO, COOR⁴, CONHR⁴, CSNHR⁴, COOR¹⁵ et CONHR¹⁵ ;
et e, g et h indépendamment les uns des autres sont les nombres 0, 1, 2 ou 3.

5. Utilisation des composés de formule I selon l'une ou plusieurs des revendications 1 à 4 dans toutes leurs formes stéréoisomères et leurs mélanges dans tous rapports ainsi que leurs sels compatibles physiologiquement, où dans la formule I W est R¹-A-C(R¹³) et R¹³ est (C₁-C₆)-alkyle, (C₆-C₁₄)-aryl-(C₁-C₈)-alkyle éventuellement substitué dans le radical aryle ou (C₃-C₈)cycloalkyle.

6. Utilisation des composés de formule I selon l'une ou plusieurs des revendications 1 à 5 dans toutes leurs formes stéréoisomères et leurs mélanges dans tous rapports ainsi que leurs sels compatibles physiologiquement, où dans la formule I R³ est (C₆-C₁₄)-aryle éventuellement substitué, COOR⁴, R¹¹NH ou CONHR⁴, où -NHR⁴ est un radical d'un α-aminoacide, ses ω-amino-(C₂-C₈)-alkylamides; ses (C₁-C₈)-alkylesters ou ses (C₆-C₁₄)-aryl-(C₁-C₄)-alkylesters, de préférence le radical des α-aminoacides valine, lysine, phénylglycine, phénylalanine ou tryptophane ou leurs (C₁-C₈)-alkylesters ou (C₆-C₁₄)-aryl-(C₁-C₄)-alkylesters.

7. Utilisation des composés de formule I selon l'une ou plusieurs des revendications 1 et 3 à 6 dans toutes leurs formes stéréoisomères et leurs mélanges dans tous rapports ainsi que leurs sels compatibles physiologiquement, où dans la formule I en même temps
W est R¹-A-C(R¹³);
Y est un groupe carbonyle;
Z est N(R⁰) ;
A est l'éthylène, le triméthylène, tétraméthylène, pentamèthylène, cyclohexylène, phénylène, phénylèneméthyle, méthylènephényle ou methylènephénylméthyle ;
B est un radical méthylène substitué ou non substitué ;
D est C(R²)(R³) ;
E est R¹⁰CO
R est l'hydrogène ou (C₁-C₄)-alkyle, en particulier l'hydrogène, le méthyle ou l'éthyle ;
R⁰ est (C₁-C₈)-alkyle, (C₃-C₈)-cycloalkyle, (C₆-C₁₄)-aryle éventuellement substitué, ou (C₆-C₁₄)-aryl-(C₁-C₈)-alkyle éventuellement substitué dans le radical aryle;
R¹ est le radical R²⁸N(R²¹)-C(O)- ;
R² est l'hydrogène;
R³ est le radical CONHR⁴ ;
R⁴ est le méthyle, qui est substitué par l'hydroxycarbonyle et un radical de la série de (C₁-C₄)-alkyle, phényle et benzyle, ou est le méthyle, qui est substitué par (C₁-C₈)-alcoxycarbonyle, de préférence (C₁- C₄)-alcoxycarbonyle, et un radical de la série de (C₁-C₄)-alkyle, phényle et benzyle ;
R¹⁰ est l'hydroxy ou (C₁-C₈)-alcoxy, de préférence (C₁-C₄)-alcoxy ;
R¹³ est (C₁-C₆)-alkyle, (C₃-C₇)-cycloalkyle ou benzyle, en particulier méthyle ;
b, c et d valent 1 et e, f et g valent 0;
h vaut 1 ou 2, de préférence 1.

8. Utilisation des composés de formule I selon l'une ou plusieurs des revendications 1, 3 et 4 dans toutes leurs formes stéréoisomères et leurs mélanges dans tous rapports ainsi que leurs sels compatibles physiologiquement, où dans la formule I en même temps
W est R¹-A-CH=C et dans lequel A est un radical phénylène ou un radical méthylènephényle ou W est R¹-A-C(R¹³) et dans lequel A est un radical bivalent de la série du méthylène, éthylène, triméthylène, tétraméthylène, pentaméthylène, cyclohexylène, phénylène, phénylèneméthyle, méthylènephényle, méthylène- phénylméthyle ;
B est un radical bivalent de la série du méthylène, éthylène, triméthylène, tétraméthylène, vinylène, phénylène ou méthylène ou éthylène substitué ;
E signifie R¹⁰ CO;
R signifie l'hydrogène ou (C₁-C₆)-alkyle ;
R⁰ est (C₁-C₈)-alkyle, (C₃-C₈)-cycloalkyle, (C₆-C₁₄)-aryle éventuellement substitué, ou (C₆-C₁₄)-aryl-(C₁-C₈)-alkyle éventuellement substitué dans le radical aryle ;
R¹ est un des radicaux R²¹O-, R²¹O-N(R²³)-, R²⁴NH-, R²⁵N(R²⁵)-, HO-((C₁-C₈)-alkyl)-N(R²⁶)-, R²¹O-C(O)- etR²⁸N(R²¹)-C(O)- ;
R² est l'hydrogène ou un radical (C₁-C₈)alkyle ;
R3 est CONHR¹⁵ ou CONHR⁴, où R⁴ ici est un radical (C₁-C₆)-alkyle non substitué au substitué par un ou plusieurs radicaux (C₆-C₁₄)- aryle ;
R¹⁵ est R¹⁶-(C₁-C₆)-alkyle ou R¹⁶, où R¹⁶ est un radical bicyclique ou tricyclique ponté de 7 à 12 membres, qui est saturé ou partiellement insaturé et qui peut contenir un à quatre hétéroatomes identiques ou différents de la série de l'azote, l'oxygène et le soufre et qui également peut être substitué par un ou plusieurs substituants identiques ou différents de la série de (C₁-C₄)-alkyle et oxo, et en particulier R¹⁵ est un radical adamantyle ou un radical adamantylméthyle ;
et e, g et h indépendamment les uns des autres sont les nombres 0, 1, 2 ou 3 et b, c et d sont le nombre 1.

9. Utilisation des composés de formule I selon l'une ou plusieurs des revendications 1, 3, 4, 5 et 8 dans toutes leurs formes stéréoisomères et leurs mélanges dans tous rapports ainsi que leurs sels compatibles physiologiquement, où dans la formule I en même temps
W est R¹-A-C(R¹³) ;
Y est un groupe carbonyle ;
Z est N(R⁰);
A est l'éthylène, le triméthylène, tétraméthylène, pentaméthylène, cyclohexylène, phénylène, phénylèneméthyle, méthylènephényle ou méthylènephénylméthyle ;
B est un radical méthylène non substitué ou substitué ;
D est C(R²)(R³) ;
E est R¹⁰CO ;
R est l'hydrogène ou (C₁-C₄)-alkyle, en particulier l'hydrogène, le méthyle ou l'éthyle ;
R⁰ est (C₁-C₈)-alkyle, (C₃-C₈)-cycloalkyle, (C₆-C₁₄)-aryle éventuellement substitué ou (C₆-C₁₄)-aryl-(C₁-C₈)-alkyle éventuellement substitué dans le radical aryle;
R¹ est le radical R²⁸N(R²¹)-C(O)-;
R² est l'hydrogène ;
R³ est CONHR¹⁵ ou CONHR⁴, où R⁴ ici est un radical (C₁-C₆)-alkyle non substitué ou substitué par un ou plusieurs radicaux (C₆-C₁₀)- aryle ;
R¹⁰ est un hydroxy ou (C₁-C₈)-alcoxy, de préférence (C₁-C₄)alcoxy ;
R¹³ est (C₁-C₆)-alkyle, (C₃-C₇)-cycloalkyle ou benzyle, en particulier méthyle ;
R¹⁵ est un radical adamantyle ou un radical adamantylméthyle ;
b, c et d valent, 1 et e, f et g valent 0 ;
h vaut 1 ou 2, de préférence 1.

10. Utilisation des composés de formule I selon l'une ou plusieurs des revendications 1, 3, 4 et 5 dans toutes leurs formes stéréoisomères et leurs mélanges dans tous, rapports ainsi que leurs sels compatibles physiologiquement, où dans la formule I en même temps
W est R¹-A-C(R¹³) ;
Y est un groupe carbonyle ;
Z est N(R⁰) ;
A est l'éthylène, le triméthylène, tétraméthylène, pentaméthylène, cyclohexylène, phénylène, phénylèneméthyle, méthylènephényle ou méthylènephénylméthyle ;
B est un radical méthylène ou éthylène non substitué ou substitué ;
D est C(R²)(R³);
E est R¹⁰CO;
R est l'hydrogène ou (C₁-C₄)-alkyle, en particulier l'hydrogène, le méthyle ou l'éthyle ;
R⁰ est (C₁-C₈)-alkyle, (C₃-C₈)-cycloalkyle, (C₆-C₁₄)-aryle éventuellement substitué ou (C₆-C₁₄)-aryl-(C₁-C₈)-alkyle éventuellement substitué dans le radical aryle ;
R¹ est un des radicaux R²¹O-, R²⁴NH-, R²⁵N(R²⁵)-, HO-((C₁-C₈)-alkyl)- N(R²⁶)-, R²¹O-C(O)- et R²⁸N(R²¹)-C(O)- ;
R² est l'hydrogène ;
R³ est un radial phényle ou un radical naphtyle non substitué, un radical phényle ou un radical naphtyle substitué par un, deux ou trois radicaux identiques ou différents de la série de (C₁-C₄)-alkyle, (C₁-C₄)-alcoxy, hydroxy, halogène, trifluorométhyle, nitro, méthylènedioxy, éthylènedioxy, hydroxycarbonyle, (C₁-C₄)- alcoxycarbonyle, aminocarbonyle, cyano, phényle, phénoxy et benzyloxy, un radical pyridyle, un radical (C₁-C₄)-alkyle, un radical (C₂-C₄)-alcènyle, un radical (C₂-C₄)-alcynyle ou un radical (C₅-C₆)- cycloalkyle, et en particulier R³ est un radical phényle ou un radical naphtyle non substitué ou substitué ;
R¹⁰ est un hydroxy ou (C₁-C₈)-alcoxy, en particulier (C₁-C₄)-alcoxy et de préférence R¹⁰ est un radical de la série de l'hydroxy, méthoxy, éthoxy, propoxy et isopropoxy ;
R¹³ est (C₁-C₆)-alkyle, (C₃-C₇)-cycloalkyle ou benzyle, en particulier méthyle ;
b, c et d valent 1 et e, f et g valent 0 ;
h vaut 1 ou 2, de préférence 1.

11. Utilisation des composés de formule I selon l'une ou plusieurs des revendications 1, 3, 4 et 5 dans toutes leurs formes stéréoisomères et leurs mélanges dans tous rapports ainsi que leurs sels compatibles physiologiquement, où dans la formule I en même temps
W est R¹-A-C(R¹³);
Y est un groupe carbonyle ;
Z est N(R⁰) ;
A est l'éthylène, le triméthylène, tétraméthylène, pentaméthylène, cyclohexylène, phénylène, phénylèneméthyle, méthylènephényle ou méthylènephénylméthyle ;
B est un radical méthylène ou éthylène non substitué ou substitué ;
D est C(R²)(R³) ;
E est R¹⁰CO ;
R est l'hydrogène ou (C₁-C₄)-alkyle, en particulier l'hydrogène, le méthyle ou l'éthyle ;
R⁰ est (C₁-C₈)-alkyle, (C₃-C₈)-cycloalkyle, (C₆-C₁₄)-aryle éventuellement substitué ou (C₆-C₁₄)-aryl-(C₁-C₈)-alkyle éventuellement substitué dans le radical aryle ;
R¹ est un des radicaux R²¹O-, R²⁴NH-, R²⁵N(R²⁵)-, HO-((C₁-C₈)-alkyl)- N(R²⁶)-, R²¹O-C(O)- et R²⁸N(R²¹)-C(O)- ;
R² est l'hydrogène ;
R³ est R¹¹NH ;
R¹⁰ est un hydroxy ou (C₁-C₈)-alcoxy, en particulier (C₁-C₄)-alcoxy et de préférence R¹⁰ est un radical de la série de l'hydroxy, méthoxy, éthoxy, propoxy et isopropoxy ;
R¹³ est (C₁-C₆)-alkyle, (C₃-C₇)-cycloalkyle ou benzyle, en particulier méthyle ;
b, c, d et e sont 1 et f et g sont 0 ;
h vaut 0.

12. Utilisation des composés de formule I selon l'une ou plusieurs des revendications 1 et 3 à 11 dans toutes leurs formes stéréoisomères et leurs mélanges dans tous rapports ainsi que leurs sels compatibles physiologiquement, où un radical méthylène substitué ou un radical éthylène substitué du groupe B porte comme substituants un radial de la série de (C₁-C₈)-alkyle, (C₂-C₆)-alcényle, (C₂-C₆)-alcynyle, (C₃-C₈)-cycloalkyle, en particulier (C₅-C₆)-cycloalkyle, (C₃-C₈)-cycloalkyl-(C₁-C₄)-alkyle, en particulier (C₅-C₆)-cycloalkyl-(C₁-C₄)-alkyle, (C₆-C₁₀)-aryle éventuellement substitué, (C₆-C₁₀)-aryl(C₁-C₄)-alkyle éventuellement substitué dans le radical aryle, hétéroaryle éventuellement substitué et hétéroaryl-(C₁-C₄)-alkyle éventuellement substitué dans le radical hétéroaryle.

13. Utilisation des composés de formule I selon l'une ou plusieurs des revendications 1 et 3 à 12 dans toutes leurs formes stéréoisomèrès et leurs mélanges dans tous rapports ainsi que leurs sels compatibles physiologiquement, où B est un radical méthylène non substitué ou un radical méthylène qui est substitué par un radical (C₁-C₈)-alkyle.

14. Utilisation des composés de formule I selon l'une ou plusieurs des revendications 1 à 13 et/ou leurs sels compatibles physiologiquement pour la préparation de médicaments pour l'inhibition de l'inflammation.

15. Utilisation des composés de formule I selon l'une ou plusieurs dès revendications 1 à 13 et/ou leurs sels compatibles physiologiquement pour la préparation de médicaments pour le traitement ou la prophylaxie de l'arthrite rhumatoïde, de la maladie inflammatoire de l'estomac, de l'érythèmatose lupus systémique ou des maladies inflammatoires du système nerveux central.

16. Utilisation des composés de formule I selon l'une ou plusieurs des revendications 1 à 13 et/ou leurs sels compatibles physiologiquement pour la préparation de médicaments pour le traitement ou la prophylaxie de l'asthme ou des allergies.

17. Utilisation des composés de formule I selon l'une ou plusieurs des revendications 1 à 13 et/ou leurs sels compatibles physiologiquement pour la préparation de médicaments pour le traitement ou la prophylaxie des maladies cardiovasculaires, de l'artériosclérose, de resténoses ou du diabète, pour la diminution du dommage des transplants d'organes, pour l'inhibition de la croissance tumorale ou des métastases tumorales ou pour la thérapie de malaria.

18. Utilisation des composés de formule I selon l'une ou plusieurs des revendications 1 à 13 et/ou leurs sels compatibles physiologiquement pour la préparation de médicaments pour l'inhibition de l'adhésion et/ou de la migration des leucocytes ou pour l'inhibition du récepteur VLA-4.

19. Utilisation des composés de formule I selon l'une ou plusieurs des revendications 1 à 13 et/ou leurs sels compatibles physiologiquement pour la préparation de médicaments pour le traitement ou la prophylaxie de maladies, pour lesquelles l'adhésion des leucocytes et/ou la migration des leucocytes présente une étendue non souhaitée, ou de maladies, pour lesquelles un phénomène d'adhésion dépendant de VLA-4 joue un rôle, pour le traitement ou la prophylaxie de l'arthrite rhumatoïde, de la maladie inflammatoire de l'estomac, de érythématose lupus systémique, des maladies inflammatoires du système nerveux central, de l'asthme, des allergies, maladies cardiovasculaires, de artériosclérose, de resténoses, du diabète, pour la diminution du dommages des transplants d'organes, pour l'inhibition de la croissance tumorale ou des métastases tumorales, pour la thérapie de malaria, ou comme inhibiteur d'inflammation.

20. Composés de formule Ib, 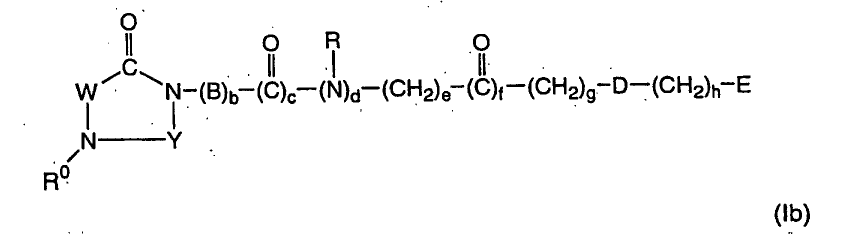 dans laquelle,
W est R¹-A-C(R¹³) ou R¹-A-CH=C ;
Y est un groupe carbonyle, thiocarbonyle ou méthylène ;
A est un radical bivalent de la série (C₁-C₆)-alkylène, (C₃-C₁₂)- cycloalkylène ; (C₁-C₆)-alkylène-(C₃-C₁₂)-cycloalkyle, phénylène, phénylène-(C₁-C₆)-alkyle, (C₁-C₆)-alkylène-phényle, (C₁-C₆)- alkylène-phényl-(C₁-C₆)-alkyle, phénylène-(C₂-C₆)-alcényle ou un radical bivalent d'un cycle saturé ou insaturé à 5 ou 6 membres, qui contient 1 ou 2 atomes d'azote et qui peut être substitué une ou deux fois par un groupe (C₁-C₆)-alkyle ou l'oxygène ou le soufre lié deux fois ;
B est un radical bivalent de la série (C₁-C₆)-alkylène, (C₂-C₆)- alcénylène, phénylène, phénylène-(C₁-C₃)-alkyle, (C₁-C₃)-alkylène- phényle ;
D est C(R²)(R³), N(R³) où CH=C(R³) ;
E signifie tétrazolyle, (R⁸O)₂P(O), HOS(O)₂, R⁹NHS(O)₂ ou R¹⁰CO ;
R et R⁰ indépendamment l'un de l'autre signifient l'hydrogène, (C₁-C₈)- alkyle, (C₃-C₁₂)-cycloalkyle, (C₃-C₁₂)-cycloalkyl-(C₁-C₈)-alkyle, (C₆- C₁₄)-aryle éventuellement substitué, (C₆-C₁₄)-aryl-(C₁-C₈)-alkyle éventuellement substitué dans le radical aryle, hétéroaryle éventuellement substitué ou hétéroaryl-(C₁-C₈)-alkyle éventuellement substitué dans le radical hétéroaryle, où les radicaux alkyle peuvent être substitués une ou plusieurs fois par le fluor ;
R¹ signifie le radical R²⁸N(R²¹)-C(O)- ;
R² signifie l'hydrogène, (C₁-C₈)-alkyle, (C₆-C₁₄)-aryle éventuellement substitué, (C₆-C₁₄)-aryl-(C₁-C₈)-alkyle éventuellement substitué dans le radical aryle ou (C₃-C₈)-cycloalkyle;
R³ signifie l'hydrogène, (C₁-C₈)-alkyle, (C₆-C₁₄)-aryle éventuellement substitué, (C₆-C₁₄)-aryl-(C₁-C₈)-alkyle éventuellement substitué dans le radical aryle, (C₃-C₈)-cycloalkyle, (C₂-C₈)-alcényle, (C₂-C₈)- alcynyle, (C₂-C₈)-alcénylcarbonyle, (C₂-C₈)-alcynylcarbonyle, pyridyle, R¹¹NH, R⁴CO, COOR⁴, CON(CH₃)R⁴, CONHR⁴, CSNHR⁴, COOR¹⁵, CON(CH₃)R¹⁵ ou CONHR¹⁵;
R⁴ signifie l'hydrogène ou (C₁-C₂₈)-alkyle, qui peut être substitué éventuellement une ou plusieurs fois par des radicaux identiques ou différents de la série hydroxy, hydroxycarbonyle, aminocarbonyle, mono- ou di-((C₁-C₁₈)-alkyl)-aminocarbonyle, amino-(C₂-C₁₈)- alkylaminocarbonyle, amino-(C₁-C₃)-alkyl-phényl-(C₁-C₃)-alkyl- aminocarbonyle, (C₁-C₁₈)-alkylcarbonylamino-(C₁-C₃)-alkylphényl- (C₁-C₃)-alkylaminocarbonyle, (C₁-C₁₈)-alkylcarbonylamino-(C₂-C₁₈)- alkylaminocarbonyle, (C₆-C₁₄)-aryl-(C₁-C₈)-alcoxycarbonyle, qui peut être substitué également dans le radical aryle, amino, mercapto, (C₁-C₁₈)-alcoxy, (C₁-C₁₈)-alcoxycarbonyle, (C₃-C₈)- cycloalkyle éventuellement substitué, HOS(O)₂-(C₁-C₃)-alkyle, R⁹NHS(O)₂-(C₁-C₃)-alkyle, (R⁸O)₂P(O)-(C₁-C₃)-alkyle, tétrazolyl-(C₁- C₃)-alkyle, halogène, nitro, trifluorométhyle et par le radical R⁵ ;
R⁵ signifie (C₆-C₁₄)-aryle éventuellement substitué, (C₆-C₁₄)-aryle-(C₁- C₈)-alkyle éventuellement substitué dans le radical aryle, un cycle hétérocyclique mono- ou bicyclique de 5 à 12 membres,qui peut être aromatique, en partie hydrogéné ou complètement hydrogéné et qui peut contenir un, deux ou trois hétéroatomes identiques ou différents de la série de l'azote, l'oxygène et le soufre, un radical R⁶ ou un radial R⁶CO-, dans lequel le radical aryle et le radical hétérocyclique indépendamment de celui-ci peut être substitué une ou plusieurs fois par des radicaux identiques ou différents de la série (C₁-C₁₈)-alkyle, (C₁-C₁₈)-alcoxy, halogène, nitro, amino ou trifluorométhyle ;
R⁶ est R⁷R⁸N, R⁷O ou R⁷S ou une chaîne latérale acide aminé, un radical acide aminé naturel ou non naturel, acide imino, acide azaamino éventuellement N-(C₁-C₈)-alkylé ou N-((C₆-C₁₄)-aryl-(C₁- C₈)-alkylé) ou un radical dipeptidique, qui peut être également substitué dans le radical aryle et/ou pour lequel la liaison peptide est réduite en -NH-CH₂-, ainsi que leur esters et amides, où à la place des groupes fonctionnels libres peut signifier éventuellement l'hydrogène ou un hydroxyméthyle et/ou dans lequel des groupes fonctionnels libres peuvent être protégés par des groupes protecteurs classiques dans la chimie des peptides ;
R⁷ signifie l'hydrogène, (C₁-C₁₈)-alkyle, (C₆-C₁₄)-aryl-(C₁-C₈)-alkyle, (C₁-C₁₈)-alkylcarbonyle, (C₁-C₁₈)-alcoxycarbonyle, (C₆-C₁₄)- arylcarbonyle, (C₆-C₁₄)-aryl-(C₁-C₈)-alkylcarbonyle ou (C₆-C₁₄)-aryl- (C₁-C₁₈)-alkyloxycarbonyle, où les groupes alkyle peuvent être éventuellement substitués par un groupe amino et/ou les restes aryle peuvent être éventuellement substitués une ou plusieurs fois, de préférence une fois, par des radicaux identiques ou différents de la série (C₁-C₈)-alkyle, (C₁-C₈)-alcoxy, halogène, nitro, amino et trifluorométhyle, un radical acide aminé naturel ou non naturel, acide imino, acide azaamino éventuellement N-(C₁-C₈)-alkylé ou N- ((C₆-C₁₄)-aryl-(C₁-C₈)-alkylé) ou un radical dipeptidique, qui peut être également substitué dans le radical aryle et/ou dans lequel la liaison peptide est réduite en -NH-CH₂- ;
R⁸ signifie l'hydrogène, (C₁-C₁₈)-alkyle, (C₆-C₁₄)-aryle éventuellement substitué, (C₆-C₁₄)-aryl-(C₁-C₈)-alkyle, qui peut être également substitué dans le radical aryle ;
R⁹ signifie l'hydrogène, aminocarbonyle, (C₁-C₁₈)-alkylaminocarbonyle, (C₃-C₈)-cycloalkylaminocarbonyle, (C₆-C₁₄)-arylaminocarbonyle éventuellement substitué, (C₁-C₁₈)-alkyle, (C₆-C₁₄)-aryle éventuellement substitué ou (C₃-C₈)-cycloalkyle;
R¹⁰ signifie hydroxy, (C₁-C₁₈)-alcoxy, (C₆-C₁₄)-aryl-(C₁-C₈)-alcoxy, qui peut également être substitué dans le radical aryle, (C₆-C₁₄)-aryloxy éventuellement substitué, amino ou mono- ou di-((C₁-C₁₈)-alkyl)- amino ;
R¹¹ signifie l'hydrogène, (C₁-C₁₈)-alkyle, R¹²CO, R^{12a}CS, (C₆-C₁₄)-aryl- S(O)₂ éventuellement substitué, (C₁-C₁₈)-alkyl-S(O)₂, (C₆-C₁₄)-aryl- (C₁-C₈)-alkyle éventuellement substitué dans le radical aryle, R⁹NHS(O)₂ ou le radical R¹⁵ ;
R¹² signifie l'hydrogène, (C₁-C₁₈)-alkyle, (C₂-C₈)-alcényle, (C₂-C₈)- alcynyle, (C₆-C₁₄)-aryle éventuellement substitué, (C₁-C₁₈)-alcoxy, (C₆-C₁₄)-aryl-(C₁-C₈)-alcoxy, qui peut également être substitué dans le radical aryle, (C₆-C₁₄)-aryloxy éventuellement substitué, le radical R¹⁵, le radical R¹⁵-O-, amino, mono- ou di-((C₁-C₁₈)-alkyl)-amino, (C₂-C₈)-alcényl-amino, (C₂-C₈)-alcynyl-amino, (C₃-C₁₂)-cycloalkyl- amino, (C₃-C₁₂)-cycloalkyl-(C₁-C₈)-alkyl-amino, le radical R¹⁵-NH-, (C₆-C₁₄)-aryl-NH éventuellement substitué, (C₆-C₁₄)-aryl-(C₁-C₈)- alkyl-NH, qui peut également être substitué dans le radical aryle, hétéroaryl-NH éventuellement substitué ou hétéroaryl-(C₁-C₈)-alkyl- NH éventuellement substitué dans le radical hétéroaryle ;
R^{12a} signifie amino, mono- ou di-((C₁-C₁₈)-alkyl)-amino, (C₂-C₈)-alcényl- amino, (C₂-C₈)-alcynyl-amino, (C₃-C₁₂)-cycloalkyl-amino, (C₃-C₁₂)- cycloalkyl-(C₁-C₈)-alkyl-amino, le radical R¹⁵-NH-, (C₆-C₁₄)-aryl-NH éventuellement substitué, (C₆-C₁₄)-aryl-(C₁-C₈)-alkyl-NH, qui peut être également substitué dans le radical aryle, hétéroaryl-NH éventuellement substitué ou hétéroaryl-(C₁-C₈)-alkyl-NH éventuellement substitué dans le radical hétéroaryle ;
R¹³ signifie l'hydrogène, (C₁-C₆)-alkyle, (C₆-C₁₄)-aryl-(C₁-C₈)-alkyle éventuellement substitué dans le radical aryle ou (C₃-C₈)- cycloalkyle ;
R¹⁵ est R¹⁶-(C₁-C₆)-alkyle ou R¹⁶ ;
R¹⁶ est un radical bicyclique ou tricyclique de 6 à 24 membres, qui est saturé ou partiellement insaturé et qui également peut contenir un à quatre hétéroatomes identiques ou différents de la série de l'azote, l'oxygène et le soufre et qui peut être également substitué par un ou plusieurs substituants identiques ou différents de le série de (C₁- C₄)-alkyle et oxo ;
R²¹ est l'hydrogène, (C₁-C₈)-alkyle, (C₃-C₁₂)-cycloalkyle, (C₃-C₁₂)- cycloalkyl-(C₁-C₈)-alkyle, (C₆-C₁₄)-aryle, (C₆-C₁₄)-aryl-(C₁-C₈)-alkyle, le radical hét- ou hét-(C₁-C₈)-alkyle, où les radicaux alkyle peuvent être substitués une fois ou plusieurs fois par le fluor où le radical R²¹ peut être identique ou différent pour des apparitions multiples ;
R²⁶ a les significations de R²¹ ou signifie HO-((C₁-C₈)-alkyle), où les radicaux alkyle peuvent être substitués une ou plusieurs fois par le fluor ;
R²⁸ est un des radicaux R²¹-, R²¹O-, R²⁶N(R²⁶)-, R²¹C(O)-, R²¹O-C(O)-, ((C₁-C₁₈)-alkyl-O-C(O)-((C₁-C₆)-alkyl)-O-C(O)-, R²¹N(R²¹)-C(O)-, R²¹N(R²¹)-C(=N(R²¹))- ou R²¹C(O)-N(R²¹)- ;
hét est un cycle aromatique ou non aromatique, mono ou polycyclique, de 4 à 14 membres, qui contient 1, 2, 3 ou 4 hétéroatomes identiques ou différents de la série de N, O et S comme membres cycliques et peut être éventuellement substitué par un ou plusieurs substituants identiques ou différents ;
b, c, d, et f sont indépendamment les uns des autres 0 ou 1, mais ne peuvent pas tous être en même temps 0 ;
e, g et h sont indépendamment les uns des autres 0, 1, 2, 3, 4, 5 ou 6 ;
dans toutes leurs formes stéréoisomères et leurs mélanges dans tous rapports ainsi que leurs sels compatibles physiologiquement.

21. Composés de formule Ib selon la revendication 20 et/ou leurs sels compatibles physiologiquement pour leur utilisation comme médicaments.

22. Préparation pharmaceutique, **caractérisée en ce qu'**elle contient un ou plusieurs composés de formule Ib selon la revendication 20 et/ou leurs sels compatibles physiologiquement à côté de matériaux supports et/ou d'additifs sûrs du point de vue pharmaceutique.

23. Composés de formule Ic, 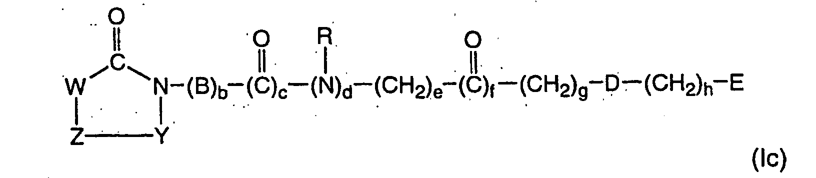 dans laquelle,
W est R¹-A-C(R¹³) ou R¹-A-CH=C ;
Y est un groupe carbonyle, thiocarbonyle ou méthylène ;
Z est N(R⁰), l'oxygène, le soufre ou un groupe méthylène ;
A est un radical (C₁-C₆)-alkylène bivalent,
B signifie un radical bivalent de la série (C₁-C₆)-alkylène, (C₂-C₆)- alcénylène, phénylène, phénylène-(C₁-C₃)-alkyle, (C₁-C₃)-alkylène- phényle ;
D est C(R²)(R³), N(R³) ou CH=C(R³) ;
E signifie tétrazolyle, (R⁸O)₂P(O), HOS(O)₂, R⁹NHS(O)₂ ou R¹⁰CO ;
R et R⁰ indépendamment l'un de l'autre signifient l'hydrogène, (C₁-C₈)- alkyle, (C₃-C₁₂)-cyclpalkyle, (C₃-C₁₂)-cycloalkyl-(C₁-C₈)-alkyle, (C₆- C₁₄)-aryle éventuellement substitué, (C₆-C₁₄)-aryl-(C₁-C₈)-alkyle éventuellement substitué dans le radical aryle, hétéroaryle éventuellement substitué ou hétéroaryl-(C₁-C₈)-alkyle éventuellement substitué dans le radical hétéroaryle, où les radicaux alkyle peuvent être substitués une ou plusieurs fois par le fluor ;
R¹ signifie l'hydrogène, (C₁-C₁₀)-alkyle qui peut être éventuellement substitué une ou plusieurs fois par le fluor, ou un des radicaux R⁴¹O-, R⁴¹O-N(R⁴²)-, R⁴²N(R⁴²)-, HO-((C₁-C₈)alkyll)-N(R⁴³)-, HC(O)-NH-, R⁴¹C(O)-N(R⁴²)-, R⁴¹C(O)-, R⁴¹O-C(O)-, R⁴⁴N(R⁴¹)-C(O)-, R⁴¹O-N=, O= et S= ;
R² signifie l'hydrogène, (C₁-C₈)-alkyle, (C₆-C₁₄)-aryle éventuellement substitué, (C₆-C₁₄)-aryl-(C₁-C₈)-alkyle éventuellement substitué dans le radical aryle ou (C₃-C₈)-cycloalkyle;
R³ signifie l'hydrogène, (C₁-C₈)-alkyle, (C₆-C₁₄)-aryle éventuellement substitué, (C₆-C₁₄)-aryl-(C₁-C₈)-alkyle éventuellement substitué dans le radical aryle, (C₃-C₈)-cycloalkyle, (C₂-C₈)-alcényle, (C₂-C₈)- alcynyle, (C₂-C₈)-alcénylcarbonyle, (C₂-C₈)-alcynylcarbonyle, pyridyle, R¹¹NH, R⁴CO, COOR⁴, CON(CH₃)R⁴, CONHR⁴, CSNHR⁴, COOR¹⁵, CON(CH₃)R¹⁵ ou CONHR¹⁵ ;
R⁴ signifie l'hydrogène ou (C₁-C₂₈)-alkyle, qui peut être substitué éventuellement une ou plusieurs fois par des radicaux identiques ou différents de la série hydroxy, hydroxycarbonyle, aminocarbonyle, mono- ou di-((C₁-C₁₈)-alkyl)-aminocarbonyle, amino-(C₂-C₁₈)- alkylaminocarbonyle, amino-(C₁-C₃)-alkyl-phényl-(C₁-C₃)-alkyl- aminocarbonyle, (C₁-C₁₈)-alkylcarbonylamino-(C₁-C₃)-alkylphényl- (C₁-C₃)-alkylaminocarbonyle, (C₁-C₁₈)-alkylcarbonylamino-(C₂-C₁₈)- alkyl-aminocarbonyle, (C₆-C₁₄)-aryl-(C₁-C₈)-alcoxycarbonyle, qui peut être substitué également dans le radical aryle, amino, mercapto, (C₁-C₁₈)-alcoxy, (C₁-C₁₈)-alcoxycarbonyle, (C₃-C₈)- cycloalkyle éventuellement substitué, HOS(O)₂-(C₁-C₃)-alkyle, R⁹NHS(O)₂-(C₁-C₃)-alkyle, (R⁸O)₂P(O)-(C₁-C₃)-alkyle, tétrazolyl-(C₁- C₃)-alkyle, halogène, nitro, trifluorométhyle et par le radical R⁵ ;
R⁵ signifie (C₆-C₁₄)-aryle éventuellement substitué, (C₆-C₁₄)-aryle-(C₁- C₈)-alkyle éventuellement substitué dans le radical aryle, un cycle hétérocyclique mono- ou bicyclique de 5 à 12 membres,qui peut être aromatique, en partie hydrogéné ou complètement hydrogéné et qui peut contenir un, deux ou trois hétéroatomes identiques ou différents de la série de l'azote, l'oxygène et le soufre, un radical R⁶ ou un radial R⁶CO-, dans lequel le radical aryle et le radical hétérocyclique indépendamment de celui-ci peut être substitué une ou plusieurs fois par des radicaux identiques ou différents de la série (C₁-C₁₈)-alkyle, (C₁-C₁₈)-alcoxy, halogène, nitro, amino ou trifluorométhyle;
R⁶ est R⁷R⁸N, R⁷O ou R⁷S ou une chaîne latérale acide aminé, un radical acide aminé naturel ou non naturel, acide imino, acide azaamino éventuellement N-(C₁-C₈)-alkylé ou N-((C₆-C₁₄)-aryl-(C₁- C₈)-alkylé) ou un radical dipeptidique, qui peut être également substitué dans le radical aryle et/ou pour lequel la liaison peptide est réduite en -NH-CH₂-, ainsi que leur esters et amides, où à la place des groupes fonctionnels libres peut signifier éventuellement l'hydrogène ou un hydroxyméthyle et/ou dans lequel des groupes fonctionnels libres peuvent être protégés par des groupes protecteurs classiques dans la chimie des peptides ;
R⁷ signifie l'hydrogène, (C₁-C₁₈)-alkyle, (C₆-C₁₄)-aryl-(C₁-C₈)-alkyle, (C₁-C₁₈)-alkylcarbonyle, (C₁-C₁₈)-alcoxycarbonyle, (C₆-C₁₄)- arylcarbonyle, (C₆-C₁₄)-aryl-(C₁-C₈)-alkylcarbonyle ou (C₆-C₁₄)-aryl- (C₁-C₁₈)-alkyloxycarbonyle, où les groupes alkyle peuvent être éventuellement substitués par un groupe amino et/ou les restes aryle peuvent être éventuellement substitués une ou plusieurs fois, de préférence une fois, par des radicaux identiques ou différents de la série (C₁-C₈)-alkyle, (C₁-C₈)-alcoxy, halogène, nitro, amino et trifluorométhyle, un radical acide aminé naturel ou non naturel, acide imino, acide azaamino éventuellement N-(C₁-C₈)-alkylé ou N- ((C₆-C₁₄)-aryl-(C₁-C₈)-alkylé) ou un radical dipeptidique, qui peut être également substitué, dans le radical aryle et/ou dans lequel la liaison peptide est réduite en -NH-CH₂- ;
R⁸ signifie l'hydrogène, (C₁-C₁₈)-alkyle, (C₆-C₁₄)-aryle éventuellement substitué, (C₆-C₁₄)-aryl-(C₁-C₈)-alkyle, qui peut être également substitué dans le radical aryle ;
R⁹ signifie l'hydrogène, aminocarbonyle, (C₁-C₁₈)-alkylamlnocarbonylé, (C₃-C₈)-cycloalkylaminocarbonyle, (C₆-C₁₄)-arylaminocarbonyle éventuellement substitué, (C₁-C₁₈)-alkyle, (C₆-C₁₄)-aryle éventuellement substitué ou (C₃-C₈)-cycloalkyle ;
R¹⁰ signifie hydroxy, (C₁-C₁₈)-alcoxy, (C₆-C₁₄)-aryl-(C₁-C₈)-alcoxy, qui peut également être substitué dans le radical aryle, (C₆-C₁₄)-aryloxy éventuellement substitué, amino ou mono- ou di-((C₁-C₁₈)-alkyl)- amino ;
R¹¹ signifie l'hydrogène, (C₁-C₁₈)-alkyle, R¹²CO, R^{12a}CS, (C₆-C₁₄)-aryl- S(O)₂ éventuellement substitué, (C₁-C₁₈)-alkyl-S(O)₂, (C₆-C₁₄)-aryl- (C₁-C₈)-alkyle éventuellement substitué dans le radical aryle, R⁹NHS(O)₂ ou le radical R¹⁵ ;
R¹² signifie l'hydrogène, (C₁-C₁₈)-alkyle, (C₂-C₈)-alcényle, (C₂-C₈)- alcynyle, (C₆-C₁₄)-aryle éventuellement substitué, (C₁-C₁₈)-alcoxy, (C₆-C₁₄)-aryl-(C₁-C₈)-alcoxy, qui peut également être substitué dans le radical aryle, (C₆-C₁₄)-aryloxy éventuellement substitué, le radical R¹⁵, le radical R¹⁵-O-, amine, mono- ou di-((C₁-C₁₈)-alkyl)-amino, (C₂-C₈)-alcényl-amino, (C₂-C₈)-alcynyl-amino, (C₃-C₁₂)-cycloalkyl- amino, (C₃-C₁₂)-cycloalkyl-(C₁-C₈)-alkyl-amino, le radical R¹⁵-NH-, (C₆-C₁₄)-aryl-NH éventuellement substitué, (C₆-C₁₄)-aryl-(C₁-C₈)- alkyl-NH, qui peut également être substitué dans le radical aryle, hétéroaryl-NH éventuellement substitué ou hétéroaryl-(C₁-C₈)-alkyl- NH éventuellement substitué dans le radical hétéroaryle ;
R^{12a} signifie amino, mono- ou di-((C₁-C₁₈)-alkyl)-amino, (C₂-C₈)-alcényl- amino, (C₂-C₈)-alcynyl-amino, (C₃-C₁₂)-cycloalkyl-amino, (C₃-C₁₂)- cycloalkyl-(C₁-C₈)-alkyl-amino, le radical R¹⁵-NH-, (C₆-C₁₄)-aryl-NH éventuellement substitué, (C₆-C₁₄)-aryl-(C₁-C₈)-alkyl-NH, qui peut être également substitué dans le radical aryle, hétéroaryl-NH éventuellement substitué ou hétéroaryl-(C₁-C₈)-alkyl-NH éventuellement substitué dans le radical hétéroaryle ;
R¹³ signifie l'hydrogène, (C₁-C₆)-alkyle, (C₆-C₁₄)-aryl-(C₁-C₈)-alkyle éventuellement substitué dans le radical aryle ou (C₃-C₈)- cycloalkyle ;
R¹⁵ est R¹⁶-(C₁-C₆)-alkyle ou R¹⁶ ;
R¹⁶ est un radical bicyclique ou tricyclique de 6 à 24 membres, qui est saturé ou partiellement insaturé et qui également peut contenir un à quatre hétéroatomes identiques ou différents de la série de l'azote, l'oxygène et le soufre et qui peut être également substitué par un ou plusieurs substituants identiques ou différents de le série de (C₁- C₄)-alkyle et oxo ;
R⁴¹ signifie l'hydrogène ou (C₁-C₈)-alkyle, où les radicaux alkyle peuvent être substitués une ou plusieurs fois par le fluor et les radicaux R⁴¹ peuvent être pour plusieurs apparitions identiques ou différents ;
R⁴² signifie (C₁-C₈)-alkyle, où les radicaux alkyle peuvent être substitués une ou plusieurs fois par le fluor et les radicaux R⁴² peuvent être pour plusieurs apparitions identiques ou différents ;
R⁴³ a les significations de R⁴¹ ou HO-((C₁-C₈)-alkyl), où les radicaux alkyle peuvent être substitués une ou plusieurs fois par le fluor ;
R⁴⁴ est un des radicaux R⁴¹-, R⁴¹O-, R⁴³N(R⁴³)-, R⁴¹C(O)-, R⁴¹O-C(O)-, ((C₁-C₁₈)-alkyl-O-C(O)-((C₁-C₆)-alkyl-O-C(O)-, R⁴¹N(R⁴¹)-C(O)-, R⁴¹N(R⁴¹)-C(=N(R⁴¹))- ou R⁴¹C(O)-N(R⁴¹)- ;
b, c, d et f indépendamment les uns des autres sont 0 ou 1, mais ne peuvent pas être tous en même temps 0;
e, g et h indépendamment les uns des autres sont 0, 1, 2, 3, 4, 5 ou 6 ;
dans toutes leurs formes stéréoisomères et leurs mélanges dans tous rapports ainsi que leurs sels compatibles physiologiquement.

24. Composés de formule Ic selon la revendication 24 et/ou leurs sels compatibles physiologiquement pour leur utilisation comme médicaments.

25. Préparation pharmaceutique, **caractérisée en ce qu'**elle contient un ou plusieurs composés de formule le selon la revendication 23 et/ou leurs sels compatibles physiologiquement à côté de matériaux supports et/ou d'additifs sûrs du point de vue pharmaceutique.

26. Composés de formule Id, 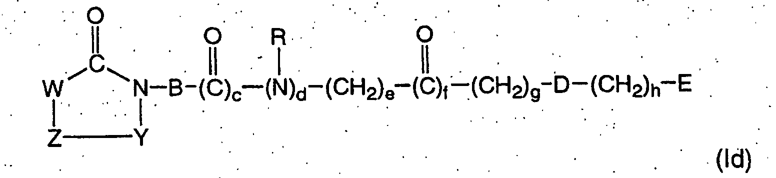 dans laquelle,
W est R¹-A-C(R¹³) ou R¹-A-CH=C ;
Y est un groupe carbonyle, thiocarbonyle ou méthylène ;
Z est N(R⁰), l'oxygène, le soufre ou un groupe méthylène ;
A est un radical bivalent de la série (C₁-C₆)-alkylène, (C₃-C₁₂)- cycloalkylène ; (C₁-C₆)-alkylène-(C₃-C₁₂)-cycloalkyle, phénylène, phénylène-(C₁-C₆)-alkyle, (C₁-C₆)-alkylène-phényle, (C₁-C₆)- alkylène-phényl-(C₁-C₆)-alkyle, phénylène-(C₂-C₆)-alcényle ou un radical bivalent d'un cycle saturé ou insaturé à 5 ou 6 membres, qui contient 1 ou 2 atomes d'azote et qui peut être substitué une ou deux fois par un groupe (C₁-C₆)-alkyle ou l'oxygène ou le soufre lié deux fois ;
B signifie un radical bivalent (C₁-C₆)-alkylène, qui est substitué par un radical de la série (C₁-C₈)-alkyle, (C₂-C₈)-alcényle, (C₂-C₈)- alcynyle, (C₃-C₁₀)-cycloalkyle, (C₃-C₁₀)-cycloalkyl-(C₁-C₆)-alkyle, (C₆-C₁₄)-aryle éventuellement substitué, (C₆-C₁₄)-aryl-(C₁-C₆)-alkyle éventuellement substitué dans le radical aryle, hétéroaryle éventuellement substitué et hétéroaryl-(C₁-C₆)-alkyle éventuellement substitué dans le radical hétéroaryle ;
D est C(R²)(R³), N(R³) ou CH=C(R³) ;
E signifie tétrazolyle, (R⁸O)₂P(O), HOS(O)₂, R⁹NHS(O)₂ ou R¹⁰CO ;
R signifie l'hydrogène, (C₁-C₈)-alkyle, (C₃-C₁₂)-cycloalkyle, (C₃-C₁₂)- cycloalkyl-(C₁-C₈)-alkyle, (C₆-C₁₄)-aryle éventuellement substitué, (C₈-C₁₄)-aryl-(C₁-C₈)-alkyle éventuellement substitué dans le radical aryle, hétéroaryle éventuellement substitué ou hétéroaryl-(C₁-C₈)- alkyle éventuellement substitué dans le radical hétéroaryle, où les radicaux alkyle peuvent être substitués une ou plusieurs fois par le fluor ;
R⁰ est l'hydrogène, (C₁-C₈)-alkyle, (C₃-C₁₂)-cycloalkyle, (C₃-C₁₂)- cycloalkyl-(C₁-C₈)-alkyle, (C₆-C₁₂)-bicycloalkyle, (C₆-C₁₂)- bicycloalkyl-(C₁-C₈)-alkyle, (C₆-C₁₂)-tricycloalkyle, (C₆-C₁₂)- tricycloalkyl-(C₁-C₈)-alkyle, (C₆-C₁₄)-aryle éventuellement substitué, (C₆-C₁₄)-aryl-(C₁-C₈)-alkyle éventuellement substitué dans le radical aryle, hétéroaryle éventuellement substitué, hétéroary(-(C₁-C₈)- alkyle éventuellement susbstitué dans le radical hétéroaryle, CHO, (C₁-C₈)-alkyle-CO, (C₃-C₁₂)-cycloalkyl-CO,(C₃-C₁₂)-cycloalkyl-(C₁- C₈)-alkyl-CO, (C₆-C₁₂)-bicycloalkyl-CO,(C₆-C₁₂)-bicycloalkyl-(C₁-C₈)- alkyl-CO, (C₆-C₁₂)-tricycloalkyl-CO,(C₆-C₁₂)-tricycloalkyl-(C₁-C₈)- alkyl-CO, (C₆-C₁₄)-aryl-CO éventuellement substitué, (C₆-C₁₄)-aryl- (C₁-C₈)-alkyl-CO éventuellement substitué dans le radical aryle, hétéroaryl-CO éventuellement substitué, hétéroaryl-(C₁-C₈)-alkyl- CO éventuellement substitué dans le radical hétéroaryle, (C₁-C₈)- alkyl-S(O)ₙ, (C₃-C₁₂)-cycloalkyl-S(O)ₙ, (C₃-C₁₂)-cycloalkyl-(C₁-C₈)- alkyl-S(O)ₙ, (C₆-C₁₂)-bicycloalkyl-S(O)ₙ, (C₆-C₁₂)-bicycloalkyl-(C₁- C₈)-alkyl-S(O)ₙ, (C₆-C₁₂)-tricycloalkyl-S(O)ₙ, (C₆-C₁₂)-tricycloalkyl- (C₁-C₈)-alkyl-S(O)ₙ, (C₆-C₁₄)-aryl-S(O)ₙ éventuellement substitué, (C₆-C₁₄)-aryl-(C₁-C₈)-alkyl-S(O)ₙ éventuellement substitué dans le radical aryle, hétéroaryl-S(O)ₙ éventuellement substitué ou hétéroaryl-(C₁-C₈)-alkyl-S(O)ₙ éventuellement substitué dans le radical hétéroaryle, n vaut 1 ou 2 ;
R¹ signifie l'hydrogène, (C₁-C₁₀)-alkyle qui peut être éventuellement substitué une ou plusieurs fois par le fluor, (C₃-C₁₂)-cycloalkyle, (C₃-C₁₂)-cycloalkyl-(C₁-C₈)-alkyle, R²¹-((C₆-C₁₄)-aryle) éventuellement substitué, (R²¹-((C₆-C₁₄)-aryl))-(C₁-C₈)-alkyle éventuellement substitué dans le radical aryle, le radical hét, hét- (C₁-C₈)-alkyle ou un des radicaux R²¹O-, R²²O-NH-, R²¹O-N(R²³)-, R²⁴NH-, R²⁵N(R²⁵)-, HO-((C₁-C₈)-alkyl)-N(R²⁶)-, R²⁷C(O)-NH-, R²¹C(O)-N(R²³)-, R²¹C(O)-, R²¹O-C(O)-, R²⁸N(R²¹)-C(O)-, R²¹O-N=, O= et S= ;
R² signifie l'hydrogène, (C₁-C₈)-alkyle, (C₆-C₁₄)-aryle éventuellement substitué, (C₆-C₁₄)-aryl-(C₁-C₈)-alkyle éventuellement substitué dans le radical aryle ou (C₃-C₈)-cycloalkyle;
R³ signifie l'hydrogène, (C₁-C₈)-alkyle, (C₆-C₁₄)-aryle éventuellement substitué, (C₆-C₁₄)-aryl-(C₁-C₈)-alkyle éventuellement substitué dans le radical aryle, (C₃-C₈)-cycloalkyle, (C₂-C₈)-alcényle, (C₂-C₈)- alcynyle, (C₂-C₈)-alcénylcarbonyle, (C₂-C₈)-alcynylcarbonyle, pyridyle, R¹¹NH, R⁴CO, COOR⁴, CON(CH₃)R⁴, CONHR⁴, CSNHR⁴, COOR¹⁵, CON(CH₃)R¹⁵ ou CONHR¹⁵ ;
R⁴ signifie l'hydrogène où (C₁-C₂₈)-alkyle, qui peut être substitué éventuellement une ou plusieurs fois par des radicaux identiques ou différents de la série hydroxy, hydroxycarbonyle, aminocarbonyle, mono- ou di-((C₁-C₁₈)-alkyl)-aminocarbonyle, amino-(C₂-C₁₈)- alkylaminocarbonyle, amino-(C₁-C₃)-alkyl-phényl-(C₁-C₃)-alkyl- aminocarbonyle, (C₁-C₁₈)-alkylcarbonylamino-(C₁-C₃)-alkylphényl- (C₁-C₃)-alkylaminocarbonyle, (C₁-C₁₈)-alkylcarbonylamino-(C₂-C₁₈)- alkyl- aminocarbonyle, (C₆-C₁₄)-aryl-(C₁-C₈)-alcoxycarbonyle, qui peut être substitué également dans le radical aryle, amino, mercapto, (C₁-C₁₈)-alcoxy, (C₁-C₁₈)-alcoxycarbonyle, (C₃-C₈)- cycloalkyle éventuellement substitué, HOS(O)₂-(C₁-C₃)-alkyle, R⁹NHS(O)₂-(C₁-C₃)-alkyle, (R⁸O)₂P(O)-(C₁-C₃)-alkyle, tétrazolyl-(C₁- C₃)-alkyle, halogène, nitro, trifluorométhyle et par le radical R⁵ ;
R⁵ signifie (C₆-C₁₄)-aryle éventuellement substitué, (C₆-C₁₄)-aryle-(C₁- C₈)-alkyle éventuellement substitué dans le radical aryle, un cycle hétérocyclique mono- ou bicyclique de 5 à 12 membres,qui peut être aromatique, en partie hydrogéné ou complètement hydrogéné et qui peut contenir un, deux ou trois hétéroatomes identiques ou différents de la série de l'azote, l'oxygène et le soufre, un radical R⁶ ou un radial R⁶CO-, dans lequel le radical aryle et le radical hétérocyclique indépendamment de celui-ci peut être substitué une ou plusieurs fois par des radicaux identiques ou différents de la série (C₁-C₁₈)-alkyle, (C₁-C₁₈)-alcoxy, halogène, nitro, amino ou trifluorométhyle ;
R⁶ est R⁷R⁸N, R⁷O ou R⁷S ou une chaîne latérale acide aminé, un radical acide aminé naturel ou non naturel, acide imino, acide azaamino éventuellement N-(C₁-C₈)-alkylé ou N-((C₆-C₁₄)-aryl-(C₁- C₈)-alkylé) ou un radical dipeptidique, qui peut être également substitué dans le radical aryle et/ou pour lequel la liaison peptide est réduite en -NH-CH₂-, ainsi que leur esters et amides, où à la place des groupes fonctionnels libres peut signifier éventuellement l'hydrogène ou un hydroxyméthyle et/ou dans lequel des groupes fonctionnels libres peuvent être protégés par des groupes protecteurs classiques dans la chimie des peptides ;
R⁷ signifie l'hydrogène, (C₁-C₁₈)-alkyle, (C₆-C₁₄)-aryl-(C₁-C₈)-alkyle, (C₁-C₁₈)-alkylcarbonyle, (C₁-C₁₈)-alcoxycarbonyle, (C₆-C₁₄)- arylcarbonyle, (C₆-C₁₄)-aryl-(C₁-C₈)-alkylcarbonyle ou (C₆-C₁₄)-aryl- (C₁-C₁₈)-alkyloxycarbonyle, où les groupes alkyle peuvent être éventuellement substitués par un groupe amino et/ou les restes aryle peuvent être éventuellement substitués une ou plusieurs fois, de préférence une fois, par des radicaux identiques ou différents de la série (C₁-C₈)-alkyle, (C₁-C₈)-alcoxy, halogène, nitro, amino et trifluorométhyle, un radical acide aminé naturel ou non naturel, acide imino, acide azaamino éventuellement N-(C₁-C₈)-alkylé ou N- ((C₆-C₁₄)-aryl-(C₁-C₈)-alkylé) ou un radical dipeptidique, qui peut être également substitué dans le radical aryle et/ou dans lequel la liaison peptide est réduite en -NH-CH₂- ;
R⁸ signifie l'hydrogène, (C₁-C₁₈)-alkyle, (C₆-C₁₄)-aryle ou (C₆-C₁₄)-aryl- (C₁-C₈)-alkyle éventuellement substitué, qui peut être également substitué dans le radical aryle ;
R⁹ signifie l'hydrogène, aminocarbonyle, (C₁-C₁₈)-alkylaminocarbonyle, (C₃-C₈)-cycloalkylaminocarbonyle, (C₆-C₁₄)-arylaminocarbonyle éventuellement substitué, (C₁-C₁₈)-alkyle, (C₆-C₁₄)-aryle éventuellement substitué ou (C₃-C₈)-cycloalkyle ;
R¹⁰ signifie hydroxy, (C₁-C₁₈)-alcoxy, (C₆-C₁₄)-aryl-(C₁-C₈)-alcoxy, qui peut également être substitué dans le radical aryle, (C₆-C₁₄)-aryloxy éventuellement substitué, amino ou mono- ou di-((C₁-C₁₈)-alkyl)- amino ;
R¹¹ signifie l'hydrogène, (C₁-C₁₈)-alkyle, R¹²CO, R^{12a}CS, (C₆-C₁₄)-aryl- S(O)₂ éventuellement substitué, (C₁-C₁₈)-alkyl-S(O)₂, (C₆-C₁₄)-aryl- (C₁-C₈)-alkyle éventuellement substitué dans le radical aryle, R⁹NHS(O)₂ ou le radical R¹⁵ ;
R¹² signifie l'hydrogène, (C₁-C₁₈)-alkyle, (C₂-C₈)-alcényle, (C₂-C₈)- alcynyle, (C₆-C₁₄)-aryle éventuellement substitué, (C₁-C₁₈)-alcoxy, (C₆-C₁₄)-aryl-(C₁-C₈)-alcoxy, qui peut également être substitué dans le radical aryle, (C₆-C₁₄)-aryloxy éventuellement substitué, le radical R¹⁵, le radical R¹⁵-O-, amino, mono- ou di-((C₁-C₁₈)-alkyl)-amino, (C₂-C₈)-alcényl-amino, (C₂-C₈)-alcynyl-amino, (C₃-C₁₂)-cycloalkyl- amino, (C₃-C₁₂)-cycloalkyl-(C₁-C₈)-alkyl-amino, le radical R¹⁵-NH-, (C₆-C₁₄)-aryl-NH éventuellement substitué, (C₆-C₁₄)-aryl-(C₁-C₈)- alkyl-NH, qui peut également être substitué dans le radical aryle, hétéroaryl-NH éventuellement substitué ou hétéroaryl-(C₁-C₈)-alkyl- NH éventuellement substitué dans le radical hétéroaryle ;
R^{12a} signifie amino, mono- ou di-((C₁-C₁₈)-alkyl)-amino, (C₂-C₈)-alcényl- amino, (C₂-C₈)-alcynyl-amino, (C₃-C₁₂)-cycloalkyl-amino, (C₃-C₁₂)- cycloalkyl-(C₁-C₈)-alkyl-amino, le radical R¹⁵-NH-, (C₆-C₁₄)-aryl-NH éventuellement substitué, (C₆-C₁₄)-aryl-(C₁-C₈)-alkyl-NH, qui peut être également substitué dans le radical aryle, hétéroaryl-NH éventuellement substitué ou hétéroaryl-(C₁-C₈)-alkyl-NH éventuellement substitué dans le radical hétéroaryle ;
R¹³ signifie l'hydrogène, (C₁-C₆)-alkyle, (C₆-C₁₄)-aryl-(C₁-C₈)-alkyle éventuellement substitué dans le radical aryle ou (C₃-C₈)- cycloalkyle ;
R¹⁵ est R¹⁶-(C₁-C₆)-alkyle ou R¹⁶ ;
R¹⁶ est un radicale bicyclique ou tricyclique de 6 à 24 membres, qui est saturé ou partiellement insaturé et qui également peut contenir un à quatre hétéroatomes identiques ou différents de la série de l'azote, l'oxygène et le soufre et qui peut être également substitué par un ou plusieurs substituants identiques ou différents de le série de (C₁- C₄)-alkyle et oxo ;
R²¹ est l'hydrogène, (C₁-C₈)-alkyle, (C₃-C₁₂)-cycloalkyle, (C₃-C₁₂)- cycloalkyl-(C₁-C₈)-alkyle, (C₆-C₁₄)-aryle, (C₆-C₁₄)-aryl-(C₁-C₈)-alkyle, le radical hét- ou hét-(C₁-C₈)-alkyle, où les radicaux alkyle peuvent être substitués une fois ou plusieurs fois par le fluor où le radical R²¹ peut être identique ou différent pour des apparitions multiples;
R²² signifie (C₃-C₁₂)-cycloalkyle, (C₃-C₁₂)-cycloalkyl-(C₁-C₈)-alkyle, (C₆- C₁₄)-aryle, le radical hét- ou hét-(C₁-C₈)-alkyle, où les radicaux alkyle peuvent être substitués une ou plusieurs fois par le fluor ;
R²³ signifie (C₁-C₈)-alkyle, (C₃-C₁₂)-cycloalkyle, (C₃-C₁₂)-cycloalkyl-(C₁- C₈)-alkyle, (C₆-C₁₄)-aryle, (C₆-C₁₄)-aryl-(C₁-C₈)-alkyle, le radical hét- ou hét-(C₁-C₈)-alkyle, où les radicaux alkyle peuvent être substitués une ou plusieurs fois par le fluor ;
R²⁴ signifie (C₃-C₁₂)-cycloalkyle, (C₃-C₁₂)-cycloalkyl-(C₁-C₈)-alkyle, (C₆- C₁₄)-aryle, (C₆-C₁₄)-aryl-(C₁-C₈)-alkyle, le radical hét- ou hét-(C₁- C₈)-alkyle, où les radicaux alkyle peuvent être substitués une ou plusieurs fois par le fluor ;
R²⁵ a les significations de R²³, où les radicaux R²⁵ peuvent être identiques ou différents ;
R²⁶ a les significations de R²¹ ou signifie HO-((C₁-C₈)-alkyle), où les radicaux alkyle peuvent être substitués une ou plusieurs fois par le fluor ;
R²⁷ signifie l'hydrogène, (C₃-C₁₂)-cycloalkyle, (C₃-C₁₂)-cycloalkyl-(C₁- C₈)-alkyle, (C₈-C₁₄)-aryl-(C₁-C₈)-alkyle, le radical hét- ou hét-(C₁- C₈)-alkyle, où les radicaux alkyle peuvent être substitués une ou plusieurs fois par le fluor ;
R²⁸ est le radical R²¹-, R²¹O-, R²⁶N(R²⁶)-, R²¹C(O)-, R²¹O-C(O)-, ((C₁-C₁₈)-alkyl-O-C(O)-((C₁-C₆)-alkyl)-O-C(O)-, R²¹N(R²¹)-C(O), R²¹N(R²¹)-C(=N(R²¹))- ou R²¹C(O)-N(R²¹)- ;
Hét est un cycle aromatique ou non aromatique, mono ou polycyclique, de 4 à 14 membres, qui contient 1, 2, 3 ou 4 hétéroatomes identiques ou différents de la série de N, O et S comme membres cycliques et peut être éventuellement substitué par un ou plusieurs substituants identiques ou différents;
c, d, et f sont indépendamment les uns des autres 0 ou 1, mais ne peuvent pas tous être en même temps 0 ;
e, g et h sont indépendamment les uns des autres 0, 1, 2, 3, 4, 5 ou 6 ;
dans toutes leurs formes stéréoisomères et leurs mélangés dans tous rapports ainsi que leurs sels compatibles physiologiquement ;
où les groupes R¹-A- ne peuvent pas être un phényle, pyrrolyle, imidazolyle ou pyridyle, où ces radiaux peuvent être substitués par (C₁-C₈)-alkyle, (C₁-C₈)-alcoxy, trifluorométhyle, hydroxy, hydroxy-(C₁-C₄)-alkyle, formyle, hydroxycarbonyle, aminocarbonyle, (C₁-C₄)-alcoxycarbonyle, phényle, phénoxy, benzyloxy, tétrazolyle, phényl-(C₁-C₄)-alkyle et (C₁-C₄)-alkyl-CO, et les groupes R¹-A- ne peuvent pas être le radial R^{x}-(C₁-C₂)-alkyle, où R^{x} est un phényle, furyle, thiényle, pyrrolyle, imidazolyle ou pyridyle, où ces radicaux peuvent être également benzanellés et peuvent être substitués par (C₁-C₈)-alkyle, (C₁-C₈)-alcoxy, halogène, nitro, amino, trifluorométhyle, hydroxy, hydroxy-(C₁-C₄)-alkyle, méthylènedioxy, éthylènedioxy, cyano, formyle,; hydroxycarbonyle, aminocarbonyle, (C₁-C₄)-alcoxycarbonyle, phényle, phénoxy, benzyloxy, tétrazolyle, phényl-(C₁-C₄)-alkyle et (C₁-C₄)-alkyl-CO.

27. Composés de formule Id selon la revendication 26, dans laquelle en même temps
W est R¹-A-C(R¹³) ;
Y est un groupe carbonyle ;
Z est N(R⁰) ;
A est un radical bivalent de la série (C₃-C₇)-cycloalkylène, phénylène, phénylène-(C₁-C₆)-alkyle, (C₁-C₆)-alkylène-phényle, ou un radical bivalent d'un cycle. saturé ou insaturé à 5 ou 6 membres, qui contient 1 ou 2 atomes d'azote et qui peut être substitué une ou deux fois par un groupe (C₁-C₆)-alkyle ou par l'oxygène ou le soufre lié deux fois ;
B est un radical méthylène ou un radical éthylène bivalent qui est substitué par un radical de la série (C₁-C₈)-alkyle, (C₂-C₈)-alcényle, (C₂-C₈)-alcynyle, (C₃-C₁₀)-cycloalkyle, (C₃-C₁₀)-cycloalkyl-(C₁-C₆)- alkyle, (C₆-C₁₄)-aryle éventuellement susbtitué, (C₆-C₁₄)-aryl-(C₁- C₆)-alkyle éventuellement substitué dans le radical aryle, hétéroaryle éventuellement substitué et hétéroaryl-(C₁-C₆)-alkyle éventuellement substitué dans le radical hétéroaryle ;
D est C(R²)(R³) ;
E signifie tétrazolyle ou R¹⁰CO ;
R signifie l'hydrogène ou (C₁-C₈)-alkyle ;
R⁰ est l'hydrogène, (C₁-C₈)-alkyle, (C₃-C₁₂)-cycloalkyle, (C₃-C₁₂)- cycloalkyl-(C₁-C₈)-alkyle, (C₆-C₁₂)-bicycloalkyle, (C₆-C₁₂)- bicycloalkyl-(C₁-C₈)-alkyle, (C₆-C₁₂)-tricycloalkyle, (C₆-C₁₂)- tricycloalkyl-(C₁-C₈)-alkyle, (C₆-C₁₄)-aryle éventuellement substitué, (C₆-C₁₄)-aryl-(C₁-C₈)-alkyle éventuellement substitué dans le radical aryle, hétéroaryle éventuellement substitué, hétéroaryl-(C₁-C₈)- alkyle éventuellement substitue dans le radical hétéroaryle, CHO, (C₁-C₈)-alkyl-CO, (C₃-C₁₂)-cycloalkyl-CO, (C₃-C₁₂)-cycloalkyl-(C₁- C₈)-alkyl-CO, (C₆-C₁₂)-bicycloalkyl-CO, (C₆-C₁₂)-bicycloalkyl-(C₁- C₈)-alkyl-CO, (C₆-C₁₂)-tricycloalkyl-CO, (C₆-C₁₂)-tricycloalkyl-(C₁- C₈)-alkyl-CO, (C₆-C₁₄)-aryl-CO éventuellement substitué, (C₆-C₁₄)- aryl-(C₁-C₈)-alkyl-CO éventuellement substitué dans le radical aryle, hétéroaryl-CO éventuellement substitué, hétéroaryl-(C₁-C₈)-alkyl- CO éventuellement substitué dans le radical hétéroaryle, (C₁-C₈)- alkyl-S(O)ₙ, (C₃-C₁₂)-cycloalkyl-S(O)ₙ, (C₃-C₁₂)-cycloalkyl-(C₁-C₈)- alkyl-S(O)ₙ, (C₆-C₁₂)-bicycloalkyl-S(O)ₙ, (C₆-C₁₂)-bicycloalkyl-(C₁- C₈)-alkyl-S(O)ₙ, (C₆-C₁₂)-tricycloalkyl-S(O)ₙ, (C₆-C₁₂-tricycloalkyl- (C₁-C₈)-alkyl-S(O)ₙ, (C₆-C₁₄)-aryl-S(O)ₙ éventuellement substitué, (C₆-C₁₄)-aryl-(C₁-C₈)-alkyl-S(O)ₙ éventuellement substitué dans le radical aryle, hétéroaryl-S(O)ₙ éventuellement substitué ou hétéroaryl-(C₁-C₈)-alkyl-S(O)ₙ éventuellement substitué dans le radical hétéroaryle, n vaut 1 ou 2 ;
R¹ est un des radicaux R²¹O-, R²⁴NH-, R²⁵N(R²⁵)-, HO-((C₁-C₈)-alkyl)- N(R²⁶)-, R²¹O-C(O)- et R²⁸N(R²¹)-C(O)- ;
R² signifie l'hydrogène ou (C₁-C₈)-alkyle ;
R³ signifie l'hydrogène, (C₁-C₈)-alkyle, (C₆-C₁₄)-aryle éventuellement substitué, (C₆-C₁₄)-aryl-(C₁-C₈)-alkyle éventuellement substitué dans le radical aryle, (C₃-C₈)-cycloalkyle, (C₂-C₈)-alcényle, (C₂-C₈)- alcynyle, (C₂-C₈)-alcénylcarbonyle, (C₂-C₈)-alcynylcarbonyle, pyridyle, R¹¹NH, CON(CH₃)R¹⁵, CONHR⁴, CON(CH₃)R¹⁵ ou CONHR¹⁵ ;
R⁴ signifie (C₁-C₁₀)-alkyle, qui peut être substitué éventuellement une ou plusieurs fois par des radicaux identiques ou différents de la série hydroxy, hydroxycarbonyle, aminocarbonyle, mono- ou di- ((C₁-C₁₈)-alkyl)-aminocarbonyle, (C₆-C₁₄)-aryl-(C₁-C₈)- alcoxycarbonyle, qui peut être substitué également dans le radical aryle, (C₁-C₈)-alcoxy, (C₁-C₈)-alcoxycarbonyle, (C₃-C₈)-cycloalkyle éventuellement substitué, tétrazolyl-(C₁-C₃)-alkyle, trifluorométhyle et R⁵ ;
R⁵ signifie (C₆-C₁₄)-aryle éventuellement substitué, (C₆-C₁₄)-aryle-(C₁- C₈)-alkyle éventuellement substitué dans le, radical aryle, un cycle hétérocyclique mono- ou bicyclique de 5 à 12 membres, qui peut être aromatique, en partie hydrogéné ou complètement hydrogéné et qui peut contenir un, deux ou trois hétéroatomes identiques ou différents de la série de l'azote, l'oxygène et le soufre, un radical R⁶ ou un radial R⁶CO-, dans lequel le radical aryle et le radical hétérocyclique indépendamment de celui-ci peut être substitué une ou plusieurs fois par des radicaux identiques ou différents de la série (C₁-C₈)-alkyle, (C₁-C₈)-alcoxy, halogène, nitro, amino ou trifluorométhyle ;
R⁶ est un radical acide aminé naturel ou non naturel, acide imino, acide azaamino éventuellement N-(C₁-C₈)-alkylé ou N-((C₆-C₁₄)- aryl-(C₁-C₈)-alkylé) ou un radical dipeptidique, qui peut être également substitué dans le radical aryle, ainsi que leur esters et amides, où des groupes fonctionnels libres peuvent être protégés par des groupes protecteurs classiques dans la chimie des peptides ;
R¹⁰ signifie hydroxy, (C₁-C₁₈)-alcoxy, (C₆-C₁₄)-aryl-(C₁-C₈)-alcoxy, qui peut également être substitué dans le radical aryle, (C₆-C₁₄)-aryloxy éventuellement substitué, amino ou mono- ou di-((C₁-C₁₈)-alkyl)- amino ;
R¹¹ signifie R¹²CO, (C₆-C₁₄)-aryl-S(O)₂ éventuellement substitué ou (C₁-C₁₈)-alkyl-S(O)₂ ;
R¹² signifie l'hydrogène, (C₁-C₁₈)-alkyle, (C₂-C₈)-alcényle, (C₂-C₈)- alcynyle, (C₆-C₁₄)-aryle éventuellement substitué, (C₁-C₁₈)-alcoxy, (C₆-C₁₄)-aryl-(C₁-C₈)-alcoxy, qui peut également être substitué dans le radical aryle, (C₆-C₁₄)-aryloxy éventuellement substitué, le radical R¹⁵ ou le radical R¹⁵-O-,
R¹³ signifie l'hydrogène ou (C₁-C₄)-alkyle ;
R¹⁵ est R¹⁶-(C₁-C₆)-alkyle ou R¹⁶ ;
R¹⁶ est un radical bicyctique ou tricyclique de 6 à 24 membres, qui est saturé ou partiellement insaturé et qui également peut contenir un à quatre hétéroatomes identiques ou différents de la série de l'azote, l'oxygène et le soufre et qui peut être également substitué par un ou plusieurs substituants identiques ou différents de le série de (C₁- C₄)-alkyle et oxo ;
c et d valent 1 et f vaut 0 ;
e et h valent indépendamment les uns des autres 0 ou 1 et g vaut 0 ;
dans toutes leurs formes stéréoisomères et leurs mélanges dans tous rapports ainsi que leurs sels compatibles physiologiquement.

28. Composés de formule Id selon la revendication 26 et/ou 27, dans lesquels le radical qui substitue le groupe B est un radical (C₁-C₈)-alkyle, dans toutes leurs formes stéréoisomères et leurs mélanges dans tous rapports ainsi que leurs sels compatibles physiologiquement.

29. Composés de formule Id selon l'une ou plusieurs des revendications 26 à 28, dans lesquels le radial R¹ est R²⁸N(R²¹)-C(O)-, dans toutes leurs formes stéréoisomères et leurs mélanges dans tous rapports ainsi que leurs sels compatibles physiologiquement.

30. Composés de formule Id selon l'une ou plusieurs des revendications 26 à 29 et/ou leurs sels compatibles physiologiquement pour l'utilisation comme médicaments.

31. Préparation pharmaceutique, **caractérisée en ce qu'**elle contient un ou plusieurs composés de formule Id selon l'une ou plusieurs des revendications 26 à 29 et/ou leurs sels compatibles physiologiquement à côté de matériau véhicules et/ou de matériaux additifs sûrs du point de vue pharmaceutique.
